(19) **Europäisches Patentamt / European Patent Office / Office européen des brevets**

(11) **EP 4 610 277 A1**

(12) **EUROPEAN PATENT APPLICATION**
published in accordance with Art. 153(4) EPC

(43) Date of publication:
**03.09.2025 Bulletin 2025/36**

(21) Application number: **23882663.0**

(22) Date of filing: **25.10.2023**

(51) International Patent Classification (IPC):
$C07K\ 16/28^{(2006.01)}$    $A61K\ 35/17^{(2025.01)}$
$A61K\ 35/22^{(2015.01)}$    $A61K\ 35/28^{(2015.01)}$
$A61K\ 35/30^{(2015.01)}$    $A61K\ 35/32^{(2015.01)}$
$A61K\ 35/34^{(2015.01)}$    $A61K\ 35/39^{(2015.01)}$
$A61K\ 35/407^{(2015.01)}$    $A61K\ 35/545^{(2015.01)}$
$A61K\ 39/395^{(2006.01)}$    $A61P\ 37/06^{(2006.01)}$
$C12N\ 5/10^{(2006.01)}$    $C12N\ 5/071^{(2010.01)}$
$C12N\ 5/0783^{(2010.01)}$    $C12N\ 15/13^{(2006.01)}$
$C12Q\ 1/02^{(2006.01)}$

(52) Cooperative Patent Classification (CPC):
A61K 35/17; A61K 35/22; A61K 35/28;
A61K 35/30; A61K 35/32; A61K 35/34;
A61K 35/39; A61K 35/407; A61K 35/545;
A61K 39/395; A61P 37/06; C07K 16/00;
C07K 16/28; C12N 5/06; C12N 5/10;    (Cont.)

(86) International application number:
**PCT/JP2023/038440**

(87) International publication number:
**WO 2024/090458 (02.05.2024 Gazette 2024/18)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **25.10.2022 JP 2022170981**

(71) Applicant: **Daiichi Sankyo Company, Limited
Tokyo 103-8426 (JP)**

(72) Inventors:
- **UENO, Suguru**
 **Tokyo 103-8426 (JP)**
- **IKEDA, Kazuya**
 **Tokyo 103-8426 (JP)**
- **NAKAYAMA, Makiko**
 **Tokyo 103-8426 (JP)**
- **HIURA, Satoshi**
 **Tokyo 103-8426 (JP)**
- **YOSHIDA, Sayaka**
 **Tokyo 103-8426 (JP)**
- **NAKAMURA, Kensuke**
 **Tokyo 103-8426 (JP)**
- **KUWASAKI, Yuto**
 **Tokyo 103-8426 (JP)**
- **NISHIKAWA, Yosuke**
 **Tokyo 103-8426 (JP)**
- **KIMURA, Takako**
 **Tokyo 103-8426 (JP)**

(74) Representative: **Marks & Clerk LLP
15 Fetter Lane
London EC4A 1BW (GB)**

Remarks:
The complete document including Reference Table(s) and the Sequence Listing(s) can be downloaded from the EPO website

(54) **METHOD FOR AVOIDING IMMUNE REJECTION USING AGONIST FOR INHIBITORY KIR**

(57) This invention provides a substance having activity of binding to inhibitory KIR and inhibiting the cytocidal activity of an NK cell that is useful for the allogeneic technology associated with organ transplantation or cell therapy and a method for producing such substance.

This invention provides a substance having activity of binding to inhibitory KIR and inhibiting the cytocidal activity of an NK cell and a cell comprising such substance expressed on the surface.

EP 4 610 277 A1

(52) Cooperative Patent Classification (CPC): (Cont.)
**C12Q 1/02**

**Description**

Technical Field

The present invention relates to a substance having activity of binding to inhibitory KIR and inhibiting the cytocidal activity of an NK cell that is useful for allogeneic technologies related to organ transplantation or cell therapy, a method for producing such substance, a method for using such substance, and the like.

Background Art

Immunity is classified into natural immunity and adaptive immunity. Natural immunity is an immediate immune response to a wide variety of pathogens, which is primarily performed by immune cells, such as natural killer (NK) cells, macrophages, and granulocytes. In contrast, adaptive immunity is a selective and effective immune response primarily performed by immune cells, such as T cells, B cells, and dendritic cells.

Human pluripotent stem cells may be able to treat a wide variety of diseases. However, clinical application of human pluripotent stem cells is very limited because a recipient immune system rejects the transplanted cells due to differences in major histocompatibility antigen complex (MHC) types.

A typical MHC is a cell surface multicomponent molecule that mediates the interactions of leukocytes with other leukocytes or other cells observed in all vertebrates. The MHC gene family is classified into 3 groups: i.e., the class I group, the class II group, and the class III group, and the MHC gene family of a human is referred to as the human leukocyte antigen (HLA). The HLA class I (HLA-I) protein is expressed in all nucleated cells, and it is composed of the HLA class I heavy chain (or $\alpha$ chain) and $\beta$-2 microglobulin (B2M). The HLA class I protein displays a peptide on a CD8+ cytotoxic T cell. To date, 6 HLA class I $\alpha$ chains have been identified, and examples thereof include 3 classical (HLA-A, HLA-B, and HLA-C) and 3 non-classical (HLA-E, HLA-F, and HLA-G) $\alpha$ chains. Peptide-binding specificity to the peptide-binding cleft of the HLA class I molecule is determined by the $\alpha$ chain. CD8+ T cells recognize a peptide displayed by the HLA class I molecule and induce cell-mediated immunity.

In allogeneic transplantation, a non-autologous HLA class I protein is recognized as presenting a foreign antigen in the recipient, and recognition of the non-autologous HLA class I protein becomes a major obstacle when pluripotent cells are used for transplantation or replacement therapy. At present, HLA-matched or partially HLA-matched cells with HLA types consistent with those of patients have been developed for transplantation from the stem cell bank or induced pluripotent stem cell (iPSC) cells. However, development of cell lines matching each patient necessitates enormous costs, cell culture for several months, highly-trained personnel, and extensive examination of final products and all of which must be approved. In addition, each of the cell lines may act differently in terms of gene expression patterns, culture properties, differentiation potentials, and genetic mutation. Accordingly, it is very difficult to adjust such properties depending on cell lines.

Preparation of personalized stem cells or the stem cell bank with a variety of HLAs can dissolve current problems of transplantation. However, it is necessary to characterize a plurality of cell lines, differentiate the cell lines into therapeutic cellular products, and approve administration thereof to humans. Such time-consuming, technically difficult, and expensive process become a major obstacle to apply stem-cell-based therapeutic methods to clinical trials. Thus, development of a cell-based therapeutic method that is more effective but less expensive and would not cause immune rejection, has been awaited.

A T cell recognizes an HLA molecule on the cell surface via a T cell receptor (TCR). HLA displays an endogenous autologous peptide on a common cell and it displays a virus-derived non-autologous peptide on an infected cell. Thus, HLA regulates immune responses and maintains homeostasis.

A natural killer (NK) cell has a function known as missing-self recognition, in which the NK cell recognizes its own HLA type and exerts cytocidal activity on cells expressing different types of HLA (Non Patent Literature 1). Concerning the function of missing-self recognition, a receptor, such as NKG2 or KIR, expressed on the NK cell surface plays a role in self HLA type recognition. Such receptor is classified as an inhibitory receptor or an activating receptor. Upon binding of HLA of the same type as the NK cell to an inhibitory receptor on the NK cell and transmission of an inhibitory signal in the NK cell, an autologous cell inhibits the cytocidal activity of the NK cell, and NK cell-mediated rejection is thus avoided.

Concerning such inhibitory receptors on NK cells, some NK cells in an individual are known to show a plurality of phenotypes of inhibitory receptors. For example, an approximately half of the CD56$^+$CD3$^-$NK cells included in human peripheral mononuclear cells (PBMCs) is NKG2A+, and the other half is known to be a population of NKG2A- cells that expresses inhibitory receptors of different types, such as KIR and LILB (Non Patent Literature 2).

NKG2A (CD159) is a type of an inhibitory receptor on the NK cell surface and it recognizes HLA-E as a ligand. The activating receptor counterpart of NKG2A is known as NKG2C/D/E (Non Patent Literature 3).

A killer cell immunoglobulin-like receptor (KIR) is a single transmembrane protein, it is expressed on some NK cells, NKT cells, or T cells, and it recognizes HLA-A/B/C as major ligands (Non Patent Literature 2). In particular, a human-derived NK cell that recognizes a ligand based on the HLA type of a host, such as HLA-C1/C1 (HLA-C in which amino acid 77 is S and amino acid 80 is N is referred to as "C1 type"), attacks when it detects HLA-C2 (HLA-C in which amino acid 77 is N and amino acid 80 is K is referred to as "C2 type"), and vice versa (Non Patent Literature 1). Such mechanism is referred to as the "missing-self" mechanism. Concerning molecules that control the missing-self mechanism, KIR2DL2/3 functions as an inhibitory receptor when a host carries HLA-C1, and KIR2DL1 functions as an inhibitory receptor when a host carries HLA-C2. An inhibitory receptor contains an immune receptor tyrosine-based inhibitor motif (ITIM) in its intracellular domain, it forms a cluster upon binding with HLA, and it transmits an inhibitory signal via an adaptor molecule such as SHP-1 in the cell (Non Patent Literature 4).

As antibodies against KIR2DL1/2/3, Lirilumab (BMS-986015, human IgG4) that has been in clinical trials as an antitumor agent and a mouse antibody Pan2D (NKVFS1, mouse IgG1) are available. As an antibody against KIR3DL1, the antibody disclosed in a patent literature (WO 2018/148223 A1) is also reported as a candidate substance for an antitumor agent. A cancer cell decreases expression of an activating ligand to an immune cell receptor or increases expression of an inhibitory ligand, so as to avoid the cytocidal activity. HLA expressed on a cancer cell surface also acts on an inhibitory receptor of an NK cell and utilizes the missing-self mechanism, so as to avoid the attack by the NK cell. While Lirilumab and the Pan2D antibody have been reported to bind to inhibitory KIR; i.e., KIR2DL1/2/3, to inhibit their binding to HLA and activate NK cells against cancer (Non Patent Literature 5, Patent Literature 1), agonist activity on inhibitory KIR is not described.

In order to dissolve the problem of rejection mediated by immune cells caused by HLA mismatch between donors and recipients, Dr. David Russel et al. reported a technique of deleting component of an HLA complex; i.e., beta-2 microglobulin (B2M), by gene editing to eliminate expression of HLA on a cell membrane and avoid immune rejection by CD8[+] T cells. Thus, cells without HLA-A, HLA-B, HLA-C, HLA-E, HLA-F, and HLA-G on the surfaces can be prepared. In order to avoid NK cell-mediated rejection, in contrast, HLA-E (a heterotrimer in nature) was forced to express as a single-stranded protein on the B2M-deficient cells. As a result, cells selectively expressing HLA-E without expressing other HLAs were obtained (hereafter referred to as the "universal cell technique"), and the cytocidal activity of the NK cells was inhibited (Patent Literature 2).

As another technique for avoiding immune rejection, it has been reported that cells expressing agonists on their surfaces targeting CD45 inhibit the cytocidal activity of CD45-expressing immune cells, such as NK cells and T cells (Patent Literature 3).

Patent Literature

Patent Literature 1: WO 2006/003179
Patent Literature 2: WO 2012/145384
Patent Literature 3: WO 2021/113853

Non Patent Literature

Non Patent Literature 1: NK cell receptors: of missing sugar and missing self, P. Parham et al., Curr. Biol., Mar 9, 2000; 10 (5): R195-7
Non Patent Literature 2: MHC class I-specific inhibitory receptors and their ligands structure diverse human NK-cell repertoires toward a balance of missing self-response, Yawata et al., Blood, Sep 15, 2008; 112 (6): 2369-80
Non Patent Literature 3: The CD94/NKG2 family of receptors: from molecules and cells to clinical relevance, F. Borrego et al., Immunol. Res., 2006; 35 (3): 263-78
Non Patent Literature 4: Negative signaling by inhibitory receptors: the NK cell paradigm, E. O. Long et al., Immunol. Rev., Aug 2008; 224: 70-84
Non Patent Literature 5: Preclinical characterization of 1-7F9, a novel human anti-KIR receptor therapeutic antibody that augments natural killer-mediated killing of tumor cells, F. Romagne et al., Blood, Sep 24, 2009; 114 (13): 2667-77

Summary of Invention

Technical Problem

The universal cell technique disclosed in Patent Literature 1 has the problem described below. All NK cells in an individual human do not express NKG2A. Generally, while an approximately half of NK cells contained in human peripheral blood monocytes (PBMCs) and identified to be CD56+CD3- cells express NKG2A, the other half does not express NKG2A but expresses other inhibitory receptors, such as KIR and LILB. Thus, despite the use of the universal cell technique, the cells may be attacked by NKG2A- NK cells.

Concerning the technique disclosed in Patent Literature 2, when a CD45 agonist is expressed on an allogeneic chimeric antigen receptor (CAR)-T cell, for example, such cells may inhibit their functions and activity each other because CD45 is expressed on a T cell. Since CD45 is expressed on an extensive range of immune cells, targeting CD45 may cause side effects or prevent immune elimination of cell therapy products in case they become cancerous. Accordingly, it is necessary to develop a technique of avoiding immune rejection with the use of an agonistic substance acting on a target receptor that shows a minimal expression pattern on immune cells rather than a broad expression pattern and reflects the biological properties of immune cells.

The distribution of NKG2A expression and that of KIR expression on the NK cell surface are reversely correlated (Non Patent Literature 2). The NK cells with low NKG2A expression levels show high KIR expression levels. This suggests that cells expressing HLA-E using the universal cell technique are effective against NK cells with high levels of NKG2A expression, whereas it may not be effective against the NK cells with low levels or no NKG2A expression and expressing inhibitory KIR. Since a ligand for inhibitory KIR is HLA-ABC, its introduction inevitably fails to avoid TCR recognition. Accordingly, a substance with an HLA-like agonist activity for inhibitary KIR is necessary.

While Lirilumab (BMS-986015, human IgG4) known as an antibody against KIR and a mouse antibody Pan2D (NKVFS1, mouse IgG1) are reported to antagonize the activity of inhibitory signals by blocking the binding of a natural ligand to inhibitory KIR, their activity in the absence of ligands remains unknown.

Solution to Problem

The present inventors discovered that, in the absence of a ligand, Lirilumab would exert agonist activity on inhibitory KIR of NK cells. They also discovered that cells expressing a single-chain Fv (ScFv) comprising the sequence of Lirilumab on their surface would be able to evade attacks by NK cells. They also discovered that Pan2D (NKVSF1) of both soluble form and on the cell surface acting on KIR exert agonistic activity on inhibitory KIR. However, Lirilumab and Pan2D were found to bind not only to inhibitory KIR but also to activating KIR, enhancing the cytocidal activity of NK cells by acting as agonists on activating KIR (e.g., KIR 2DS1, 2, and 4).

Under the above circumstances, the present inventors intended to obtain an agonist binder that would selectively bind to inhibitory KIR and act thereon while refraining from binding the activating receptor. As a result, they succeeded in identifying a substance that would bind to inhibitory KIR2DL2 and KIR2DL3 and exert agonist activity thereon but would not bind to KIR2DS1, KIR2DS2, and KIR2DS4, a substance that would bind to KIR 2DL1 and exert agonist activity thereon but would not bind to KIR2DS1, KIR2DS2, and KIR2DS4, and a substance that would bind to KIR3DL1 and exert agonist activity thereon but would not bind to KIR3DS1 by phage panning using the human antibody phage library. They further conducted studies, thereby completing the present invention.

Specifically, the present invention provides the following.

[1] A substance having activity of binding to human inhibitory KIR and inhibiting the cytocidal activity of an NK cell.

[2] The substance according to [1], which is a polypeptide comprising a site binding to human inhibitory KIR.

[3] The substance according to [2], wherein the site binding to human inhibitory KIR comprises an amino acid sequence derived from a heavy chain variable region and a light chain variable region of a monoclonal antibody binding to human inhibitory KIR or an antigen-binding fragment.

[4] The substance according to [1], wherein the activity of binding to human inhibitory KIR is higher than the activity of binding to human activating KIR corresponding thereto.

[5] The substance according to [1], wherein the agonist activity on human inhibitory KIR is higher than the agonist activity on human activating KIR corresponding thereto.

[6] The substance according to [1], which exerts substantially no agonist activity on the corresponding human activating KIR.

[7] The substance according to [2], wherein the polypeptide is any of (1) to (3) below:

(1) a polypeptide in which the site binding to KIR2DL2 and/or KIR2DL3 comprise a C1 epitope motif sequence contained in HLA-Cw1, Cw3, Cw7, Cw8, Cw9, Cw10, Cw12, Cw14, or Cw16 that belongs to the HLA-C1 type or HLA-B46 or B73 (in which amino acid 77 is S and amino acid 80 is N) and a TCR-binding capability is lost or attenuated;

(2) a polypeptide in which the site binding to KIR2DL1 comprise a C2 epitope motif sequence contained in HLA-Cw2, Cw4, Cw5, Cw6, Cw15, or Cw17 that belongs to the HLA-C2 type (in which amino acid 77 is N and amino acid 80 is K) and a TCR-binding capability is lost or attenuated; and

(3) a polypeptide in which the site binding to KIR3DL1 comprise a Bw4 epitope motif sequence contained in HLA-B5, B27, B37, B38, B44, B49, B51, B52, B53, B57, B58, B59, B77, A23, A24, A25, or A32 that belongs to the HLA-Bw4 type (in which amino acid 77 is N, amino acid 80 is I or T, and amino acid 83 is R) and a TCR-binding capability is lost or attenuated.

[8] The substance according to [3], wherein the human inhibitory KIR is KIR2DL2 and/or KIR2DL3 and the binding site comprises an amino acid sequence derived from a heavy chain variable region and a light chain variable region of a monoclonal antibody binding to KIR2DL2 and/or KIR2DL or an antigen-binding fragment.

[9] The substance according to [8], wherein the monoclonal antibody binding to KIR2DL2 and/or KIR2DL3 or an antigen-binding fragment thereof competes with Lirilumab, Pan2D, or scFv consisting of an amino acid sequence according to any of (i) to (xii) below when binding to KIR2DL2 and/or KIR2DL3:

(i) an amino acid sequence comprising the amino acid sequence of a heavy chain variable region shown in SEQ ID NO: 33 in combination with the amino acid sequence of a light chain variable region shown in SEQ ID NO: 34;
(ii) an amino acid sequence comprising the amino acid sequence of a heavy chain variable region shown in SEQ ID NO: 35 in combination with the amino acid sequence of a light chain variable region shown in SEQ ID NO: 36;
(iii) an amino acid sequence comprising the amino acid sequence of a heavy chain variable region shown in SEQ ID NO: 37 in combination with the amino acid sequence of a light chain variable region shown in SEQ ID NO: 38;
(iv) an amino acid sequence comprising the amino acid sequence of a heavy chain variable region shown in SEQ ID NO: 39 in combination with the amino acid sequence of a light chain variable region shown in SEQ ID NO: 40;
(v) an amino acid sequence comprising the amino acid sequence of a heavy chain variable region shown in SEQ ID NO: 41 in combination with the amino acid sequence of a light chain variable region shown in SEQ ID NO: 42;
(vi) an amino acid sequence comprising the amino acid sequence of a heavy chain variable region shown in SEQ ID NO: 43 in combination with the amino acid sequence of a light chain variable region shown in SEQ ID NO: 44;
(vii) an amino acid sequence comprising the amino acid sequence of a heavy chain variable region shown in SEQ ID NO: 45 in combination with the amino acid sequence of a light chain variable region shown in SEQ ID NO: 46;
(viii) an amino acid sequence comprising the amino acid sequence of a heavy chain variable region shown in SEQ ID NO: 47 in combination with the amino acid sequence of a light chain variable region shown in SEQ ID NO: 48;
(ix) an amino acid sequence comprising the amino acid sequence of a heavy chain variable region shown in SEQ ID NO: 49 in combination with the amino acid sequence of a light chain variable region shown in SEQ ID NO: 50;
(x) an amino acid sequence comprising the amino acid sequence of a heavy chain variable region shown in SEQ ID NO: 51 in combination with the amino acid sequence of a light chain variable region shown in SEQ ID NO: 52;
(xi) an amino acid sequence comprising the amino acid sequence of a heavy chain variable region shown in SEQ ID NO: 61 in combination with the amino acid sequence of a light chain variable region shown in SEQ ID NO: 62; and
(xii) an amino acid sequence comprising the amino acid sequence of a heavy chain variable region shown in SEQ ID NO: 63 in combination with the amino acid sequence of a light chain variable region shown in SEQ ID NO: 64.

[9-2] The substance according to [9], which competes with scFv consisting of an amino acid sequence comprising the amino acid sequence of a heavy chain variable region shown in SEQ ID NO: 51 in combination with the amino acid sequence of a light chain variable region shown in SEQ ID NO: 52.

[10] The substance according to [8], wherein the site binding to KIR2DL2 and/or KIR2DL3 comprises a variable region comprising amino acids located adjacent to 1 to 6 amino acids including Asp-47 in a region of amino acids 44 to 49, 2 to 4 amino acids including Gly-67 and Pro-68 in a region of amino acids 65 to 68, and 1 to 4 amino acids including Asp-72 in a region of amino acids 70 to 73 in the amino acid sequence of KIR2DL2 of SEQ ID NO: 6.

[11] The substance according to [10], wherein, in the site binding to KIR2DL2 and/or KIR2DL3, an amino acid interacting with Asp-47 of SEQ ID NO: 6 is Tyr, an amino acid interacting with Gly-67 of SEQ ID NO: 6 is Gly, an amino acid interacting with Pro-68 of SEQ ID NO: 6 is Tyr, and an amino acid interacting with Asp-72 of SEQ ID NO: 6 is Thr, Asn, Gly, His, Ile, Ser, Val, or Ala.

[12] The substance according to [11], wherein the amino acid included in the variable region identified in [11] belongs to CDR3.

[13] The substance according to [8], which is a polypeptide comprising a binding site according to any of (I) to (XII) below as the site binding to KIR2DL2 and/or KIR2DL and optionally comprising mutation of 1 or 2 amino acids in each CDR:

(I) a binding site comprising a first variable region comprising the amino acid sequence derived from the amino acid sequence of CDR1, CDR2, and CDR3 shown in SEQ ID NO: 33 in combination with a second variable region comprising the amino acid sequence derived from the amino acid sequence of CDR1, CDR2, and CDR3 shown in SEQ ID NO: 34;

(II) a binding site comprising a first variable region comprising the amino acid sequence derived from the amino acid sequence of CDR1, CDR2, and CDR3 shown in SEQ ID NO: 35 in combination with a second variable region comprising the amino acid sequence derived from the amino acid sequence of CDR1, CDR2, and CDR3 shown in SEQ ID NO: 36;

(III) a binding site comprising a first variable region comprising the amino acid sequence derived from the amino acid sequence of CDR1, CDR2, and CDR3 shown in SEQ ID NO: 37 in combination with a second variable region comprising the amino acid sequence derived from the amino acid sequence of CDR1, CDR2, and CDR3 shown in SEQ ID NO: 38;

(IV) a binding site comprising a first variable region comprising the amino acid sequence derived from the amino acid sequence of CDR1, CDR2, and CDR3 shown in SEQ ID NO: 39 in combination with a second variable region comprising the amino acid sequence derived from the amino acid sequence of CDR1, CDR2, and CDR3 shown in SEQ ID NO: 40;

(V) a binding site comprising a first variable region comprising the amino acid sequence derived from the amino acid sequence of CDR1, CDR2, and CDR3 shown in SEQ ID NO: 41 in combination with a second variable region comprising the amino acid sequence derived from the amino acid sequence of CDR1, CDR2, and CDR3 shown in SEQ ID NO: 42;

(VI) a binding site comprising a first variable region comprising the amino acid sequence derived from the amino acid sequence of CDR1, CDR2, and CDR3 shown in SEQ ID NO: 43 in combination with a second variable region comprising the amino acid sequence derived from the amino acid sequence of CDR1, CDR2, and CDR3 shown in SEQ ID NO: 44;

(VII) a binding site comprising a first variable region comprising the amino acid sequence derived from the amino acid sequence of CDR1, CDR2, and CDR3 shown in SEQ ID NO: 45 in combination with a second variable region comprising the amino acid sequence derived from the amino acid sequence of CDR1, CDR2, and CDR3 shown in SEQ ID NO: 46;

(VIII) a binding site comprising a first variable region comprising the amino acid sequence derived from the amino acid sequence of CDR1, CDR2, and CDR3 shown in SEQ ID NO: 47 in combination with a second variable region comprising the amino acid sequence derived from the amino acid sequence of CDR1, CDR2, and CDR3 shown in SEQ ID NO: 48;

(IX) a binding site comprising a first variable region comprising the amino acid sequence derived from the amino acid sequence of CDR1, CDR2, and CDR3 shown in SEQ ID NO: 49 in combination with a second variable region comprising the amino acid sequence derived from the amino acid sequence of CDR1, CDR2, and CDR3 shown in SEQ ID NO: 50;

(X) a binding site comprising a first variable region comprising the amino acid sequence derived from the amino acid sequence of CDR1, CDR2, and CDR3 shown in SEQ ID NO: 51 in combination with a second variable region comprising the amino acid sequence derived from the amino acid sequence of CDR1, CDR2, and CDR3 shown in SEQ ID NO: 52;

(XI) a binding site comprising a first variable region comprising the amino acid sequence derived from the amino acid sequence of CDR1, CDR2, and CDR3 shown in SEQ ID NO: 61 in combination with a second variable region comprising the amino acid sequence derived from the amino acid sequence of CDR1, CDR2, and CDR3 shown in SEQ ID NO: 62; and

(XII) a binding site comprising a first variable region comprising the amino acid sequence derived from the amino acid sequence of CDR1, CDR2, and CDR3 shown in SEQ ID NO: 63 in combination with a second variable region comprising the amino acid sequence derived from the amino acid sequence of CDR1, CDR2, and CDR3 shown in SEQ ID NO: 64.

[13-2] The substance according to [13], which is a polypeptide comprising a binding site comprising a first variable region comprising the amino acid sequence derived from the amino acid sequence of CDR1, CDR2, and CDR3 shown in SEQ ID NO: 51 in combination with a second variable region comprising the amino acid sequence derived from the amino acid sequence of CDR1, CDR2, and CDR3 shown in SEQ ID NO: 52.

[14] The substance according to [13], which optionally comprises mutation of 1 or 2 amino acids on the C and/or N terminal sides of each CDR.

[14-2] The substance according to [13] or [14], wherein, in the second variable region in (x) above, Thr-94 of SEQ ID NO: 52 may be substituted with Asn, Gly, His, Ile, Ser, Val, or Ala.

[15] The substance according to [13], which is a polypeptide comprising a binding site according to any of (I) to (XII) below as the site binding to KIR2DL2 and/or KIR2DL:

(I) a binding site comprising a first variable region comprising the amino acid sequence shown in SEQ ID NO: 33 in combination with a second variable region comprising the amino acid sequence shown in SEQ ID NO: 34;

(II) a binding site comprising a first variable region comprising the amino acid sequence shown in SEQ ID NO: 35 in combination with a second variable region comprising the amino acid sequence shown in SEQ ID NO: 36;
(III) a binding site comprising a first variable region comprising the amino acid sequence shown in SEQ ID NO: 37 in combination with a second variable region comprising the amino acid sequence shown in SEQ ID NO: 38;
(IV) a binding site comprising a first variable region comprising the amino acid sequence shown in SEQ ID NO: 39 in combination with a second variable region comprising the amino acid sequence shown in SEQ ID NO: 40;
(V) a binding site comprising a first variable region comprising the amino acid sequence shown in SEQ ID NO: 41 in combination with a second variable region comprising the amino acid sequence shown in SEQ ID NO: 42;
(VI) a binding site comprising a first variable region comprising the amino acid sequence shown in SEQ ID NO: 43 in combination with a second variable region comprising the amino acid sequence shown in SEQ ID NO: 44;
(VII) a binding site comprising a first variable region comprising the amino acid sequence shown in SEQ ID NO: 45 in combination with a second variable region comprising the amino acid sequence shown in SEQ ID NO: 46;
(VIII) a binding site comprising a first variable region comprising the amino acid sequence shown in SEQ ID NO: 47 in combination with a second variable region comprising the amino acid sequence shown in SEQ ID NO: 48;
(IX) a binding site comprising a first variable region comprising the amino acid sequence shown in SEQ ID NO: 49 in combination with a second variable region comprising the amino acid sequence shown in SEQ ID NO: 50;
(X) a binding site comprising a first variable region comprising the amino acid sequence shown in SEQ ID NO: 51 in combination with a second variable region comprising the amino acid sequence shown in SEQ ID NO: 52;
(XI) a binding site comprising a first variable region comprising the amino acid sequence shown in SEQ ID NO: 61 in combination with a second variable region comprising the amino acid sequence shown in SEQ ID NO: 62; and
(XII) a binding site comprising a first variable region comprising the amino acid sequence shown in SEQ ID NO: 63 in combination with a second variable region comprising the amino acid sequence shown in SEQ ID NO: 64.

[15-2] The substance according to [15], which is a polypeptide comprising a binding site comprising a first variable region comprising the amino acid sequence shown in SEQ ID NO: 51 in combination with a second variable region comprising the amino acid sequence shown in SEQ ID NO: 52.
[16] The substance according to [8], wherein the activity of binding to KIR2DL2 and/or KIR2DL3 is higher than the activity of binding to KIR2DS1, KIR2DS2, and/or KIR2DS4.
[17] The substance according to [8], wherein the agonist activity on KIR2DL2 and/or KIR2DL3 is higher than the agonist activity on KIR2DS1, KIR2DS2, and/or KIR2DS4.
[18] The substance according to [17], which exerts substantially no agonist activity on KIR2DS1, KIR2DS2, and/or KIR2DS4.
[19] The substance according to [17], which does not substantially bind to KIR2DS1, KIR2DS2, and KIR2DS4.
[20] The substance according to [19], which comprises an antigen-binding fragment obtained by the method comprising:

step A: a step of obtaining a population of display bodies displaying an antigen-binding site binding to KIR2DL2 or KIR2DL3 comprising mixing a labeled soluble protein comprising an extracellular domain of KIR2DL2 or KIR2DL3 in a suspension containing a population of polyclonal display bodies including display bodies displaying the antigen-binding site binding to KIR2DL2 or KIR2DL3 on the surface, bringing the suspension into contact with a carrier comprising, immobilized thereon, a substance binding specifically to the labeled part, and collecting the display bodies bound to the carrier;
step B: a step of removing display bodies displaying the antigen-binding site binding to KIR2DS1, KIR2DS2, and KIR2DS4 from the population of display bodies comprising mixing a labeled soluble protein comprising extracellular domains of KIR2DS1, KIR2DS2, and KIR2DS4 in the suspension containing the population of display bodies obtained in step A, bringing the suspension into contact with a carrier comprising, immobilized thereon, a substance binding to the labeled part, and collecting the display bodies not bound to the carrier;
step C: a step of identifying a sequence of a gene encoding the antigen-binding site by performing genetic analysis of the display bodies obtained by the method comprising the steps described above; and
step D: a step of expressing the identified gene comprising the antigen-binding site in a cell.

[21] The substance according to [19], which comprises an antigen-binding fragment obtained by the method comprising:

step A: a step of obtaining a population of display bodies displaying an antigen-binding site binding specifically to KIR2DL2 or KIR2DL3 comprising mixing a labeled soluble protein comprising an extracellular domain of KIR2DL2 or KIR2DL3 and an unlabeled soluble protein comprising extracellular domains of KIR2DS1, KIR2DS2, and KIR2DS4 in a suspension containing a population of polyclonal display bodies including display bodies displaying the antigen-binding site binding to KIR2DL2 or KIR2DL3 on the surface, bringing the suspension into contact with a carrier comprising, immobilized thereon, a substance binding specifically to the labeled part, and collecting the display bodies bound to the carrier;

step B: a step of identifying a sequence of a gene encoding the antigen-binding site by performing genetic analysis of the display bodies obtained by the method comprising the steps described above; and

step C: a step of expressing the identified gene comprising the antigen-binding site in a cell.

[22] The substance according to [20] or [21], wherein a combination of a label and a label-binding substance is a combination of biotin and avidin.

[23] The substance according to [20] or [21], which is obtained by a method further comprising a step of selecting a substance having an agonist activity on KIR2DL2 or KIR2DL3.

[24] The substance according to any of [8] to [23], wherein the polypeptide is a monoclonal antibody or an antigen-binding fragment.

[25] The substance according to [24], which is a single-stranded antibody or scFv comprising a first variable region linked to a second variable region via a GS linker or a G4S linker.

[26] The substance according to any of [8] to [23], wherein the polypeptide is a membrane protein having an extracellular domain comprising the site binding to KIR2DL2 and/or KIR2DL3 and a transmembrane domain.

[27] The substance according to [3], wherein the inhibitory KIR is KIR3DL1 and the binding site comprises an amino acid sequence derived from a heavy chain variable region and a light chain variable region of the monoclonal antibody binding to KIR3DL1 or an antigen-binding fragment.

[28] The substance according to [27], wherein the monoclonal antibody binding to KIR3DL1 or an antigen-binding fragment thereof competes with scFv consisting of an amino acid sequence according to any of (xiii) to (xviii) below when binding to KIR3DL1:

(xiii) an amino acid sequence comprising the amino acid sequence of a heavy chain variable region shown in SEQ ID NO: 56 in combination with the amino acid sequence of a light chain variable region shown in SEQ ID NO: 55;

(xiv) an amino acid sequence comprising the amino acid sequence of a heavy chain variable region shown in SEQ ID NO: 137 in combination with the amino acid sequence of a light chain variable region shown in SEQ ID NO: 138;

(xv) an amino acid sequence comprising the amino acid sequence of a heavy chain variable region shown in SEQ ID NO: 139 in combination with the amino acid sequence of a light chain variable region shown in SEQ ID NO: 140;

(xvi) an amino acid sequence comprising the amino acid sequence of a heavy chain variable region shown in SEQ ID NO: 141 in combination with the amino acid sequence of a light chain variable region shown in SEQ ID NO: 142;

(xvii) an amino acid sequence comprising the amino acid sequence of a heavy chain variable region shown in SEQ ID NO: 143 in combination with the amino acid sequence of a light chain variable region shown in SEQ ID NO: 144; and

(xviii) an amino acid sequence comprising the amino acid sequence of a heavy chain variable region shown in SEQ ID NO: 145 in combination with the amino acid sequence of a light chain variable region shown in SEQ ID NO: 146.

[29] The substance according to [27], which is a polypeptide comprising a binding site according to any of (XIII) to (XVIII) below as the site binding to KIR3DL1 and optionally comprising mutation of 1 or 2 amino acids in each CDR:

(XIII) a binding site comprising a first variable region comprising the amino acid sequence derived from the amino acid sequence of CDR1, CDR2, and CDR3 shown in SEQ ID NO: 56 in combination with a second variable region comprising the amino acid sequence derived from the amino acid sequence of CDR1, CDR2, and CDR3 shown in SEQ ID NO: 55;

(XIV) a binding site comprising a first variable region comprising the amino acid sequence derived from the amino acid sequence of CDR1, CDR2, and CDR3 shown in SEQ ID NO: 137 in combination with a second variable region comprising the amino acid sequence derived from the amino acid sequence of CDR1, CDR2, and CDR3 shown in SEQ ID NO: 138;

(XV) a binding site comprising a first variable region comprising the amino acid sequence derived from the amino acid sequence of CDR1, CDR2, and CDR3 shown in SEQ ID NO: 139 in combination with a second variable region comprising the amino acid sequence derived from the amino acid sequence of CDR1, CDR2, and CDR3 shown in SEQ ID NO: 140;

(XVI) a binding site comprising a first variable region comprising the amino acid sequence derived from the amino acid sequence of CDR1, CDR2, and CDR3 shown in SEQ ID NO: 141 in combination with a second variable region comprising the amino acid sequence derived from the amino acid sequence of CDR1, CDR2, and CDR3 shown in SEQ ID NO: 142;

(XVII) a binding site comprising a first variable region comprising the amino acid sequence derived from the amino acid sequence of CDR1, CDR2, and CDR3 shown in SEQ ID NO: 143 in combination with a second variable region comprising the amino acid sequence derived from the amino acid sequence of CDR1, CDR2, and CDR3 shown in SEQ ID NO: 144; and

(XVIII) a binding site comprising a first variable region comprising the amino acid sequence derived from the amino acid sequence of CDR1, CDR2, and CDR3 shown in SEQ ID NO: 145 in combination with a second variable region comprising the amino acid sequence derived from the amino acid sequence of CDR1, CDR2, and CDR3 shown in SEQ ID NO: 146.

[30] The substance according to [29], which optionally comprises mutation of 1 or 2 amino acids on the C and/or N terminal sides of each CDR.

[31] The substance according to [27], which is a polypeptide comprising a binding site according to any of (XIII) to (XVIII) below as the site binding to KIR3DL1;

(XIII) a binding site comprising a first variable region comprising the amino acid sequence shown in SEQ ID NO: 56 in combination with a second variable region comprising the amino acid sequence shown in SEQ ID NO: 55;

(XIIII) a binding site comprising a first variable region comprising the amino acid sequence shown in SEQ ID NO: 137 in combination with a second variable region comprising the amino acid sequence shown in SEQ ID NO: 138;

(XV) a binding site comprising a first variable region comprising the amino acid sequence shown in SEQ ID NO: 139 in combination with a second variable region comprising the amino acid sequence shown in SEQ ID NO: 140;

(XVI) a binding site comprising a first variable region comprising the amino acid sequence shown in SEQ ID NO: 141 in combination with a second variable region comprising the amino acid sequence shown in SEQ ID NO: 142;

(XVII) a binding site comprising a first variable region comprising the amino acid sequence shown in SEQ ID NO: 143 in combination with a second variable region comprising the amino acid sequence shown in SEQ ID NO: 144; and

(XVIII) a binding site comprising a first variable region comprising the amino acid sequence shown in SEQ ID NO: 145 in combination with a second variable region comprising the amino acid sequence shown in SEQ ID NO: 146.

[32] The substance according to [27], wherein the activity of binding to KIR3DL1 is higher than the activity of binding to KIR3DS1.

[33] The substance according to [27], wherein the agonist activity on KIR3DL1 is higher than the agonist activity on KIR3DS1.

[34] The substance according to [33], which exerts substantially no agonist activity on KIR3DS1.

[35] The substance according to [34], which does not substantially bind to KIR3DS1.

[36] The substance according to [35], which comprises an antigen-binding fragment obtained by the method comprising:

step A: a step of obtaining a population of display bodies displaying an antigen-binding site binding to KIR3DL1 comprising mixing a labeled soluble protein comprising an extracellular domain of KIR3DL1 in a suspension containing a population of polyclonal display bodies including display bodies displaying, on the surface, the antigen-binding site binding to KIR3DL1, bringing the suspension into contact with a carrier comprising, immobilized thereon, a substance binding specifically to the labeled part, and collecting the display bodies bound to the carrier;

step B: a step of removing display bodies displaying the antigen-binding site binding to KIR3DS1 from the population of display bodies comprising mixing a labeled soluble protein comprising an extracellular domain of KIR3DS1 in the suspension containing the population of display bodies obtained in step A, bringing the suspension into contact with a carrier comprising, immobilized thereon, a substance binding to the labeled part, and collecting the display bodies not bound to the carrier;

step C: a step of identifying a sequence of a gene encoding the antigen-binding site by performing genetic analysis of the display bodies obtained by the method comprising the steps described above; and

step D: a step of expressing the identified gene comprising the antigen-binding site in a cell.

[37] The substance according to [36], which comprises an antigen-binding fragment obtained by the method comprising:

step A: a step of obtaining a population of display bodies displaying an antigen-binding site binding specifically to KIR3DL1 comprising mixing a labeled soluble protein comprising an extracellular domain of KIR3DL1 and an unlabeled soluble protein comprising an extracellular domain of KIR3DS1 in a suspension containing a population of polyclonal display bodies including display bodies displaying, on the surface, the antigen-binding site binding to KIR3DL1, bringing the suspension into contact with a carrier comprising, immobilized thereon, a substance binding specifically to the labeled part, and collecting the display bodies bound to the carrier;

step B: a step of identifying a sequence of a gene encoding the antigen-binding site by performing genetic analysis of the display bodies obtained by the method comprising the steps described above; and

step C: a step of expressing the identified gene comprising the antigen-binding site in a cell.

[38] The substance according to [36] or [37], wherein a combination of a label and a label-binding substance is a combination of biotin and avidin.

[39] The substance according to any of [36] to [38], which is obtained by a method further comprising a step of selecting a substance having an agonist activity on KIR3DL1.

[40] The substance according to any of [27] to [39], wherein the polypeptide is a monoclonal antibody or an antigen-binding fragment.

[41] The substance according to [40], which is a single-stranded antibody or scFv comprising a first variable region linked to a second variable region via a GS linker or a G4S linker.

[42] The substance according to any of [27] to [34], wherein the polypeptide is a membrane protein having an extracellular domain comprising the site binding to KIR3DL1 and a transmembrane domain.

[43] The substance according to [3], wherein the inhibitory KIR is KIR2DL1 and the binding site comprises an amino acid sequence derived from a heavy chain variable region and a light chain variable region of a monoclonal antibody binding to KIR2DL1 or an antigen-binding fragment.

[44] The substance according to [43], wherein the monoclonal antibody binding to KIR2DL1 or an antigen-binding fragment competes with Pan2D when binding to KIR2DL1.

[45] The substance according to [43], wherein the monoclonal antibody binding to KIR2DL1 or an antigen-binding fragment thereof competes with scFv consisting of an amino acid sequence according to any of (ci) to (cxv) below when binding to KIR2DL1:

(ci) an amino acid sequence comprising the amino acid sequence of a heavy chain variable region shown in SEQ ID NO: 107 in combination with the amino acid sequence of a light chain variable region shown in SEQ ID NO: 108;

(cii) an amino acid sequence comprising the amino acid sequence of a heavy chain variable region shown in SEQ ID NO: 109 in combination with the amino acid sequence of a light chain variable region shown in SEQ ID NO: 110;

(ciii) an amino acid sequence comprising the amino acid sequence of a heavy chain variable region shown in SEQ ID NO: 111 in combination with the amino acid sequence of a light chain variable region shown in SEQ ID NO: 112;

(civ) an amino acid sequence comprising the amino acid sequence of a heavy chain variable region shown in SEQ ID NO: 113 in combination with the amino acid sequence of a light chain variable region shown in SEQ ID NO: 114;

(cv) an amino acid sequence comprising the amino acid sequence of a heavy chain variable region shown in SEQ ID NO: 115 in combination with the amino acid sequence of a light chain variable region shown in SEQ ID NO: 116;

(cvi) an amino acid sequence comprising the amino acid sequence of a heavy chain variable region shown in SEQ ID NO: 117 in combination with the amino acid sequence of a light chain variable region shown in SEQ ID NO: 118;

(cvii) an amino acid sequence comprising the amino acid sequence of a heavy chain variable region shown in SEQ ID NO: 119 in combination with the amino acid sequence of a light chain variable region shown in SEQ ID NO: 120;

(cviii) an amino acid sequence comprising the amino acid sequence of a heavy chain variable region shown in SEQ ID NO: 121 in combination with the amino acid sequence of a light chain variable region shown in SEQ ID NO: 122;

(cviiii) an amino acid sequence comprising the amino acid sequence of a heavy chain variable region shown in SEQ ID NO: 123 in combination with the amino acid sequence of a light chain variable region shown in SEQ ID NO: 124;

(cx) an amino acid sequence comprising the amino acid sequence of a heavy chain variable region shown in SEQ ID NO: 125 in combination with the amino acid sequence of a light chain variable region shown in SEQ ID NO: 126;

(cxi) an amino acid sequence comprising the amino acid sequence of a heavy chain variable region shown in SEQ ID NO: 127 in combination with the amino acid sequence of a light chain variable region shown in SEQ ID NO: 128;

(cxii) an amino acid sequence comprising the amino acid sequence of a heavy chain variable region shown in SEQ ID NO: 129 in combination with the amino acid sequence of a light chain variable region shown in SEQ ID NO: 130;

(cxiii) an amino acid sequence comprising the amino acid sequence of a heavy chain variable region shown in SEQ ID NO: 131 in combination with the amino acid sequence of a light chain variable region shown in SEQ ID NO: 132;

(cxiv) an amino acid sequence comprising the amino acid sequence of a heavy chain variable region shown in SEQ ID NO: 133 in combination with the amino acid sequence of a light chain variable region shown in SEQ ID NO: 134; and

(cxv) an amino acid sequence comprising the amino acid sequence of a heavy chain variable region shown in SEQ ID NO: 135 in combination with the amino acid sequence of a light chain variable region shown in SEQ ID NO: 136.

[46] The substance according to [43], which is a polypeptide comprising a binding site according to any of (CI) to (CXV) below as the site binding to KIR2DL1 and optionally comprising mutation of 1 or 2 amino acids in each CDR:

(CI) a binding site comprising a first variable region comprising the amino acid sequence derived from the amino acid sequence of CDR1, CDR2, and CDR3 shown in SEQ ID NO: 107 in combination with a second variable region comprising the amino acid sequence derived from the amino acid sequence of CDR1, CDR2, and CDR3 shown in SEQ ID NO: 108;

(CII) a binding site comprising a first variable region comprising the amino acid sequence derived from the amino acid sequence of CDR1, CDR2, and CDR3 shown in SEQ ID NO: 109 in combination with a second variable region comprising the amino acid sequence derived from the amino acid sequence of CDR1, CDR2, and CDR3 shown in SEQ ID NO: 110;

(CIII) a binding site comprising a first variable region comprising the amino acid sequence derived from the amino acid sequence of CDR1, CDR2, and CDR3 shown in SEQ ID NO: 111 in combination with a second variable region comprising the amino acid sequence derived from the amino acid sequence of CDR1, CDR2, and CDR3 shown in SEQ ID NO: 112;

(CIV) a binding site comprising a first variable region comprising the amino acid sequence derived from the amino acid sequence of CDR1, CDR2, and CDR3 shown in SEQ ID NO: 113 in combination with a second variable region comprising the amino acid sequence derived from the amino acid sequence of CDR1, CDR2, and CDR3 shown in SEQ ID NO: 114 (preferably, a variable region comprising the amino acid sequence shown in SEQ ID NO: 114);

(CV) a binding site comprising a first variable region comprising the amino acid sequence derived from the amino acid sequence of CDR1, CDR2, and CDR3 shown in SEQ ID NO: 115 in combination with a second variable region comprising the amino acid sequence derived from the amino acid sequence of CDR1, CDR2, and CDR3 shown in SEQ ID NO: 116;

(CVI) a binding site comprising a first variable region comprising the amino acid sequence derived from the amino acid sequence of CDR1, CDR2, and CDR3 shown in SEQ ID NO: 117 in combination with a second variable region comprising the amino acid sequence derived from the amino acid sequence of CDR1, CDR2, and CDR3 shown in SEQ ID NO: 118;

(CVII) a binding site comprising a first variable region comprising the amino acid sequence derived from the amino acid sequence of CDR1, CDR2, and CDR3 shown in SEQ ID NO: 119 in combination with a second variable region comprising the amino acid sequence derived from the amino acid sequence of CDR1, CDR2, and CDR3 shown in SEQ ID NO: 120;

(CVIII) a binding site comprising a first variable region comprising the amino acid sequence derived from the amino acid sequence of CDR1, CDR2, and CDR3 shown in SEQ ID NO: 121 in combination with a second variable region comprising the amino acid sequence derived from the amino acid sequence of CDR1, CDR2, and CDR3 shown in SEQ ID NO: 122;

(CVIIII) a binding site comprising a first variable region comprising the amino acid sequence derived from the amino acid sequence of CDR1, CDR2, and CDR3 shown in SEQ ID NO: 123 in combination with a second variable region comprising the amino acid sequence derived from the amino acid sequence of CDR1, CDR2, and CDR3 shown in SEQ ID NO: 124;

(CX) a binding site comprising a first variable region comprising the amino acid sequence derived from the amino acid sequence of CDR1, CDR2, and CDR3 shown in SEQ ID NO: 125 in combination with a second variable region comprising the amino acid sequence derived from the amino acid sequence of CDR1, CDR2, and CDR3 shown in SEQ ID NO: 126;

(CXI) a binding site comprising a first variable region comprising the amino acid sequence derived from the amino acid sequence of CDR1, CDR2, and CDR3 shown in SEQ ID NO: 127 in combination with a second variable region comprising the amino acid sequence derived from the amino acid sequence of CDR1, CDR2, and CDR3 shown in SEQ ID NO: 128;

(CXII) a binding site comprising a first variable region comprising the amino acid sequence derived from the amino acid sequence of CDR1, CDR2, and CDR3 shown in SEQ ID NO: 129 in combination with a second variable region comprising the amino acid sequence derived from the amino acid sequence of CDR1, CDR2, and CDR3 shown in SEQ ID NO: 130;

(CXIII) a binding site comprising a first variable region comprising the amino acid sequence derived from the amino acid sequence of CDR1, CDR2, and CDR3 shown in SEQ ID NO: 131 in combination with a second variable region comprising the amino acid sequence derived from the amino acid sequence of CDR1, CDR2, and CDR3 shown in SEQ ID NO: 132;

(CXIV) a binding site comprising a first variable region comprising the amino acid sequence derived from the amino acid sequence of CDR1, CDR2, and CDR3 shown in SEQ ID NO: 133 in combination with a second variable region comprising the amino acid sequence derived from the amino acid sequence of CDR1, CDR2, and CDR3 shown in SEQ ID NO: 134; and

(CXV) a binding site comprising a first variable region comprising the amino acid sequence derived from the amino acid sequence of CDR1, CDR2, and CDR3 shown in SEQ ID NO: 135 in combination with a second variable region comprising the amino acid sequence derived from the amino acid sequence of CDR1, CDR2, and CDR3 shown in SEQ ID NO: 136.

[47] The substance according to [46], which optionally comprises mutation of 1 or 2 amino acids on the C and/or N terminal sides of each CDR.

[48] The substance according to [46], which is a polypeptide comprising a binding site according to any of (CI) to (CXV) below as the site binding to KIR2DL1:

(CI) a binding site comprising a first variable region comprising the amino acid sequence shown in SEQ ID NO: 107 in combination with a second variable region comprising the amino acid sequence shown in SEQ ID NO: 108;

(CII) a binding site comprising a first variable region comprising the amino acid sequence shown in SEQ ID NO: 109 in combination with a second variable region comprising the amino acid sequence shown in SEQ ID NO: 110;

(CIII) a binding site comprising a first variable region comprising the amino acid sequence shown in SEQ ID NO: 111 in combination with a second variable region comprising the amino acid sequence shown in SEQ ID NO: 112;

(CIV) a binding site comprising a first variable region comprising the amino acid sequence shown in SEQ ID NO: 113 in combination with a second variable region comprising the amino acid sequence shown in SEQ ID NO: 114 (preferably, a variable region comprising the amino acid sequence shown in SEQ ID NO: 114);

(CV) a binding site comprising a first variable region comprising the amino acid sequence shown in SEQ ID NO: 115 in combination with a second variable region comprising the amino acid sequence shown in SEQ ID NO: 116;

(CVI) a binding site comprising a first variable region comprising the amino acid sequence shown in SEQ ID NO: 117 in combination with a second variable region comprising the amino acid sequence shown in SEQ ID NO: 118;

(CVII) a binding site comprising a first variable region comprising the amino acid sequence shown in SEQ ID NO: 119 in combination with a second variable region comprising the amino acid sequence shown in SEQ ID NO: 120;

(CVIII) a binding site comprising a first variable region comprising the amino acid sequence shown in SEQ ID NO: 121 in combination with a second variable region comprising the amino acid sequence shown in SEQ ID NO: 122;

(CVIIII) a binding site comprising a first variable region comprising the amino acid sequence shown in SEQ ID NO: 123 in combination with a second variable region comprising the amino acid sequence shown in SEQ ID NO: 124;

(CX) a binding site comprising a first variable region comprising the amino acid sequence shown in SEQ ID NO: 125 in combination with a second variable region comprising the amino acid sequence shown in SEQ ID NO: 126;

(CXI) a binding site comprising a first variable region comprising the amino acid sequence shown in SEQ ID NO: 127 in combination with a second variable region comprising the amino acid sequence shown in SEQ ID NO: 128;

(CXII) a binding site comprising a first variable region comprising the amino acid sequence shown in SEQ ID NO: 129 in combination with a second variable region comprising the amino acid sequence shown in SEQ ID NO: 130;

(CXIII) a binding site comprising a first variable region comprising the amino acid sequence shown in SEQ ID NO: 131 in combination with a second variable region comprising the amino acid sequence shown in SEQ ID NO: 132;

(CXIV) a binding site comprising a first variable region comprising the amino acid sequence shown in SEQ ID NO: 133 in combination with a second variable region comprising the amino acid sequence shown in SEQ ID NO: 134; and

(CXV) a binding site comprising a first variable region comprising the amino acid sequence shown in SEQ ID NO: 135 in combination with a second variable region comprising the amino acid sequence shown in SEQ ID NO: 136.

[49] The substance according to [43], wherein the activity of binding to KIR2DL1 is higher than the activity of binding to KIR2DS1.

[50] The substance according to [43], wherein the agonist activity on KIR2DL1 is higher than the agonist activity on KIR2DS1.

[51] The substance according to [50], which exerts substantially no agonist activity on KIR2DS1.

[52] The substance according to [51], which does not substantially bind to KIR2DS1, KIR2DS2, and KIR2DS4.

[53] The substance according to [52], which comprises an antigen-binding fragment obtained by the method comprising:

step A: a step of obtaining a population of display bodies displaying an antigen-binding site binding to KIR2DL1 comprising mixing a labeled soluble protein comprising an extracellular domain of KIR2DL1 in a suspension containing a population of polyclonal display bodies including display bodies displaying, on the surface, the antigen-binding site binding to KIR2DL1, bringing the suspension into contact with a carrier comprising, immobilized thereon, a substance binding specifically to the labeled part, and collecting the display bodies bound to the carrier;

step B: a step of removing display bodies displaying the antigen-binding site binding to KIR2DS1, KIR2DS2, and KIR2DS4 from the population of display bodies comprising mixing a labeled soluble protein comprising extracellular domains of KIR2DS1, KIR2DS2, and KIR2DS4 in the suspension containing the population of display bodies obtained in step A, bringing the suspension into contact with a carrier comprising, immobilized thereon, a substance binding to the labeled part, and collecting the display bodies not bound to the carrier;

step C: a step of identifying a sequence of a gene encoding the antigen-binding site by performing genetic analysis of the display bodies obtained by the method comprising the steps described above; and

step D: a step of expressing the identified gene comprising the antigen-binding site in a cell.

[54] The substance according to [52], which comprises an antigen-binding fragment obtained by the method comprising:

step A: a step of obtaining a population of display bodies displaying an antigen-binding site binding specifically to KIR2DL1 comprising mixing a labeled soluble protein comprising an extracellular domain of KIR2DL1 and an unlabeled soluble protein comprising extracellular domains of KIR2DS1, KIR2DS2, and KIR2DS4 in a suspension containing a population of polyclonal display bodies including display bodies displaying, on the surface, the antigen-binding site binding to KIR2DL1, bringing the suspension into contact with a carrier comprising, immobilized thereon, a substance binding specifically to the labeled part, and collecting the display bodies bound to the carrier;

step B: a step of identifying a sequence of a gene encoding the antigen-binding site by performing genetic analysis of the display bodies obtained by the method comprising the steps described above; and

step C: a step of expressing the identified gene comprising the antigen-binding site in a cell.

[55] The substance according to [53] or [54], wherein a combination of a label and a label-binding substance is a combination of biotin and avidin.

[56] The substance according to any of [53] to [55], which is obtained by a method further comprising a step of selecting a substance having an agonist activity on KIR3DL1.

[57] The substance according to any of [43] to [56], wherein the polypeptide is a monoclonal antibody or an antigen-binding fragment.

[58] The substance according to [57], which is a single-stranded antibody or scFv comprising a first variable region linked to a second variable region via a GS linker.

[59] The substance according to any of [43] to [56], wherein the polypeptide is a membrane protein having an extracellular domain comprising the site binding to KIR2DL1 and a transmembrane domain.

[60] A pharmaceutical composition comprising, as an active ingredient, the substance according to any of [1] to [59].

[61] The pharmaceutical composition according to [60], which is an immune rejection inhibitor.

[62] A cell comprising a substance having activity of binding to the inhibitory KIR according to any of [1] to [59] and inhibiting the cytocidal activity of an NK cell expressed on the surface.

[63] The cell according to [62], which further comprises a ligand for NKG2A expressed on the cell surface.

[64] The cell according to [63], wherein the ligand for NKG2A is HLA-E.

[65] The cell according to [62], wherein the expression of at least one class I HLA is quenched or attenuated on the cell surface.

[66] The cell according to [65], wherein the expression of at least one class I HLA is quenched or attenuated on the cell surface by modification of class I HLA, B2M, TAP1, TAP2, and TAPBP regions on the genome by gene editing.

[67] The cell according to [65], which is a cell line established from an individual in which HLA expression is genetically quenched.

[68] The cell according to [62], which further comprises a ligand for NKG2A expressed on the cell surface and shows quenched or attenuated expression of at least one class I HLA on the cell surface.

[69] The cell according to [62], which is a pluripotent stem cell, T cell, NK cell, peripheral blood mononuclear cell (PBMC), mesenchymal stem cell, myocardial cell, cartilage cell, retinal cell, hepatic cell, kidney cell, or pancreatic cell.

[70] The cell according to [69], wherein the pluripotent stem cell is an iPS cell or ES cell.

[71] The cell according to any of [62] to [70], which further comprises a chimeric receptor expressed on the cell surface.

[72] The cell according to [62], wherein the inhibitory KIR is KIR2DL2 and/or KIR2DL3 and the substance having activity of binding to KIR2DL2 and/or KIR2DL3 and inhibiting the cytocidal activity of an NK cell is the membrane protein according to [26].

[73] The cell according to [72], wherein the membrane protein binding to KIR2DL2 and/or KIR2DL3 comprises a binding site competing with Lirilumab, Pan2D, or scFv consisting of an amino acid sequence according to any of (i) to (xii) below when binding to KIR2DL2 and/or KIR2DL3:

(i) an amino acid sequence comprising the amino acid sequence of a heavy chain variable region shown in SEQ ID NO: 33 in combination with the amino acid sequence of a light chain variable region shown in SEQ ID NO: 34;
(ii) an amino acid sequence comprising the amino acid sequence of a heavy chain variable region shown in SEQ ID NO: 35 in combination with the amino acid sequence of a light chain variable region shown in SEQ ID NO: 36;
(iii) an amino acid sequence comprising the amino acid sequence of a heavy chain variable region shown in SEQ ID NO: 37 in combination with the amino acid sequence of a light chain variable region shown in SEQ ID NO: 38;
(iv) an amino acid sequence comprising the amino acid sequence of a heavy chain variable region shown in SEQ ID NO: 39 in combination with the amino acid sequence of a light chain variable region shown in SEQ ID NO: 40;
(v) an amino acid sequence comprising the amino acid sequence of a heavy chain variable region shown in SEQ ID NO: 41 in combination with the amino acid sequence of a light chain variable region shown in SEQ ID NO: 42;
(vi) an amino acid sequence comprising the amino acid sequence of a heavy chain variable region shown in SEQ ID NO: 43 in combination with the amino acid sequence of a light chain variable region shown in SEQ ID NO: 44;
(vii) an amino acid sequence comprising the amino acid sequence of a heavy chain variable region shown in SEQ ID NO: 45 in combination with the amino acid sequence of a light chain variable region shown in SEQ ID NO: 46;
(viii) an amino acid sequence comprising the amino acid sequence of a heavy chain variable region shown in SEQ ID NO: 47 in combination with the amino acid sequence of a light chain variable region shown in SEQ ID NO: 48;
(ix) an amino acid sequence comprising the amino acid sequence of a heavy chain variable region shown in SEQ ID NO: 49 in combination with the amino acid sequence of a light chain variable region shown in SEQ ID NO: 50;
(x) an amino acid sequence comprising the amino acid sequence of a heavy chain variable region shown in SEQ ID NO: 51 in combination with the amino acid sequence of a light chain variable region shown in SEQ ID NO: 52;
(xi) an amino acid sequence comprising the amino acid sequence of a heavy chain variable region shown in SEQ ID NO: 61 in combination with the amino acid sequence of a light chain variable region shown in SEQ ID NO: 62; and
(xii) an amino acid sequence comprising the amino acid sequence of a heavy chain variable region shown in SEQ ID NO: 63 in combination with the amino acid sequence of a light chain variable region shown in SEQ ID NO: 64.

[74] The cell according to [72], which comprises, as the site binding to KIR2DL2 and/or KIR2DL3, a membrane protein comprising a binding site according to any of (I) to (XII) below and optionally comprising mutation of 1 or 2 amino acids in each CDR expressed on the cell surface:

(I) a binding site comprising a first variable region comprising the amino acid sequence derived from the amino acid sequence of CDR1, CDR2, and CDR3 shown in SEQ ID NO: 33 in combination with a second variable region comprising the amino acid sequence derived from the amino acid sequence of CDR1, CDR2, and CDR3 shown in SEQ ID NO: 34;
(II) a binding site comprising a first variable region comprising the amino acid sequence derived from the amino acid sequence of CDR1, CDR2, and CDR3 shown in SEQ ID NO: 33 in combination with a second variable region comprising the amino acid sequence derived from the amino acid sequence of CDR1, CDR2, and CDR3 shown in SEQ ID NO: 36;
(III) a binding site comprising a first variable region comprising the amino acid sequence derived from the amino acid sequence of CDR1, CDR2, and CDR3 shown in SEQ ID NO: 37 in combination with a second variable region comprising the amino acid sequence derived from the amino acid sequence of CDR1, CDR2, and CDR3 shown in SEQ ID NO: 38;
(IV) a binding site comprising a first variable region comprising the amino acid sequence derived from the amino acid sequence of CDR1, CDR2, and CDR3 shown in SEQ ID NO: 39 in combination with a second variable region comprising the amino acid sequence derived from the amino acid sequence of CDR1, CDR2, and CDR3 shown in SEQ ID NO: 40;
(V) a binding site comprising a first variable region comprising the amino acid sequence derived from the amino acid sequence of CDR1, CDR2, and CDR3 shown in SEQ ID NO: 41 in combination with a second variable region comprising the amino acid sequence derived from the amino acid sequence of CDR1, CDR2, and CDR3 shown in SEQ ID NO: 42;

(VI) a binding site comprising a first variable region comprising the amino acid sequence derived from the amino acid sequence of CDR1, CDR2, and CDR3 shown in SEQ ID NO: 43 in combination with a second variable region comprising the amino acid sequence derived from the amino acid sequence of CDR1, CDR2, and CDR3 shown in SEQ ID NO: 44;

(VII) a binding site comprising a first variable region comprising the amino acid sequence derived from the amino acid sequence of CDR1, CDR2, and CDR3 shown in SEQ ID NO: 45 in combination with a second variable region comprising the amino acid sequence derived from the amino acid sequence of CDR1, CDR2, and CDR3 shown in SEQ ID NO: 46;

(VIII) a binding site comprising a first variable region comprising the amino acid sequence derived from the amino acid sequence of CDR1, CDR2, and CDR3 shown in SEQ ID NO: 47 in combination with a second variable region comprising the amino acid sequence derived from the amino acid sequence of CDR1, CDR2, and CDR3 shown in SEQ ID NO: 48;

(IX) a binding site comprising a first variable region comprising the amino acid sequence derived from the amino acid sequence of CDR1, CDR2, and CDR3 shown in SEQ ID NO: 49 in combination with a second variable region comprising the amino acid sequence derived from the amino acid sequence of CDR1, CDR2, and CDR3 shown in SEQ ID NO: 50;

(X) a binding site comprising a first variable region comprising the amino acid sequence derived from the amino acid sequence of CDR1, CDR2, and CDR3 shown in SEQ ID NO: 51 in combination with a second variable region comprising the amino acid sequence derived from the amino acid sequence of CDR1, CDR2, and CDR3 shown in SEQ ID NO: 52;

(XI) a binding site comprising a first variable region comprising the amino acid sequence derived from the amino acid sequence of CDR1, CDR2, and CDR3 shown in SEQ ID NO: 61 in combination with a second variable region comprising the amino acid sequence derived from the amino acid sequence of CDR1, CDR2, and CDR3 shown in SEQ ID NO: 62; and

(XII) a binding site comprising a first variable region comprising the amino acid sequence derived from the amino acid sequence of CDR1, CDR2, and CDR3 shown in SEQ ID NO: 63 in combination with a second variable region comprising the amino acid sequence derived from the amino acid sequence of CDR1, CDR2, and CDR3 shown in SEQ ID NO: 64.

[75] The cell according to [74], which optionally comprises mutation of 1 or 2 amino acids on the C and/or N terminal sides of each CDR.

[76] The cell according to [62], wherein the inhibitory KIR is KIR3DL1 and the substance having activity of binding to KIR3DL1 and inhibiting the cytocidal activity of an NK cell is the membrane protein according to [42].

[77] The cell according to [76], wherein the membrane protein binding to KIR3DL1 comprises a binding site competing with scFv consisting of an amino acid sequence according to any of (xiii) to (xviii) below when binding to KIR3DL1:

(xiii) an amino acid sequence comprising the amino acid sequence of a heavy chain variable region shown in SEQ ID NO: 56 in combination with the amino acid sequence of a light chain variable region shown in SEQ ID NO: 55;

(xiv) an amino acid sequence comprising the amino acid sequence of a heavy chain variable region shown in SEQ ID NO: 137 in combination with the amino acid sequence of a light chain variable region shown in SEQ ID NO: 138;

(xv) an amino acid sequence comprising the amino acid sequence of a heavy chain variable region shown in SEQ ID NO: 139 in combination with the amino acid sequence of a light chain variable region shown in SEQ ID NO: 140;

(xvi) an amino acid sequence comprising the amino acid sequence of a heavy chain variable region shown in SEQ ID NO: 141 in combination with the amino acid sequence of a light chain variable region shown in SEQ ID NO: 142;

(xvii) an amino acid sequence comprising the amino acid sequence of a heavy chain variable region shown in SEQ ID NO: 143 in combination with the amino acid sequence of a light chain variable region shown in SEQ ID NO: 144; and

(xviii) an amino acid sequence comprising the amino acid sequence of a heavy chain variable region shown in SEQ ID NO: 145 in combination with the amino acid sequence of a light chain variable region shown in SEQ ID NO: 146.

[78] The cell according to [75], which comprises, as the site binding to KIR3DL1, a membrane protein comprising a binding site according to any of (XIII) to (XVIII) below and optionally comprising mutation of 1 or 2 amino acids in each CDR expressed on the cell surface:

(XIII) a binding site comprising a first variable region comprising the amino acid sequence derived from the amino acid sequence of CDR1, CDR2, and CDR3 shown in SEQ ID NO: 56 in combination with a second variable region comprising the amino acid sequence derived from the amino acid sequence of CDR1, CDR2, and CDR3 shown in SEQ ID NO: 55;

(XIV) a binding site comprising a first variable region comprising the amino acid sequence derived from the amino acid sequence of CDR1, CDR2, and CDR3 shown in SEQ ID NO: 137 in combination with a second variable region comprising the amino acid sequence derived from the amino acid sequence of CDR1, CDR2, and CDR3 shown in SEQ ID NO: 138 (preferably, a variable region comprising the amino acid sequence shown in SEQ ID NO: 138);

(XV) a binding site comprising a first variable region comprising the amino acid sequence derived from the amino acid sequence of CDR1, CDR2, and CDR3 shown in SEQ ID NO: 139 in combination with a second variable region comprising the amino acid sequence derived from the amino acid sequence of CDR1, CDR2, and CDR3 shown in SEQ ID NO: 140;

(XVI) a binding site comprising a first variable region comprising the amino acid sequence derived from the amino acid sequence of CDR1, CDR2, and CDR3 shown in SEQ ID NO: 141 in combination with a second variable region comprising the amino acid sequence derived from the amino acid sequence of CDR1, CDR2, and CDR3 shown in SEQ ID NO: 142;

(XVII) a binding site comprising a first variable region comprising the amino acid sequence derived from the amino acid sequence of CDR1, CDR2, and CDR3 shown in SEQ ID NO: 143 in combination with a second variable region comprising the amino acid sequence derived from the amino acid sequence of CDR1, CDR2, and CDR3 shown in SEQ ID NO: 144; and

(XVIII) a binding site comprising a first variable region comprising the amino acid sequence derived from the amino acid sequence of CDR1, CDR2, and CDR3 shown in SEQ ID NO: 145 in combination with a second variable region comprising the amino acid sequence derived from the amino acid sequence of CDR1, CDR2, and CDR3 shown in SEQ ID NO: 146.

[79] The cell according to [78], which optionally comprises mutation of 1 or 2 amino acids on the C and/or N terminal sides of each CDR.

[80] The cell according to [62], wherein the inhibitory KIR is KIR2DL1 and the substance having activity of binding to KIR2DL1 and inhibiting the cytocidal activity of an NK cell is the membrane protein according to [59].

[81] The cell according to [80], wherein the membrane protein binding to KIR2DL1 comprises a binding site competing with Pan2D when binding to KIR2DL1.

[82] The cell according to [78], which comprises, as the site binding to KIR2DL1, a membrane protein comprising the binding site (P) below and optionally comprising mutation of 1 or 2 amino acids in each CDR expressed on the cell surface:

(P) a binding site comprising a first variable region comprising the amino acid sequence derived from the amino acid sequence of CDR1, CDR2, and CDR3 shown in SEQ ID NO: 31 in combination with a second variable region comprising the amino acid sequence derived from the amino acid sequence of CDR1, CDR2, and CDR3 shown in SEQ ID NO: 32.

[83] The cell according to [82], which optionally comprises mutation of 1 or 2 amino acids on the C and/or N terminal sides of each CDR.

[84] The cell according to [80], wherein the membrane protein binding to KIR2DL1 comprises a binding site competing with scFv consisting of an amino acid sequence according to any of (ci) to (cxv) below when binding to KIR2DL1:

(ci) an amino acid sequence comprising the amino acid sequence of a heavy chain variable region shown in SEQ ID NO: 107 in combination with the amino acid sequence of a light chain variable region shown in SEQ ID NO: 108;

(cii) an amino acid sequence comprising the amino acid sequence of a heavy chain variable region shown in SEQ ID NO: 109 in combination with the amino acid sequence of a light chain variable region shown in SEQ ID NO: 110;

(ciii) an amino acid sequence comprising the amino acid sequence of a heavy chain variable region shown in SEQ ID NO: 111 in combination with the amino acid sequence of a light chain variable region shown in SEQ ID NO: 112;

(civ) an amino acid sequence comprising the amino acid sequence of a heavy chain variable region shown in SEQ ID NO: 113 in combination with the amino acid sequence of a light chain variable region shown in SEQ ID NO: 114;

(cv) an amino acid sequence comprising the amino acid sequence of a heavy chain variable region shown in SEQ ID NO: 115 in combination with the amino acid sequence of a light chain variable region shown in SEQ ID NO: 116;

(cvi) an amino acid sequence comprising the amino acid sequence of a heavy chain variable region shown in SEQ ID NO: 117 in combination with the amino acid sequence of a light chain variable region shown in SEQ ID NO: 118;

(cvii) an amino acid sequence comprising the amino acid sequence of a heavy chain variable region shown in SEQ ID NO: 119 in combination with the amino acid sequence of a light chain variable region shown in SEQ ID NO: 120;

(cviii) an amino acid sequence comprising the amino acid sequence of a heavy chain variable region shown in SEQ ID NO: 121 in combination with the amino acid sequence of a light chain variable region shown in SEQ ID NO:

122;

(cviiii) an amino acid sequence comprising the amino acid sequence of a heavy chain variable region shown in SEQ ID NO: 123 in combination with the amino acid sequence of a light chain variable region shown in SEQ ID NO: 124;

(cx) an amino acid sequence comprising the amino acid sequence of a heavy chain variable region shown in SEQ ID NO: 125 in combination with the amino acid sequence of a light chain variable region shown in SEQ ID NO: 126;

(cxi) an amino acid sequence comprising the amino acid sequence of a heavy chain variable region shown in SEQ ID NO: 127 in combination with the amino acid sequence of a light chain variable region shown in SEQ ID NO: 128;

(cxii) an amino acid sequence comprising the amino acid sequence of a heavy chain variable region shown in SEQ ID NO: 129 in combination with the amino acid sequence of a light chain variable region shown in SEQ ID NO: 130;

(cxiii) an amino acid sequence comprising the amino acid sequence of a heavy chain variable region shown in SEQ ID NO: 131 in combination with the amino acid sequence of a light chain variable region shown in SEQ ID NO: 132;

(cxiv) an amino acid sequence comprising the amino acid sequence of a heavy chain variable region shown in SEQ ID NO: 133 in combination with the amino acid sequence of a light chain variable region shown in SEQ ID NO: 134; and

(cxv) an amino acid sequence comprising the amino acid sequence of a heavy chain variable region shown in SEQ ID NO: 135 in combination with the amino acid sequence of a light chain variable region shown in SEQ ID NO: 136.

[85] The cell according to [80], which comprises, as the site binding to KIR2DL1, a membrane protein comprising a binding site according to any of (CI) to (CXV) below and optionally comprising mutation of 1 or 2 amino acids in each CDR expressed on the cell surface:

(CI) a binding site comprising a first variable region comprising the amino acid sequence derived from the amino acid sequence of CDR1, CDR2, and CDR3 shown in SEQ ID NO: 107 in combination with a second variable region comprising the amino acid sequence derived from the amino acid sequence of CDR1, CDR2, and CDR3 shown in SEQ ID NO: 108;

(CII) a binding site comprising a first variable region comprising the amino acid sequence derived from the amino acid sequence of CDR1, CDR2, and CDR3 shown in SEQ ID NO: 109 in combination with a second variable region comprising the amino acid sequence derived from the amino acid sequence of CDR1, CDR2, and CDR3 shown in SEQ ID NO: 110;

(CIII) a binding site comprising a first variable region comprising the amino acid sequence derived from the amino acid sequence of CDR1, CDR2, and CDR3 shown in SEQ ID NO: 111 in combination with a second variable region comprising the amino acid sequence derived from the amino acid sequence of CDR1, CDR2, and CDR3 shown in SEQ ID NO: 112;

(CIV) a binding site comprising a first variable region comprising the amino acid sequence derived from the amino acid sequence of CDR1, CDR2, and CDR3 shown in SEQ ID NO: 113 in combination with a second variable region comprising the amino acid sequence derived from the amino acid sequence of CDR1, CDR2, and CDR3 shown in SEQ ID NO: 114 (preferably, a variable region comprising the amino acid sequence shown in SEQ ID NO: 114);

(CV) a binding site comprising a first variable region comprising the amino acid sequence derived from the amino acid sequence of CDR1, CDR2, and CDR3 shown in SEQ ID NO: 115 in combination with a second variable region comprising the amino acid sequence derived from the amino acid sequence of CDR1, CDR2, and CDR3 shown in SEQ ID NO: 116;

(CVI) a binding site comprising a first variable region comprising the amino acid sequence derived from the amino acid sequence of CDR1, CDR2, and CDR3 shown in SEQ ID NO: 117 in combination with a second variable region comprising the amino acid sequence derived from the amino acid sequence of CDR1, CDR2, and CDR3 shown in SEQ ID NO: 118;

(CVII) a binding site comprising a first variable region comprising the amino acid sequence derived from the amino acid sequence of CDR1, CDR2, and CDR3 shown in SEQ ID NO: 119 in combination with a second variable region comprising the amino acid sequence derived from the amino acid sequence of CDR1, CDR2, and CDR3 shown in SEQ ID NO: 120;

(CVIII) a binding site comprising a first variable region comprising the amino acid sequence derived from the amino acid sequence of CDR1, CDR2, and CDR3 shown in SEQ ID NO: 121 in combination with a second variable region comprising the amino acid sequence derived from the amino acid sequence of CDR1, CDR2, and CDR3 shown in SEQ ID NO: 122;

(CVIIII) a binding site comprising a first variable region comprising the amino acid sequence derived from the amino acid sequence of CDR1, CDR2, and CDR3 shown in SEQ ID NO: 123 in combination with a second variable region comprising the amino acid sequence derived from the amino acid sequence of CDR1, CDR2, and CDR3 shown in SEQ ID NO: 124;

(CX) a binding site comprising a first variable region comprising the amino acid sequence derived from the amino acid sequence of CDR1, CDR2, and CDR3 shown in SEQ ID NO: 125 in combination with a second variable region comprising the amino acid sequence derived from the amino acid sequence of CDR1, CDR2, and CDR3 shown in SEQ ID NO: 126;

(CXI) a binding site comprising a first variable region comprising the amino acid sequence derived from the amino acid sequence of CDR1, CDR2, and CDR3 shown in SEQ ID NO: 127 in combination with a second variable region comprising the amino acid sequence derived from the amino acid sequence of CDR1, CDR2, and CDR3 shown in SEQ ID NO: 128;

(CXII) a binding site comprising a first variable region comprising the amino acid sequence derived from the amino acid sequence of CDR1, CDR2, and CDR3 shown in SEQ ID NO: 129 in combination with a second variable region comprising the amino acid sequence derived from the amino acid sequence of CDR1, CDR2, and CDR3 shown in SEQ ID NO: 130;

(CXIII) a binding site comprising a first variable region comprising the amino acid sequence derived from the amino acid sequence of CDR1, CDR2, and CDR3 shown in SEQ ID NO: 131 in combination with a second variable region comprising the amino acid sequence derived from the amino acid sequence of CDR1, CDR2, and CDR3 shown in SEQ ID NO: 132;

(CXIV) a binding site comprising a first variable region comprising the amino acid sequence derived from the amino acid sequence of CDR1, CDR2, and CDR3 shown in SEQ ID NO: 133 in combination with a second variable region comprising the amino acid sequence derived from the amino acid sequence of CDR1, CDR2, and CDR3 shown in SEQ ID NO: 134; and

(CXV) a binding site comprising a first variable region comprising the amino acid sequence derived from the amino acid sequence of CDR1, CDR2, and CDR3 shown in SEQ ID NO: 135 in combination with a second variable region comprising the amino acid sequence derived from the amino acid sequence of CDR1, CDR2, and CDR3 shown in SEQ ID NO: 136.

[86] The cell according to [85], which optionally comprises mutation of 1 or 2 amino acids on the C and/or N terminal sides of each CDR.

[87] A pharmaceutical composition comprising, as an active ingredient, the cell according to any of [62] to [86] and exhibiting a lower risk of immune rejection in a target of administration or transplantation.

[88] The pharmaceutical composition according to [87], which is an immune rejection inhibitor.

[89] A method for lowering or inhibiting immune rejection against a therapeutic cell by a subject comprising a step of expressing the substance according to any of [1] to [59] on the surface of the therapeutic cell.

[90] A method for producing a therapeutic cell exhibiting a lowered or inhibited risk of immune rejection by a subject, which comprises a step of expressing the substance according to any of [1] to [59] on the surface of the therapeutic cell.

[91] A method for evaluating activity of a test substance for avoiding immune rejection caused by the agonist activity on inhibitory KIR2DL2 or KIR2DL3 comprising:

(A) a step of bringing NK cells derived from a HLA-C1/C1 homozygous subject into contact with target cells derived from a HLA-C2/C2 homozygous subject *ex vivo* in the presence of a test substance binding to KIR2DL2 or KIR2DL3 (wherein the Bw4 motif-containing HLA phenotype of the donor of the NK cells is identical to that of the donor of the target cells); and
(B) a subsequent step of measuring the viable target cell count.

[92] A method for evaluating activity of a test substance for avoiding immune rejection caused by the agonist activity on inhibitory KIR2DL1 comprising:

(A) a step of bringing NK cells derived from a HLA-C2/C2 homozygous subject into contact with target cells derived from a HLA-C1/C1 homozygous subject *ex vivo* in the presence of a test substance binding to KIR2DL1 (wherein the Bw4 motif-containing HLA phenotype of the donor of the NK cells is identical to that of the donor of the target cells); and
(B) a subsequent step of measuring the viable target cell count.

[93] A method for evaluating activity of a test substance for avoiding immune rejection caused by the agonist activity on inhibitory KIR3DL1 comprising:

(A) a step of bringing NK cells derived from a HLA-Bw4$^+$/Bw4$^+$ homozygous subject or a Bw4$^+$/Bw4$^-$ heterozygous subject into contact with target cells derived from a HLA-Bw4$^-$/Bw4$^-$ homozygous subject *ex vivo* in the presence of a test substance binding to KIR3DL1 (wherein the HLA-C phenotype of the donor of the NK cells is identical to that of the donor of the target cells); and
(B) a subsequent step of measuring the viable target cell count.

[94] A method for screening for a substance having activity of avoiding immune rejection comprising a step of selecting a test substance exhibiting a high viable target cell count in the step B as a substance having activity of avoiding immune rejection by the method according to any of [91] to [93].
[95] The method according to any of [91] to [93], wherein, in step (A), the test substance is expressed as a membrane protein on the surface of the target cells.
[96] A method for producing a substance having activity of binding to human inhibitory KIR and inhibiting NK cells comprising:

step A: a step of obtaining a population of display bodies displaying an antigen-binding site binding to inhibitory KIR comprising mixing a labeled soluble protein comprising an extracellular domain of inhibitory KIR in a suspension containing a population of polyclonal display bodies including display bodies displaying an antigen-binding site binding to inhibitory KIR on the surface, bringing the suspension into contact with a carrier comprising, immobilized thereon, a substance binding specifically to the labeled part, and collecting the display bodies bound to the carrier;
step B: a step of removing display bodies displaying the antigen-binding site binding to the activating KIR from the population of display bodies comprising mixing a labeled soluble protein comprising an extracellular domain of activating KIR corresponding to the inhibitory KIR in the suspension containing the population of display bodies obtained in step A, bringing the suspension into contact with a carrier comprising, immobilized thereon, a substance binding to the labeled part, and collecting the display bodies not bound to the carrier;
step C: a step of identifying a sequence of a gene encoding the antigen-binding site by performing genetic analysis of the display bodies obtained by the method comprising the steps described above; and
step D: a step of expressing the identified gene comprising the antigen-binding site in a cell.

[97] A method for producing a substance having activity of binding to human inhibitory KIR and inhibiting NK cells comprising:

step A: a step of obtaining a population of display bodies displaying an antigen-binding site binding specifically to inhibitory KIR comprising mixing a labeled soluble protein comprising an extracellular domain of inhibitory KIR and an unlabeled soluble protein comprising an extracellular domain of activating KIR corresponding to the inhibitory KIR in a suspension containing a population of polyclonal display bodies including display bodies displaying the antigen-binding site binding to the inhibitory KIR on the surface, bringing the suspension into contact with a carrier comprising, immobilized thereon, a substance binding specifically to the labeled part, and collecting the display bodies bound to the carrier;
step B: a step of identifying a sequence of a gene encoding the antigen-binding site by performing genetic analysis of the display bodies obtained by the method comprising the steps described above; and
step C: a step of expressing the identified gene comprising the antigen-binding site in a cell.

[98] The method according to [96] or [97], wherein a combination of a label and a label-binding substance is a combination of biotin and avidin.
[99] The method according to any of [96] to [98], which further comprises a step of selecting a substance having an agonist activity on the inhibitory KIR.
[100] The method according to any of [96] to [99], wherein the inhibitory KIR is KIR2DL2 or KIR2DL3 and activating KIR corresponding thereto is KIR2DS1, KIR2DS2, or KIR2DS4.
[101] The method according to any of [96] to [99], wherein the inhibitory KIR is KIR3DL1 and activating KIR corresponding thereto is KIR3DS1.
[102] The method according to any of [96] to [99], wherein the inhibitory KIR is KIR2DL1 and activating KIR corresponding thereto is KIR2DS1, KIR2DS2, or KIR2DS4.

The description incorporates the contents disclosed by JP Patent Application No. 2022-170981, based on which the priority of the present application claims.

Advantageous Effects of Invention

A substance having activity of binding to inhibitory KIR and inhibiting the cytocidal activity of an NK cell or a cell expressing the same inhibits the cytocidal activity of an NK cell, and such substance or cell can thus inhibit rejection at the time of organ or therapeutic cell transplantation. Accordingly, a substance having activity of binding to inhibitory KIR and inhibiting the cytocidal activity of an NK cell or a cell expressing the same can be used for a novel allogeneic technology that can significantly change the versatility of organ transplantation or cell therapy.

Brief Description of Drawings

[Figure 1-1]

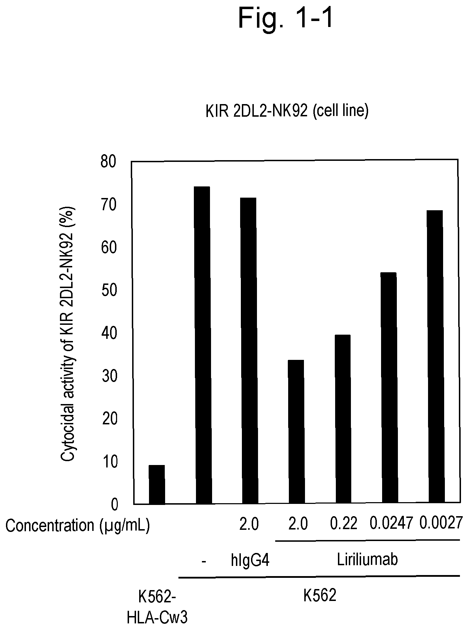

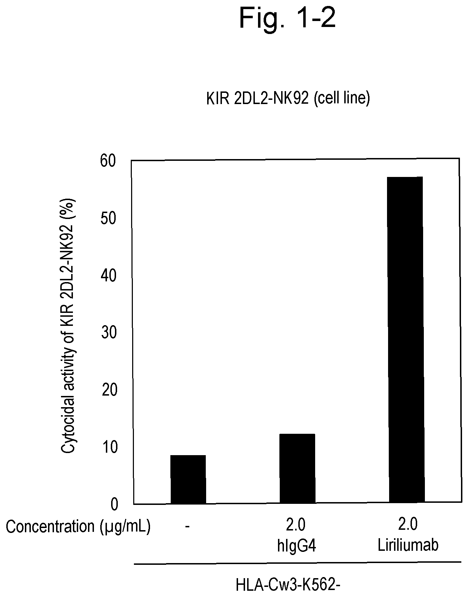

Figure 1-1 shows the results of evaluation of the cytocidal activity of KIR2DL2-expressing NK92 on K562 and shows the agonist activity of Lirilumab on inhibitory KIR2DL2.
[Figure 1-2]
Figure 1-2 shows the results of evaluation of the cytocidal activity of KIR2DL2-expressing NK92 on HLA-Cw3-K562 and shows the inhibitory activity of Lirilumab on an agonist for the inhibitory KIR2DL2.
[Figure 2]

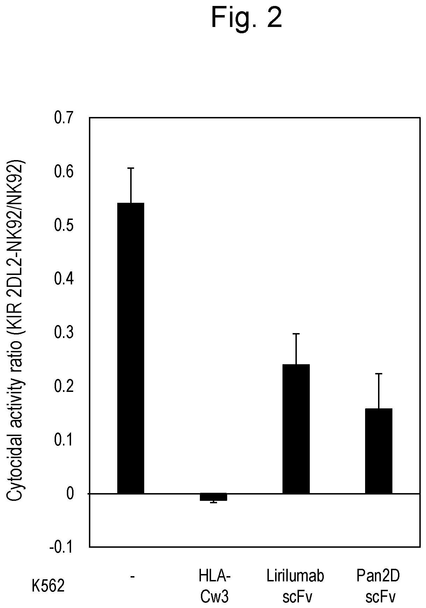

Figure 2 demonstrates that the anti-KIR antibody, Lirilumab or Pan2D, expressed in the form of an scFv-membrane protein attenuates the cytocidal activity on the KIR2DL2-NK92-expressing cell line.
[Figure 3]

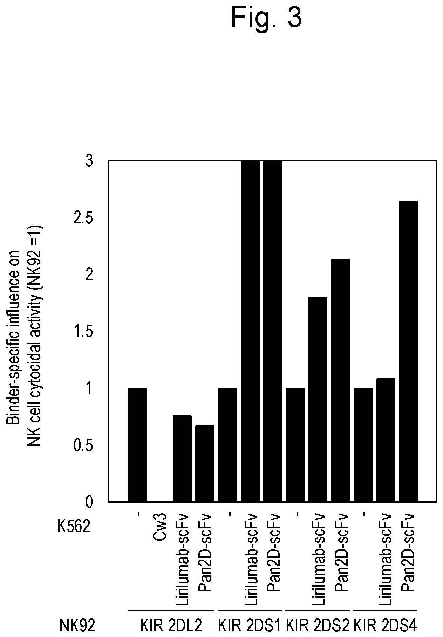

Figure 3 demonstrates that a binder exhibiting a binding capability to activating KIR potentiates activity of an NK cell line expressing activating KIR.
[Figure 4]

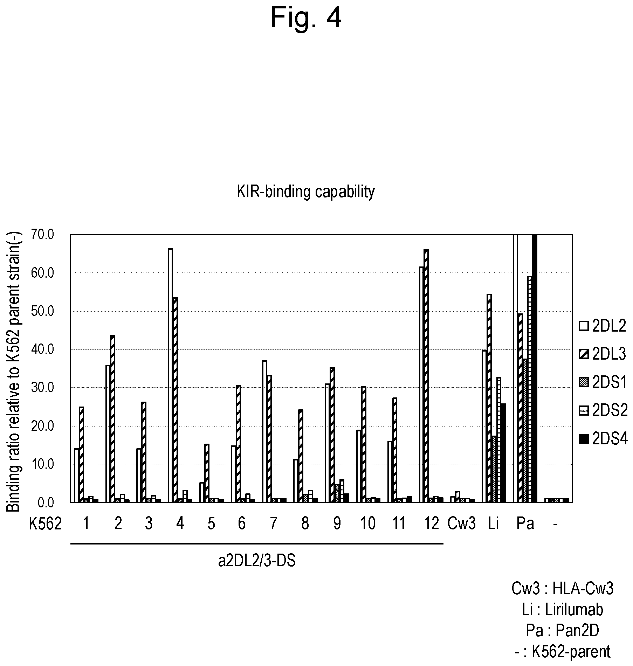

Figure 4 shows a binding capability of a novel KIR2DL2/3-specific binder obtained by phage panning to inhibitory KIR and activating KIR.
[Figure 5]

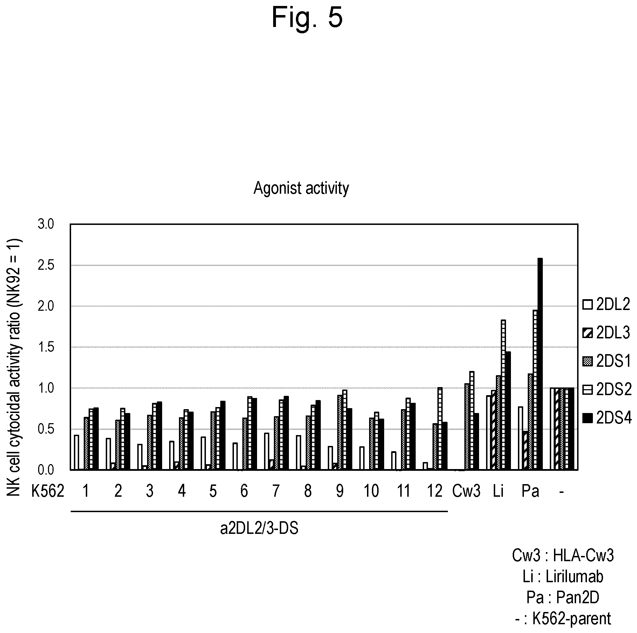

Figure 5 shows the agonist activity of a novel KIR2DL2/3-specific binder obtained by phage panning on inhibitory KIR.
[Figure 6]

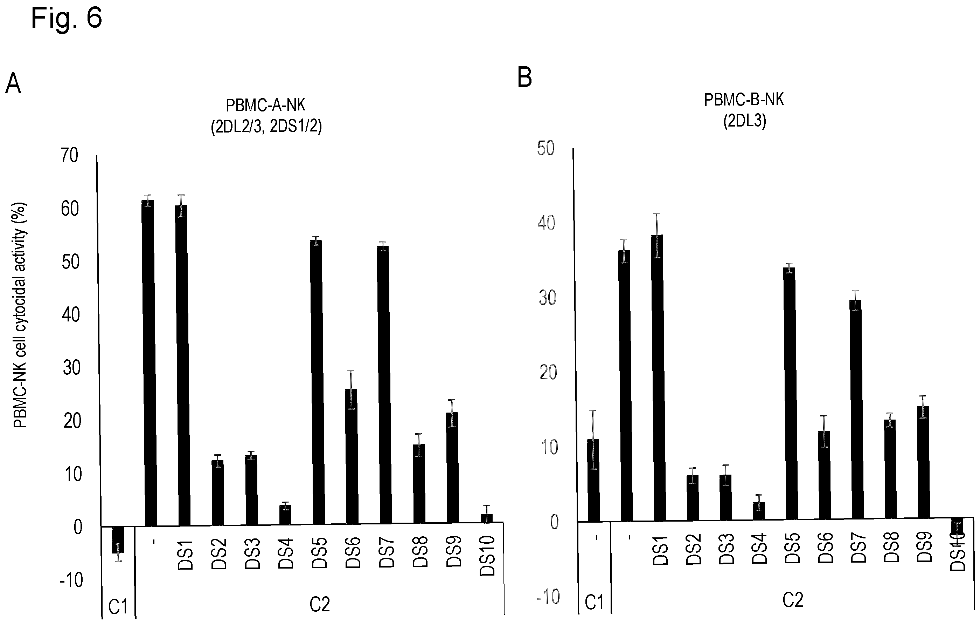

Figure 6 shows the results of evaluation of agonist activity of a KIR2DL2/3-specific binder using a primary NK cell. Figure 6A shows the results obtained with the use of NK cells having the KIR2DL2, KIR2DL3, KIR2DS1, and KIR2DS2 genes, and Figure 6B shows the results obtained with the use of NK cells having the KIR2DL3 gene.
[Figure 7]

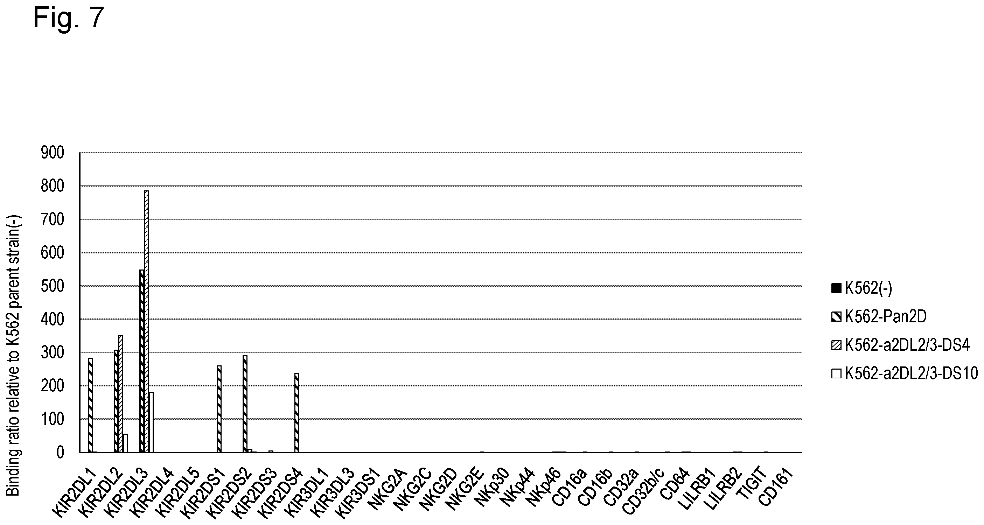

Figure 7 demonstrates that a novel binder binds to 2DL2 or 3 and inhibits activation of NK cells.
[Figure 8]

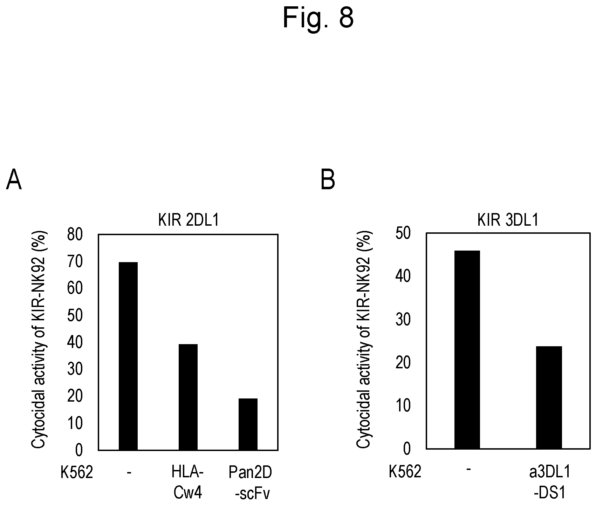

Figure 8 shows the results of evaluation as to whether or not a binder exhibiting a binding capability to KIR2DL1 or KIR3DL1 (scFv) would change the cytocidal activity on NK cell lines expressing receptors.
[Figure 9]

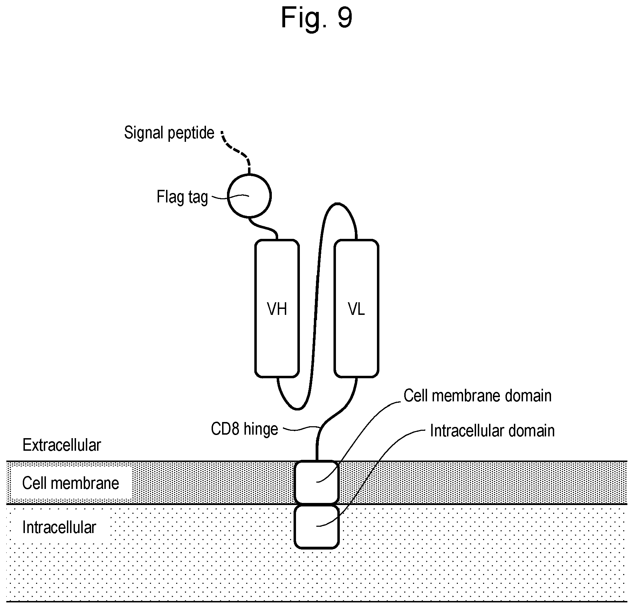

Figure 9 schematically shows a membrane scFv binder.
[Figure 10]

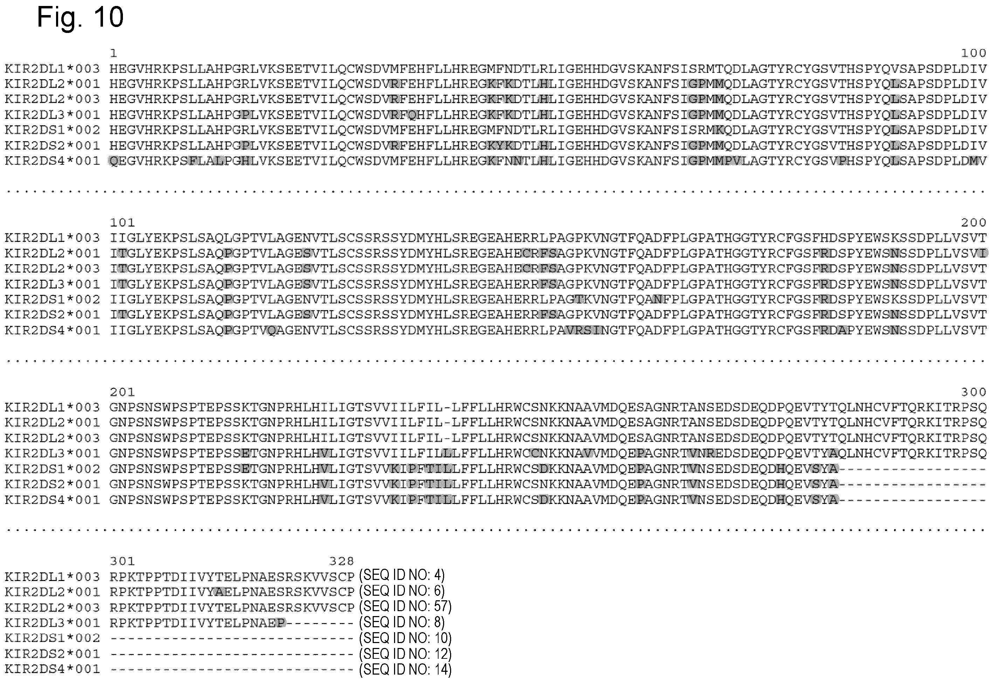

Figure 10 shows the alignment of full-length amino acid sequences of KIR2DL2*001 (SEQ ID NO: 6; extracellular region: 1 to 224), KIR2DL2*003 (SEQ ID NO: 57; extracellular region: 1 to 224), KIR2DL3*001 (SEQ ID NO: 8; extracellular region: 1 to 224), KIR2DS1*002 (SEQ ID NO: 10; extracellular region: 1 to 224), KIR2DS2*001 (SEQ ID NO: 12; extracellular region: 1 to 224), and KIR2DS4*001 (SEQ ID NO: 14; extracellular region: 1 to 224), relative to KIR2DL1*003 (SEQ ID NO: 4; extracellular region: 1 to 222).
[Figure 11]

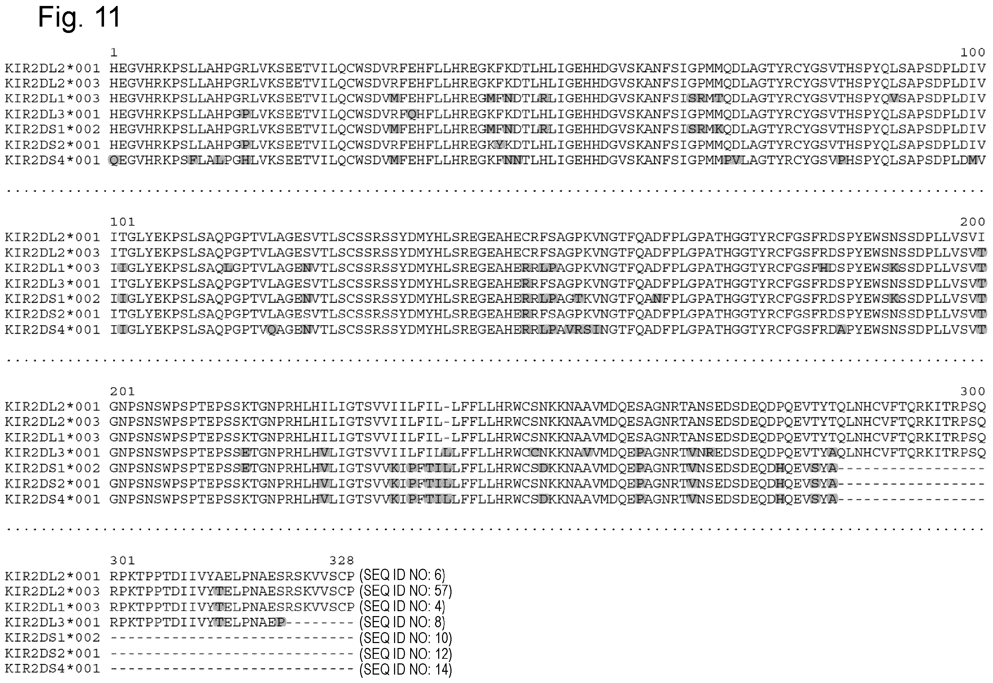

Figure 11 shows the alignment of full-length amino acid sequences of KIR2DL2*003 (SEQ ID NO: 57; extracellular region: 1 to 224), KIR2DL1*003 (SEQ ID NO: 4; extracellular region: 1 to 224), KIR2DL3*001 (SEQ ID NO: 8; extracellular region: 1 to 224), KIR2DS1*002 (SEQ ID NO: 10; extracellular region: 1 to 224), KIR2DS2*001 (SEQ ID NO: 12; extracellular region: 1 to 224), and KIR2DS4*001 (SEQ ID NO: 14; extracellular region: 1 to 224), relative to KIR2DL2*001 (SEQ ID NO: 6; extracellular region: 1 to 224).
[Figure 12]

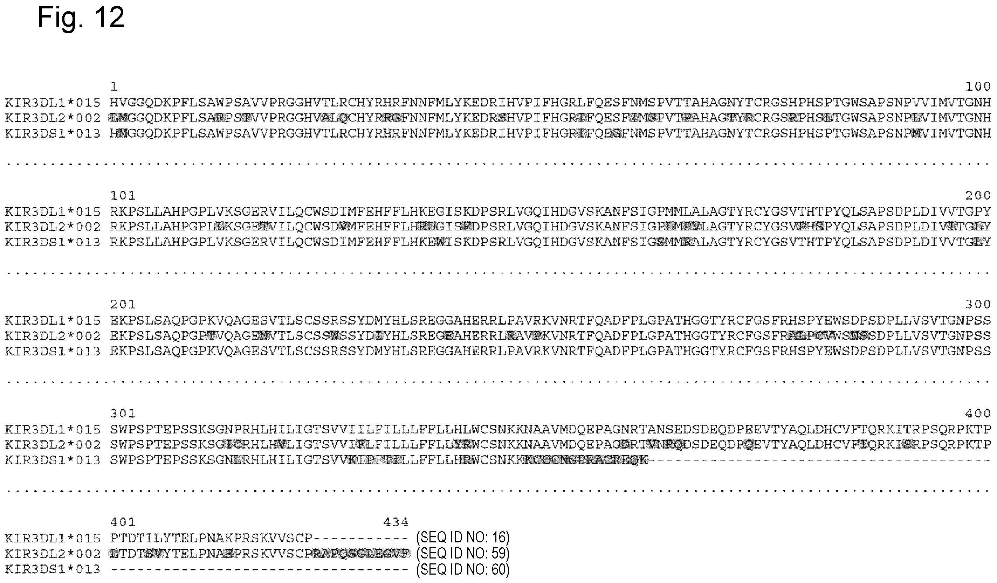

Figure 12 shows the alignment of full-length amino acid sequences of KIR3DL2*002 (SEQ ID NO: 59; extracellular region: 1 to 319) and KIR3DS1*013 (SEQ ID NO: 60; extracellular region: 1 to 319), relative to KIR3DL1*015 (SEQ ID NO: 16; extracellular region: 1 to 319).
[Figure 13]

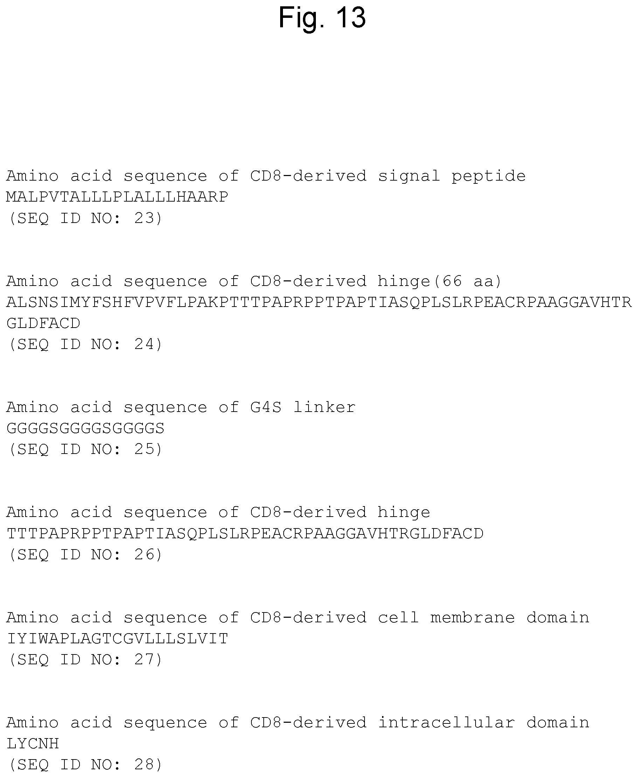

Figure 13 shows the amino acid sequences of the CD8-derived signal peptide (SEQ ID NO: 23), the CD8-derived hinge (SEQ ID NO: 24), the G4S linker (SEQ ID NO: 25), the CD8-derived hinge (SEQ ID NO: 26), the CD8-derived cell membrane domain (SEQ ID NO: 27), and the CD8-derived intracellular domain (SEQ ID NO: 28).

[Figure 14]

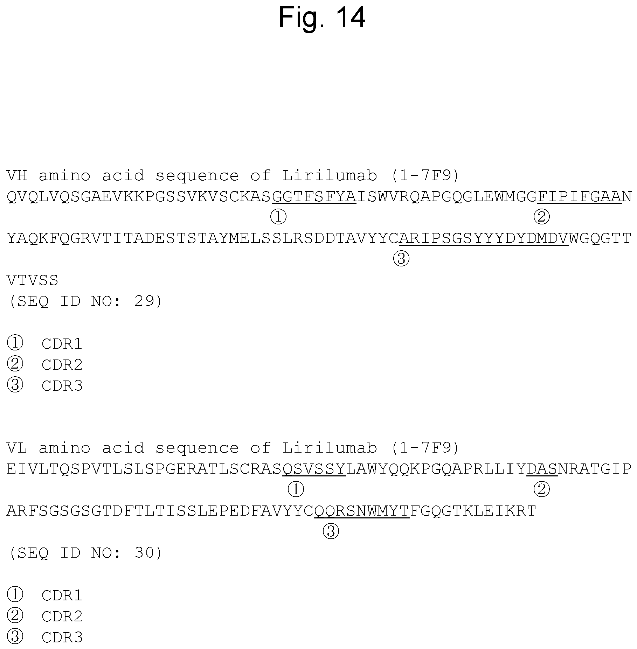

Figure 14 shows the VH amino acid sequence of Lirilumab (1-7F9) (SEQ ID NO: 29) and the VL amino acid sequence of Lirilumab (1-7F9) (SEQ ID NO: 30). Underlined sequences indicate the sequences of CDR1 to CDR3.

[Figure 15]

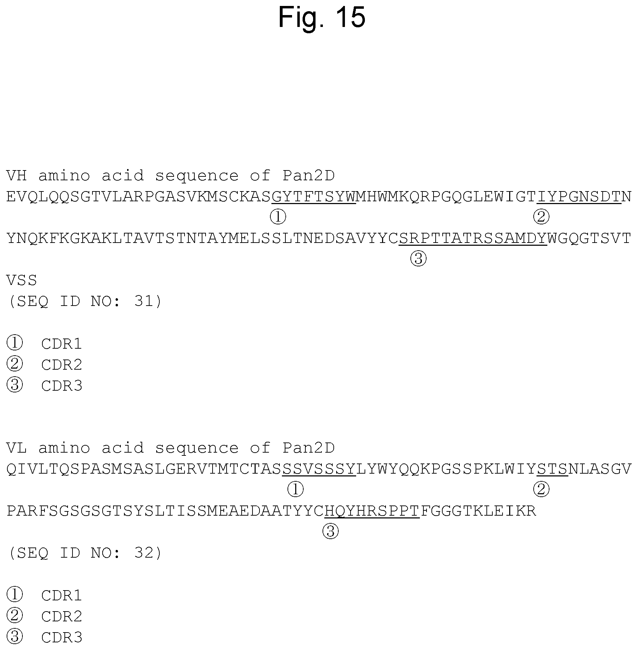

Figure 15 shows the VH amino acid sequence of Pan2D (SEQ ID NO: 31) and the VL amino acid sequence of Pan2D (SEQ ID NO: 32). Underlined sequences indicate the sequences of CDR1 to CDR3.

[Figure 16]

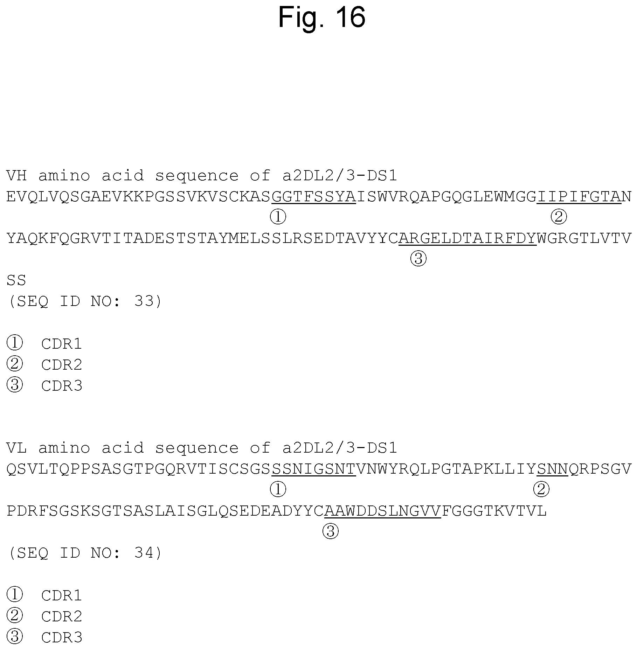

Figure 16 shows the VH amino acid sequence of a2DL2/3-DS1 (SEQ ID NO: 33) and the VL amino acid sequence of a2DL2/3-DS1 (SEQ ID NO: 34). Underlined sequences indicate the sequences of CDR1 to CDR3.

[Figure 17]

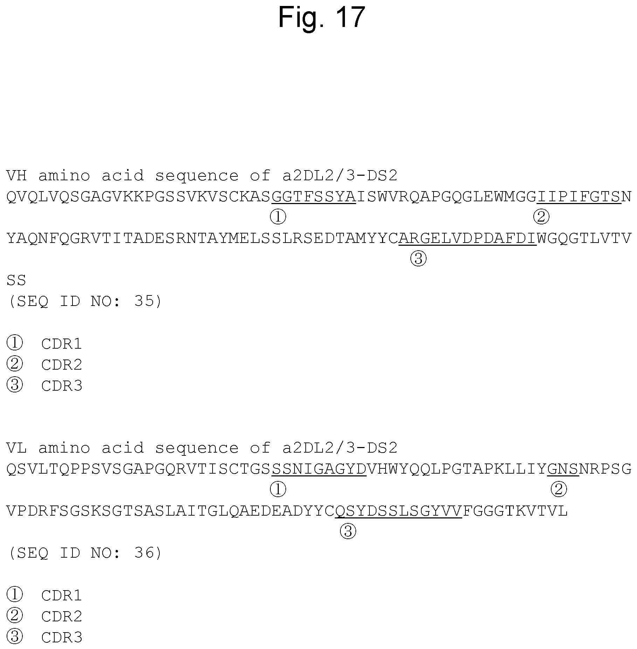

Figure 17 shows the amino acid sequence of a2DL2/3-DS2 VH (SEQ ID NO: 35) and the VL amino acid sequence of a2DL2/3-DS2 (SEQ ID NO: 36). Underlined sequences indicate the sequences of CDR1 to CDR3.

[Figure 18]

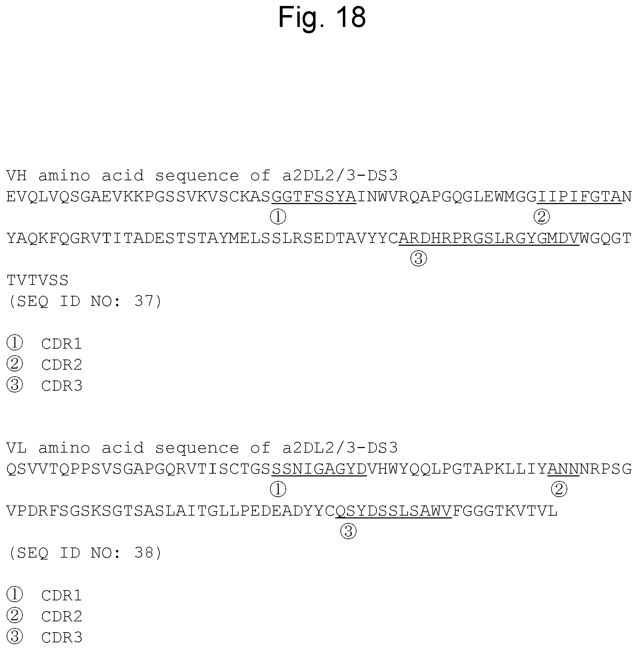

Figure 18 shows the VH amino acid sequence of a2DL2/3-DS3 (SEQ ID NO: 37) and the VL amino acid sequence of a2DL2/3-DS3 (SEQ ID NO: 38). Underlined sequences indicate the sequences of CDR1 to CDR3.

[Figure 19]

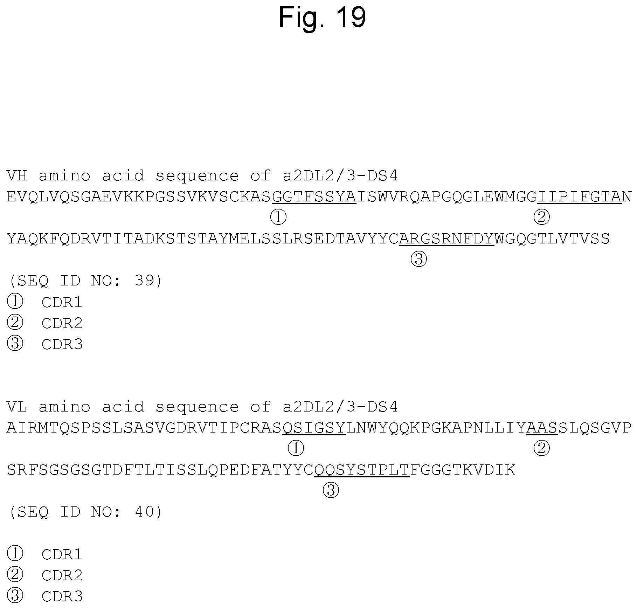

Figure 19 shows the VH amino acid sequence of a2DL2/3-DS4 (SEQ ID NO: 39) and the VL amino acid sequence of a2DL2/3-DS4 (SEQ ID NO: 40). Underlined sequences indicate the sequences of CDR1 to CDR3.

[Figure 20]

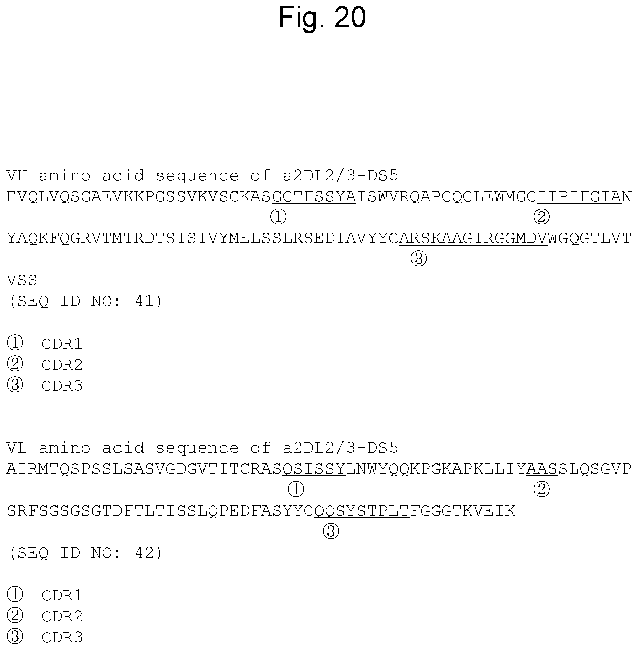

Figure 20 shows the VH amino acid sequence of a2DL2/3-DS5 (SEQ ID NO: 41) and the VL amino acid sequence of a2DL2/3-DS5 (SEQ ID NO: 42). Underlined sequences indicate the sequences of CDR1 to CDR3.

[Figure 21]

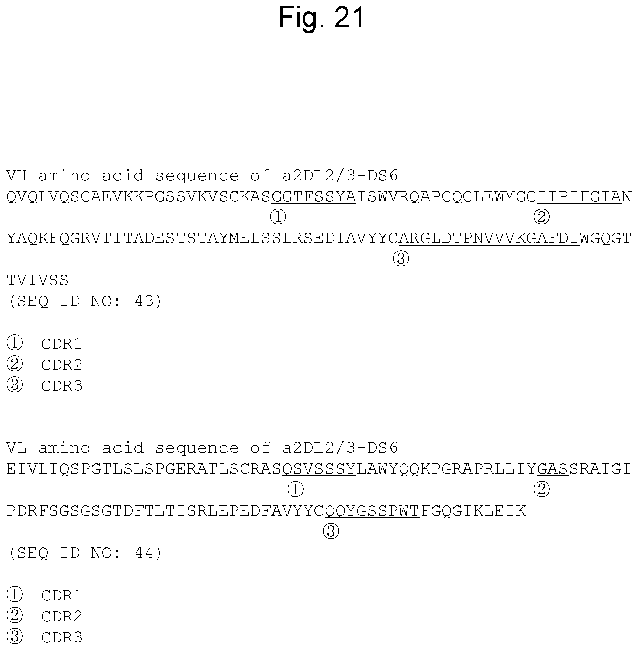

Figure 21 shows the VH amino acid sequence of a2DL2/3-DS6 (SEQ ID NO: 43) and the VL amino acid sequence of a2DL2/3-DS6 (SEQ ID NO: 44). Underlined sequences indicate the sequences of CDR1 to CDR3.

[Figure 22]

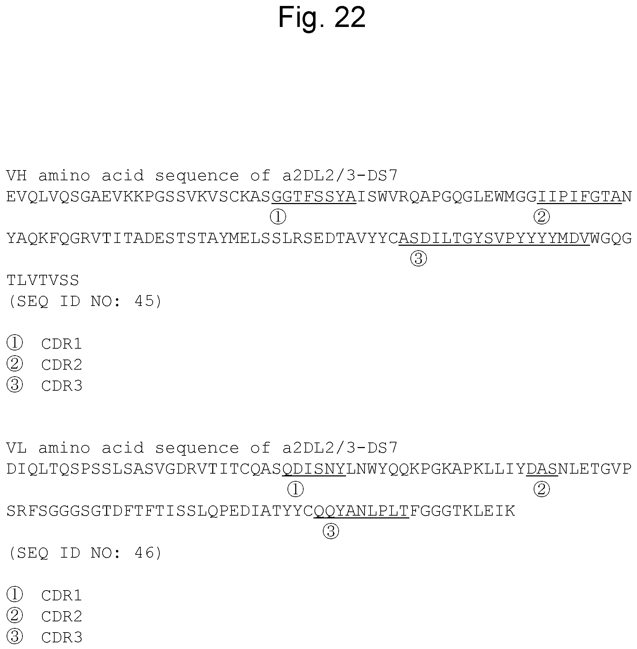

Figure 22 shows the VH amino acid sequence of a2DL2/3-DS7 (SEQ ID NO: 45) and the VL amino acid sequence of a2DL2/3-DS7 (SEQ ID NO: 46). Underlined sequences indicate the sequences of CDR1 to CDR3.

[Figure 23]

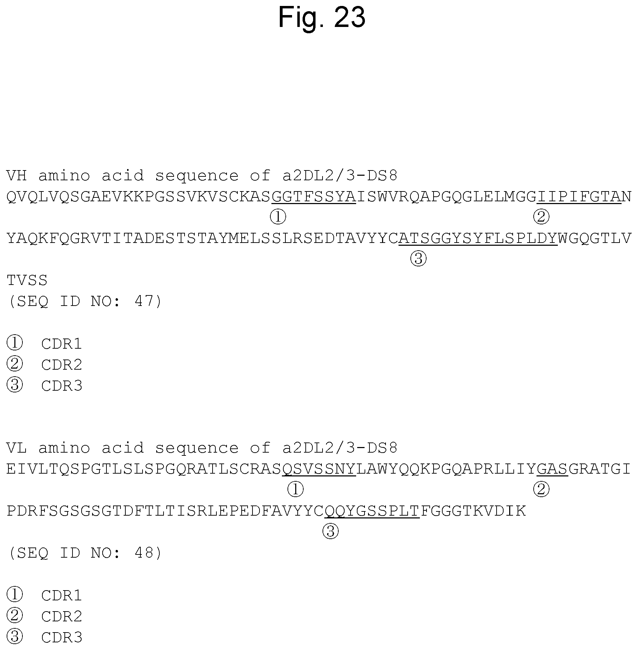

Figure 23 shows the VH amino acid sequence of a2DL2/3-DS8 (SEQ ID NO: 47) and the VL amino acid sequence of a2DL2/3-DS8 (SEQ ID NO: 48). Underlined sequences indicate the sequences of CDR1 to CDR3.

[Figure 24]

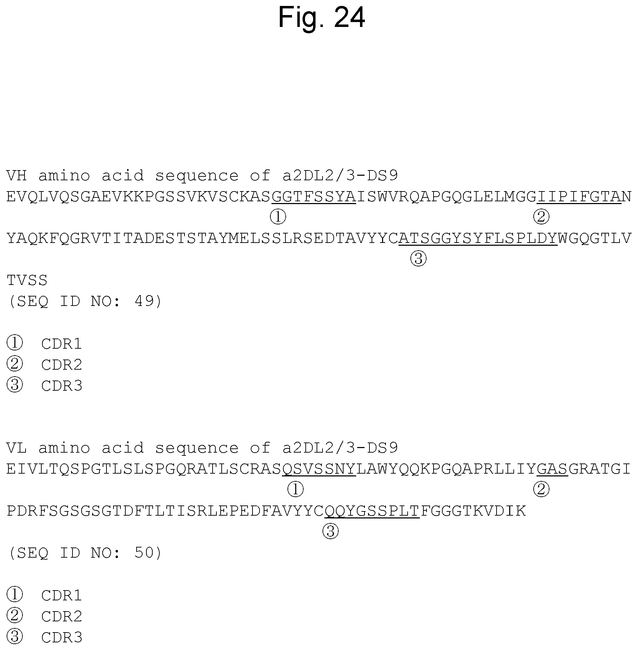

Figure 24 shows the VH amino acid sequence of a2DL2/3-DS9 (SEQ ID NO: 49) and the VL amino acid sequence of a2DL2/3-DS9 (SEQ ID NO: 50). Underlined sequences indicate the sequences of CDR1 to CDR3.

[Figure 25]

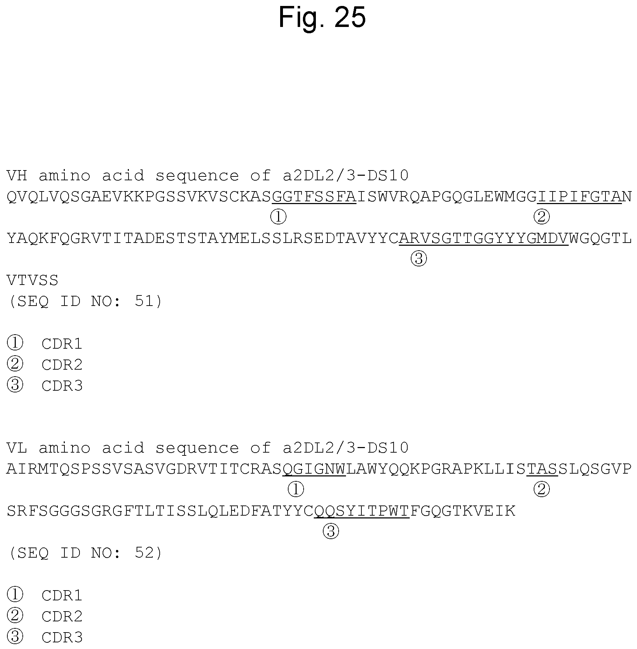

Figure 25 shows the VH amino acid sequence of a2DL2/3-DS10 (SEQ ID NO: 51) and the VL amino acid sequence of a2DL2/3-DS10 (SEQ ID NO: 52). Underlined sequences indicate the sequences of CDR1 to CDR3.

[Figure 26]

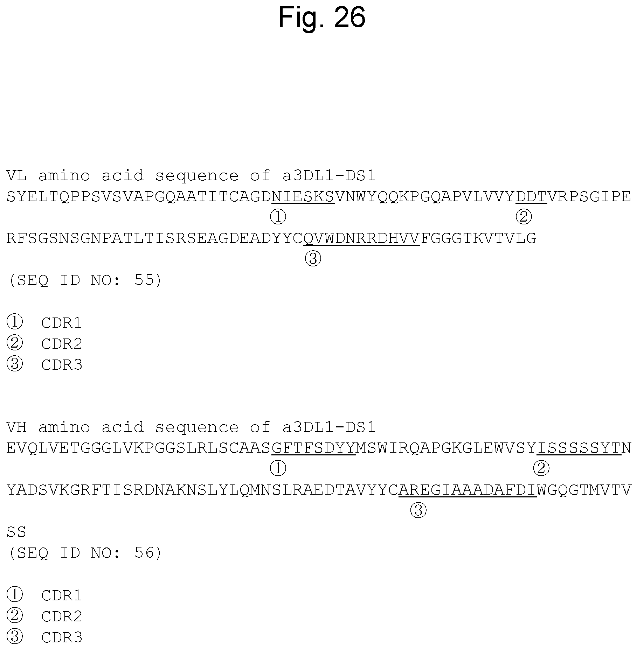

Figure 26 shows the VL amino acid sequence of a3DL1-DS1 (SEQ ID NO: 55) and the VH amino acid sequence of a3DL1-DS1 (SEQ ID NO: 56). Underlined sequences indicate the sequences of CDR1 to CDR3.

[Figure 27-1]

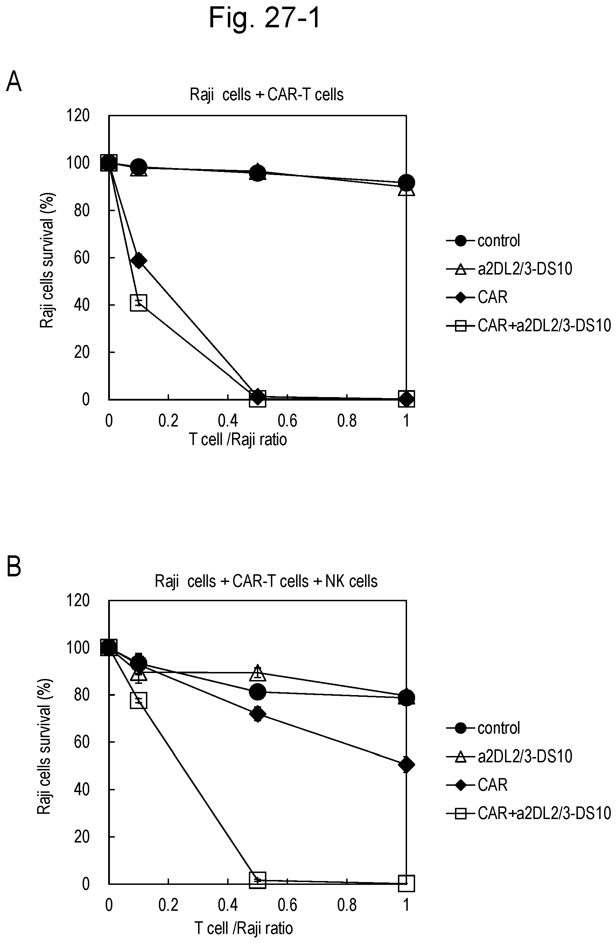

Figure 27-1 demonstrates that the CAR-T cell expresses the KIR2DL2/3-specific binder to inhibit the cytocidal activity of the NK cell against the CAR-T cell and maintain the cytocidal activity of the CAR-T cell against the target cell. Figure 27-1A shows the cytocidal activity of the CAR-T cells in wells not supplemented with the NK cell, and Figure 27-1B shows the cytocidal activity of the CAR-T cells in wells supplemented with the NK cell.

[Figure 27-2]

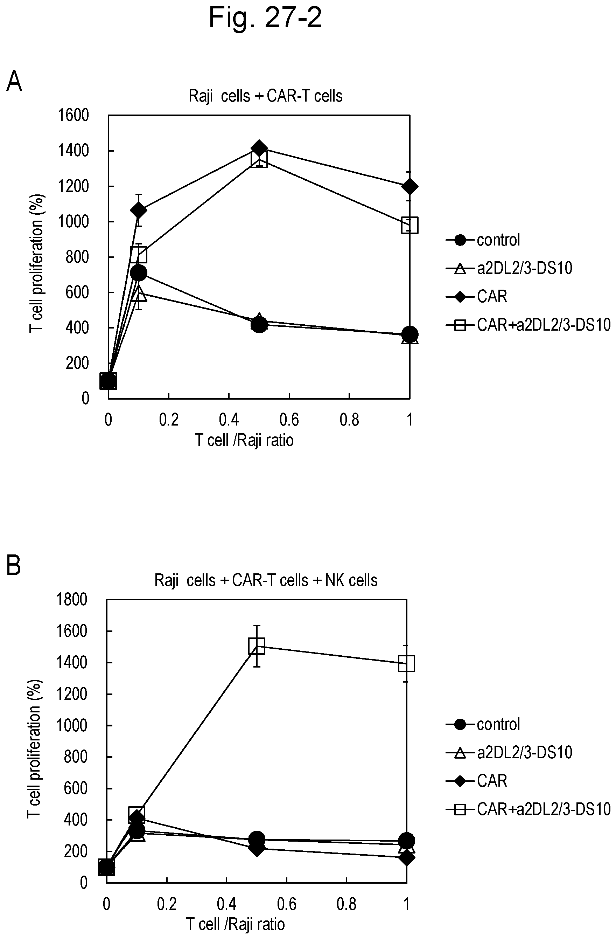

Figure 27-2 shows the number of PBMC-C-derived T cells in wells in which PBMC-A-derived NK cells are cocultured.

[Figure 28]

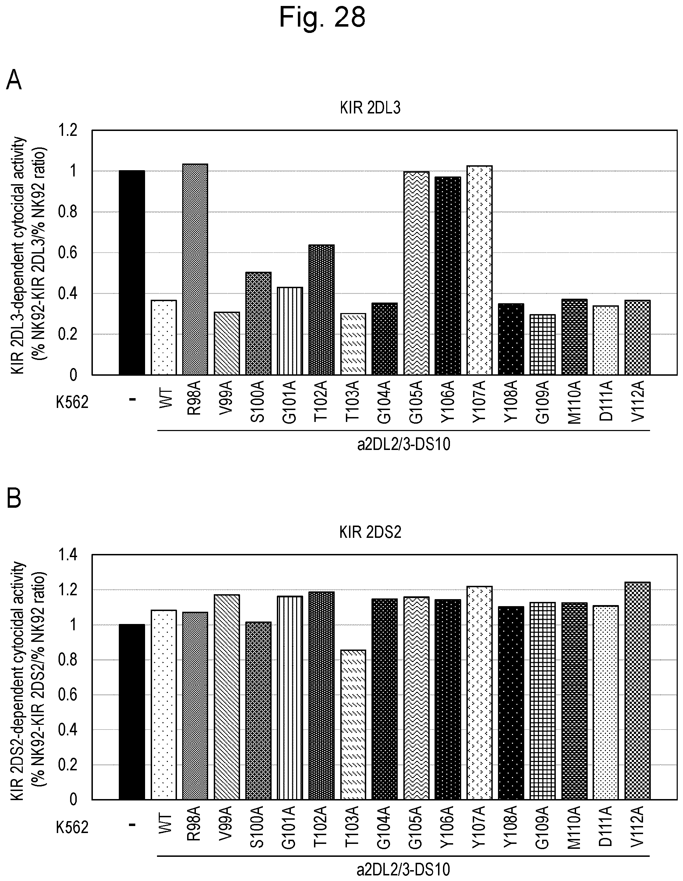

Figure 28 shows the agonist activity of variants resulting from substitution of each of amino acids of a variable region (VH) CDR3 of the KIR2DL2/3-specific binder, a2DL2/3-DS10, with alanine on inhibitory KIR.

[Figure 29]

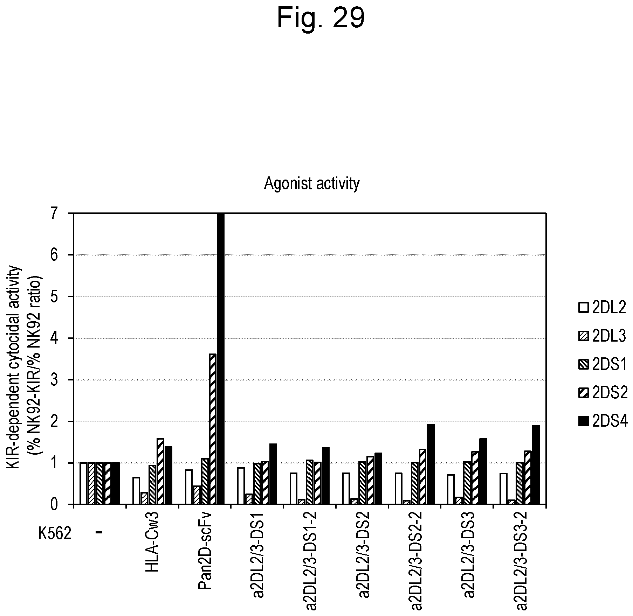

Figure 29 shows the agonist activity of a CDR-grafted body constructed by transferring CDR of the KIR2DL2/3-specific binder to a constant region of a different scFv on inhibitory KIR.

[Figure 30]

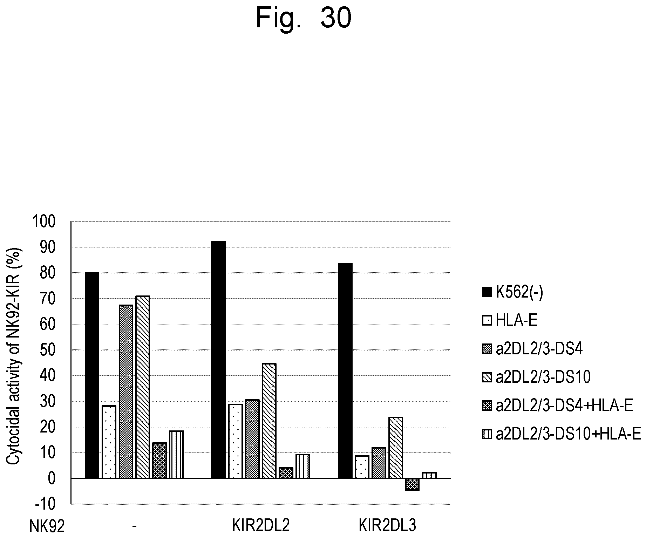

Figure 30 shows the additive inhibitory activity of cells coexpressing the KIR2DL2/3-specific binder and HLA-E on the cytocidal activity of an NK cell expressing inhibitory KIR and NKG2A.

[Figure 31]

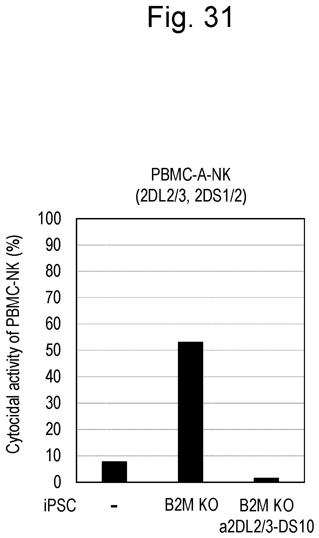

Figure 31 shows the inhibitory activity of iPS cells not expressing class I HLA but expressing the KIR 2DL2/3-specific binder on the cytocidal activity of an NK cell.

[Figure 32-1]

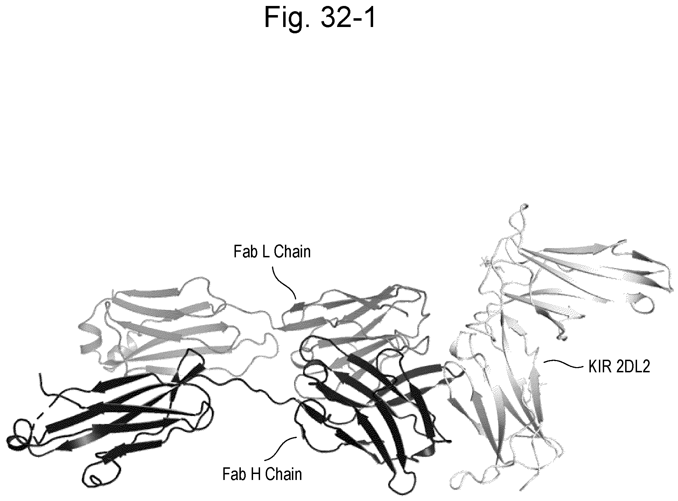

Figure 32-1 shows a model diagram of binding between an Fab fragment of the KIR2DL2/3-specific binder, a2DL2/3-DS10, identified by X-ray crystallography and KIR2DL2.

[Figure 32-2]

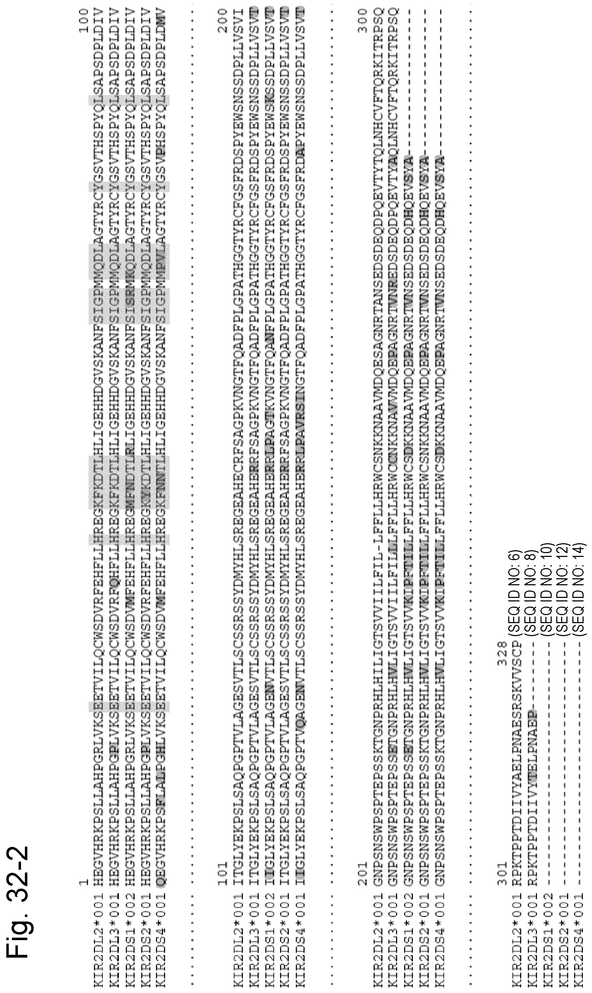

Figure 32-2 shows the results of epitope mapping of the KIR2DL2/3-specific binder, a2DL2/3-DS10, identified by X-ray crystallography on KIR2DL2.

[Figure 33]

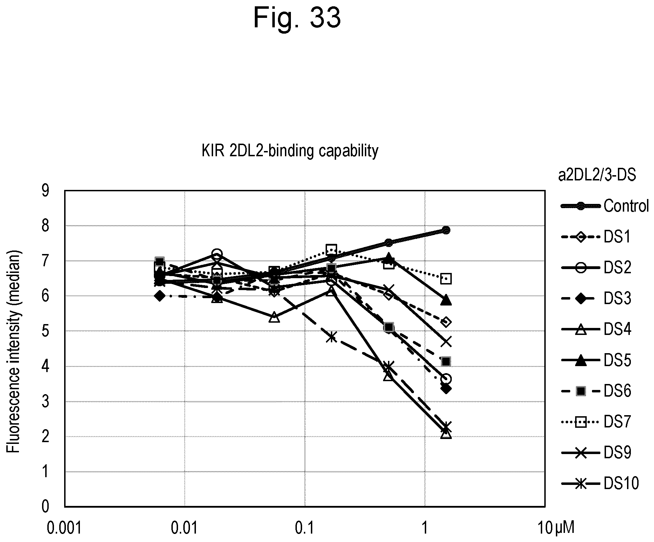

Figure 33 shows the results of examination of competition of the KIR2DL2/3-specific binder with a2DL2/3-DS10 by the antigen binding test.

[Figure 34]

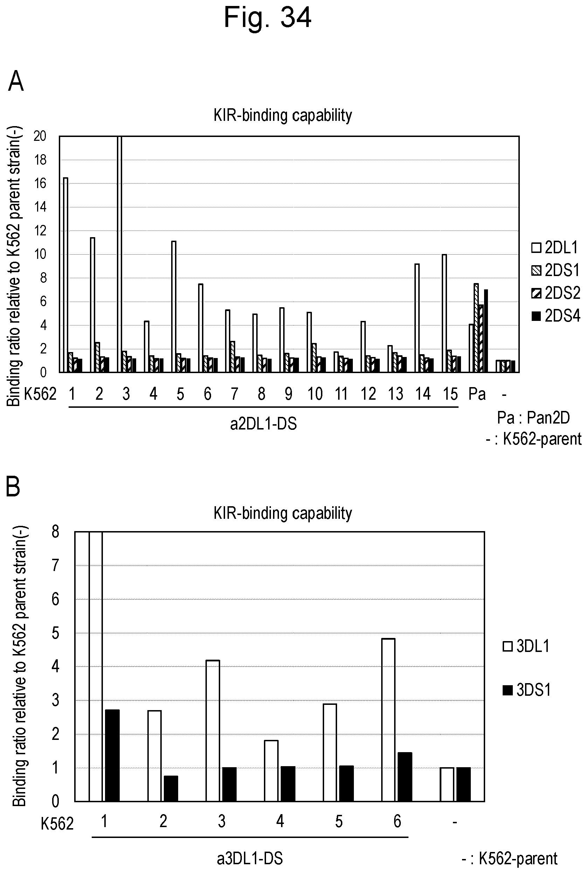

Figure 34 shows the binding capability of the novel KIR2DL1-specific binder or KIR3DL1-specific binder obtained by phage panning to inhibitory KIR and activating KIR.

[Figure 35]

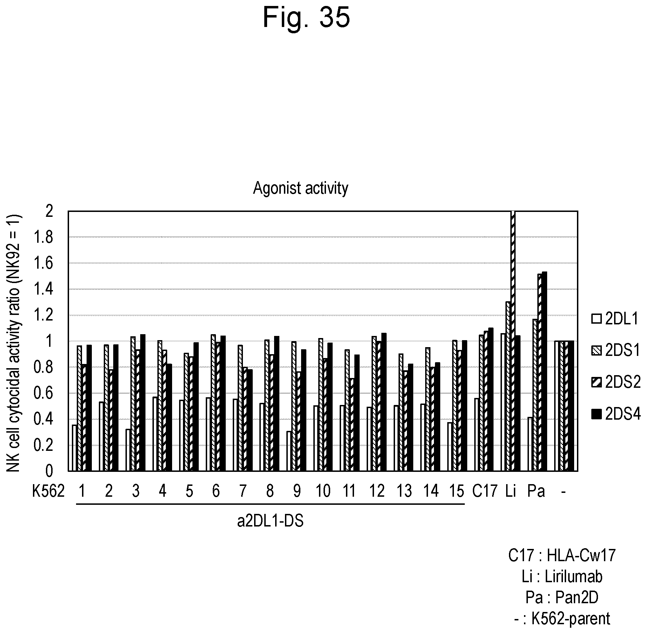

Figure 35 shows the agonist activity of the novel KIR2DL1-specific binder obtained by phage panning on inhibitory KIR.

[Figure 36]

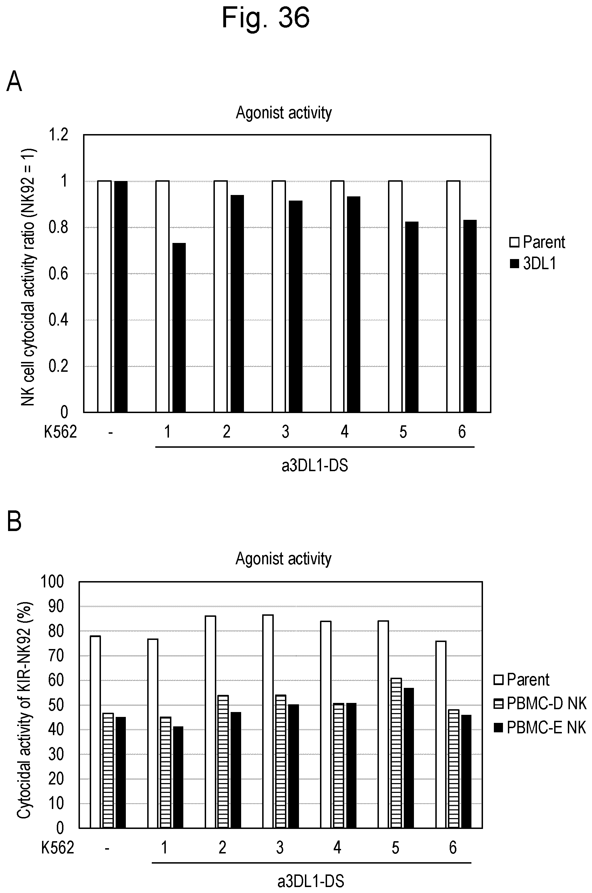

Figure 36 shows the agonist activity of the novel KIR3DL1-specific binder obtained by phage panning on inhibitory KIR.

[Figure 37-1]

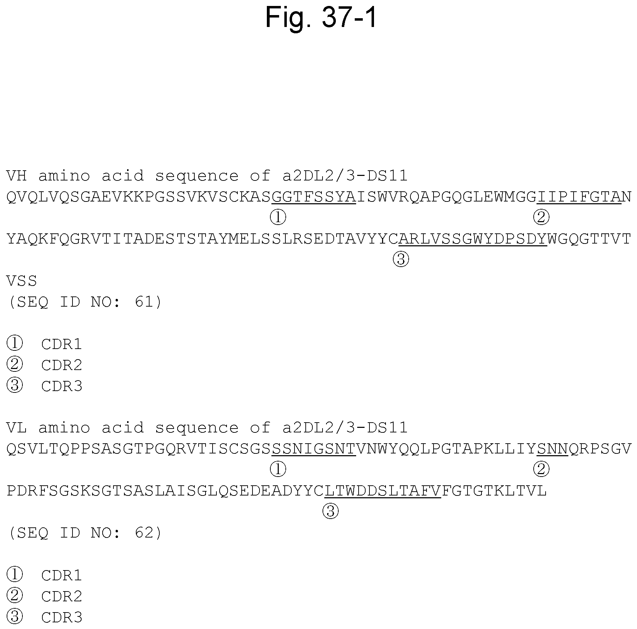

Figure 37-1 shows the VH amino acid sequence of a2DL2/3-DS11 (SEQ ID NO: 61) and the VL amino acid sequence of a2DL2/3-DS11 (SEQ ID NO: 62).

[Figure 37-2]

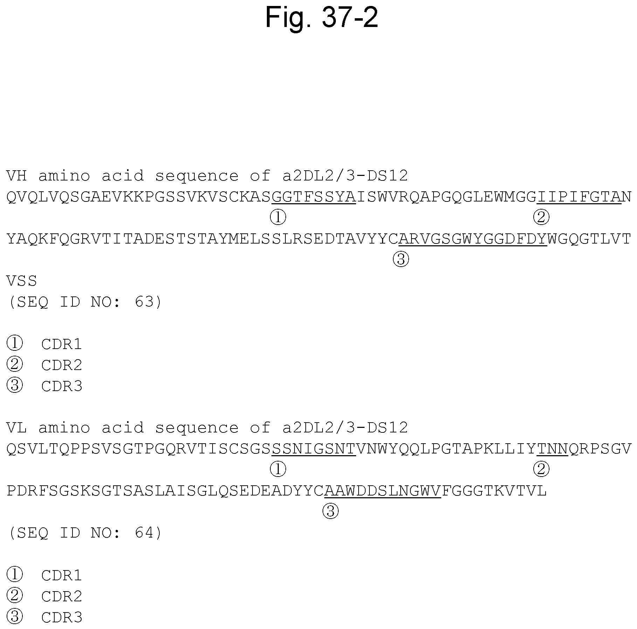

Figure 37-2 shows the VH amino acid sequence of a2DL2/3-DS12 (SEQ ID NO: 63) and the VL amino acid sequence of a2DL2/3-DS12 (SEQ ID NO: 64).

[Figure 38]

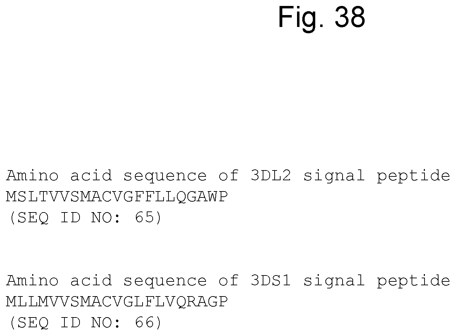

Figure 38 shows the amino acid sequence of the 3DL2 signal peptide (SEQ ID NO: 65) and the amino acid sequence of the 3DS1 signal peptide (SEQ ID NO: 66).

[Figure 39]

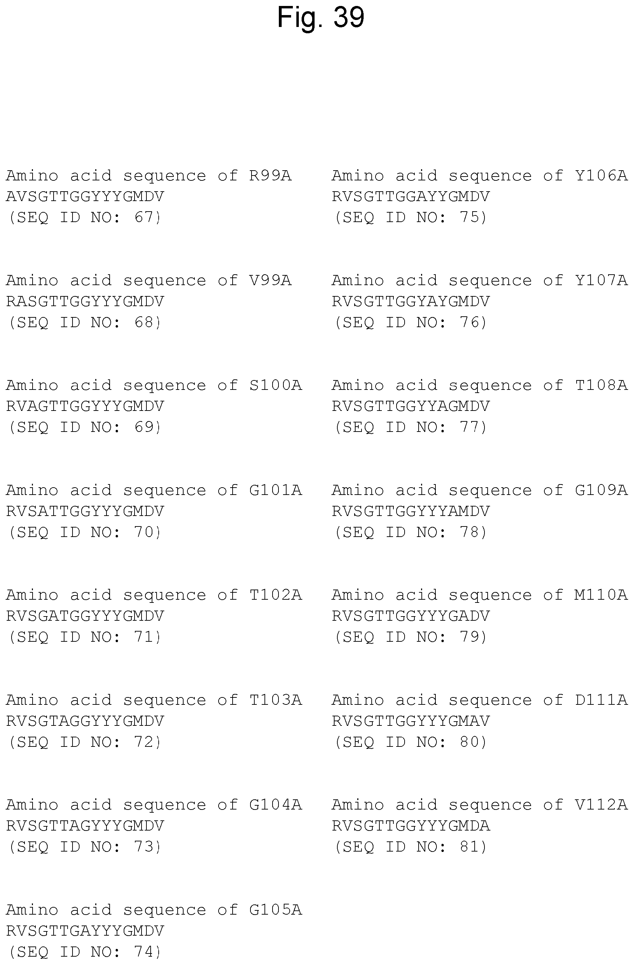

Figure 39 shows the amino acid sequence of R99A (SEQ ID NO: 67), the amino acid sequence of V99A (SEQ ID NO: 68), the amino acid sequence of S100A (SEQ ID NO: 69), the amino acid sequence of G101A (SEQ ID NO: 70), the amino acid sequence of T102A (SEQ ID NO: 71), the amino acid sequence of T103A (SEQ ID NO: 72), the amino acid sequence of G104A (SEQ ID NO: 73), the amino acid sequence of G105A (SEQ ID NO: 74), the amino acid sequence of Y106A (SEQ ID NO: 75), the amino acid sequence of Y107A (SEQ ID NO: 76), the amino acid sequence of T108A (SEQ ID NO: 77), the amino acid sequence of G109A (SEQ ID NO: 78), the amino acid sequence of M110A (SEQ ID NO: 79), the amino acid sequence of D111A (SEQ ID NO: 80), and the amino acid sequence of V112A (SEQ ID NO: 81).

[Figure 40-1]

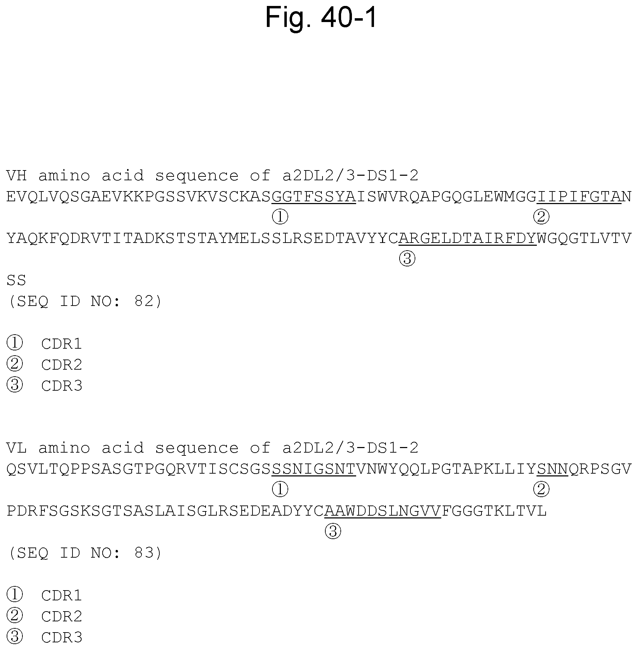

Figure 40-1 shows the VH amino acid sequence of a2DL2/3-DS1-2 (SEQ ID NO: 82) and the VL amino acid sequence of a2DL2/3-DS1-2 (SEQ ID NO: 83).

[Figure 40-2]

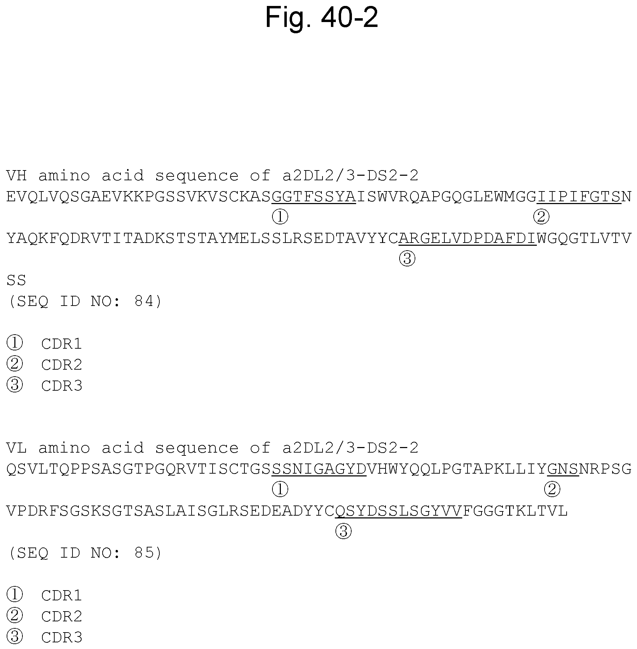

Figure 40-2 shows the VH amino acid sequence of a2DL2/3-DS2-2 (SEQ ID NO: 84) and the VL amino acid sequence of a2DL2/3-DS2-2 (SEQ ID NO: 85).

[Figure 40-3]

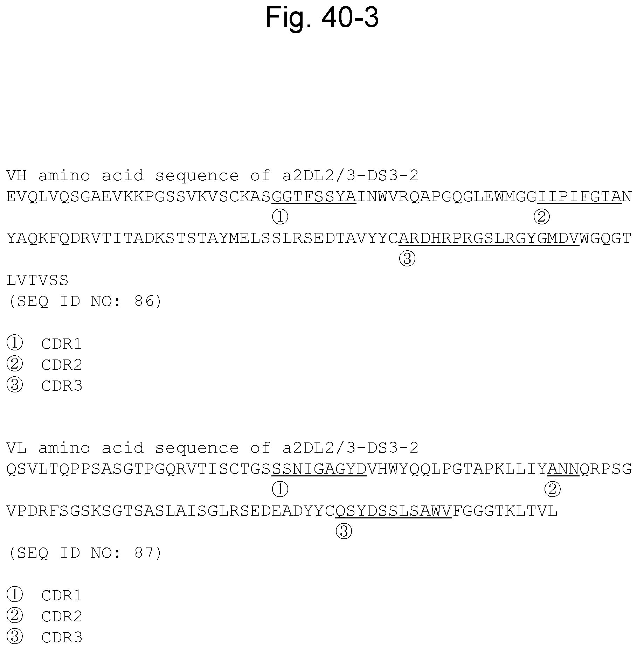

Figure 40-3 shows the VH amino acid sequence of a2DL2/3-DS3-2 (SEQ ID NO: 86) and the VL amino acid sequence of a2DL2/3-DS3-2 (SEQ ID NO: 87).

[Figure 41]

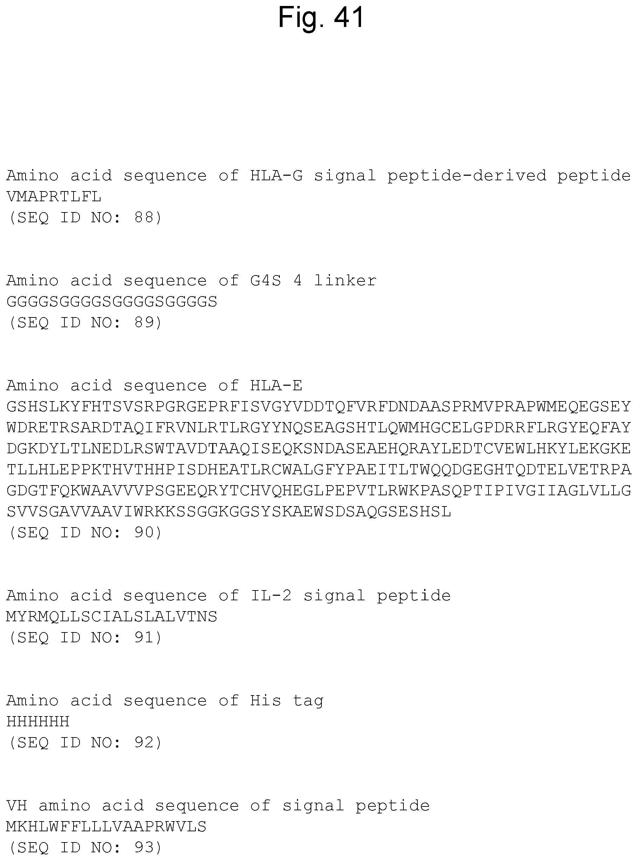

Figure 41 shows the amino acid sequence of the HLA-G signal peptide-derived peptide (SEQ ID NO: 88), the amino acid sequence of the G4S linker (SEQ ID NO: 89), the amino acid sequence of HLA-E (SEQ ID NO: 90), the amino acid sequence of the IL-2 signal peptide (SEQ ID NO: 91), the amino acid sequence of the His tag (SEQ ID NO: 92), and the amino acid sequence of the VH signal peptide (SEQ ID NO: 93).

[Figure 42]

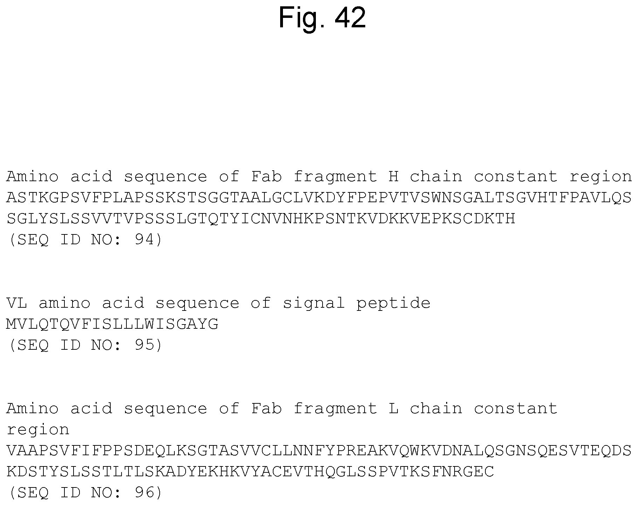

Figure 42 shows the amino acid sequence of the Fab fragment H-chain constant region (SEQ ID NO: 94), the amino acid sequence of the VI signal peptide (SEQ ID NO: 95), and the amino acid sequence of the Fab fragment L-chain constant region (SEQ ID NO: 96).

[Figure 43]

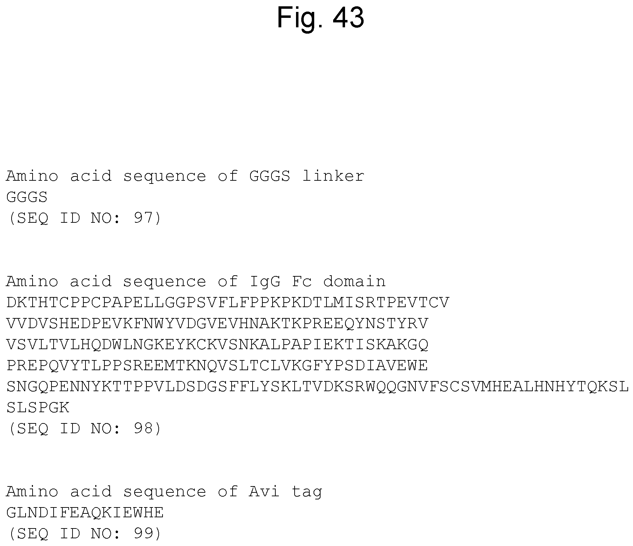

Figure 43 shows the amino acid sequence of the GGGS linker (SEQ ID NO: 97), the amino acid sequence of the IgG Fc domain (SEQ ID NO: 98), the amino acid sequence of the GAA linker (GAA), and the amino acid sequence of the Avi tag (SEQ ID NO: 99).

[Figure 44]

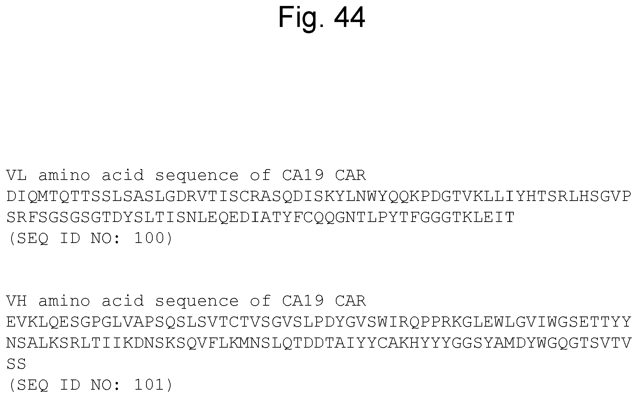

Figure 44 shows the VL amino acid sequence of CA19CAR (SEQ ID NO: 100) and the VH amino acid sequence of CA19CAR (SEQ ID NO: 101).

[Figure 45]

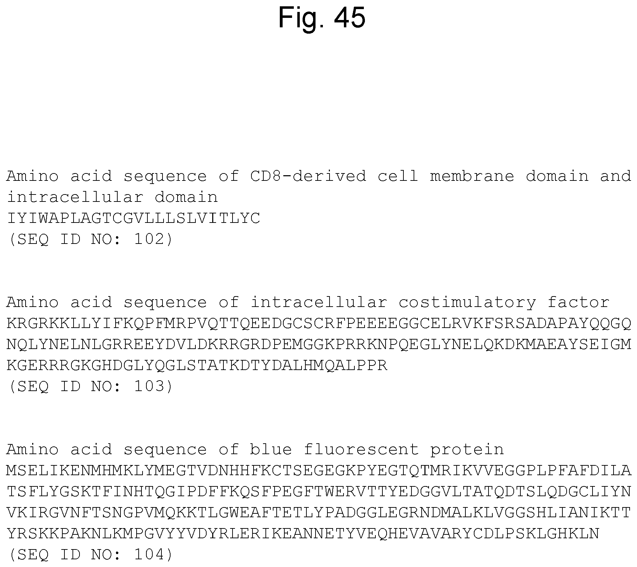

Figure 45 shows the amino acid sequence of the CD8-derived cell membrane domain and intracellular domain (SEQ ID NO: 102), the amino acid sequence of the intracellular costimulatory molecule (SEQ ID NO: 103), and the amino acid sequence of the blue fluorescent protein (SEQ ID NO: 104).

[Figure 46]

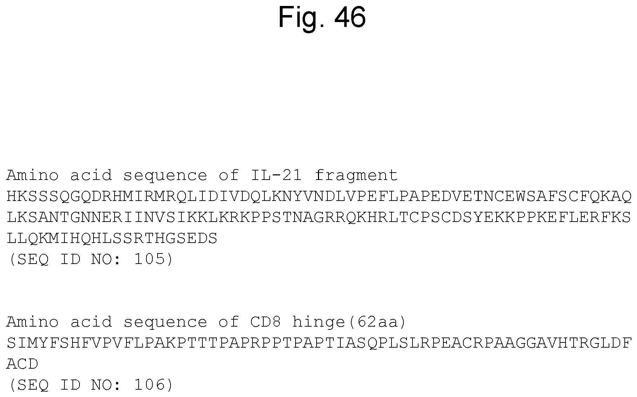

Figure 46 shows the amino acid sequence of the IL-21 fragment (SEQ ID NO: 105) and the amino acid sequence of the CD8 hinge (62 amino acids) (SEQ ID NO: 106).

[Figure 47-1]

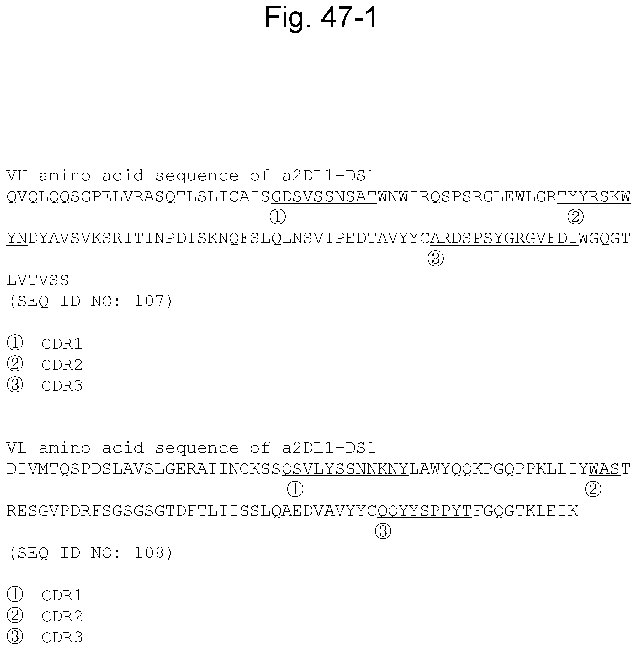

Figure 47-1 shows the VH amino acid sequence of a2DL1-DS1 (SEQ ID NO: 107) and the VL amino acid sequence of a2DL1-DS1 (SEQ ID NO: 108).

[Figure 47-2]

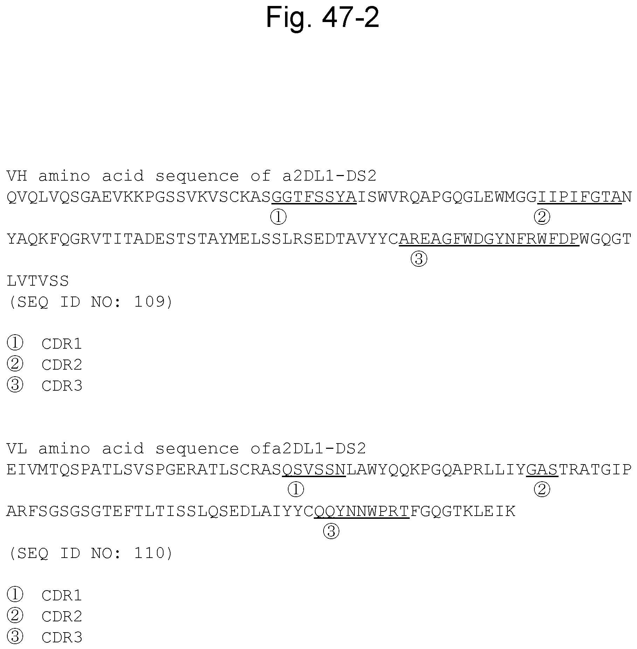

Figure 47-2 shows the VH amino acid sequence of a2DL1-DS2 (SEQ ID NO: 109) and the VL amino acid sequence of a2DL1-DS2 (SEQ ID NO: 110).

[Figure 47-3]

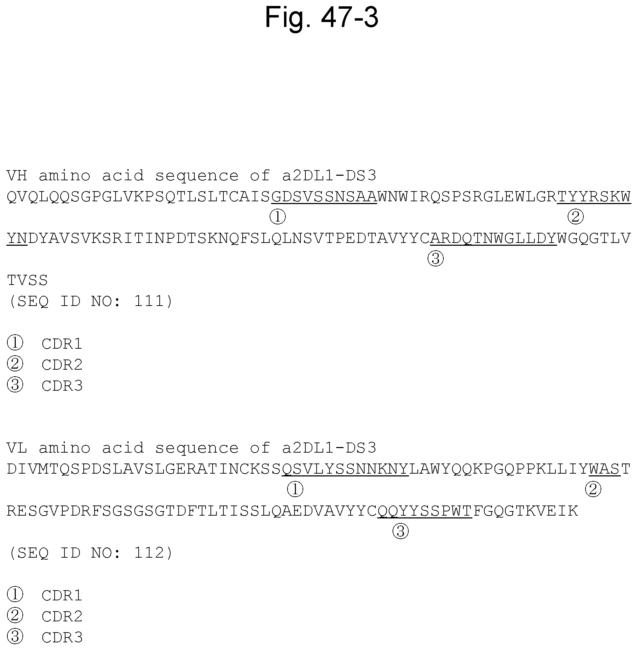

Figure 47-3 shows the VH amino acid sequence of a2DL1-DS3 (SEQ ID NO: 111) and the VL amino acid sequence of a2DL1-DS1 (SEQ ID NO: 112).

[Figure 47-4]

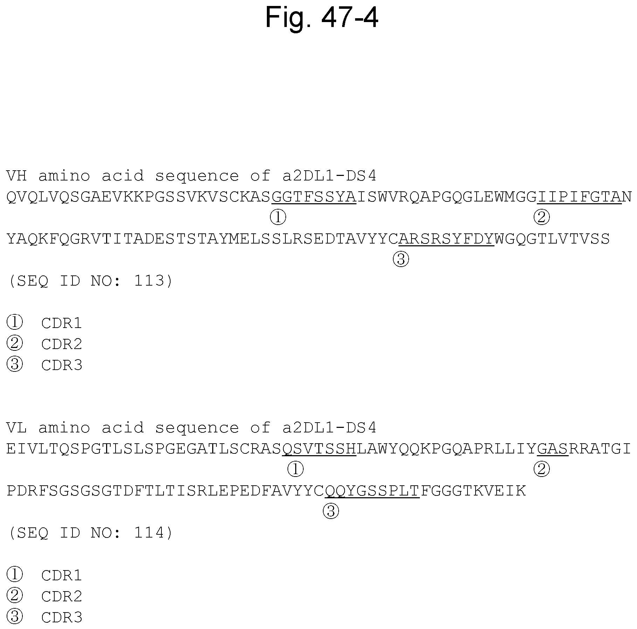

Figure 47-4 shows the VH amino acid sequence of a2DL1-DS4 (SEQ ID NO: 113) and the VL amino acid sequence of a2DL1-DS4 (SEQ ID NO: 114).

[Figure 47-5]

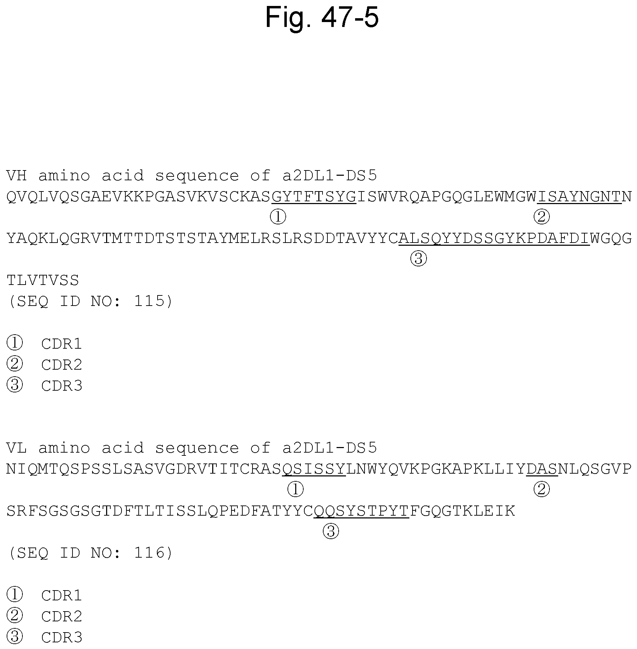

Figure 47-5 shows the VH amino acid sequence of a2DL1-DS5 (SEQ ID NO: 115) and the VL amino acid sequence of a2DL1-DS5 (SEQ ID NO: 116).

[Figure 47-6]

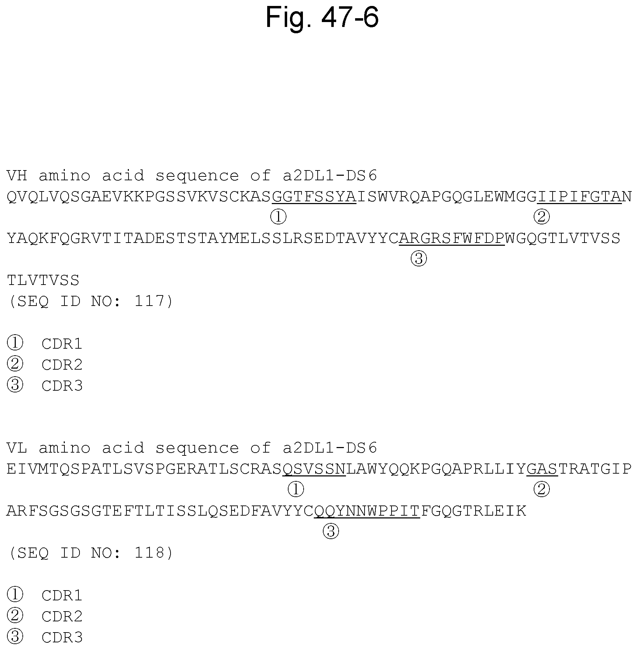

Figure 47-6 shows the VH amino acid sequence of a2DL1-DS6 (SEQ ID NO: 117) and the VL amino acid sequence of a2DL1-DS6 (SEQ ID NO: 118).

[Figure 47-7]

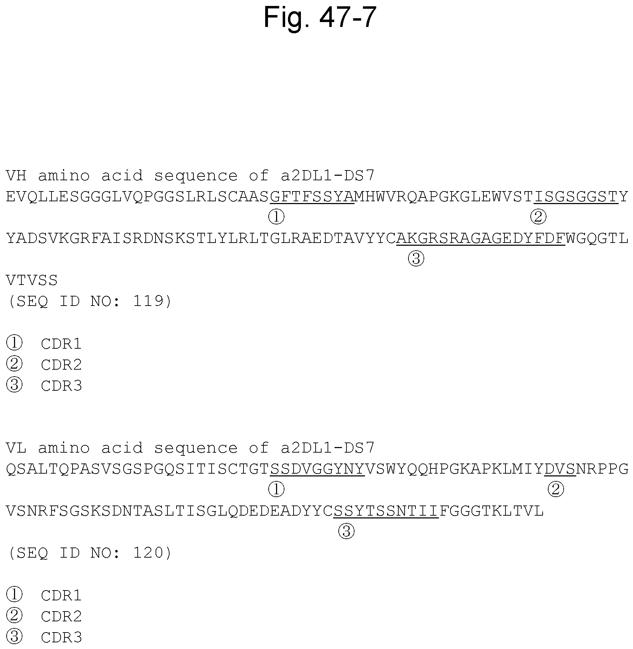

Figure 47-7 shows the VH amino acid sequence of a2DL1-DS7 (SEQ ID NO: 119) and the VL amino acid sequence of a2DL1-DS7 (SEQ ID NO: 120).

[Figure 47-8]

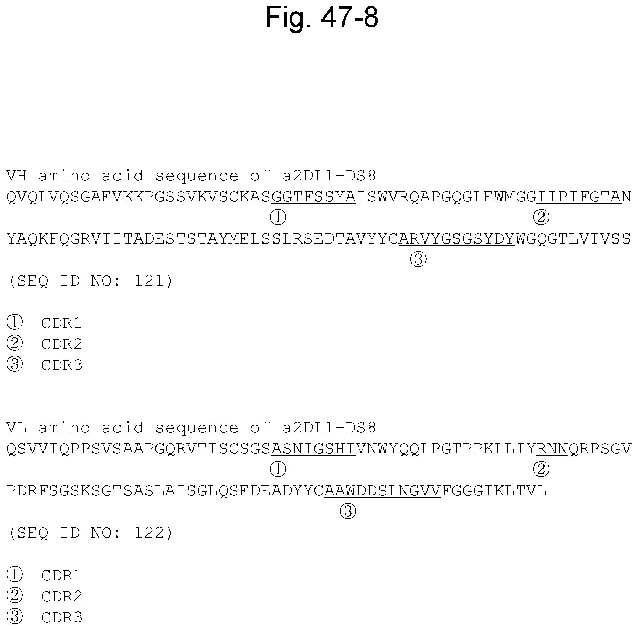

Figure 47-8 shows the VH amino acid sequence of a2DL1-DS8 (SEQ ID NO: 121) and the VL amino acid sequence of a2DL1-DS8 (SEQ ID NO: 122).

[Figure 47-9]

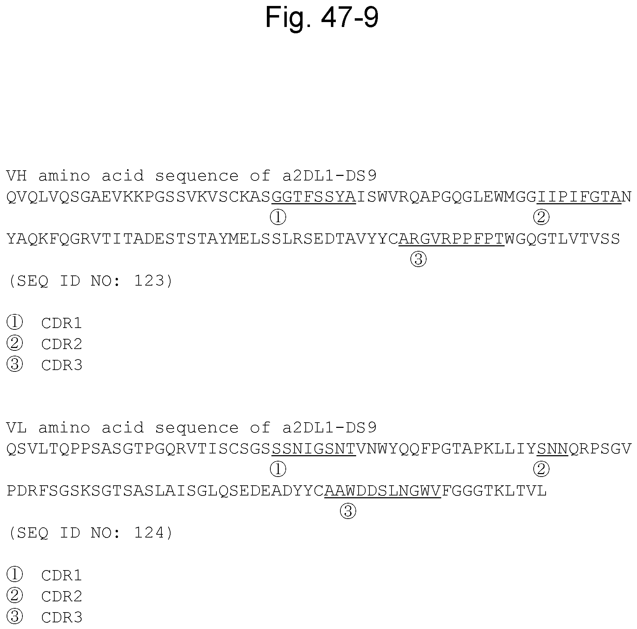

Figure 47-9 shows the VH amino acid sequence of a2DL1-DS9 (SEQ ID NO: 123) and the VL amino acid sequence of a2DL1-DS9 (SEQ ID NO: 124).

[Figure 47-10]

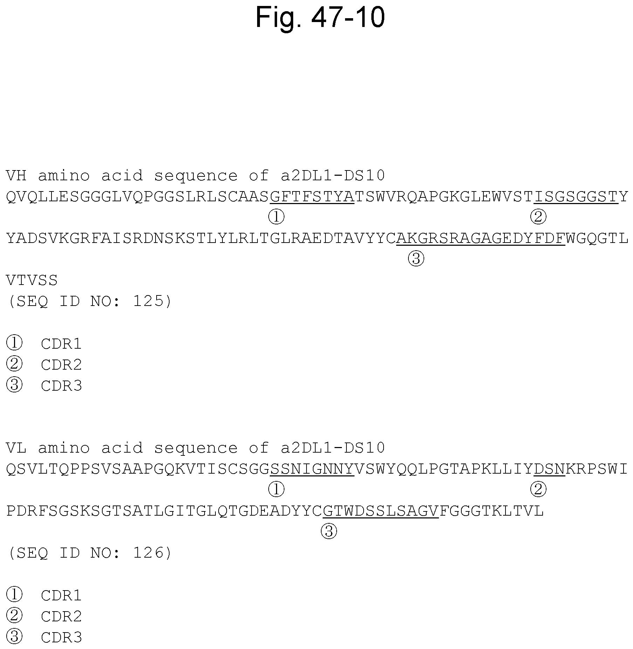

Figure 47-10 shows the VH amino acid sequence of a2DL1-DS10 (SEQ ID NO: 125) and the VL amino acid sequence of a2DL1-DS10 (SEQ ID NO: 126).

[Figure 47-11]

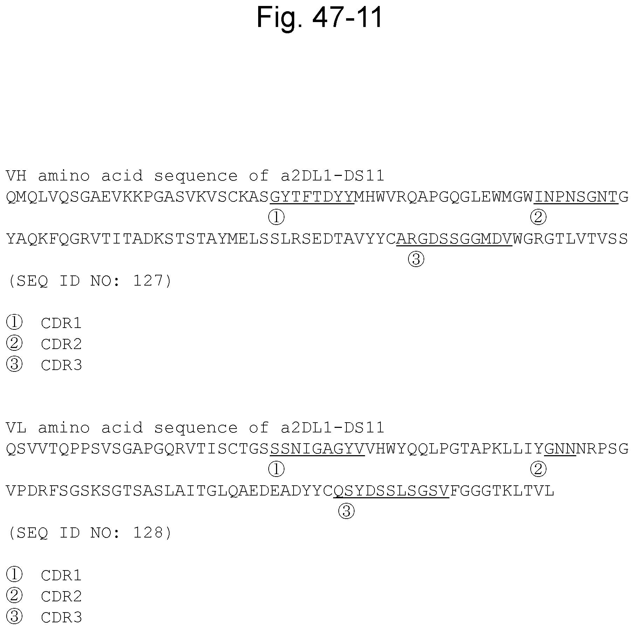

Figure 47-11 shows the VH amino acid sequence of a2DL1-DS11 (SEQ ID NO: 127) and the VL amino acid sequence of a2DL1-DS11 (SEQ ID NO: 128).

[Figure 47-12]

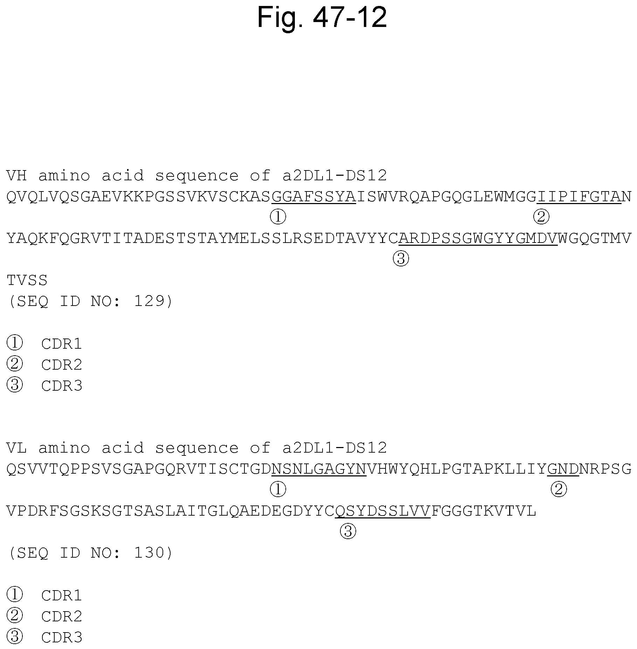

Figure 47-12 shows the VH amino acid sequence of a2DL1-DS12 (SEQ ID NO: 129) and the VL amino acid sequence of a2DL1-DS12 (SEQ ID NO: 130).

[Figure 47-13]

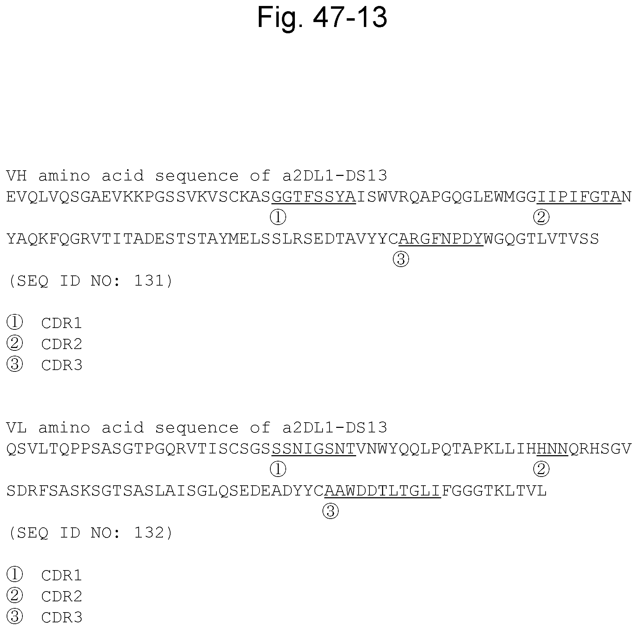

Figure 47-13 shows the VH amino acid sequence of a2DL1-DS13 (SEQ ID NO: 131) and the VL amino acid sequence of a2DL1-DS13 (SEQ ID NO: 132).

[Figure 47-14]

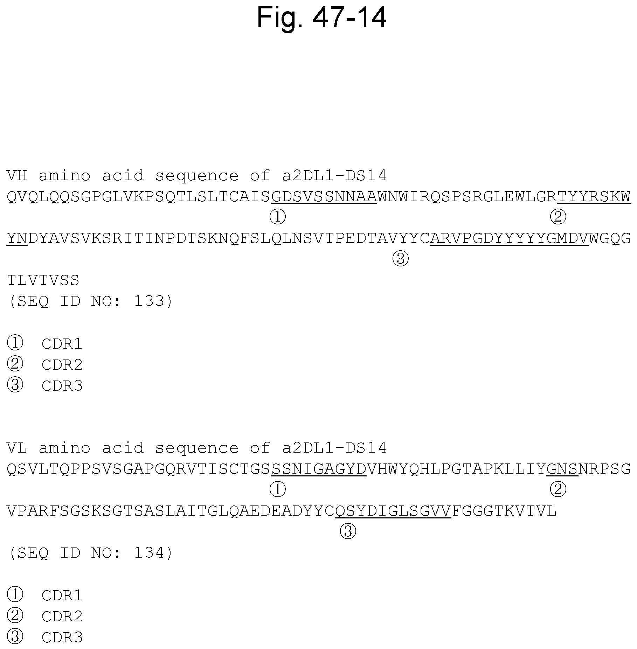

Figure 47-14 shows the VH amino acid sequence of a2DL1-DS14 (SEQ ID NO: 133) and the VL amino acid sequence of a2DL1-DS14 (SEQ ID NO: 134).

[Figure 47-15]

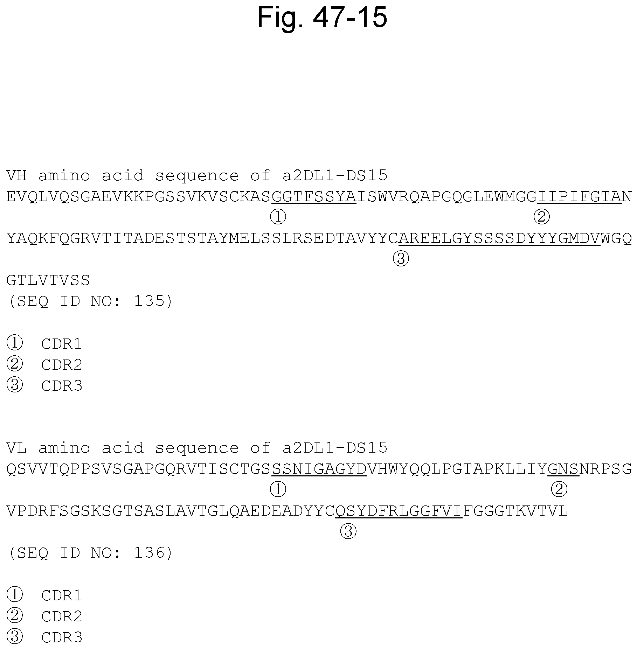

Figure 47-15 shows the VH amino acid sequence of a2DL1-DS15 (SEQ ID NO: 135) and the VL amino acid sequence of a2DL1-DS15 (SEQ ID NO: 136).

[Figure 48-1]

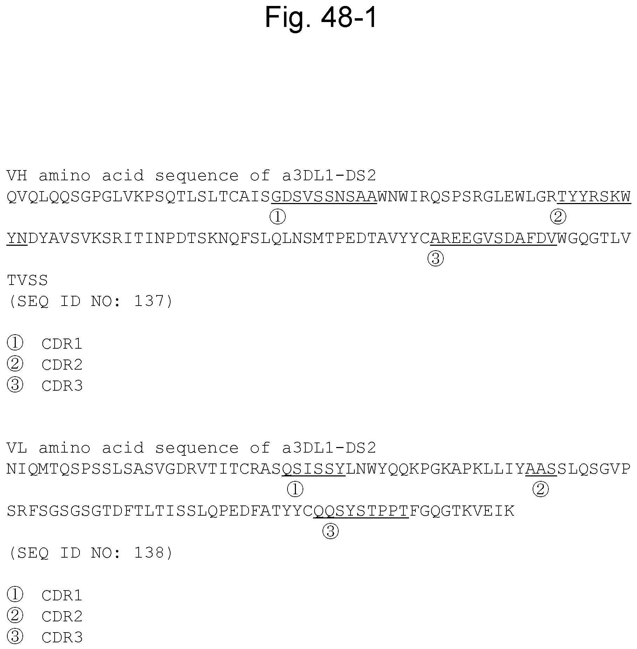

Figure 48-1 shows the VH amino acid sequence of a3DL1-DS2 (SEQ ID NO: 137) and the VL amino acid sequence of a3DL1-DS2 (SEQ ID NO: 138).

[Figure 48-2]

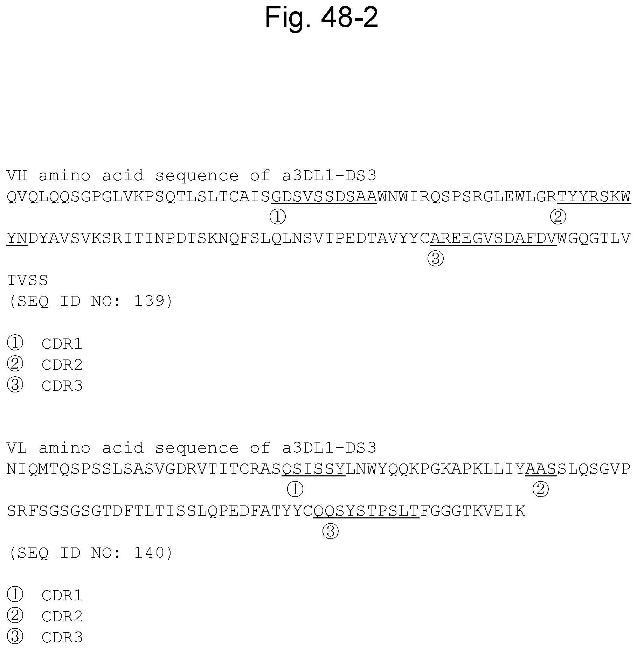

Figure 48-2 shows the VH amino acid sequence of a3DL1-DS3 (SEQ ID NO: 139) and the VL amino acid sequence of a3DL1-DS3 (SEQ ID NO: 140).

[Figure 48-3]

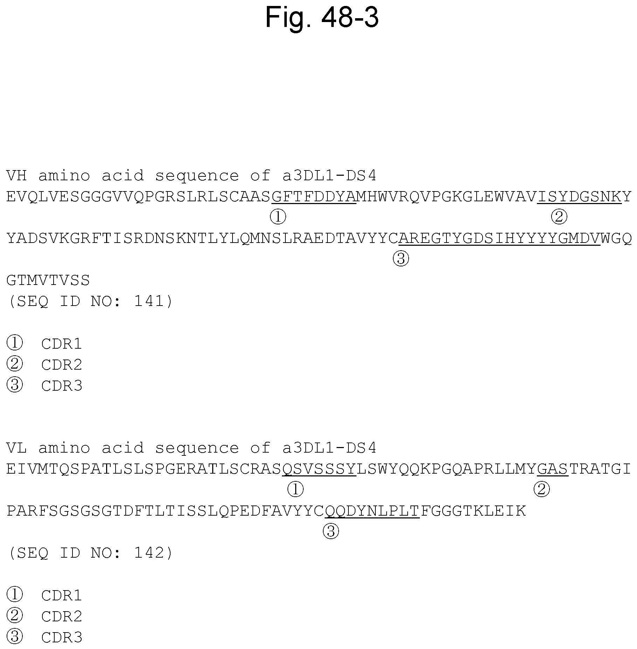

Figure 48-3 shows the VH amino acid sequence of a3DL1-DS4 (SEQ ID NO: 141) and the VL amino acid sequence of a3DL1-DS4 (SEQ ID NO: 142).

[Figure 48-4]

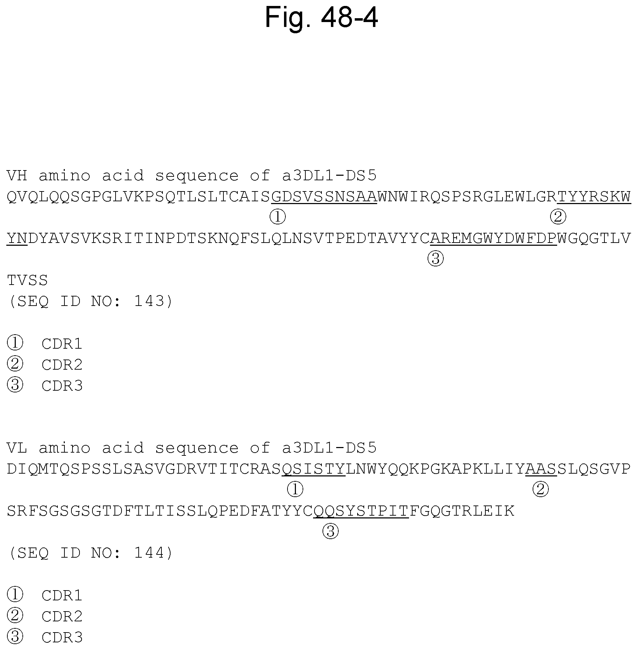

Figure 48-4 shows the VH amino acid sequence of a3DL1-DS5 (SEQ ID NO: 143) and the VL amino acid sequence of a3DL1-DS5 (SEQ ID NO: 144).

[Figure 48-5]

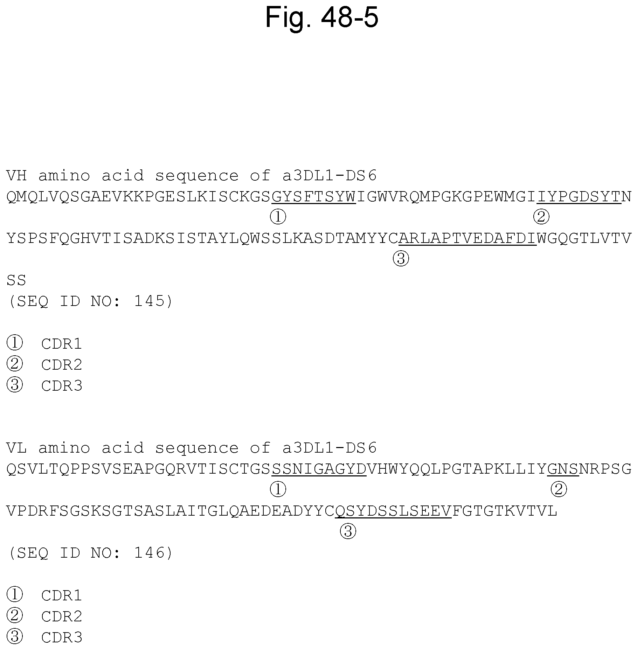

Figure 48-5 shows the VH amino acid sequence of a3DL1-DS6 (SEQ ID NO: 145) and the VL amino acid sequence of a3DL1-DS6 (SEQ ID NO: 145).

Description of Embodiments

Hereafter, the present invention is descried in detail.

1. Substance having activity of binding to human inhibitory KIR (killer-cell immunoglobulin-like receptor) and inhibiting cytocidal activity of NK cell

The present invention provides a substance having activity of binding to inhibitory KIR and inhibiting the cytocidal activity of an NK cell. Such substance may be referred to as a substance having an agonist activity on inhibitory KIR. Specifically, activity of binding to inhibitory KIR and inhibiting the cytocidal activity of an NK cell may be referred to as "agonist activity." In the present invention, a substance binding to KIR is referred to as a "binder," and a substance having activity of binding to KIR and activating a signal from the KIR to an NK cell is referred to as an "agonist binder." Specifically, an agonist binder for inhibitory KIR has activity of inhibiting activity of an NK cell, and an agonist binder for activating KIR has activity of activating an NK cell.

KIR is expressed on the NK cell surface and it is involved in a function of an NK cell to distinguish self from others, which is referred to as the function of "missing-self recognition." KIR is classified as inhibitory KIR or activating KIR, and inhibitory KIR and activating KIR each has a plurality of subtypes. A signal from inhibitory KIR inhibits the cytocidal activity of an NK cell. When an NK cell in the steady state recognizes a surface antigen on the target cell, specifically, such NK cell has a mechanism of activating a signal to an inhibitory receptor when such signal is not activated, and acquiring activity of damaging or killing the recognized cell (hereafter, such activity is referred to as the "cytocidal activity of an NK cell"). When the target cell expresses HLA of the same type as an NK cell, specifically, HLA of a cell binds to inhibitory KIR on an NK cell, and an inhibitory signal is transmitted into an NK cell to inhibit the cytocidal activity of an NK cell. As a result, a cell expressing HLA of the same type as the NK cell can avoid NK cell-mediated rejection.

In contrast, activating KIR recognizes HLA that displays a virus-derived peptide on a cell expressing HLA of the same type as that of the NK cell and the peptide displayed thereon, transmits an activated signal into the NK cell, and activates the cytocidal activity of an NK cell. Unlike inhibitory KIR, activating KIR functions to recognize and eliminate viral peptides displayed on HLA.

In the present invention, the term "binding" is also referred to as "recognition." The term "recognition;" i.e., "binding," used herein refers to binding, which is not non-specific adsorption. Whether or not KIR recognizes; i.e., binds, can be determined with the use of, for example, a dissociation constant (hereafter referred to as "KD"). A KD value of a preferable antibody or the like of the present invention to an antigen protein is $1 \times 10^{-5}$ M or lower, $5 \times 10^{-6}$ M or lower, $2 \times 10^{-6}$ M or lower, or $1 \times 10^{-6}$ M or lower. In the present invention, binding can be assayed or evaluated by, for example, biomolecular interaction analysis, such as SPR or BLI, ELISA, or RIA. Binding between a substance expressed on a cell surface and a recombinant protein of inhibitory KIR can be assayed by flow cytometry or other techniques.

A substance having activity of binding to inhibitory KIR and inhibiting the cytocidal activity of an NK cell binds to inhibitory KIR, activates inhibitory KIR, transmits an inhibitory signal into an NK cell, and inhibits the cytocidal activity of an NK cell.

Examples of inhibitory KIR include those belonging to the KIR2DL subclass and the KIR3DL subclass. Examples of inhibitory KIRs belonging to the KIR2DL subclass include KIR2DL1, KIR2DL2, KIR2DL3, and KIR2DL5. Examples of inhibitory KIRs belonging to the KIR3DL subclass include KIR3DL1, KIR3DL2, and KIR3DL3. Examples of activating KIR subtypes include KIR2DS1, KIR2DS2, KIR2DS3, KIR2DS4, KIR2DS5, and KIR3DS1. KIR2DL4 is a subtype that has both inhibitory and activating properties. Table 1 shows ligands binding to such inhibitory KIRs and activating KIRs (HLA).

[Table 1]

| KIR subtype | Influence on cytocidal activity of NK cell | Ligand |
|---|---|---|
| KIR2DL1 | Inhibitory | HLA-C2 type (Cw2, Cw4, Cw5, Cw6, Cw15, Cw17) |
| KIR2DL2 | Inhibitory | HLA-C1 type (Cw1, Cw3, Cw7, Cw8, Cw9, Cw10, Cw12, Cw14, Cw16, B46) |
| KIR2DL3 | Inhibitory | HLA-C1 type (Cw1, Cw3, Cw7, Cw8, Cw9, Cw10, Cw12, Cw14, Cw16, B46) |
| KIR2DL4 | Inhibitory/activating | HLA-G |
| KIR2DL5 | Inhibitory | Not reported |
| KIR3DL1 | Inhibitory | HLA-Bw4 type (A23, A24, A25, A32, B5, B27, B37, B38, B44, B49, B51, B52, B53, B57, B58, B59, B77) |
| KIR3DL2 | Inhibitory | HLA-A03, A11 |
| KIR3DL3 | Inhibitory | Not reported |
| KIR2DS1 | Activating | HLA-C2 type (Cw2, Cw4, Cw5, Cw6, Cw15, Cw17) |
| KIR2DS2 | Activating | HLA-C1 type (Cwl, Cw3, Cw7, Cw8, Cw9, Cw10, Cw12, Cw14, Cw16, B46) |
| KIR2DS3 | Activating | Not expressed extracellularly |
| KIR2DS4 | Activating | HLA-A11 |
| KIR3DS1 | Activating | Not reported |

The inhibitory KIR and the activating KIR of the present invention each have various variants. In the examples of the present invention, variants comprising amino acid sequences exhibiting high expression frequency were selected and used. Accordingly, functions and effects of the present invention are not limited to each of variants but are applicable to KIRs of relevant subtypes, cells and hosts having HLA types corresponding to such KIRs, and the like. KIR2DL1*003 (SEQ ID NO: 4; IPD Accession Number: KIR00003) was used as a KIR2DL1 variant, KIR2DL2*001 (SEQ ID NO: 6; IPD Accession Number: KIR00010) and KIR2DL2*003 (SEQ ID NO: 57; IPD Accession Number: KIR00012) were used as KIR2DL2 variants, KIR2DL3*001 (SEQ ID NO: 8; IPD Accession Number: KIR00014) was used as a KIR2DL3 variant, KIR2DS1*002 (SEQ ID NO: 10; IPD Accession Number: KIR00034) was used as a KIR2DS1 variant, KIR2DS2*001 (SEQ ID NO: 12; IPD Accession Number: KIR00037) was used as a KIR2DS2 variant, KIR2DS4*001 (SEQ ID NO: 14; IPD Accession Number: KIR00045) was used as a KIR2DS4 variant, and KIR3DL1*015 (SEQ ID NO: 16; IPD Accession Number: KIR00102) was used as a KIR3DL1.

As shown in Figure 11, the sequence identity between the amino acid sequence of KIR2DL2*003 and that of KIR2DL3*001 is high (the amino acid sequence identity in the extracellular region is particularly high). In addition, the amino acid sequence identity thereof with the amino acid sequences of activating KIRs; i.e., KIR2DS1*002, KIR2DS2*001, and KIR2DS4*001, is also high.

The substance having activity of binding to human inhibitory KIR and inhibiting the cytocidal activity of an NK cell of the present invention has activity of binding to at least one of the inhibitory KIR subclasses indicated above and inhibiting the cytocidal activity of an NK cell expressing the KIR to which the substance binds. While the inhibitory KIR to which the substance of the present invention binds is not particularly limited, such substance has activity of binding preferably to KIR2DL1, KIR2DL2, KIR2DL3, and/or KIR3DL1, more preferably to KIR2DL1, KIR2DL2, and/or KIR2DL3, and inhibiting the cytocidal activity of an NK cell. Such substance binds to inhibitory KIR and exerts agonist activity thereon.

KIR2DL1 recognizes the C2 epitopes (Cw2, Cw4, Cw5, Cw6, Cw15, and Cw17) of HLA-C, which is an MHC class I molecule.

KIR2DL2 and KIR2DL3 recognize the C1 epitopes (Cw1, Cw3, Cw7, Cw8, Cw9, Cw10, Cw12, Cw14, and Cw16) of HLA-C, which is an MHC class I molecule, and the C1 epitopes (B46 and B73) of HLA-B, which is an MHC class I molecule.

KIR3DL1 recognizes the Bw4 epitopes (A23, A24, A25, A32, B5, B27, B37, B38, B44, B49, B51, B52, B53, B57, B58, B59, and B77) of HLA-B, which is an MHC class I molecule.

KIR3DL2 recognizes the A3 and A11 epitopes of HLA-A, which is an MHC class I molecule.

The activity of the substance having activity of binding to inhibitory KIR and inhibiting the cytocidal activity of an NK cell according to the present invention to activate the cytocidal activity of an NK cell is low. For example, the activity of the substance having activity of binding to inhibitory KIR and inhibiting the cytocidal activity of an NK cell according to the present invention to inhibit the cytocidal activity of an NK cell is higher than the activity of the substance to bind to the corresponding activating KIR and activate the cytocidal activity of an NK cell. The activating KIRs corresponding to the inhibitory KIR; i.e., KIR2DL1, KIR2DL2, or KIR2DL3, are KIR2DS1, KIR2DS2, KIR2DS3, and KIR2DS4, and particularly important activating KIRs are KIR2DS1, KIR2DS2, and KIR2DS4. The activating KIR corresponding to KIR3DL1 is KIR3DS1. It is preferable that the substance having activity of binding to inhibitory KIR and inhibiting the cytocidal activity of an NK cell of the present invention do not bind to activating KIR. Specifically, such substance exerts substantially no agonist activity on activating KIR. Accordingly, the substance having activity of binding to inhibitory KIR and inhibiting the cytocidal activity of an NK cell preferably binds to a partial sequence having a small number of identical sequences or having a different access to a three-dimensional protein structure in the amino acid sequence alignment of KIR2DL2, KIR2DL3, KIR2DS1, KIR2DS2, and KIR2DS4 shown in Figure 10.

An NK cell, activation of which is inhibited by the substance of the present invention, comprises target inhibitory KIR expressed on the surface thereof. As such NK cell, a blood sample of a subject having an HLA type corresponding to the inhibitory KIR or the population of NK cells isolated from the blood may be used. Alternatively, NK cells prepared from a commercially available NK cell line to express the target inhibitory KIR by genetic engineering may be used.

The agonist activity of the substance of the present invention on inhibitory KIR can be evaluated by assaying the cytocidal activity of the effector cells described below on the target cells described below in the presence and the absence of the test substance, and calculating the percentage of cytocidal activity inhibition in the presence of the test substance. As effector cells, NK cells expressing the target inhibitory KIR are used. As target cells, cells that do not express HLA corresponding to the target inhibitory KIR are used. In agonist activity assays, the test substance may be added in a soluble form in a culture solution, or it may be expressed in the form of a membrane protein described below on the target cell surface.

The agonist activity of, for example, a substance binding to KIR2DL2/3 is assayed with the use of, as an effector cell, an NK cell derived from an HLA-C1 homozygote or an HLA-C1/C2 heterozygote and expressing KIR2DL2 and/or KIR2DL3 and, as a target cell, a cell not expressing an HLA-C1 type molecule (e.g., a cell sampled from an HLA-C2/C2 homozygote). It is preferable that the effector cell and the target cell have Bw4 of the same type (negative homozygous or positive homozygous cell).

The agonist activity of, for example, a substance binding to KIR2DL1 is assayed with the use of, as an effector cell, an NK cell derived from an HLA-C2 homozygote or an HLA-C1/C2 heterozygote and expressing KIR2DL1 and, as a target cell, a cell not expressing an HLA-C2 type molecule (e.g., a cell sampled from a HLA C1/C1 homozygous subject). It is preferable that the effector cell and the target cell have Bw4 of the same type (negative homozygous or positive homozygous).

The agonist activity of, for example, a substance binding to KIR3DL1 is assayed with the use of, as an effector cell, an NK cell derived from an HLA-Bw4 type molecule and expressing KIR3DL1 and, as a target cell, a cell not expressing an HLA-Bw4 type molecule (e.g., a cell sampled from a HLA Bw4$^+$/Bw4$^-$ homozygous subject). It is preferable that the effector cell and the target cell have HLA-C of the same type (i.e., both cells are C1/C1 homozygous, C2/C2 homozygous, or C1/C2 heterozygous).

The agonist activity of, for example, a substance binding to KIR3DL2 is assayed with the use of, as an effector cell, an NK cell derived from an HLA-A03 or A11 type molecule and expressing KIR3DL2 and, as a target cell, a cell not expressing an HLA-A03 or A11 type molecule. It is preferable that the effector cell and the target cell have HLA-C of the same type (i.e., both cells are C1/C1 homozygous, C2/C2 homozygous, or C1/C2 heterozygous) and BW4 of the same type (i.e., both cells are BW4$^-$ homozygous, BW4$^+$ homozygous, or BW4$^+$/BW4$^-$ heterozygous).

An example of a substance having activity of binding to human inhibitory KIR and inhibiting the cytocidal activity of an NK cell is a substance having a site binding to inhibitory KIR. A specific example of such substance is a polypeptide having a site binding to inhibitory KIR and binding to inhibitory KIR.

Examples of polypeptides each having a site binding to inhibitory KIR and binding to inhibitory KIR include the polypeptides (1) to (3) below.

(1) Antibody against inhibitory KIR or antigen-binding fragment

Examples of antibodies against inhibitory KIR include Lirilumab, Pan2D, and monoclonal antibodies comprising CDRs or variable regions included in the scFv (i) to (x) described below grafted thereinto. In the present invention, antibodies are not limited to those described above, and all antibodies having activity of binding to human inhibitory KIR and inhibiting the cytocidal activity of an NK cell are within the scope of the present invention.

Antibodies having activity of binding to inhibitory KIR and inhibiting the cytocidal activity of an NK cell and antigen-binding fragments binding to inhibitory KIR (hereafter, they may be referred to as "the antibodies and the like of the present invention") may be monoclonal antibodies or polyclonal antibodies. Isotypes of the monoclonal antibodies of the present invention are not particularly limited. Examples of isotypes include IgG, such as IgG1, IgG2, IgG3, and IgG4, IgM, IgA, such as IgA1 and IgA2, IgD, and Ig. The isotype and the subclass of a monoclonal antibody can be determined by, for example, the Ouchterlony method, ELISA, or RIA. Examples of the monoclonal antibody of the present invention include a non-human animal-derived antibody (a non-human animal antibody), a human antibody, a chimerized antibody (also referred to as a "chimera antibody"), and a humanized antibody, with the use of a human antibody being preferable. An antibody variant is within the scope of the antibody of the present invention. For example, a human antibody variant is within the scope of the human antibody.

Examples of non-human animal antibodies include antibodies derived from vertebrates, such as mammals and birds. Examples of mammal-derived antibodies include an antibody derived from a rodent, such as a mouse antibody or rat antibody, and a single-chain antibody comprising a single variable region derived from camel, alpaca, or llama. An example of a bird-derived antibody is a chicken antibody.

An example of a chimerized antibody is, but is not limited to, an antibody composed of a variable region derived from a non-human animal antibody and a constant region derived from a human antibody (human immunoglobulin) bound to each other.

Examples of humanized antibodies include, but are not limited to, an antibody constructed by grafting CDR in a variable region of a non-human animal antibody into a human antibody (a variable region of human immunoglobulin), an antibody constructed by grafting a partial sequence of a framework region of a non-human animal antibody in addition to CDR into a human antibody, and an antibody constructed by substituting at least one amino acid derived from any non-human animal antibody with a human amino acid.

Examples of CDR-grafted antibodies include, but are not limited to, an antibody or an antigen-binding fragment constructed by grafting CDR in a variable region of a given antibody or an antigen-binding fragment into a variable region of another immunoglobulin, an antibody or an antigen-binding fragment constructed by grafting a partial sequence of a framework region of an original antibody or an antigen-binding fragment in addition to CDR into a variable region of another immunoglobulin, and an antibody or an antigen-binding fragment constructed by substituting at least one amino acid derived from any of the antibodies described below or antigen-binding fragments with another amino acid.

When substituting an amino acid in CDR with another amino acid at the time of CDR grafting, it is preferable that 1 to 6 amino acids selected from 1, 2, or 3 amino acids on the N terminal side and/or C terminal side in the CDR region be substituted, it is more preferable that 1 to 4 amino acids selected from 2 amino acids on the N terminal side and/or C terminal side be substituted, and it is further preferable that 1 or 2 amino acids selected from 1 amino acid on the N terminal side and/or C terminal side be substituted.

An antibody can be prepared by various known techniques. Examples of known techniques include a method involving the use of a hybridoma, cell immunization, and genetic recombination. A method of obtaining a human antibody selected from the human antibody phage library is also known. For example, the phage display method comprising expressing a variable region of a human antibody as scFv on a phage surface and selecting an antigen-binding phage may be employed. The gene of the phage selected upon binding to the antigen may be analyzed, so as to determine a DNA sequence encoding a variable region of an antigen-binding human antibody. When a DNA sequence of antigen-binding scFv is identified, an expression vector comprising such sequence may be prepared, introduced into an adequate host, and expressed therein. Thus, a human antibody can be obtained (WO 92/01047, WO 92/20791, WO 93/06213, WO 93/11236, WO 93/19172, WO 95/01438, WO 95/15388, Annu. Rev. Immunol., 1994, 12, 433-455). A single-chain antibody consisting of a heavy chain is an antibody possessed by a camel, alpaca, llama, or the like. Such antibody can be obtained by immunizing such animal or using a phage library derived from such animal (see, for example, David R. Maass et al., J. Immunol. Methods, Jul 31, 2007; 324 (1-2): 13-25; and Lukas Roth et al., Methods Mol. Biol., 2020; 2070: 173-189).

In the present invention, the term "antigen-binding fragment" refers to a peptide composed of 1 or 2 variable regions and having antigen-binding activity. Examples of "antigen-binding fragments" include, but are not limited to, antigen-binding fragments, such as scFv, Fab, F(ab')$_2$, Fab', Fv, and single-domain antibody (sdAb). Such antigen-binding fragment may be obtained by treating a full-length molecule of an antibody protein with an enzyme, such as papain or pepsin, or it may be a recombinant protein produced in an adequate host cell using a recombinant gene. When an antigen-binding fragment having a heavy chain variable region and a light chain variable region is produced by genetic engineering, an antigen-binding fragment may be produced by grafting a heavy chain variable region and a light chain variable region (which may exclusively be a heavy chain variable region in the case of a single-stranded antibody) included in a monoclonal antibody binding to a target antigen or an antigen-binding fragment or CDR1, CDR2, and CDR3 included therein into a framework sequence of an antigen-binding fragment of interest. An antigen-binding fragment derived from a single-chain antibody consisting of a heavy chain may be integrated into an expression format suitable for only one variable region with the use of a phage- or yeast-displaying body library carrying a gene encoding a variable region of a single-chain antibody derived from a camel, alpaca, or llama binding to a target antigen in accordance with a known method or the method disclosed herein in the same manner as the method for producing scFv or membrane scFv (see, for example, David R. Maass et al. J. Immunol. Methods, Jul 31, 2007; 324 (1-2): 13-25; and Lukas Roth et al., Methods Mol. Biol., 2020; 2070: 173-189). When grafting a variable region or CDR, an antibody constructed by substituting at least one amino acid in the sequence of an original antibody with an amino acid of the antigen-binding fragment of interest may be used, although the antibody is not limited thereto. When the antigen-binding fragment of the present invention is a single-chain antibody such as scFv, a binding capability is known to be optionally maintained even if a heavy chain is replaced with a light chain when grafting a variable region or CDR from the original antibody or an antigen-binding fragment to the antigen-binding fragment of interest. The present invention encompasses an antibody-binding fragment in which a heavy chain variable region is replaced with a light chain variable region (Effects of variable domain orientation on anti-HER2 single-chain variable fragment antibody expressed in the *Escherichia coli* cytoplasm, Ilkay Kocer et al., Biotechnol. Prog., Mar 2021; 37 (2): 33102).

In particular, scFv is preferable. scFv is an antigen-binding fragment that is obtained by linking a heavy chain variable region to a light chain variable region with a polypeptide linker (Pluckthun A., The Pharmacology of Monoclonal Antibodies 113, Rosenburg and Moore (ed.), Springer Verlag, New York, 269-315, 1994, Nature Biotechnology, 2005, 23, 1126-1136). As a linker sequence, a variety of sequences known as linker sequences of a single-stranded antibody or scFv, such as artificial sequences, can be used. As an artificial linker sequence, an amino acid sequence rich in glycine and serine is known. Examples thereof include a GS linker consisting of 3 amino acids (GSG) or 4 amino acids (GSGS) (SEQ ID NO: 3) and a linker sequence represented by a formula (G4S)n. In the formula, n is 1 to 10, and n is preferably 3 (GGGGSGGGGSGGGGS: SEQ ID NO: 25). A linker domain is also referred to as a peptide linker, and a known peptide linker can be used as an extracellular linker domain. A sequence represented by the formula (G4S)n is referred to as a "G4S linker."

scFv binding to a target antigen can be obtained by the phage display method comprising expressing a variable region library as a single-stranded antibody (scFv) on the phage surface and selecting an antigen-binding phage (Nature Biotechnology, 2005, 23, (9), pp. 1105-1116). The gene of the phage selected upon binding to the antigen may be analyzed, so as to determine a DNA sequence encoding an antigen-binding variable region. When the sequences of heavy chain and light chain variable regions are identified, the sequences of CDRs in the variable regions can be identified. The sequences of CDRs and the variable regions are integrated into the sequence format of an antibody or an antigen-binding fragment of interest to prepare an expression vector, and the expression vector is introduced into an adequate host to express the antibody or an antigen-binding fragment. Thus, an antibody or an antigen-binding fragment having antigen-binding properties of interest can be obtained (WO 92/01047, WO 92/20791, WO 93/06213, WO 93/11236, WO 93/19172, WO 95/01438, WO 95/15388, Annu. Rev. Immunol., 1994, 12, pp. 433-455; Nature Biotechnology, 2005, 23 (9), pp. 1105-1116).

CDR can be modified, provided that the agonist activity of the polypeptide of the present invention is not changed. Specifically, CDR of the polypeptide of the present invention may be examined as to whether or not a particular amino acid residue is substituted with alanine and the agonist activity on inhibitory KIR is maintained. An amino acid that maintains the agonist activity of the polypeptide on inhibitory KIR after substitution with alanine can be determined to be insignificant for the agonist activity. Such amino acid in CDR can be substituted with another amino acid, and the polypeptide resulting from amino acid substitution has reactivity similar to that of the polypeptide before amino acid substitution. When substituting an amino acid in CDR with another amino acid, it is preferable that 1 to 6 amino acids selected from 1, 2, or 3 amino acids on the N terminal side and/or C terminal side in the CDR region be substituted, it is more preferable that 1 to 4 amino acids selected from 2 amino acids on the N terminal side and/or C terminal side be substituted, and it is further preferable that 1 or 2 amino acids selected from 1 amino acid on the N terminal side and/or C terminal side be substituted.

An example of scFv for the inhibitory KIR2DL2 or KIR2DL3 of the present invention is scFv consisting of an amino acid sequence according to any of (i) to (xii). A heavy chain variable region can be linked to a light chain variable region with, for example, a GS linker or a G4S linker:

(i) an amino acid sequence comprising the amino acid sequence of a heavy chain variable region shown in SEQ ID NO: 33 in combination with the amino acid sequence of a light chain variable region shown in SEQ ID NO: 34;

(ii) an amino acid sequence comprising the amino acid sequence of a heavy chain variable region shown in SEQ ID NO: 35 in combination with the amino acid sequence of a light chain variable region shown in SEQ ID NO: 36;

(iii) an amino acid sequence comprising the amino acid sequence of a heavy chain variable region shown in SEQ ID NO: 37 in combination with the amino acid sequence of a light chain variable region shown in SEQ ID NO: 38;

(iv) an amino acid sequence comprising the amino acid sequence of a heavy chain variable region shown in SEQ ID NO: 39 in combination with the amino acid sequence of a light chain variable region shown in SEQ ID NO: 40;

(v) an amino acid sequence comprising the amino acid sequence of a heavy chain variable region shown in SEQ ID NO: 41 in combination with the amino acid sequence of a light chain variable region shown in SEQ ID NO: 42;

(vi) an amino acid sequence comprising the amino acid sequence of a heavy chain variable region shown in SEQ ID NO: 43 in combination with the amino acid sequence of a light chain variable region shown in SEQ ID NO: 44;

(vii) an amino acid sequence comprising the amino acid sequence of a heavy chain variable region shown in SEQ ID NO: 45 in combination with the amino acid sequence of a light chain variable region shown in SEQ ID NO: 46;

(viii) an amino acid sequence comprising the amino acid sequence of a heavy chain variable region shown in SEQ ID NO: 47 in combination with the amino acid sequence of a light chain variable region shown in SEQ ID NO: 48;

(ix) an amino acid sequence comprising the amino acid sequence of a heavy chain variable region shown in SEQ ID NO: 49 in combination with the amino acid sequence of a light chain variable region shown in SEQ ID NO: 50;
(x) an amino acid sequence comprising the amino acid sequence of a heavy chain variable region shown in SEQ ID NO: 51 in combination with the amino acid sequence of a light chain variable region shown in SEQ ID NO: 52;
(xi) an amino acid sequence comprising the amino acid sequence of a heavy chain variable region shown in SEQ ID NO: 61 in combination with the amino acid sequence of a light chain variable region shown in SEQ ID NO: 62; and
(xii) an amino acid sequence comprising the amino acid sequence of a heavy chain variable region shown in SEQ ID NO: 63 in combination with the amino acid sequence of a light chain variable region shown in SEQ ID NO: 64.

An example of scFv for the inhibitory KIR3DL1 of the present invention is scFv consisting of an amino acid sequence according to any of (xiii) to (xviii). A heavy chain variable region can be linked to a light chain variable region with, for example, a GS linker or a G4S linker:

(xiii) an amino acid sequence comprising the amino acid sequence of a heavy chain variable region shown in SEQ ID NO: 56 in combination with the amino acid sequence of a light chain variable region shown in SEQ ID NO: 55;
(xiv) an amino acid sequence comprising the amino acid sequence of a heavy chain variable region shown in SEQ ID NO: 137 in combination with the amino acid sequence of a light chain variable region shown in SEQ ID NO: 138;
(xv) an amino acid sequence comprising the amino acid sequence of a heavy chain variable region shown in SEQ ID NO: 139 in combination with the amino acid sequence of a light chain variable region shown in SEQ ID NO: 140;
(xvi) an amino acid sequence comprising the amino acid sequence of a heavy chain variable region shown in SEQ ID NO: 141 in combination with the amino acid sequence of a light chain variable region shown in SEQ ID NO: 142;
(xvii) an amino acid sequence comprising the amino acid sequence of a heavy chain variable region shown in SEQ ID NO: 143 in combination with the amino acid sequence of a light chain variable region shown in SEQ ID NO: 144; and
(xviii) an amino acid sequence comprising the amino acid sequence of a heavy chain variable region shown in SEQ ID NO: 145 in combination with the amino acid sequence of a light chain variable region shown in SEQ ID NO: 146.

An example of scFv for the inhibitory KIR2DL1 of the present invention is scFv consisting of an amino acid sequence according to any of (ci) to (cxv) below. A heavy chain variable region can be linked to a light chain variable region with, for example, a GS linker or a G4S linker:

(ci) an amino acid sequence comprising the amino acid sequence of a heavy chain variable region shown in SEQ ID NO: 107 in combination with the amino acid sequence of a light chain variable region shown in SEQ ID NO: 108;
(cii) an amino acid sequence comprising the amino acid sequence of a heavy chain variable region shown in SEQ ID NO: 109 in combination with the amino acid sequence of a light chain variable region shown in SEQ ID NO: 110;
(ciii) an amino acid sequence comprising the amino acid sequence of a heavy chain variable region shown in SEQ ID NO: 111 in combination with the amino acid sequence of a light chain variable region shown in SEQ ID NO: 112;
(civ) an amino acid sequence comprising the amino acid sequence of a heavy chain variable region shown in SEQ ID NO: 113 in combination with the amino acid sequence of a light chain variable region shown in SEQ ID NO: 114;
(cv) an amino acid sequence comprising the amino acid sequence of a heavy chain variable region shown in SEQ ID NO: 115 in combination with the amino acid sequence of a light chain variable region shown in SEQ ID NO: 116;
(cvi) an amino acid sequence comprising the amino acid sequence of a heavy chain variable region shown in SEQ ID NO: 117 in combination with the amino acid sequence of a light chain variable region shown in SEQ ID NO: 118;
(cvii) an amino acid sequence comprising the amino acid sequence of a heavy chain variable region shown in SEQ ID NO: 119 in combination with the amino acid sequence of a light chain variable region shown in SEQ ID NO: 120;
(cviii) an amino acid sequence comprising the amino acid sequence of a heavy chain variable region shown in SEQ ID NO: 121 in combination with the amino acid sequence of a light chain variable region shown in SEQ ID NO: 122;
(cviiii) an amino acid sequence comprising the amino acid sequence of a heavy chain variable region shown in SEQ ID NO: 123 in combination with the amino acid sequence of a light chain variable region shown in SEQ ID NO: 124;
(cx) an amino acid sequence comprising the amino acid sequence of a heavy chain variable region shown in SEQ ID NO: 125 in combination with the amino acid sequence of a light chain variable region shown in SEQ ID NO: 126;
(cxi) an amino acid sequence comprising the amino acid sequence of a heavy chain variable region shown in SEQ ID NO: 127 in combination with the amino acid sequence of a light chain variable region shown in SEQ ID NO: 128;
(cxii) an amino acid sequence comprising the amino acid sequence of a heavy chain variable region shown in SEQ ID NO: 129 in combination with the amino acid sequence of a light chain variable region shown in SEQ ID NO: 130;
(cxiii) an amino acid sequence comprising the amino acid sequence of a heavy chain variable region shown in SEQ ID NO: 131 in combination with the amino acid sequence of a light chain variable region shown in SEQ ID NO: 132;
(cxiv) an amino acid sequence comprising the amino acid sequence of a heavy chain variable region shown in SEQ ID NO: 133 in combination with the amino acid sequence of a light chain variable region shown in SEQ ID NO: 134; and

(cxv) an amino acid sequence comprising the amino acid sequence of a heavy chain variable region shown in SEQ ID NO: 135 in combination with the amino acid sequence of a light chain variable region shown in SEQ ID NO: 136.

In addition, the polypeptide having a site binding to inhibitory KIR and binding to inhibitory KIR encompasses a polypeptide having a binding site consisting of an amino acid sequence derived from an antibody or scFv recognizing inhibitory KIR and binding thereto. Such polypeptide can be in any of various forms, and preferable examples include a monoclonal antibody, an antigen-binding fragment such as scFv, and a membrane protein having an amino acid sequence of an antigen-binding fragment outside the cell.

The amino acid sequence derived from an antibody or scFv recognizing inhibitory KIR and binding thereto comprises an amino acid sequence of a site binding to inhibitory KIR in an antibody or scFv recognizing inhibitory KIR and binding thereto, an amino acid sequence derived from the amino acid sequence of a site binding to inhibitory KIR in an antibody or scFv recognizing inhibitory KIR and binding thereto by substitution, deletion, or addition (addition encompasses insertion) (hereafter, collectively referred to as "mutation") of one or several amino acids, and an amino acid sequence having activity of binding to inhibitory KIR and inhibiting the cytocidal activity of an NK cell. The term "several" in the term "one or several" refers to 2 to 10, preferably 7 or less, more preferably 5 or less, and further preferably 4, 3, 2, or 1.

Specific examples of sites binding to inhibitory KIR include sites comprising amino acid sequences derived from CDR1, CDR2, and CDR3 included in each of the light chain variable region and the heavy chain variable region of a monoclonal antibody or scFv binding to inhibitory KIR, an amino acid sequence derived from a variable region including the CDR1, CDR2, and CDR3, or amino acid sequences derived from the light chain variable region and the heavy chain variable region. The amino acid sequence derived from the antibody against inhibitory KIR or scFv is as defined above.

For example, a polypeptide having a site binding to inhibitory KIR2DL2 and/or KIR2DL3 and binding to inhibitory KIR2DL2 and/or KIR2DL3 has a heavy chain variable region in combination with a light chain variable region shown in Table 2. In Table 2, "a2DL2/3" indicates that a binder binds to inhibitory KIR2DL2 and/or KIR2DL3.

[Table 2]

| Binder | Variable region | CDR1 | CDR2 | CDR3 |
|---|---|---|---|---|
| a2DL2/3-DS1 | VH (SEQ ID NO: 33) | 26-33 | 51-58 | 97-109 |
|  | VL (SEQ ID NO: 34) | 27-34 | 52-54 | 91-101 |
| a2DL2/3-DS2 | VH (SEQ ID NO: 35) | 26-33 | 51-58 | 97-109 |
|  | VL (SEQ ID NO: 36) | 26-34 | 52-54 | 91-102 |
| a2DL2/3-DS3 | VH (SEQ ID NO: 37) | 26-33 | 51-58 | 97-113 |
|  | VL (SEQ ID NO: 38) | 26-34 | 52-54 | 91-101 |
| a2DL2/3-DS4 | VH (SEQ ID NO: 39) | 26-33 | 51-58 | 97-105 |
|  | VL (SEQ ID NO: 40) | 27-32 | 50-52 | 89-97 |
| a2DL2/3-DS5 | VH (SEQ ID NO: 41) | 26-33 | 51-58 | 97-110 |
|  | VL (SEQ ID NO: 42) | 27-32 | 50-52 | 89-97 |
| a2DL2/3-DS6 | VH (SEQ ID NO: 43) | 26-33 | 51-58 | 97-113 |
|  | VL (SEQ ID NO: 44) | 27-33 | 51-53 | 90-98 |
| a2DL2/3-DS7 | VH (SEQ ID NO: 45) | 26-33 | 51-58 | 97-114 |
|  | VL (SEQ ID NO: 46) | 27-32 | 50-52 | 89-97 |
| a2DL2/3-DS8 | VH (SEQ ID NO: 47) | 26-33 | 51-58 | 97-111 |
|  | VL (SEQ ID NO: 48) | 27-33 | 51-53 | 90-98 |
| a2DL2/3-DS9 | VH (SEQ ID NO: 49) | 26-33 | 51-58 | 97-111 |
|  | VL (SEQ ID NO: 50) | 27-33 | 51-53 | 90-98 |
| a2DL2/3-DS10 | VH (SEQ ID NO: 51) | 27-33 | 51-58 | 97-112 |
|  | VL (SEQ ID NO: 52) | 27-32 | 50-52 | 89-97 |
| a2DL2/3-DS11 | VH (SEQ ID NO: 61) | 26-33 | 51-58 | 97-110 |
|  | VL (SEQ ID NO: 62) | 26-33 | 51-53 | 90-100 |

(continued)

| Binder | Variable region | CDR1 | CDR2 | CDR3 |
|---|---|---|---|---|
| a2DL2/3-DS12 | VH (SEQ ID NO: 63) | 26-33 | 51-58 | 97-110 |
| | VL (SEQ ID NO: 64) | 26-33 | 51-53 | 90-100 |

| * In the table, CDR1, CDR2, and CDR3 are each indicated with the amino acid numbers in the amino acid sequence of relevant variable region. The amino acid numbers are specified in accordance with the IMGT definitions. |
|---|

A polypeptide having a site binding to inhibitory KIR2DL1 and binding to inhibitory KIR2DL1 comprises a heavy chain variable region in combination with a light chain variable region shown in Table 3. In Table 3, "a2DL1" indicates that a binder binds to inhibitory KIR2DL1.

[Table 3]

| Binder | Variable region | CDR1 | CDR2 | CDR3 |
|---|---|---|---|---|
| a2DL1-DS1 | VH (SEQ ID NO: 107) | 26-35 | 53-61 | 100-113 |
| | VL (SEQ ID NO: 108) | 27-38 | 56-58 | 95-103 |
| a2DL1-DS2 | VH (SEQ ID NO: 109) | 26-33 | 51-58 | 97-113 |
| | VL (SEQ ID NO: 110) | 27-32 | 50-52 | 89-97 |
| a2DL1-DS3 | VH (SEQ ID NO: 111) | 26-35 | 53-61 | 100-111 |
| | VL (SEQ ID NO: 112) | 27-38 | 56-58 | 95-103 |
| a2DL1-DS4 | VH (SEQ ID NO: 113) | 26-33 | 51-58 | 97-105 |
| | VL (SEQ ID NO: 114) | 27-33 | 51-53 | 90-98 |
| a2DL1-DS5 | VH (SEQ ID NO: 115) | 26-33 | 51-58 | 97-114 |
| | VL (SEQ ID NO: 116) | 27-32 | 50-52 | 89-97 |
| a2DL1-DS6 | VH (SEQ ID NO: 117) | 26-33 | 51-58 | 97-106 |
| | VL (SEQ ID NO: 118) | 27-32 | 50-52 | 89-98 |
| a2DL1-DS7 | VH (SEQ ID NO: 119) | 26-33 | 51-58 | 97-112 |
| | VL (SEQ ID NO: 120) | 26-34 | 52-54 | 91-100 |
| a2DL1-DS8 | VH (SEQ ID NO: 121) | 26-33 | 51-58 | 97-107 |
| | VL (SEQ ID NO: 122) | 26-33 | 51-53 | 90-100 |
| a2DL1-DS9 | VH (SEQ ID NO: 123) | 26-33 | 51-58 | 97-106 |
| | VL (SEQ ID NO: 124) | 26-33 | 51-53 | 90-100 |
| a2DL1-DS10 | VH (SEQ ID NO: 125) | 26-33 | 51-58 | 97-112 |
| | VL (SEQ ID NO: 126) | 26-33 | 51-53 | 90-100 |
| a2DL1-DS11 | VH (SEQ ID NO: 127) | 26-33 | 51-58 | 97-105 |
| | VL (SEQ ID NO: 128) | 26-33 | 51-53 | 90-100 |
| a2DL1-DS12 | VH (SEQ ID NO: 129) | 26-33 | 51-58 | 97-111 |
| | VL (SEQ ID NO: 130) | 26-34 | 52-54 | 91-99 |
| a2DL1-DS13 | VH (SEQ ID NO: 131) | 26-33 | 51-58 | 97-104 |
| | VL (SEQ ID NO: 132) | 26-33 | 51-53 | 90-100 |
| a2DL1-DS14 | VH (SEQ ID NO: 133) | 26-35 | 53-61 | 100-114 |
| | VL (SEQ ID NO: 134) | 26-34 | 52-54 | 91-101 |
| a2DL1-DS15 | VH (SEQ ID NO: 135) | 26-33 | 51-58 | 97-115 |

(continued)

| Binder | Variable region | CDR1 | CDR2 | CDR3 |
|---|---|---|---|---|
| | VL (SEQ ID NO: 136) | 26-34 | 52-54 | 91-102 |

A polypeptide having a site binding to inhibitory KIR3DL1 and binding to inhibitory KIR3DL1 comprises a heavy chain variable region in combination with a light chain variable region shown in Table 4. In Table 4, "a3DL1" indicates that a binder binds to inhibitory KIR3DL1.

[Table 4]

| Binder | Variable region | CDR1 | CDR2 | CDR3 |
|---|---|---|---|---|
| a3DL1-DS1 | VH (SEQ ID NO: 56) | 26-33 | 51-58 | 97-109 |
| | VL (SEQ ID NO: 55) | 26-33 | 49-51 | 88-98 |
| a3DL1-DS2 | VH (SEQ ID NO: 137) | 26-35 | 53-61 | 100-111 |
| | VL (SEQ ID NO: 138) | 27-32 | 50-52 | 89-97 |
| a3DL1-DS3 | VH (SEQ ID NO: 139) | 26-35 | 53-61 | 100-111 |
| | VL (SEQ ID NO: 140) | 27-32 | 50-52 | 89-98 |
| a3DL1-DS4 | VH (SEQ ID NO: 141) | 26-33 | 51-58 | 97-115 |
| | VL (SEQ ID NO: 142) | 27-33 | 51-53 | 90-98 |
| a3DL1-DS5 | VH (SEQ ID NO: 143) | 26-35 | 53-61 | 100-111 |
| | VL (SEQ ID NO: 144) | 27-32 | 50-52 | 89-97 |
| a3DL1-DS6 | VH (SEQ ID NO: 145) | 26-33 | 51-58 | 97-109 |
| | VL (SEQ ID NO: 146) | 26-34 | 52-54 | 91-101 |

* In the table, CDR1, CDR2, and CDR3 are each indicated with the amino acid numbers in the amino acid sequence of relevant variable region. The amino acid numbers are specified in accordance with the IMGT definitions.

An example of the polypeptide having a site binding to inhibitory KIR2DL2 or KIR2DL3 and binding to inhibitory KIR according to an aspect of the present invention is a polypeptide comprising the amino acid sequence according to any of (I) to (XII) below (preferably, a monoclonal antibody, an antigen-binding fragment, scFv consisting of such amino acid sequence, or a membrane protein comprising such scFv in an extracellular domain):

(I) an amino acid sequence comprising a first variable region comprising the amino acid sequence derived from the amino acid sequence of CDR1, CDR2, and CDR3 shown in SEQ ID NO: 33 (preferably, a variable region comprising the amino acid sequence shown in SEQ ID NO: 33) in combination with a second variable region comprising the amino acid sequence derived from the amino acid sequence of CDR1, CDR2, and CDR3 in SEQ ID NO: 34 (preferably, a variable region comprising the amino acid sequence shown in SEQ ID NO: 34);

(II) an amino acid sequence comprising a first variable region comprising the amino acid sequence derived from the amino acid sequence of CDR1, CDR2, and CDR3 shown in SEQ ID NO: 35 (preferably, a variable region comprising the amino acid sequence shown in SEQ ID NO: 35) in combination with a second variable region comprising the amino acid sequence derived from the amino acid sequence of CDR1, CDR2, and CDR3 shown in SEQ ID NO: 36 (preferably, a variable region comprising the amino acid sequence shown in SEQ ID NO: 36);

(III) an amino acid sequence comprising a first variable region comprising the amino acid sequence derived from the amino acid sequence of CDR1, CDR2, and CDR3 shown in SEQ ID NO: 37 (preferably, a variable region comprising the amino acid sequence shown in SEQ ID NO: 37) in combination with a second variable region comprising the amino acid sequence derived from the amino acid sequence of CDR1, CDR2, and CDR3 shown in SEQ ID NO: 38 (preferably, a variable region comprising the amino acid sequence shown in SEQ ID NO: 38);

(IV) an amino acid sequence comprising a first variable region comprising the amino acid sequence derived from the amino acid sequence of CDR1, CDR2, and CDR3 shown in SEQ ID NO: 39 (preferably, a variable region comprising the amino acid sequence shown in SEQ ID NO: 39) in combination with a second variable region comprising the amino acid sequence derived from the amino acid sequence of CDR1, CDR2, and CDR3 shown in SEQ ID NO: 40 (preferably, a variable region comprising the amino acid sequence shown in SEQ ID NO: 40);

(V) an amino acid sequence comprising a first variable region comprising the amino acid sequence derived from the amino acid sequence of CDR1, CDR2, and CDR3 shown in SEQ ID NO: 41 (preferably, a variable region comprising the amino acid sequence shown in SEQ ID NO: 41) in combination with a second variable region comprising the amino acid sequence derived from the amino acid sequence of CDR1, CDR2, and CDR3 shown in SEQ ID NO: 42 (preferably, a variable region comprising the amino acid sequence shown in SEQ ID NO: 42);

(VI) an amino acid sequence comprising a first variable region comprising the amino acid sequence derived from the amino acid sequence of CDR1, CDR2, and CDR3 shown in SEQ ID NO: 43 (preferably, a variable region comprising the amino acid sequence shown in SEQ ID NO: 43) in combination with a second variable region comprising the amino acid sequence derived from the amino acid sequence of CDR1, CDR2, and CDR3 shown in SEQ ID NO: 44 (preferably, a variable region comprising the amino acid sequence shown in SEQ ID NO: 44);

(VII) an amino acid sequence comprising a first variable region comprising the amino acid sequence derived from the amino acid sequence of CDR1, CDR2, and CDR3 shown in SEQ ID NO: 45 (preferably, a variable region comprising the amino acid sequence shown in SEQ ID NO: 45) in combination with a second variable region comprising the amino acid sequence derived from the amino acid sequence of CDR1, CDR2, and CDR3 shown in SEQ ID NO: 46 (preferably, a variable region comprising the amino acid sequence shown in SEQ ID NO: 46);

(VIII) an amino acid sequence comprising a first variable region comprising the amino acid sequence derived from the amino acid sequence of CDR1, CDR2, and CDR3 shown in SEQ ID NO: 47 (preferably, a variable region comprising the amino acid sequence shown in SEQ ID NO: 47) in combination with a second variable region comprising the amino acid sequence derived from the amino acid sequence of CDR1, CDR2, and CDR3 shown in SEQ ID NO: 48 (preferably, a variable region comprising the amino acid sequence shown in SEQ ID NO: 48);

(IX) an amino acid sequence comprising a first variable region comprising the amino acid sequence derived from the amino acid sequence of CDR1, CDR2, and CDR3 shown in SEQ ID NO: 49 (preferably, a variable region comprising the amino acid sequence shown in SEQ ID NO: 49) in combination with a second variable region comprising the amino acid sequence derived from the amino acid sequence of CDR1, CDR2, and CDR3 shown in SEQ ID NO: 50 (preferably, a variable region comprising the amino acid sequence shown in SEQ ID NO: 50);

(X) an amino acid sequence comprising a first variable region comprising the amino acid sequence derived from the amino acid sequence of CDR1, CDR2, and CDR3 shown in SEQ ID NO: 51 (preferably, a variable region comprising the amino acid sequence shown in SEQ ID NO: 51) in combination with a second variable region comprising the amino acid sequence derived from the amino acid sequence of CDR1, CDR2, and CDR3 shown in SEQ ID NO: 52 (preferably, a variable region comprising the amino acid sequence shown in SEQ ID NO: 52);

(XI) a binding site comprising a first variable region comprising the amino acid sequence derived from the amino acid sequence of CDR1, CDR2, and CDR3 shown in SEQ ID NO: 61 in combination with a second variable region comprising the amino acid sequence derived from the amino acid sequence of CDR1, CDR2, and CDR3 shown in SEQ ID NO: 62; and

(XII) a binding site comprising a first variable region comprising the amino acid sequence derived from the amino acid sequence of CDR1, CDR2, and CDR3 shown in SEQ ID NO: 63 in combination with a second variable region comprising the amino acid sequence derived from the amino acid sequence of CDR1, CDR2, and CDR3 shown in SEQ ID NO: 64.

The polypeptide having a site binding to inhibitory KIR3DL1 and binding to the inhibitory KIR3DL1 according to an aspect of the present invention is, for example, a polypeptide comprising an amino acid sequence according to any of (XIII) to (XVIII) below (preferably, a monoclonal antibody, an antigen-binding fragment, scFv consisting of the amino acid sequence, or a membrane protein comprising such scFv in an extracellular domain):

(XIII) an amino acid sequence comprising a first variable region comprising the amino acid sequence derived from the amino acid sequence of CDR1, CDR2, and CDR3 shown in SEQ ID NO: 56 (preferably, a variable region comprising the amino acid sequence shown in SEQ ID NO: 56) in combination with a second variable region comprising the amino acid sequence derived from the amino acid sequence of CDR1, CDR2, and CDR3 shown in SEQ ID NO: 55 (preferably, a variable region comprising the amino acid sequence shown in SEQ ID NO: 55);

(XIV) an amino acid sequence comprising a first variable region comprising the amino acid sequence derived from the amino acid sequence of CDR1, CDR2, and CDR3 shown in SEQ ID NO: 137 (preferably, a variable region comprising the amino acid sequence shown in SEQ ID NO: 137) in combination with a second variable region comprising the amino acid sequence derived from the amino acid sequence of CDR1, CDR2, and CDR3 shown in SEQ ID NO: 138 (preferably, a variable region comprising the amino acid sequence shown in SEQ ID NO: 138);

(XV) an amino acid sequence comprising a first variable region comprising the amino acid sequence derived from the amino acid sequence of CDR1, CDR2, and CDR3 shown in SEQ ID NO: 139 (preferably, a variable region comprising the amino acid sequence shown in SEQ ID NO: 139) in combination with a second variable region comprising the amino acid sequence derived from the amino acid sequence of CDR1, CDR2, and CDR3 shown in SEQ ID NO: 140 (preferably, a variable region comprising the amino acid sequence shown in SEQ ID NO: 140);

(XVI) an amino acid sequence comprising a first variable region comprising the amino acid sequence derived from the amino acid sequence of CDR1, CDR2, and CDR3 shown in SEQ ID NO: 141 (preferably, a variable region comprising the amino acid sequence shown in SEQ ID NO: 141) in combination with a second variable region comprising the amino acid sequence derived from CDR1, CDR2, and CDR3 of SEQ ID NO: 142 (preferably, a variable region comprising the amino acid sequence shown in SEQ ID NO: 142);

(XVII) an amino acid sequence comprising a first variable region comprising the amino acid sequence derived from the amino acid sequence of CDR1, CDR2, and CDR3 shown in SEQ ID NO: 143 (preferably, a variable region comprising the amino acid sequence shown in SEQ ID NO: 143) in combination with a second variable region comprising the amino acid sequence derived from the amino acid sequence of CDR1, CDR2, and CDR3 shown in SEQ ID NO: 144 (preferably, a variable region comprising the amino acid sequence shown in SEQ ID NO: 144); and

(XVIII) an amino acid sequence comprising a first variable region comprising the amino acid sequence derived from the amino acid sequence of CDR1, CDR2, and CDR3 shown in SEQ ID NO: 145 (preferably, a variable region comprising the amino acid sequence shown in SEQ ID NO: 145) in combination with a second variable region comprising the amino acid sequence derived from the amino acid sequence of CDR1, CDR2, and CDR3 shown in SEQ ID NO: 146 (preferably, a variable region comprising the amino acid sequence shown in SEQ ID NO: 146).

The polypeptide having a site binding to the inhibitory KIR2DL1 and binding to the inhibitory KIR2DL1 according to an aspect of the present invention is, for example, a polypeptide comprising the amino acid sequence (P) (preferably, a monoclonal antibody, an antigen-binding fragment, scFv consisting of the amino acid sequence, or a membrane protein comprising such scFv in an extracellular domain):

(P) an amino acid sequence comprising a first variable region comprising the CDR1-, CDR2-, and CDR3-derived amino acid sequence shown in SEQ ID NO: 31 (preferably, a variable region comprising the amino acid sequence shown in SEQ ID NO: 31) in combination with a second variable region comprising the amino acid sequence derived from the amino acid sequence of CDR1, CDR2, and CDR3 shown in SEQ ID NO: 32 (preferably, a variable region comprising the amino acid sequence shown in SEQ ID NO: 32).

The polypeptide having a site binding to the inhibitory KIR2DL1 and binding to the inhibitory KIR2DL1 according to another aspect of the present invention is, for example, a polypeptide comprising an amino acid sequence according to any of (CI) to (CXV) below (preferably, a monoclonal antibody, an antigen-binding fragment, scFv consisting of the amino acid sequence, or a membrane protein comprising such scFv in an extracellular domain):

(CI) an amino acid sequence comprising a first variable region comprising the amino acid sequence derived from the amino acid sequence of CDR1, CDR2, and CDR3 shown in SEQ ID NO: 107 (preferably, a variable region comprising the amino acid sequence shown in SEQ ID NO: 107) in combination with a second variable region comprising the amino acid sequence derived from the amino acid sequence of CDR1, CDR2, and CDR3 shown in SEQ ID NO: 108 (preferably, a variable region comprising the amino acid sequence shown in SEQ ID NO: 108);

(CII) an amino acid sequence comprising a first variable region comprising the amino acid sequence derived from the amino acid sequence of CDR1, CDR2, and CDR3 shown in SEQ ID NO: 109 (preferably, a variable region comprising the amino acid sequence shown in SEQ ID NO: 109) in combination with a second variable region comprising the amino acid sequence derived from the amino acid sequence of CDR1, CDR2, and CDR3 shown in SEQ ID NO: 110 (preferably, a variable region comprising the amino acid sequence shown in SEQ ID NO: 110);

(CIII) an amino acid sequence comprising a first variable region comprising the amino acid sequence derived from the amino acid sequence of CDR1, CDR2, and CDR3 shown in SEQ ID NO: 111 (preferably, a variable region comprising the amino acid sequence shown in SEQ ID NO: 111) in combination with a second variable region comprising the amino acid sequence derived from the amino acid sequence of CDR1, CDR2, and CDR3 shown in SEQ ID NO: 112 (preferably, a variable region comprising the amino acid sequence shown in SEQ ID NO: 112);

(CIV) an amino acid sequence comprising a first variable region comprising the amino acid sequence derived from the amino acid sequence of CDR1, CDR2, and CDR3 shown in SEQ ID NO: 113 (preferably, a variable region comprising the amino acid sequence shown in SEQ ID NO: 113) in combination with a second variable region comprising the amino acid sequence derived from the amino acid sequence of CDR1, CDR2, and CDR3 shown in SEQ ID NO: 114 (preferably, a variable region comprising the amino acid sequence shown in SEQ ID NO: 114);

(CV) an amino acid sequence comprising a first variable region comprising the amino acid sequence derived from the amino acid sequence of CDR1, CDR2, and CDR3 shown in SEQ ID NO: 115 (preferably, a variable region comprising the amino acid sequence shown in SEQ ID NO: 115) in combination with a second variable region comprising the amino acid sequence derived from the amino acid sequence of CDR1, CDR2, and CDR3 shown in SEQ ID NO: 116 (preferably, a variable region comprising the amino acid sequence shown in SEQ ID NO: 116);

(CVI) an amino acid sequence comprising a first variable region comprising the amino acid sequence derived from the amino acid sequence of CDR1, CDR2, and CDR3 shown in SEQ ID NO: 117 (preferably, a variable region comprising the amino acid sequence shown in SEQ ID NO: 117) in combination with a second variable region comprising the amino acid sequence derived from the amino acid sequence of CDR1, CDR2, and CDR3 shown in SEQ ID NO: 118 (preferably, a variable region comprising the amino acid sequence shown in SEQ ID NO: 118);

(CVII) an amino acid sequence comprising a first variable region comprising the amino acid sequence derived from the amino acid sequence of CDR1, CDR2, and CDR3 shown in SEQ ID NO: 119 (preferably, a variable region comprising the amino acid sequence shown in SEQ ID NO: 119) in combination with a second variable region comprising the amino acid sequence derived from the amino acid sequence of CDR1, CDR2, and CDR3 shown in SEQ ID NO: 120 (preferably, a variable region comprising the amino acid sequence shown in SEQ ID NO: 120);

(CVIII) an amino acid sequence comprising a first variable region comprising the amino acid sequence derived from the amino acid sequence of CDR1, CDR2, and CDR3 shown in SEQ ID NO: 121 (preferably, a variable region comprising the amino acid sequence shown in SEQ ID NO: 121) in combination with a second variable region comprising the amino acid sequence derived from the amino acid sequence of CDR1, CDR2, and CDR3 shown in SEQ ID NO: 122 (preferably, a variable region comprising the amino acid sequence shown in SEQ ID NO: 122);

(CVIIII) an amino acid sequence comprising a first variable region comprising the amino acid sequence derived from the amino acid sequence of CDR1, CDR2, and CDR3 shown in SEQ ID NO: 123 (preferably, a variable region comprising the amino acid sequence shown in SEQ ID NO: 123) in combination with a second variable region comprising the amino acid sequence derived from the amino acid sequence of CDR1, CDR2, and CDR3 shown in SEQ ID NO: 124 (preferably, a variable region comprising the amino acid sequence shown in SEQ ID NO: 124);

(CX) an amino acid sequence comprising a first variable region comprising the amino acid sequence derived from the amino acid sequence of CDR1, CDR2, and CDR3 shown in SEQ ID NO: 125 (preferably, a variable region comprising the amino acid sequence shown in SEQ ID NO: 125) in combination with a second variable region comprising the amino acid sequence derived from the amino acid sequence of CDR1, CDR2, and CDR3 shown in SEQ ID NO: 126 (preferably, a variable region comprising the amino acid sequence shown in SEQ ID NO: 126);

(CXI) an amino acid sequence comprising a first variable region comprising the amino acid sequence derived from the amino acid sequence of CDR1, CDR2, and CDR3 shown in SEQ ID NO: 127 (preferably, a variable region comprising the amino acid sequence shown in SEQ ID NO: 127) in combination with a second variable region comprising the amino acid sequence derived from the amino acid sequence of CDR1, CDR2, and CDR3 shown in SEQ ID NO: 128 (preferably, a variable region comprising the amino acid sequence shown in SEQ ID NO: 128);

(CXII) an amino acid sequence comprising a first variable region comprising the amino acid sequence derived from the amino acid sequence of CDR1, CDR2, and CDR3 shown in SEQ ID NO: 129 (preferably, a variable region comprising the amino acid sequence shown in SEQ ID NO: 129) in combination with a second variable region comprising the amino acid sequence derived from the amino acid sequence of CDR1, CDR2, and CDR3 shown in SEQ ID NO: 130 (preferably, a variable region comprising the amino acid sequence shown in SEQ ID NO: 130);

(CXIII) an amino acid sequence comprising a first variable region comprising the amino acid sequence derived from the amino acid sequence of CDR1, CDR2, and CDR3 shown in SEQ ID NO: 131 (preferably, a variable region comprising the amino acid sequence shown in SEQ ID NO: 131) in combination with a second variable region comprising the amino acid sequence derived from the amino acid sequence of CDR1, CDR2, and CDR3 shown in SEQ ID NO: 132 (preferably, a variable region comprising the amino acid sequence shown in SEQ ID NO: 132);

(CXIV) an amino acid sequence comprising a first variable region comprising the amino acid sequence derived from the amino acid sequence of CDR1, CDR2, and CDR3 shown in SEQ ID NO: 133 (preferably, a variable region comprising the amino acid sequence shown in SEQ ID NO: 133) in combination with a second variable region comprising the amino acid sequence derived from the amino acid sequence of CDR1, CDR2, and CDR3 shown in SEQ ID NO: 134 (preferably, a variable region comprising the amino acid sequence shown in SEQ ID NO: 134); and

(CXV) an amino acid sequence comprising a first variable region comprising the amino acid sequence derived from the amino acid sequence of CDR1, CDR2, and CDR3 shown in SEQ ID NO: 135 (preferably, a variable region comprising the amino acid sequence shown in SEQ ID NO: 135) in combination with a second variable region comprising the amino acid sequence derived from the amino acid sequence of CDR1, CDR2, and CDR3 shown in SEQ ID NO: 136 (preferably, a variable region comprising the amino acid sequence shown in SEQ ID NO: 136).

CDR1, CDR2, and CDR3 in the heavy chain variable region and CDR1, CDR2, and CDR3 in the light chain variable region may have amino acid mutation, provided that reactivity thereof with the antigens would not be lost. An example of such mutation is substitution of 1 or 2 amino acids in the amino acid sequence of CDR, and a preferable example is substitution of 1 or 2 amino acids at the C and/or N terminus of the amino acid sequence of CDR.

In the polypeptides (I) to (XIII), (P), and (CI) to (CXV) described above, structures other than the variable regions can be adequately selected in accordance with forms of polypeptides to be selected (e.g., a monoclonal antibody or an antigen-binding fragment). In the case of a single-stranded polypeptide, the first variable region, the linker sequence, and the second variable region may be positioned in that order from the N terminus, or the second variable region, the linker sequence, and the first variable region may be positioned in that order from the N terminus. In the case of a polypeptide divided into a heavy chain and a light chain, the first variable region may be positioned in the heavy chain and the second variable region may be positioned in the light chain. Alternatively, the first variable region may be positioned in the light chain and the second variable region may be positioned in the heavy chain.

An aspect of the present invention provides a substance having the agonist activity specific to inhibitory KIR. The "agonist activity specific to inhibitory KIR" is the agonist activity on inhibitory KIR, and the agonist activity on activating KIR corresponding to the inhibitory KIR is 70% or lower, 60% or lower, 50% or lower, 40% or lower, 30% or lower, 20% or lower, 15% or lower, 10% or lower, or 5% or lower than the agonist activity on the inhibitory KIR. It is the most preferable that the substance would not substantially exert agonist activity on activating KIR. The term "activating KIR corresponding to inhibitory KIR" refers to activating KIR comprising an amino acid sequence of the extracellular domain exhibiting high homology to the amino acid sequence of the extracellular domain of the target inhibitory KIR. Examples of activating KIR corresponding to KIR2DL2/3 include KIR2DS1, KIR2DS2, and KIR2DS4. Activating KIR corresponding to KIR2DL1 is KIR2DS1, activating KIR corresponding to KIR2DL2/3 is KIR2DS2, and activating KIR corresponding to KIR3DL1 and KIR3DL2 is KIR3DS1.

Such antibody or the like can be obtained by, for example, bringing the antibody binding to the inhibitory KIR and exerting agonist activity thereon obtained by the method described above or an antigen-binding fragment into contact with NK cells expressing the activating KIR corresponding thereto, assaying the cytocidal activity of the NK cells on the target cells, and selecting the antibody or the antigen-binding fragment exhibiting the activity level equivalent to or lower than the given activity level. In order to attain such agonist activity, the antibody or an antigen-binding fragment may be added as a soluble protein or expressed in the form of a membrane protein described below on the target cell surface. In order to obtain such antibody or the like, an antibody binding to inhibitory KIR and exhibiting a low binding capability to activating KIR can be obtained by, for example, the phage display method comprising expressing an antibody variable region as a single-stranded antibody (scFv) on the phage surface, and selecting the phage binding to the antigen (Nature Biotechnology, 2005, 23, (9), pp. 1105-1116). According to a method of obtaining a binder that binds specifically to inhibitory KIR by the phage display method, for example, a phage that expresses scFv binding to inhibitory KIR but not binding to activating KIR can be selected by collecting and concentrating phages expressing scFv binding to inhibitory KIR from the human antibody phage library (positive selection) and removing phages expressing scFv that binds to activating KIR from the phage mixture (depletion). As inhibitory or activating KIR, a recombinant protein/peptide consisting of an amino acid sequence of an extracellular region of a molecule, which comprises a partial sequence important for the agonist activity of inhibitory KIR, can be employed. In the step of collecting and concentrating phages or the step of removing phages, various methods used for selecting a substance having a binding capability or affinity can be employed. For example, one of two molecules exhibiting a high binding capability (e.g., biotin in combination with avidin) is allowed to bind to a KIR fragment (labeled KIR), and the other molecule is allowed to bind to a carrier (e.g., a magnetic bead). After the phage solution is mixed with the labeled KIR, the mixture is brought into contact with the carrier. Thus, phages binding to the labeled KIR are collected and concentrated on the carrier side. In positive selection, inhibitory KIR is labeled, and phages concentrated and collected on the carrier side are obtained. In depletion, in contrast, activating KIR is labeled, phages in the solution that did not bind to a carrier are collected, and phages that do not bind to activating KIR can be thus obtained. Alternatively, an excess amount of unlabeled activating KIR fragments may be added in the step of positive selection. Thus, phages exhibiting a high binding capability to the labeled KIR can be collected and concentrated (deselection). Such method may be performed a plurality of times or several methods may be performed in combination. Thus, it is highly likely that scFv with a high binding capability to inhibitory KIR is achieved.

Specifically, an antibody or the like that binds to inhibitory KIR but does not bind to activating KIR can be prepared by the method comprising the step (1) or (2) described below. The present invention provides such method of production, a substance produced by such method, and the like. The term "display body" used herein refers to a gene-carrying body of a cell having a function of displaying a protein or peptide encoded by the gene integrated with the aid of a vector on the lipid membrane surface, and examples thereof include a phage, a yeast, and a cell. When a phage is used as a display body, a known technique, such as the phage display method or the phage panning method described above, can be employed. As a method involving the use of a yeast as a display body, a method known as a yeast display method (see, for example, Lukas Roth, et al., Methods Mol. Biol., 2020, Vol. 207, pp. 173-189, doi: 10.1007/978-1-4939-9853-1_10) can be employed.

(1) A method for producing a substance having activity of binding to human inhibitory KIR and inhibiting a NK cell comprises:

step A: a step of obtaining a population of display bodies displaying an antigen-binding site binding to inhibitory KIR comprising mixing a labeled soluble protein comprising an extracellular domain of inhibitory KIR in a suspension containing a population of polyclonal display bodies including display bodies displaying the antigen-binding site binding to the inhibitory KIR on the surface, bringing the suspension into contact with a carrier comprising, immobilized thereon, a substance binding specifically to the label part of said labeled soluble protein, and collecting the display bodies bound to the carrier;

step B: a step of removing display bodies displaying the antigen-binding site binding to activating KIR from the population of display bodies obtained in Step A, comprising mixing a labeled soluble protein comprising an extracellular domain of activating KIR corresponding to the inhibitory KIR in the suspension containing the population of display bodies obtained in step A, bringing the suspension into contact with a carrier comprising, immobilized thereon, a substance binding to the label part of said labeled foluble protein, and collecting the display bodies not bound to the carrier;

step C: a step of identifying a sequence of a gene encoding the antigen-binding site by performing genetic analysis of the display bodies obtained the step B; and

step D: a step of expressing a gene comprising a gene encoding the identified antigen-binding site integrated into an expression format of the antibody or the antigen-binding fragment in a cell.

(2) A method for producing a substance having activity of binding to human inhibitory KIR and inhibiting a NK cell comprises:

step A: a step of obtaining a population of display bodies displaying an antigen-binding site binding specifically to inhibitory KIR comprising mixing a labeled soluble protein comprising an extracellular domain of inhibitory KIR and an unlabeled soluble protein comprising an extracellular domain of activating KIR corresponding to the inhibitory KIR in a suspension containing a population of polyclonal display bodies including display bodies displaying the antigen-binding site binding to the inhibitory KIR on the surface, bringing the cell-containing solution into contact with a carrier comprising, immobilized thereon, a substance binding specifically to the labeled part; and collecting the display bodies bound to the carrier;

step B: a step of identifying a sequence of a gene encoding the antigen-binding site by performing genetic analysis of the display bodies obtained by the method comprising the steps described above; and

step C: a step of expressing a gene comprising a gene encoding the identified antigen-binding site integrated into an expression format of the antibody or the antigen-binding fragment in a cell.

Each of the steps A and B of the method (1) and the step A of the method (2) may be performed a plurality of times, or both of such steps may be performed in combination.

In the method (1) or (2), a combination of a label and a label-binding substance may be any of various known substances. An example thereof is a combination of biotin and avidin.

Concerning the antibody expressed in the method (1) or (2) or an antigen-binding fragment (a soluble or membrane protein), the method may further comprise a step of selecting the substance having an agonist activity on inhibitory KIR in accordance with the method described herein.

In the method described above, inhibitory KIR may be KIR2DL2 or KIR2DL3, and activating KIR corresponding thereto may be KIR2DS1, KIR2DS2, or KIR2DS4. Alternatively, inhibitory KIR may be KIR3DL1, and activating KIR corresponding thereto may be KIR3DS1. Further, inhibitory KIR may be KIR2DL1, and activating KIR corresponding thereto may be KIR2DS1, KIR2DS2, or KIR2DS4.

A population of polyclonal display bodies displaying an antigen-binding site binding to inhibitory KIR on the surface can be obtained or produced by known techniques. For example, phage libraries that display various variable regions used for the phage display method used to obtain antibodies or the like or yeast libraries used for the yeast display method can be used. According to another aspect, an animal is immunized with an antigen protein comprising an extracellular domain of inhibitory KIR, a cell that carries the gene of an antigen-specific antibody, such as a B cell, is collected from the spleen cell of an animal that produces an antibody against the antigen or an antigen-binding fragment, a sequence of a gene encoding a variable region of the antibody gene of the cell is amplified, and the amplified sequence is integrated into a vector that displays the variable region in a given form on the lipid membrane surface. Thus, a library of display bodies can be produced. Such method is described in, for example, David R. Maass et al., J. Immunol. Methods, Jul 31, 2007; 324 (1-2): 13-25; Lukas Roth et al., Methods Mol. Biol., 2020; 2070: 173-189. When a display body is a phage, a phagemid vector is used.

In the method (1) or (2), an antibody or an antigen-binding fragment can be expressed in various cells, such as animal cells or *E. coli* cells, in accordance with the intended use thereof. When an antibody or an antigen-binding fragment is expressed in the form of a membrane protein, for example, target cells used for agonist activity assay or cells for cell transplantation can be used (examples thereof include, but are not limited to, blood cells, T cells, and pluripotent stem cells).

Examples of substances having agonist activity specific to inhibitory KIR2DL2/3 include scFv according to any of (i) to (xii) described above and the polypeptide according to any of (I) to (XII) described above. Examples of substances having agonist activity specific to inhibitory KIR3DL1 include the scFv (xiii) described above and the polypeptide (XIII) described above.

Examples of substances having agonist activity specific to inhibitory KIR2DL1 include scFv according to any of (ci) to (cxv) described above and the polypeptide according to any of (CI) to (CXV) described above.

Another aspect of the present invention provides a polypeptide comprising, as a site binding to inhibitory KIR, a binding site derived from a heavy chain variable region and a light chain variable region included in a monoclonal antibody or an antigen-binding fragment (e.g., a competitively binding antibody) competing with a monoclonal antibody binding to inhibitory KIR or an antigen-binding fragment when binding to inhibitory KIR. Such competitively binding antibody or the like recognizes and binds to the same epitope as that of the monoclonal antibody or scFv described above, an epitope that overlaps with the aforementioned epitope, or an epitope that is positioned in the vicinity of the aforementioned epitope. Accordingly, it is highly likely that such competitively binding antibody or the like exhibits agonist activity equivalent to the agonist activity of the monoclonal antibody or an antigen-binding fragment described above on the inhibitory KIR. Accordingly, a polypeptide having agonist activity of interest on inhibitory KIR can be obtained by obtaining a plurality of antibodies or antigen-binding fragments competitively binding to the antibodies or antigen-binding fragments verified to have the agonist activity in the examples herein from among antibodies against inhibitory KIR and selecting antibodies or antigen-binding fragments exhibiting agonist activity of interest (in particular, agonist activity specific to inhibitory KIR).

For example, the polypeptide comprising the site binding to inhibitory KIR2DL2 and/or KIR2DL3 of the present invention comprises, when binding to inhibitory KIR2DL2 and/or KIR2DL3, a binding site derived from a heavy chain variable region and a light chain variable region of a monoclonal antibody or an antigen-binding fragment competing with scFv consisting of the amino acid sequence according to any or (i) to (xii) above, Lirilumab, or Pan2D.

For example, the polypeptide comprising the site binding to KIR2DL1 comprises, when binding to KIR2DL1, a binding site derived from a heavy chain variable region and a light chain variable region of a monoclonal antibody or an antigen-binding fragment competing with Pan2D or (P) described above.

For example, the polypeptide comprising the site binding to KIR2DL1 comprises, when binding to KIR2DL1, a binding site derived from a heavy chain variable region and a light chain variable region of a monoclonal antibody or an antigen-binding fragment competing with scFv consisting of an amino acid sequence according to any of (ci) to (cxv) above.

For example, the polypeptide comprising the site binding to inhibitory KIR3DL1 of the present invention comprises, when binding to inhibitory KIR3DL1, a binding site derived from a heavy chain variable region and a light chain variable region of a monoclonal antibody or an antigen-binding fragment competing with scFv consisting of an amino acid sequence according to any of (xiii) to (xviii) above.

The epitope of the inhibitory KIR that is recognized by the polypeptide comprising the site binding to inhibitory KIR of the present invention can be analyzed by X-ray crystallography of the complex of the polypeptide comprising the site binding to inhibitory KIR and the inhibitory KIR. The epitope analysis by X-ray crystallography can be performed by, for example, crystallizing the complex of the polypeptide comprising the site binding to inhibitory KIR of the present invention and KIR and performing X-ray structure analysis. In this case, the Fab fragment can be used as the polypeptide comprising the site binding to inhibitory KIR. In the analysis, for example, a ribbon model may be obtained, and amino acid residues located in the vicinity of the Fab fragment, such as up to 4Å distance away therefrom, may be identified, and the identified amino acid residues may be determined to be the epitope-constituting amino acids.

Amino acids constituting an epitope of the inhibitory KIR2DL2 when binding to a2DL2/3-DS10 (VH: SEQ ID NO: 5; VL: SEQ ID NO: 52) are, for example, Glu42 (amino acid 21 in SEQ ID NO: 6), His61 (amino acid 40 in SEQ ID NO: 6), Lys65 (amino acid 44 in SEQ ID NO: 6), Phe66 (amino acid 45 in SEQ ID NO: 6), Lys67 (amino acid 46 in SEQ ID NO: 6), Asp68 (amino acid 47 in SEQ ID NO: 6), Thr69 (amino acid 48 in SEQ ID NO: 6), Leu70 (amino acid 49 in SEQ ID NO: 6), Ser86 (amino acid 65 in SEQ ID NO: 6), Ile87 (amino acid 66 in SEQ ID NO: 6), Gly88 (amino acid 67 in SEQ ID NO: 6), Pro89 (amino acid 68 in SEQ ID NO: 6), Met91 (amino acid 70 in SEQ ID NO: 6), Gln92 (amino acid 71 in SEQ ID NO: 6), Asp93 (amino acid 72 in SEQ ID NO: 6), Leu94 (amino acid 73 in SEQ ID NO: 6), Tyr101 (amino acid 80 in SEQ ID NO: 6), and Leu111 (amino acid 90 in SEQ ID NO: 6). The amino acids different from those in the amino acid sequence of the extracellular domain of activating KIR2DS1 are Lys65, Lys67, Gly88, Pro89, and Met91, the amino acid different from that in the amino acid sequence of the extracellular domain of activating KIR2DS2 is Phe66, and the amino acids different from those in the amino acid sequence of the extracellular domain of activating KIR2DS4 are Lys67, Asp68, Gln92, and Asp93. Accordingly, it is important that the KIR2DL2/3-specific binder interacts with such amino acids and amino acids in the vicinity thereof.

In the analysis of the interaction between a2DL2/3-DS10 and KIR2DL2, Tyr107 in the VH chain of a2DL2/3-DS10 (amino acid 107 in SEQ ID NO: 51; hereafter referred to as "DS10-VH-Y107") was found to form a hydrogen bond with Asp68 of KIR2DL2 and be positioned adjacent to Phe66. In contrast, the analysis of the interaction between a2DL2/3-DS10 and activating KIR corresponding thereto indicates that DS10-VH-Y107 would form a weak hydrogen bond or would not form a hydrogen bond with Asn68 (amino acid 47 in SEQ ID NO: 14) of KIR2DS4 corresponding to Asp68 and that DS10-VH-Y107 would not be positioned adjacent to Tyr66 (Tyr45 in SEQ ID NO: 12) of KIR2DS2 corresponding to Phe66 because of the steric hindrance therewith. This may prevent a2DL2/3-DS10 from binding to KIR2DS2 and KIR2DS4. Specifically, an amino acid in the variable region adjacent to Phe66 and Asp68 on KIR2DL2 would cause the steric hindrance when the amino acid corresponding to Phe66 of the receptor is Tyr, such amino acid is capable of forming a hydrogen bond with Asp, but its ability to form a hydrogen bond with Asn is weak or lost. Such amino acid is preferably Tyr.

In the analysis of the interaction between a2DL2/3-DS10 and KIR2DL2, Tyr106 in the VH chain of a2DL2/3-DS10 (Tyr: amino acid 106 in SEQ ID NO: 51; hereafter referred to as "DS10-VH-Y106") was found to form a hydrogen bond with a carbonyl group in the main chain of Pro89 of KIR2DL2. In contrast, the analysis of the interaction between a2DL2/3-DS10 and activating KIR corresponding thereto indicates that DS10-VH-Y106 would not form a hydrogen bond or would form a weak hydrogen bond with a carbonyl group in the main chain of Arg89 (Arg68 in SEQ ID NO: 10) of KIR2DS1 corresponding to Pro89. This may prevent a2DL2/3-DS10 from binding to KIR2DS1. Specifically, an amino acid in the variable region adjacent to Pro89 of KIR2DL2 is capable of forming a hydrogen bond with Pro, but its ability to form a hydrogen bond with Arg is weak or lost. Such amino acid is preferably Tyr.

In the analysis of the interaction between a2DL2/3-DS10 and KIR2DL2, Gly104 in the VH chain of a2DL2/3-DS10 (Gly: amino acid 104 in SEQ ID NO: 51; hereafter referred to as "DS10-VH-G104") was found to form a hydrogen bond with an imino group in the main chain of Gly88 of KIR2DL2 at the carbonyl group in its main chain. In contrast, the analysis of the interaction between a2DL2/3-DS10 and activating KIR corresponding thereto indicates that DS10-VH-G104 would not form a hydrogen bond or would form a weak hydrogen bond with Ser88 (Ser67 in SEQ ID NO: 10) of KIR2DS1 corresponding to Gly88 between the main chains because of the steric hindrance. This may prevent a2DL2/3-DS10 from binding to KIR2DS1. Specifically, an amino acid in the variable region adjacent to Gly88 on KIR2DL2 can form a hydrogen bond with an imino group in the main chain of Gly, but its ability to form hydrogen bond with an imino group in the main chain of Ser is weak or lost. Such amino acid is preferably Gly.

In the analysis of the interaction between a2DL2/3-DS10 and KIR2DL2, Thr94 in the VL chain of a2DL2/3-DS10 (Thr: amino acid 94 in SEQ ID NO: 52; hereafter referred to as "DS10-VL-T94") was found to form a hydrogen bond with a side chain of Asp93 on KIR2DL2 at the imino group in the main chain. In contrast, the analysis of the interaction between a2DL2/3-DS10 and activating KIR corresponding thereto indicates that DS10-VL-T94 would not form a hydrogen bond or would form a weak hydrogen bond with Val93 of KIR2DS4 corresponding to Asp93 (Val72 in SEQ ID NO: 14). This may prevent a2DL2/3-DS10 from binding KIR2DS4. This specificity for the interaction with KIR2DL2 was found to be maintained when DS10-VL-T94 was substituted with Asn, Gly, His, Ile, Ser, Val, or Ala. Specifically, an amino acid in the variable region adjacent to Asp93 on KIR2DL2 is capable of forming a hydrogen bond with a side chain of Asp, but its ability to form a hydrogen bond with Val is weak or lost. Such amino acid is preferably Thr, Asn, Gly, His, Ile, Ser, Val, or Ala, and more preferably Thr.

The KIR2DL2- or KIR2DL3-specific agonist binder according to an aspect of the present invention is a substance (i.e., an antibody or an antigen-binding fragment) comprising a variable region that interacts with 1 to 6 (preferably 3 to 6, more preferably 4 to 6, and further preferably 5 or 6) amino acids including amino acid 47 in a region of amino acids 44 to 49, 2 to 4 (preferably 2, 3, or 4) amino acids including amino acids 67 and 68 in a region of amino acids 65 to 68, and 1 to 4 (preferably 2, 3, or 4) amino acids including amino acid 72 in a region of amino acids 70 to 73 in the amino acid sequence of KIR2DL2 shown in SEQ ID NO: 6. Such substance is preferably a binder comprising a variable region that interacts with amino acids in a region of amino acids 45 to 47, a region of amino acids 45 to 48, a region of amino acids 44 to 47, a region of amino acids 45 to 48, a region of amino acids 44 to 48, a region of amino acids 45 to 49, a region of amino acids 44 to 49, a region of amino acids 67 to 68, a region of amino acids 66 to 68, a region of amino acids 65 to 68, a region of amino acids 72 to 73, a region of amino acids 71 to 73, or a region of amino acids 70 to 73 in the amino acid sequence of KIR2DL2 shown in SEQ ID NO: 6.

The KIR2DL2-specific agonist according to an aspect of the present invention is an antibody or an antigen-binding fragment in which an amino acid that interacts with Asp47 in SEQ ID NO: 6 is Tyr, an amino acid that interacts with Gly67 in SEQ ID NO: 6 is Gly, an amino acid that interacts with Pro68 in SEQ ID NO: 6 is Tyr, and an amino acid that interacts with Asp72 in SEQ ID NO: 6 is Thr, Asn, Gly, His, Ile, Ser, Val, or Ala in a variable region of KIR2DL2. In such antibody or antigen-binding fragment, an amino acid located adjacent to Phe45 in SEQ ID NO: 6 is preferably Tyr in addition to the amino acids indicated above, and all the amino acids are more preferably located in CDR3 of the variable region. Such antibody or antigen-binding fragment preferably has, as the first variable region, CDR3 comprising the amino acid sequence consisting of amino acids 97 to 112 in SEQ ID NO: 51 and, as the second variable region, CDR3 comprising an amino acid sequence derived from the amino acid sequence comprising amino acids 89 to 97 in SEQ ID NO: 52 by substitution of Thr94 with Asn, Gly, His, Ile, Ser, Val, or Ala. Such antibody or antigen-binding fragment more preferably has, as the first variable region, the amino acid sequence shown in SEQ ID NO: 51 and, as the second variable region, an amino acid sequence derived from the amino acid sequence shown in SEQ ID NO: 52 by substitution of Thr94 with Asn, Gly, His, Ile, Ser, Val, or Ala.

In an antigen-binding test, polypeptides comprising sites binding to inhibitory KIR are allowed to compete with each other, and epitopes can be analyzed by performing a competition test. For example, the Fab fragment of a polypeptide that is different from the K562 cell line expressing scFv of the polypeptide having a site binding to inhibitory KIR is mixed with recombinant KIR, and binding of the K562 cell line expressing scFv to recombinant KIR may be allowed to compete with that of the Fab fragment. Whether or not binding of the K562 cell line expressing scFv to recombinant KIR would be inhibited by competition may be examined, and the result thereof may be used as the indicator to evaluate the epitope site of each binder. In the present invention, the term "recombinant" is identical to the term "soluble."

The polypeptide having a site binding to inhibitory KIR of the present invention encompasses other polypeptides that recognize epitopes recognized by the polypeptides each having a site binding to inhibitory KIR analyzed by the method described above. Other polypeptides each compete with the polypeptide having a site binding to inhibitory KIR of the present invention when binding to inhibitory KIR and have inhibitory selectivity.

The membrane protein of the present invention has an extracellular domain comprising a site binding to inhibitory KIR and a transmembrane domain. When the membrane protein is expressed in a cell, accordingly, a polypeptide that has a site binding to inhibitory KIR and recognizes and binds to the inhibitory KIR can be present on the cell membrane surface. The membrane protein of the present invention may further comprise an intracellular domain.

The polypeptide having a site binding to inhibitory KIR and binding to inhibitory KIR is linked to a transmembrane domain via an extracellular linker domain.

The term "domain" used herein refers to a region in a polypeptide, which folds independently of other regions to form a specific structure. In a membrane protein, the polypeptide having a site binding to inhibitory KIR and binding to inhibitory KIR can be referred to as a binding domain. When a binding domain comprises the first variable region and the second variable region derived from the heavy chain variable region and the light chain variable region of the monoclonal antibody or an antigen-binding fragment described in (1) above, the linker sequence that can be used for the scFv described above can be used for such two variable regions. The first variable region, the linker sequence, and the second variable region may be positioned in that order from the N terminus of the polypeptide, or the second variable region, the linker sequence, and the first variable region may be positioned in that order from the N terminus.

A transmembrane domain has affinity to a lipid bilayer that constitutes a cell membrane. Examples thereof include, but are not limited to, transmembrane domains of HLA, CD28, CD3, CD8, CD19, CD4, and 4-1BB, and transmembrane sequences derived from various naturally-occurring membrane proteins or artificially designed transmembrane sequences can be used. A CD8α-derived transmembrane domain (SEQ ID NO: 27) is preferable.

As extracellular linkers, full-length or partial sequences of extracellular domains of HLA, CD28, CD8, CD19, CD3, CD4, and 4-1BB are exemplified, although extracellular linkers are not limited thereto. For example, linker sequences derived from various naturally-occurring proteins, hinge sequences such as a hinge sequence of immunoglobulin, and artificially designed flexible linker sequences can be used. Preferable examples include a CD8α-derived hinge sequence comprising 45 amino acids (SEQ ID NO: 26) and a CD8α-derived hinge sequence comprising 66 amino acids (SEQ ID NO: 24).

An intracellular domain is necessary to maintain stability of scFv. A part of an intracellular domain (preferably comprising up to 10 amino acids, up to 9 amino acids, up to 8 amino acids, up to 7 amino acids, up to 6 amino acids, up to 5 amino acids, up to 4 amino acids, up to 3 amino acids, up to 2 amino acids, or 1 amino acid) that is not involved in intracellular signal transmission of HLA, CD28, CD3, CD8, CD19, CD4, or 4-1BB can be used. When it is necessary to accelerate activation or inhibition of intracellular signals upon binding to KIR, a part of an intracellular domain that is involved in intracellular signal transmission can be used.

Examples of the membrane protein of the present invention include a membrane protein having an extracellular domain comprising the site binding to KIR2DL2 and/or KIR2DL3 and a transmembrane domain, a membrane protein having an extracellular domain comprising the site binding to KIR3DL1 and a transmembrane domain, and a membrane protein having an extracellular domain comprising the site binding to KIR2DL1 and a transmembrane domain.

(3) A polypeptide in which the site binding to inhibitory KIR comprises a sequence derived from a site binding to inhibitory KIR of an HLA molecule, which is a naturally-occurring ligand of the inhibitory KIR, and a TCR-binding capability (T cell receptor) is lost or attenuated.

When inhibitory KIR is KIR2DL2 or KIR2DL3, for example, it is possible to use a polypeptide comprising a C1 epitope motif sequence contained in HLA-Cw1, Cw3, Cw7, Cw8, Cw9, Cw10, Cw12, Cw14, or Cw16 that belongs to the HLA-C1 type and HLA-B46 and B73 (in which amino acid 77 is S and amino acid 80 is N) and comprising a domain that is not recognized by TCR due to mutation or deletion of a region recognized by TCR of the HLA molecule.

When inhibitory KIR is KIR2DL1, for example, it is possible to use a polypeptide comprising a C2 epitope motif sequence contained in HLA-Cw2, Cw4, Cw5, Cw6, Cw15, or Cw17 that belongs to the HLA-C2 type (in which amino acid 77 is N and amino acid 80 is K) and comprising a domain that is not recognized by TCR due to mutation or deletion in a region recognized by TCR of the HLA molecule.

When inhibitory KIR is KIR3DL1, for example, it is possible to use a polypeptide comprising a Bw4 epitope motif sequence contained in HLA-B5, B27, B37, B38, B44, B49, B51, B52, B53, B57, B58, B59, B77, A23, A24, A25, or A32 that belongs to the HLA-Bw4 type (in which amino acid 77 is N, amino acid 80 is I or T, and amino acid 83 is R) and comprising a domain that is not recognized by TCR due to mutation or deletion in a region recognized by TCR of the HLA molecule. Such polypeptide may be in the form of a soluble ligand comprising an amino acid sequence including the epitope motif sequence or it may be in the form of a membrane protein comprising the epitope motif sequence in the extracellular region.

When inhibitory KIR is KIR3DL2, for example, a polypeptide comprising a domain that is not recognized by TCR due to mutation or deletion in free light chains with HLA-A3, A11, and B27 or a region recognized by TCR of the HLA molecule can be employed. Such polypeptide may be in the form of a soluble ligand or a membrane protein.

An example of a substance having an agonist activity on inhibitory KIR is a low-molecular-weight compound binding to inhibitory KIR and exhibiting an agonist activity on the KIR. Such compound can be selected from, for example, a compound library in accordance with the method for evaluating agonist activity on inhibitory KIR. In addition, compounds derived from the compound selected by the method described above on the basis of the structures thereof may be assayed in terms of the binding activity to the target inhibitory KIR or the agonist activity, the structure-activity relationship may be analyzed, and derivatives thereof may further be developed. Thus, compounds with higher binding capability and/or agonist activity can be obtained.

2. Method for producing substance having activity of binding to inhibitory KIR and inhibiting cytocidal activity of NK cell

The polypeptide having a site binding to inhibitory KIR and binding to inhibitory KIR of the present invention can be prepared in the form of a secretory protein (soluble protein) or a membrane protein by, for example, recombination, genome editing, *in vitro* translation, chemical synthesis, or peptide synthesis.

For example, a polynucleotide comprising a nucleotide sequence encoding an amino acid sequence of the polypeptide is introduced into a cell, the cell is cultured, and the polypeptide is collected from the culture product. Thus, the polypeptide of the present invention can be produced.

A nucleotide encoding an amino acid sequence of each domain may be linked to the polypeptide having a site binding to inhibitory KIR and binding to inhibitory KIR and the membrane protein having a transmembrane domain, and an element, such as a promoter, an enhancer, or a polyadenylation signal, may further be operably linked thereto. Nucleotide sequences each encoding an amino acid sequence of a domain are linked to each other, inserted into a vector, and expressed in a host cell, so that an extracellular linker domain is positioned between a polypeptide binding to inhibitory KIR and a transmembrane domain. When "operably linked" herein, an element is linked, so as to exert its functions. The nucleotides may be inserted into an expression vector, a host cell may be transformed with the aid of the vector, and the host cell may then be cultured to produce and collect the nucleotides. The vector may comprise a nucleotide encoding a signal peptide that accelerates secretion of the polypeptide from the host cell. In such a case, a nucleotide encoding a signal peptide is linked in-frame to the nucleotide encoding the polypeptide. The signal peptide may be removed after the polypeptide is produced, and the polypeptide can be obtained as a mature protein. As a signal peptide, for example, a CD8 signal peptide can be used.

An expression vector is not particularly limited, as long as it can replicate a gene of interest in animal, bacterial, yeast, or other host cells, and examples thereof include known plasmids and phages. Examples of a vector used to construct an expression vector include pcDNA™ (Thermo Fisher Scientific), Flexi® vector (Promega), pUC19, pUEX2 (Amersham), pGEX-4T, pKK233-2 (Pharmacia), and pMAM-neo (Clontech). As host cells, prokaryotic cells such as *Escherichia coli* and *Bacillus subtilis* and eukaryotic cells such as yeasts and animal cells can be used, with the use of eukaryotic cells being preferable. Examples of animal cells include the human embryonic kidney cell line HEK293 and the Chinese hamster ovary (CHO) cell. It is sufficient to introduce an expression vector into a host cell by a known method to transform the host cell. Examples of methods include an electroporation method, a calcium phosphate precipitation method, and a DEAE-dextran transfection method. The produced antibody can be purified by general protein isolation or purification methods. For example, affinity chromatography or other chromatography techniques, filtration, ultrafiltration, salting out, dialysis, and the like can be suitably selected and performed in combination.

3. Cell comprising substance having activity of binding to inhibitory KIR and inhibiting cytocidal activity of NK cell expressed on its surface and method for producing the same

A cell comprising a substance having activity of binding to inhibitory KIR and inhibiting the cytocidal activity of an NK cell expressed on the surface is preferably a cell in which the polypeptide having a site binding to inhibitory KIR and binding to inhibitory KIR described in 1. (2) above and a membrane protein having a transmembrane domain is expressed. A transmembrane domain of the membrane protein is present while passing through the cell membrane, and the polypeptide having a site binding to inhibitory KIR and binding to inhibitory KIR is present on the cell surface.

A nucleotide encoding a polypeptide having a site binding to inhibitory KIR and binding to inhibitory KIR may be introduced into such cell and expressed therein.

A nucleotide may be introduced into a cell by any common method without particular limitation. When a nucleotide is introduced with the aid of a vector, examples of vectors that can be used include, but are not particularly limited to, a lentivirus vector, a retrovirus vector, a foamy virus vector, and an adeno-associated virus vector.

When expressing the membrane protein, an expression control element, such as an enhancer or promoter, is linked to a nucleotide encoding the membrane protein, the resultant is introduced into an expression vector, and a cell is transformed with the aid of the expression vector to express the membrane protein in the cell. When introducing a nucleotide, it is preferable that an adequate promoter be operably linked to a site upstream of the nucleotide to be introduced. Examples of promoters that can be used include, but are not limited to, virus promoters, such as lentivirus, retrovirus, adenovirus, SV40 virus, and cytomegalovirus promoters, and promoters derived from mammalian cells.

When introducing a nucleotide encoding the membrane protein in a cell, an expression vector may comprise a selection marker gene. As a selection marker gene, a known marker gene, such as a thymidine kinase gene, can be used.

The present invention also encompasses DNA encoding the membrane protein and RNA, which is a transcription product of the DNA.

The membrane protein of the present invention may be expressed by genome editing. Examples of genome editing techniques that can be employed include, but are not limited to, various techniques, such as CRISPR-Cas, Zinc-Finger, and TaLEN. A genetically modified cell can be prepared by a genome editing technique. According to the method described in WO 2018/018534, for example, pluripotent stem cells, such as iPS cells, can be effectively subjected to genome editing, as well as common cells.

The membrane protein can be expressed in, for example, cells used for allogeneic transplantation for the purpose of transplantation or cell therapy. Such cells are referred to as "therapeutic cells." The cell comprising the membrane protein expressed therein comprises a polypeptide having a site binding to inhibitory KIR and binding to inhibitory KIR expressed on the surface thereof, and such polypeptide binds to inhibitory KIR of an NK cell, activates the inhibitory KIR, transmits an inhibitory signal into the NK cell, and inhibits the cytocidal activity of the NK cell. As a result, immune rejection occurring at the time of transplantation may be inhibited, immune rejection may become less likely to occur, and a risk of immune rejection may be lowered.

Examples of cells used for allogeneic transplantation for the purpose of transplantation or cell therapy include pluripotent stem cells, and specific examples include ES cells and iPS cells. In such pluripotent stem cells, the membrane protein of the present invention can be expressed by genome editing as described above, or useful gene editing can be performed. Further examples include mesenchymal stem cells, T cells derived from peripheral blood mononuclear cells (PBMCs) as primary cells, NK cells, and macrophages. In cells sampled from biological tissue, NK activity can be inhibited by, for example, knocking out HLA or modifying the cells to express a KIR agonist. Mesenchymal cells may be modified to express and secrete useful therapeutic factors, such as cancer therapeutic factors, and transplanted. T cells and NK cells can be modified to have anticancer activity, such as CAR-T/NK cells, and used as therapeutic cells. Further examples of cells used for allogeneic transplantation for the purpose of transplantation or cell therapy include myocardial cells, cartilage cells, retinal cells, hepatic cells, kidney cells, and pancreatic cells. Such cells may be cells sampled from a donor or various cells artificially induced to differentiate from pluripotent stem cells. Any cell comprising a membrane protein capable of binding to inhibitory KIR of an NK cell expressed on the surface thereof can bind to inhibitory KIR of the NK cell, activate inhibitory KIR, transmit an inhibitory signal into the NK cell, and inhibit the cytocidal activity of the NK cell.

In the cells of the present invention, expression of at least one class I HLA on the cell surface may be quenched or attenuated. Expression of at least one class I HLA on the cell surface may be quenched or attenuated by modification of the class I HLA, B2M, TAP1, TAP2, and TAPBP regions on the genome by gene editing.

In addition, the cells of the present invention may be established from an individual with genetically quenched HLA expression.

The cells of the present invention may further comprise a ligand for NKG2A expressed on the surface. NKG2A (CD159) is a type of an inhibitory receptor on the NK cell surface. An example of a ligand for NKG2A is HLA-E.

The present invention encompasses a method for producing a therapeutic cell providing a reduced or inhibited risk of immune rejection in a subject comprising expressing a substance having activity of binding to human inhibitory KIR and inhibiting the cytocidal activity of an NK cell of the present invention on the surface of a therapeutic cell.

4. CAR-T

The present invention encompasses a T cell further comprising, expressed thereon, a chimeric antigen receptor (CAR) targeting an antigen of interest (CAR-T), in addition to the membrane protein. CAR-T used in the present invention is not limited, and CAR-T targets a tumor-associated antigen. Such CAR-T expresses, on its surface, a polypeptide comprising a domain that binds specifically to a tumor-cell-specific tumor-associated antigen, a transmembrane domain, and an intracellular signal transmission domain. A domain that binds specifically to a tumor-associated antigen can be, for example, a polypeptide derived from an antibody reacting with the tumor-associated antigen, such as a polypeptide comprising scFv or an Fv region of the immunoglobulin heavy chain and light chain linked thereto. CAR of the CAR-T of the present invention is present on a cell membrane, and it comprises an extracellular domain including a domain that binds specifically to a tumor-cell-specific tumor-associated antigen, a transmembrane domain, and an intracellular signal transmission domain in an intracellular domain. An extracellular domain may comprise an extracellular spacer domain, and an intracellular domain may comprise a costimulatory domain. A transmembrane domain can be, for example, a transmembrane domain of CD8$\alpha$, CD28, CD3$\epsilon$, CD3, or CD4. An extracellular spacer domain can be, for example, a spacer sequence of (G4S)3 or IgG1 hinge region. A costimulatory domain can be, for example, CD27, CD28, CD134, CD27, CD2, CD5, CD30, or CD40. An intracellular signal transmission domain can be, for example, a human CD3ζ chain, Fc receptor, FcγRIII, or FcεRI. A polynucleotide encoding CAR of the present invention can be prepared by linking polynucleotides each encoding the domain indicated above to each other. Such polynucleotide and a polynucleotide encoding the polypeptide having a site binding to inhibitory KIR of the present invention may be introduced into a T cell to prepare the CAR-T of the present invention.

The CAR-T of the present invention can be used for treatment of tumors expressing tumor-associated antigens. The CAR-T cell of the present invention comprises, a polypeptide having a site binding to inhibitory KIR expressed on the cell surface and, accordingly, has a function of avoiding NK cell-mediated immune rejection, in addition to the cytocidal activity against the target cell. The present invention encompasses a pharmaceutical composition comprising the CAR-T cell of the present invention.

5. Use of substance having activity of binding to human inhibitory KIR and inhibiting cytocidal activity of NK cell and cell comprising substance having activity of binding to inhibitory KIR and inhibiting cytocidal activity of NK cell of the present invention expressed on surface

The present invention encompasses a pharmaceutical composition comprising, as an active ingredient, a substance having activity of binding to inhibitory KIR and inhibiting the cytocidal activity of an NK cell and a cell comprising a substance having activity of binding to inhibitory KIR and inhibiting the cytocidal activity of an NK cell expressed on the surface thereof.

The pharmaceutical composition can be used as an immune rejection inhibitor at the time of organ or therapeutic cell transplantation. Specifically, the pharmaceutical composition can be used for inhibition of rejection at the time of allogeneic transplantation of the kidney or pancreatic islet, prevention or treatment of graft-versus-host disease (GVHD), inhibition of rejection at the time of allogeneic transplantation of reproduced therapeutic cells, and the like. The pharmaceutical composition can also be used for heterogenetic organ transplantation, such as transplantation of a genetically-modified pig-derived organ into a human. Such pharmaceutical composition is also referred to as an immune rejection inhibitor or a transplantation rejection inhibitor.

A cell comprising a substance having activity of binding to inhibitory KIR and inhibiting the cytocidal activity of an NK cell expressed on the cell surface can be used as a therapeutic cell that inhibits immune rejection in a subject to which the cell is to be administered or transplanted. Such therapeutic cell can be used as a pharmaceutical composition for transplantation.

In addition, the present invention encompasses a method for inhibiting immune rejection in a subject comprising administering or transplanting a substance having activity of binding to inhibitory KIR and inhibiting the cytocidal activity of an NK cell and a cell comprising the substance having activity of binding to inhibitory KIR and inhibiting the cytocidal activity of an NK cell expressed on the surface thereof to a subject who is in need of transplantation.

Further, the present invention encompasses a method for inhibiting immune rejection in a subject comprising expressing the substance having activity of binding to inhibitory KIR and inhibiting the cytocidal activity of an NK cell of the present invention on the surface of a therapeutic cell.

When used for the applications indicated above, a patient, who is the subject of treatment, has an HLA type serving as a ligand for the target inhibitory KIR. When using an agonist against inhibitory KIR2DL2 and/or KIR2DL3 or a cell comprising the agonist expressed on the cell surface, for example, immunosuppressive activity or activity of avoiding immune rejection is exerted on the HLA-C1 homozygous patient. When using an agonist against inhibitory KIR2DL1 or a cell comprising the agonist expressed on the cell surface, immunosuppressive activity or activity of avoiding immune rejection is exerted on the HLA-C2 homozygous patient. When an agonist against inhibitory KIR2DL2 and/or KIR2DL3 is used in combination with an agonist against inhibitory KIR2DL1 or a cell comprising such 2 types of agonists expressed in combination on the cell surface is used, immunosuppressive activity or activity of avoiding immune rejection is exerted on all patients with any HLA-C genotype (i.e., HLA-C1 homozygous, HLA-C2 homozygous, and HLA-C1/C2 heterozygous patients).

A HLA-Bw4⁻ patient may be treated in accordance with the HLA-C genotype. In the case of a HLA-Bw4+ positive patient, immunosuppressive activity or activity of avoiding immune rejection can be exerted by additionally administering an agonist against KIR3DL1 to the patient or with the use of a cell comprising such agonist additionally expressed on the cell surface.

The pharmaceutical composition of the present invention can contain a therapeutically effective amount of a substance having activity of binding to inhibitory KIR and inhibiting the cytocidal activity of an NK cell or a substance having activity of binding to inhibitory KIR and inhibiting the cytocidal activity of an NK cell and a cell comprising a substance having activity of binding to inhibitory KIR and inhibiting the cytocidal activity of an NK cell expressed on the surface thereof, a pharmaceutically acceptable carrier, a diluent, a solubilizer, an emulsifier, a preservative, an aid, and the like. A method for administering the pharmaceutical composition of the present invention can be suitably selected. Examples of methods that can be employed include injection, topical infusion, intraperitoneal administration, selective intravenous infusion, intravenous injection, subcutaneous injection, and organ perfusate infusion. Further, an injection solution can be formulated using a carrier comprising a salt solution, a glucose solution, or a mixture of a salt solution and a glucose solution, various types of buffer solutions, or the like. Further, a powder may be formulated and mixed with a liquid carrier to prepare an injection solution before use.

Other administration methods can be suitably selected along with development of a formulation. For example, oral solutions, powders, pills, capsules, tablets, and the like can be applied for oral administration. For oral solutions, oral liquid preparations such as suspensions and syrups can be produced using water, saccharides such as sucrose, sorbitol, and fructose, glycols such as polyethylene glycol, oils such as sesame oil and soybean oil, preservatives such as alkyl parahydroxybenzoates, flavors such as strawberry flavor and peppermint, and the like. Powders, pills, capsules, and tablets can be formulated using excipients such as lactose, glucose, sucrose, and mannitol, disintegrating agents such as starch and alginate soda, lubricants such as magnesium stearate and talc, binders such as polyvinyl alcohol, hydroxypropyl cellulose, and gelatin, surfactants such as fatty acid esters, plasticizers such as glycerin, and the like. Tablets and capsules are preferred unit dosage forms for the composition of the present invention in that they are easily administered. Solid production carriers are used to produce tablets and capsules.

A therapeutically effective dose of the substance having activity of binding to human inhibitory KIR and inhibiting the cytocidal activity of an NK cell can be changed in accordance with the patient's age and condition, and it may be finally determined by a physician. For example, one dose is 0.0001 mg to 100 mg per kg of body weight. The predetermined dose may be administered once every one to 180 days, or the dose may be divided into two doses, three doses, four doses, or more doses per day at appropriate intervals.

The dose of a substance having activity of binding to inhibitory KIR and inhibiting the cytocidal activity of an NK cell and a cell comprising, expressed on the surface, a substance having activity of binding to inhibitory KIR and inhibiting the cytocidal activity of an NK cell can be adequately determined in accordance with the type of cells to be transplanted and other conditions.

6. Method for evaluating activity of avoiding immune rejection by activity of test substance for inhibiting cytocidal activity of NK cell against inhibitory KIR

The present invention encompasses a method for evaluating activity of avoiding immune rejection by activity of a test substance for inhibiting the cytocidal activity of an NK cell against inhibitory KIR.

A test substance may be any substance having activity of binding to inhibitory KIR and inhibiting the cytocidal activity of an NK cell, and examples thereof include those described in 1 above.

The method comprises the following steps:

(A) a step of bringing NK cells derived from a HLA-C1/C1 homozygous subject into contact with target cells derived from a HLA-C2/C2 homozygous subject *ex vivo* in the presence of a test substance binding to KIR (wherein the Bw4 phenotype of the donor of the NK cells is identical to that of the donor of the target cells); and
(B) a subsequent step of measuring the viable target cell count.

Specifically, the method may comprise the following steps:

(A) a step of bringing NK cells derived from a HLA-C1/C1 homozygous subject into contact with target cells derived from a HLA-C2/C2 homozygous subject *ex vivo* in the presence of a test substance binding to KIR2DL2 or KIR2DL3 (wherein the Bw4 motif-containing HLA phenotype of the donor of the NK cells is identical to that of the donor of the target cells); and
(B) a subsequent step of measuring the viable target cell count.

The method may further comprise the following steps:

(A) a step of bringing NK cells derived from a HLA-C2/C2 homozygous subject into contact with target cells derived from a HLA-C1/C1 homozygous subject *ex vivo* in the presence of a test substance binding to KIR2DL1 (wherein the Bw4 motif-containing HLA phenotype of the donor of the NK cells is identical to that of the donor of the target cells); and
(B) a subsequent step of measuring the viable target cell count.

The method may further comprise the following steps:

(A) a step of bringing NK cells derived from a HLA-Bw4+/Bw4- homozygous or Bw4+/Bw4- heterozygous subject into contact with target cells derived from a HLA-Bw4-/Bw4- homozygous subject *ex vivo* in the presence of a test substance binding to KIR3DL1 (wherein the HLA-C phenotype of the donor of the NK cells is identical to that of the donor of the target cells); and
(B) a subsequent step of measuring the viable target cell count.

The method may further comprise the following steps:

(A) a step of bringing NK cells derived from a HLA-A3$^{+/-}$ homozygous subject or HLA-A11$^{+/-}$ homozygous subject, or HLA-A3$^{+/-}$ heterozygous subject or HLA-A11$^{+/-}$ heterozygous subject into contact with target cells derived from a HLA-A3$^{-/-}$ homozygous subject or HLA-A11$^{-/-}$ homozygous subject *ex vivo* in the presence of a test substance binding to KIR3DL2 (wherein the HLA-C and Bw4 phenotypes of the donor of the NK cells are identical to those of the donor of the target cells); and
(B) a subsequent step of measuring the viable target cell count.

As NK cells, for example, NK cells derived from the peripheral blood can be used. As target cells, for example, T cells derived from the peripheral blood can be used.

An NK cell has a function of missing-self recognition by which the NK cell recognizes its own HLA type (C1, C2, or Bw4) and exerts cytocidal activity on a target cell. For example, an NK cell sampled from a HLA-C1/C1 homozygous human subject recognizes C1 as the self. When a target cell does not have the HLA-C1 type, accordingly, a signal is transmitted into an NK cell through inhibitory KIR, and the NK cell exerts cytocidal activity. In contrast, an NK cell does not exert cytocidal activity on the target cell of the C1 type. When a substance having activity of binding to inhibitory KIR2DL2 and/or KIR2DL3 that can serve as an alternative to the C1 type and inhibiting the cytocidal activity of an NK cell (i.e., agonist binder) is expressed, the cytocidal activity can be reduced.

NK cells derived from the HLA-C1/C1 homozygous subject used for the method of evaluation can be isolated from peripheral blood mononuclear cells (PBMCs) of a HLA-C1/C1 homozygous human subject with the use of the positivity for CD56 as the indicator. For example, NK cells can be isolated with the use of CD56 microbeads or via flow cytometry. In the NK cells thus isolated, KIR2DL2 and KIR2DL3 are activated.

T cells may be used as target cells derived from the HLA-C2/C2 homozygous subject, and such T cells can be isolated from PBMCs of the HLA-C2/C2 homozygous human subject with the use of the positivity for CD3 as the indicator. Since the target cells do not express HLA of the C1 type, NK cells derived from the HLA-C1/C1 homozygous subject exert the cytocidal activity thereon.

When effector cells; i.e., the NK cells derived from the HLA-C1/C1 homozygous subject, are brought into contact with the target cells derived from the HLA-C2/C2 homozygous subject, the target cells are killed by the effector cells. In the presence of a substance having activity of binding to inhibitory KIR2DL2 and/or KIR2DL3 and inhibiting the cytocidal activity of an NK cell, however, such substance binds to KIR2DL2 and/or KIR2DL3 of the NK cells derived from the HLA-C1/C1 homozygous subject and inhibits the cytocidal activity of the NK cells. Thus, the cytocidal activity of the effector cells is reduced, and the target cells would not be killed. In the presence of a test substance binding to KIR2DL2 and/or KIR2DL3, accordingly, the NK cells derived from the HLA-C1/C1 homozygous subject are brought into contact with the target cells derived from the HLA-C2/C2 homozygous subject, and the viability of the target cells is then measured. Thus, activity of avoiding immune rejection relative to the activity of the test substance for inhibiting the cytocidal activity of an NK cell against inhibitory KIR can be evaluated.

When the target cell viability is high in the step B, the test substance can be determined to have activity of avoiding immune rejection. The present invention encompasses a method for screening for a substance having activity of avoiding immune rejection, which comprises a step of selecting a test substance exhibiting a high target cell viability in step B as a substance having activity of avoiding immune rejection.

In the step (A) of the method described above, the test substance may be expressed in the form of the membrane protein as described in 1 (2) above on the target cell surface.

Specifically, evaluation can be performed in accordance with the method described in the "Evaluation of cytocidal activity" section or the "Evaluation of agonist activity" section in the examples below.

Examples

The present invention is described in greater detail with reference to the following examples, although the present invention is not limited to these examples.

Method

The following method was employed as a technique of experimentation.

[Plasmid vector for virus preparation]

A lentivirus used to introduce a target gene into a natural killer cell line as an effector cell, a K562 cell line as a target cell, and a human T cell were prepared with the use of the pLVSIN vector deprived of the sequence of the gene encoding the green fluorescent protein (ZsGreen1) included in pLVSIN-EF1α IRES-ZsGreen1 (6191, Takara Bio). The target gene was introduced into the multicloning site of pLVSIN to prepare a plasmid vector for lentivirus preparation.

[Preparation of lentivirus]

The Lenti-X 293T cells (632180, Takara Bio) were cultured to reach confluency using the Dulbecco's Modified Eagle Medium (DMEM) containing 10(v/v)% fetal bovine serum (FBS) (10566-016, Thermo Fisher Scientific), the cells were detached using trypsin (12563029, Thermo Fisher Scientific) on the day of transfection, and the cells were then reseeded in a T flask. The cells were cultured to reach 70% to 80% confluency, the plasmid vector for lentivirus preparation, the Lentiviral High Titer Packaging Mix (6194, Takara Bio), and the TransIT-293 Transfection Reagent (MIR2704, Takara Bio) were mixed in the Opti-MEM I Reduced Serum Media (31985070, Thermo Fisher Scientific), and the mixture was added to the Lenti-X 293T cells after incubation. On the following day, the medium was exchanged with a DMEM medium containing 10(v/v)% FBS, culture was conducted for 24 hours, and the supernatant was then collected. After the supernatant was collected, a DMEM medium containing 10(v/v)% FBS was added again, according to need, culture was conducted for 24 hours, and the supernatant was then collected again. The collected supernatant was filtered, the Lenti-X concentrator (631232, Takara Bio) was added in an amount 1/3 of the amount of the filtered supernatant, the resultant was incubated at 4°C for 1 hour or longer, and the viruses were precipitated via centrifugation. The supernatant was removed by suction, pellets were dissolved in an AIM-V medium containing 5(v/v)% FBS (12055083, Thermo Fisher Scientific) (hereafter, referred to as a "basal medium"), and the resulting lentivirus concentrate was cryopreserved at -80°C or -150°C.

[Introduction of KIR gene into NK92 cell and preparation]

NK92 cells were obtained from ATCC (Cat. No: CRL-2407™), and various KIRs were adequately expressed and used as effector cells for the initial evaluation system. Culture was conducted in the MyeloCult H5100 medium (ST-05150, STEMCELL Technologies) supplemented with IL-2 at 500 IU/ml (in the range of 100 to 2000) and streptomycin/penicillin at 37°C in the presence of 5% $CO_2$.

In order to establish the NK92 cell line expressing various KIRs shown in Table 4, an artificial gene encoding a polypeptide comprising a target KIR-derived signal peptide (see Table 5), a Flag tag (SEQ ID NO: 2), GSGS (SEQ ID NO: 3), and a target receptor peptide (see Table 4) positioned in that order from the N terminus was inserted into pLVSIN EF1α in accordance with the method described in the [Preparation of lentivirus] section above to prepare the lentivirus. In order to establish NK92 cell line expressing KIR2DL2*001, an artificial gene encoding a polypeptide comprising a target KIR-derived signal peptide (see Table 5), a target receptor peptide (SEQ ID NO: 57), a self-cleaving peptide T2A (SEQ ID NO: 20), and an NGFR peptide (SEQ ID NO: 58) positioned in that order without the use of the Flag tag and the GS linker was inserted into pLVSIN EF1α in accordance with the method described in the [Preparation of lentivirus] section above to prepare the lentivirus.

KIRs were selected from among variants with high frequencies based on the Allele Frequency Net Database. In the examples herein, KIR2DL2*003 was used as KIR2DL2, unless otherwise specified.

[Table 5]

| Target KIR | IPD Accession No. | Cell line | Signal peptide sequence | Receptor sequence |
|---|---|---|---|---|
| Human KIR2DL1*003 | KIR00003 | KIR2DL1-NK92 cell line | SEQ ID NO: 1 | SEQ ID NO: 4 |
| Human KIR2DL2*001 | KIR00010 | KIR2DL2*001-NK92 cell line | SEQ ID NO: 5 | SEQ ID NO: 6 |
| Human KIR2DL2*003 | KIR00012 | KIR2DL2*003-NK92 cell line | SEQ ID NO: 5 | SEQ ID NO: 57 |
| Human KIR2DL3*001 | KIR00014 | KIR2DL3-NK92 cell line | SEQ ID NO: 7 | SEQ ID NO: 8 |
| Human KIR2DS1*002 | KIR00034 | KIR2DS1-NK92 cell line | SEQ ID NO: 9 | SEQ ID NO: 10 |
| Human KIR2DS2*001 | KIR00037 | KIR2DS2-NK92 cell line | SEQ ID NO: 11 | SEQ ID NO: 12 |
| Human KIR2DS4*001 | KIR00045 | KIR2DS4-NK92 cell line | SEQ ID NO: 13 | SEQ ID NO: 14 |
| Human KIR3DL1*015 | KIR00102 | KIR3DL1-NK92 cell line | SEQ ID NO: 15 | SEQ ID NO: 16 |

(continued)

| Target KIR | IPD Accession No. | Cell line | Signal peptide sequence | Receptor sequence |
|---|---|---|---|---|
| Human KIR3DL2*002 | KIR00066 | KIR3DL2-NK92 cell line | SEQ ID NO: 65 | SEQ ID NO: 59 |

RetroNectin (T100B, Takara Bio) was diluted to 20 to 100 μg/ml in phosphate buffered saline (PBS) and added to the non-treatment dish. The dish was allowed to stand at room temperature for 2 hours or at 4°C overnight and washed with PBS to prepare a coated plate.

NK92 cells were suspended at 1 to 2 × 10$^6$ cells/ml in the Myelocult H5100 medium. The inhibitory KIR, an activating KIR gene-encoding lentivirus concentrate, and an NK cell suspension were mixed on a RetroNectin-coated plate, and the resultant was centrifuged at 1000× g and 32°C for 1 hour. Thereafter, culture was conducted at 37°C in the presence of 5% $CO_2$. The total volume of the medium was exchanged with the Myelocult H5100 medium every 1 to 3 days from 3 days after the culture, and positive cells were selected with the use of the APC-labeled anti-Flag antibody or APC-labeled anti-NGFR antibody and the anti-APC Microbeads (130-090-855, Miltenyi Biotec) 1 week after the culture. Expression of the introduced KIRs was confirmed with the use of commercially available anti-KIR2DS1 antibody, anti-KIR2DL2/3/2DS2 antibody, and anti-KIR2DS4 antibody. According to need, the collected cells were suspended at 0.5 to 1 × 10$^7$ cells/ml in CELLBANKER 1 (CB011, Takara Bio) and cryopreserved at -80°C or -150°C. When frozen cells were used in experimentation, the cells were thawed and cultured in the NK cell culture medium before use.

[Flow cytometry and antibody to be added]

When performing flow cytometry to confirm expression of various cell surface proteins, the antibodies shown in Table 6 were used.

[Table 6]

| Antibody | Manufacturer | Catalog No. | Label |
|---|---|---|---|
| Anti-HLA-ABC antibody | BIOLEGEND | 311410 | APC |
| Anti-Flag antibody | BIOLEGEND | 637308 | APC |
| Anti-NGFR antibody | BIOLEGEND | 345108 | APC |
| Anti-CD19 antibody | BIOLEGEND | 302212 | APC |
| Anti-KIR2DL1/2DS1 antibody | Miltenyi Biotech | 130-092-685 | APC |
| Anti-KIR2DL2/3/2DS2 antibody | Miltenyi Biotech | 130-092-617 | APC |
| Anti-KIR2DS1 antibody | R&D systems | FAB8887G | AlexaFluore488 |
| Anti-KIR2DS4 antibody | R&D systems | FAB1847A | APC |
| Lirilumab(1-7F9) | CREATIVE BIOLABS | TAB-757 | - |
| Pan2D(NKVSF1) | GeneTex | GTX6000 | - |

The fluorescence-labeled antibodies were suspended at 1 to 10 μg/ml in autoMACS running buffer-MACS separation buffer (130-091-221, Miltenyi Biotec), the suspension was added to the cells, and the reaction was allowed to proceed at 4°C for 30 minutes.

The antibody-stained cells were suspended in FACS buffer containing 7-aminoactinomycin D (7-AAD, 016-25241, WAKO) at 1 to 10 μg/ml and then assayed using a flow cytometer (MACSQuant Analyzer 10, 130-096-343, Miltenyi biotec). The data were extracted as the FCS file, a cell fraction was selected by FSC/SSC plotting using the FLOW JO software (VER. 10.7.1, FLOWJO LLC), and 7-aminoactinomycin D$^-$/target$^+$ cells were counted as viable cells.

Example 1: Lirilumab inhibits cytocidal activity of K562 by NK92-expressing KIR2DL2 cell line

An evaluation system in which the cytocidal activity of the KIR2DL2-expressing NK cell line would be attenuated by recognition of HLA by KIR2DL2 was constructed, and whether or not the existing anti-KIR antibody added in a soluble state would show the agonist activity against the KIR2DL2-expressing cell line was evaluated.

[Gene introduction into K-562 cell and preparation]

K562 cells were obtained from ATCC (CCL-243™) and used as target cells for the initial evaluation system. Culture was conducted in RPMI 1640 medium with GlutaMAX (61870036, Life Technologies) (hereafter, referred to as "RPMI medium") supplemented with FCS 10 (v/v)% and streptomycin/penicillin.

In order to establish the luciferase-expressing K562 cell line (Luc-K562 cell line), an artificial gene encoding a polypeptide of firefly luciferase (SEQ ID NO: 17) was introduced into the K562 cell line using CRISPR/CAS9.

In order to establish the human HLA-Cw3-expressing K562 cell line (HLA-Cw3-Luc-K562 cell line), an artificial gene encoding a polypeptide comprising a luciferase (SEQ ID NO: 17), a self-cleaving peptide T2A (SEQ ID NO: 20), a human HLA-Cw3-derived signal peptide (SEQ ID NO: 21), and a human HLA-Cw3 peptide (SEQ ID NO: 22) linked in that order from the N terminus was introduced into the K562 cell line using CRISPR/CAS9.

The Luc-K562 cells were cloned, and clones were selected based on the results of luciferase activity assay. Expression of the HLA-Cw3-K562 cells were confirmed with the use of anti-HLA-ABC, the HLA-Cw3-K562 cells were cloned, and clones were selected based on the results of luciferase activity assay and the intensity of anti-HLA-ABC staining.

According to need, the collected cells were suspended at 0.5 to $1 \times 10^7$ cells/ml in CELLBANKER 1 (CB011, Takara Bio) and cryopreserved at -80°C or -150°C. When frozen cells were used in experimentation, the cells were thawed and cultured in the RPMI medium before use.

[Evaluation of cytocidal activity of KIR2DL2-expressing NK92 on K562]

In this example, KIR2DL2*001 was used as KIR2DL2. On the day before evaluation of the cytocidal activity, the KIR2DL2-NK92 cells stimulated with IL-2 at 500 IU/ml were added at $5 \times 10^3$ cells/well to a 96-well plate, the equivalent amount (i.e., $5 \times 10^3$ cells/well) of the Luc-K562 cells or HLA-Cw3-Luc-K562 cells were added to individual wells, and culture was further conducted. An anti-KIR antibody, lirilumab (1-7F9, human IgG4), was added to the wells containing both the KIR2DL2-NK92 cells and the Luc-K562 cells at 4 concentrations each of that represented 4 replicates of a 9-fold dilution of 2 $\mu$g/mL. As a Lirilumab control, human IgG4 (GTX63305, GeneTex) was treated in the same manner at 2 $\mu$g/ml. All culture procedures were performed using the basal medium. The cytotoxic activity was determined in terms of the number of remaining target cells by measuring the luciferase activity of the K562 cells 3 hours later, relative to the number of target cells in the wells not supplemented with the KIR2DL2-NK92 cells designated to be 100%.

The results are shown in Figure 1-1. While the KIR2DL2-NK92 cell line exerted the cytocidal activity on the K562 cell line, the cytocidal activity thereof on the HLA-Cw3-K562 cell line was attenuated. KIR2DL2 that has recognized HLA-Cw3 is considered to inhibit the cytocidal activity by introducing the inhibitory signal into the KIR2DL2-NK92 cell line. Thus, an evaluation system demonstrating the missing-self mechanism of KIR2DL2 was constructed. In the system evaluating the cytocidal activity of the KIR2DL2-NK92 cell line on the K562 cell line, Lirilumab was added to the culture solution. As a result, the cytocidal activity of the KIR2DL2 cell line on the K562 cell line was attenuated in a concentration-dependent manner, and approximately 50% of the activity was inhibited at 2 $\mu$g/ml. This indicates that, in terms of the cytocidal activity of the KIR2DL2-NK92 cell line on the K562 cell line, Lirilumab would exert the agonist activity on inhibitory KIR2DL2. No change was observed with the use of a control antibody; i.e., human IgG4, at 2 $\mu$g/ml.

In the evaluation of the cytocidal activity of the KIR2DL2-NK92 cell line on the HLA-Cw3-K562 cell line, the cytocidal activity thereof was potentiated with the addition of Lirilumab at 2 $\mu$g/ml. The results are shown in Figure 1-2. No change was observed with the use of a control antibody; i.e., human IgG4, at 2 $\mu$g/ml.

Such phenomenon is the same as that of the case concerning Lirilumab patent (PL4: WO 2006/003179_A2), Lirilumab is deduced to cancel the inhibitory signal emitted by the KIR ligand and potentiate NK cell activation, and such activity is to inhibit the agonist activity of the HLA ligand on inhibitory KIR.

As described above, an anti-KIR antibody, Lirilumab, was found to cancel the ligand activity and potentiate the cytocidal activity of an NK cell in the presence of a KIR2DL2 agonist, such as HLA-Cw3. In contrast, Lirilumab was found to function as a partial agonist exerting the agonist activity on inhibitory KIR in the absence of a KIR2DL2 ligand.

Example 2: Inhibition of cytocidal activity of NK cells by Lirilumab and Pan2D in scFv expression through inhibitory KIR

When an agonist binder of an inhibitory receptor is allowed to act as a secretory protein according to an allogeneic technology, it may affect the immune system in the vicinity of the target tissue and the immune system in the body. In order to produce immunological tolerance selectively to cells that are intended to evade the immune system, accordingly, an agonist binder was allowed to express on the surface of the cells to be transplanted, so as to achieve topical immune tolerance.

Lirilumab in a soluble state inhibited the cytocidal activity of the KIR2DL2-NK92 cells on the K562 cells. Thus, scFv derived from Lirilumab or Pan2D was expressed as a membrane protein on the surface of the K562 cells, and whether or not the cytocidal activity of the KIR2DL2-NK92 cells would be inhibited was evaluated.

[scFv gene introduction into K-562 cells and preparation]

The sequences of Lirilumab and Pan2D (NKVSF1) were quoted from WO 2006/003179 A2 (the sequence identification numbers of variable regions are shown in Table 6).

In order to establish the Lirilumab-scFv-expressing K562 cell line (Lirilumab scFv-Luc-K562 cell line) and the Pan2D-scFv-expressing K562 cell line (Pan2D-scFv-Luc-K562 cell line), an artificial gene encoding a polypeptide comprising a human CD8-derived signal peptide (SEQ ID NO: 23), a Flag tag (SEQ ID NO: 2), a linker (GSG), a variable region (VH) shown in Table 7, a GS(G4S) linker (SEQ ID NO: 25), a variable region (VL) shown in Table 7, a sequence of a CD8-derived hinge region (66 amino acids) (SEQ ID NO: 24), a CD8-derived cell membrane domain (SEQ ID NO: 27), a CD8-derived intracellular domain (SEQ ID NO: 28), a self-cleaving peptide T2A (SEQ ID NO: 20), and a firefly luciferase (SEQ ID NO: 17) linked in that order from the N terminus (hereafter, referred to as a "membrane-type scFv expression format") was inserted into pLVSIN EF1$\alpha$ in accordance with the method described in the [Preparation of lentivirus] section above to prepare the lentivirus. Figure 9 schematically shows such membrane-type scFv binders.

[Table 7]

| Binder | Variable region | CDR1 | CDR2 | CDR3 |
|---|---|---|---|---|
| Lirilumab | VH (SEQ ID NO: 29) | 26-33 | 51-58 | 97-112 |
| | VL (SEQ ID NO: 30) | 27-32 | 50-52 | 89-97 |
| Pan2D | VH (SEQ ID NO: 31) | 26-33 | 51-58 | 97-110 |
| | VL (SEQ ID NO: 32) | 27-33 | 51-53 | 90-98 |
| * In the table, CDR1, CDR2, and CDR3 are each indicated with the amino acid numbers in the amino acid sequence of relevant variable region. The amino acid numbers are specified in accordance with the IMGT definitions. | | | | |

RetroNectin (T100B, Takara Bio) was diluted to 20 to 100 $\mu$g/ml in phosphate buffered saline (PBS) and added to the non-treatment dish. The dish was allowed to stand at room temperature for 2 hours or at 4°C overnight and washed with PBS to prepare a coated plate.

K562 cells were suspended at 1 to 2 × 10$^6$ cells/ml in RPMI medium. The lentivirus concentrate encoding the Lirilumab scFv-Luc or Pan2D scFv-Luc gene was mixed with cells on a RetroNectin-coated plate, and the resultant was centrifuged at 1000× g and 32°C for 1 hour. The total volume of the medium was exchanged with the RPMI medium every 1 to 3 days from 3 days after the culture, and positive cells were selected with the use of the APC-labeled anti-Flag antibody and the anti-APC Microbeads (130-090-855, Miltenyi Biotec) 1 week after the culture.

According to need, the collected cells were suspended at 0.5 to 1 × 10$^7$ cells/ml in CELLBANKER 1 (CB011, Takara Bio) and cryopreserved at -80°C or -150°C. When frozen cells were used in experimentation, the cells were thawed and cultured in the RPMI medium before use.

[Evaluation of cytocidal activity of KIR2DL2-expressing NK92 cells on Lirilumab-scFv/Pan2D-scFv-K562 cells]

In this example, KIR2DL2*001 was used as KIR2DL2. On the day before evaluation of the cytocidal activity, the KIR2DL2-NK92 cells stimulated with IL-2 at 500 IU/ml were added to a 96-well plate, the Luc-K562 cell line, the HLA-Cw3-Luc-K562 cell line, the Lirilumab-scFv-K562 cell line, and Pan2D-scFv-K562 were added to individual wells to adjust the ratio of the NK92 cells to the K562 cells to 3:1 or 1:1, and culture was further conducted. All culture procedures were performed using the basal medium. The luciferase activity of the K562 cells was assayed 2 to 5 hours later, and the luciferase activity in the wells supplemented with the KIR2DL2-NK92 cells was shown as the number of remaining target cells by designating the luciferase activity in the wells not supplemented with the KIR2DL2-NK92 cells to be 100%. Also, the value obtained by subtracting the luciferase activity in the wells supplemented with the KIR2DL2-NK92 cells from the luciferase activity in the wells not supplemented with the KIR2DL2-NK92 cells was shown as the cytocidal activity. The luciferase activity was measured using ONE-Glo™ Luciferase Assay System (E6120, PROMEGA).

In order to determine KIR-dependent activity and calculate a binder-dependent activity ratio, the ratio of the cytocidal activity was determined in accordance with the equation shown below while designating the activity of the Luc-K-562 cell line to be 1.

$$\text{Ratio of KIR-dependent cytocidal activity} = A^{\text{KIR-NK92}}/A^{\text{NK92}}$$

[A = cytocidal activity (%) in NK cell = (luciferase activity of target cell not supplemented with effector cell - luciferase activity of target cell supplemented with effector cell) / luciferase activity of target cell not supplemented with effector cell × 100]

The results are shown in Figure 2. While the KIR2DL2-NK92 cell line exerted the cytocidal activity on the K562 cell line, the cytocidal activity thereof on the HLA-Cw3-K562 cell line was attenuated. In addition, the cytocidal activity of Lirilumab-scFv-K562 or Pan2D-scFv-K562 was attenuated, compared with that of the K562 cell line.

When an anti-KIR antibody, such as Lirilumab or Pan2D, is expressed as an scFv-membrane protein, the cytocidal activity of the KIR2DL2-NK92 cell line was inhibited, and the agonist activity was exerted on KIR2DL2.

Example 3: Lirilumab and Pan2D evoke cytocidal activity of NK cell through activating KIR

Whether or not Lirilumab and Pan2D would exert agonist activity on other inhibitory KIRs and activating KIRs with which Lirilumab and Pan2D would have cross-reactivity, as well as KIR2DL2, was evaluated.

Under the same evaluation conditions as employed in Example 2, sensitivity of Lirilumab or Pan2D-scFv-K562 to the cytocidal activity was evaluated using NK92 cell lines expressing various types of KIRs. In order to determine the KIR-dependent activity, the activity ratio was calculated in accordance with the following equation. In order to calculate the binder-dependent activity ratio, in addition, the activity ratio was calculated while designating the activity of the Luc-K-562 cell line to be 1. In this example, KIR2DL2*001 was used as KIR2DL2.

$$\text{KIR-dependent cytocidal activity ratio} = A^{\text{KIR-NK92}}/A^{\text{NK92}}$$

[A = cytocidal activity (%) in NK cell = (luciferase activity of target cell not supplemented with effector cell - luciferase activity of target cell supplemented with effector cell) / luciferase activity of target cell not supplemented with effector cell × 100]

The results are shown in Figure 3. The Lirilumab-scFv-K562 cell line or the Pan2D-scFv-K562 cell line exerted the effects of inhibiting the cytocidal activity on KIR2DL2-NK92 and exerted the agonist activity on inhibitory KIR. The results indicated above are the same as the results attained in Example 2. Since Lirilumab-scFv-K562 potentiated the activity of the KIR2DS1-NK92 cell line and the KIR2DS2-NK92 cell line, Lirilumab-scFv-K562 was found to exert the agonist activity on activating KIR2DS1/2. Since Pan2D-scFv-K562 potentiated the activity of the KIR2DS1-NK92 cell line, the KIR2DS2-NK92 cell line, and the KIR2DS4-NK92 cell line, Pan2D-scFv-K562 was found to exert the agonist activity on activating KIR2DS1/2/4.

While the binding specificity of Lirilumab and Pan2D is reported in WO 2006/003179 A2, Lirilumab is reported to show a binding capability to KIR2DS1 and KIR2DS2, and Pan2D is reported to show a binding capability to KIR2DS1, KIR2DS2, and KIR2DS4. Specifically, an antibody reacting with a known inhibitory KIR2DL2/3 was found to show a binding capability to corresponding activating KIR and potentiate the activity of the NK cell expressing activating KIR.

Example 4: Acquisition of KIR2DL2- and/or KIR2DL3-specific binders by phage panning

On the basis of the results of Example 3, an inhibitory KIR-specific binder that would not bind to activating KIR was obtained in the manner described below, so as to obtain a novel binder that would not activate an activating receptor and exert the agonist activity on an inhibitory receptor.

[Acquisition of inhibitory KIR-specific binder by phage panning]

Phage panning was performed with the use of a human antibody phage library, so as to obtain a specific binder that would bind to inhibitory KIR2DL2 and/or KIR2DL3 but would not bind to activating KIR2DS1, 2, or 4.

In order to perform phage panning, soluble KIRs shown in Table 8 were prepared. For soluble KIRs, recombinant proteins each comprising an IL-2-derived signal peptide (SEQ ID NO: 91), an extracellular domain of a KIR protein shown in Table 7, a GGGS linker (SEQ ID NO: 97), an IgG Fc domain (SEQ ID NO: 98), a GAA linker, and an Avi tag (SEQ ID NO: 99) linked in that order from the N terminus were prepared. Phage panning was performed with the use of soluble KIRs corresponding to KIRs shown in Table 7 that have or have not been biotinylated. When performing ELISA, the recombinant human KIR2DL3/CD158b2 Fc chimera protein (2014-KR-050, R&D Systems) was used as soluble KIR2DL3.

[Table 8]

| Recombinant protein | Extracellular domain |
| --- | --- |
| KIR2DL2 | Amino acids 1 to 224 in SEQ ID NO: 6 |
| KIR2DS1 | Amino acids 1 to 224 in SEQ ID NO: 10 |
| KIR2DS2 | Amino acids 1 to 224 in SEQ ID NO: 12 |
| KIR2DS4 | Amino acids 1 to 224 in SEQ ID NO: 14 |

In phage panning performed herein, "positive selection" is a procedure comprising allowing a biotinylated recombinant protein to react with an scFv-expressing phage library solution, and concentrating an scFv-expressing phage binding to a biotinylated recombinant protein using streptavidin or neutravidin beads. In phage panning performed herein, "depletion" is a procedure comprising allowing a biotinylated recombinant protein to react with an scFv-expressing phage library solution, and removing an scFv-expressing phage binding to a biotinylated recombinant protein using streptavidin or neutravidin beads. In phage panning performed herein, "deselection" is a procedure comprising, in the process of a reaction between the biotinylated recombinant protein and the scFv-expressing phage library solution during positive selection, adding a non-biotinylated recombinant protein to facilitate concentration of an scFv-expressing phage that would not bind to a non-biotinylated recombinant protein but specifically bind to a biotinylated recombinant protein, when concentrating an scFv-expressing phage that binds to a biotinylated recombinant protein with the use of streptavidin or neutravidin beads.

During each round of phage panning, inhibitory KIR was subjected to positive selection, and activating KIR was subjected to both or either of depletion and deselection. After the second round, a polyclonal phage library with the enriched KIR2DL2-specific binders was obtained.

ELISA was performed using human KIR2DL2 (22-245)-Fc-Avi (the soluble KIR2DL2 indicated above) to select scFv-expressing phages binding to KIR2DL2 from the polyclonal phage library. From the phagemids of the selected scFv-expressing phages, scFv sequences were transferred to an *E. coli* recombinant protein expression vector to introduce the scFv sequences into *E. coli,* and scFvs were prepared from the culture supernatant. scFv was subjected to ELISA using the soluble KIR shown in Table 7 or the recombinant human KIR2DL3/CD158b2 Fc chimera protein (2014-KIR-050, R&D Systems), and information on the binding capability of scFv to KIR was obtained.

A variable region of scFv that would bind to KIR2DL2 and/or KIR2DL3 but would not bind to KIR2DS1, KIR2DS2, and KIR2DS4 was selected therefrom, introduced into the membrane-type scFv expression format described in the [Introduction of scFv gene into K-562 cell and preparation] section above, and inserted into pLVSIN EF1$\alpha$. With the use of pLVSIN EF1$\alpha$ comprising membrane-type scFv introduced thereinto, lentiviruses were prepared by the method described in the [Preparation of lentivirus] section above. With the use of the prepared lentiviruses, the K562 cell line expressing each scFv in the form of membrane-type scFv was prepared by the method described in the [Introduction of scFv gene into K-562 cell and preparation] section above.

The recombinant protein or recombinant human KIR2DL3/CD158b2 Fc chimera protein (2014-KIR-050, R&D Systems) shown in Table 7 supplemented with Fc was added at 0.1 $\mu$M to the target cells (5 $\times$ 10$^4$ cells), and the reaction was allowed to proceed at 4°C for 90 minutes.

After the reaction, the target cells were washed with PBS, the APC-labeled anti-human IgG Fc antibody (409306, BIOLEGEND) was added, and the reaction was allowed to proceed at 4°C for 30 minutes. The cells were washed two times with PBS and subjected to flow cytometry to examine the binding capability. The binding capability to the K562 cell line (the median fluorescence intensity derived from APC) was designated to be 1, and the ratio relative thereto was calculated.

The results are shown in Figure 4.

While Lirilumab and Pan2D showed the binding capability to recombinant KIR2DS1, 2, or 4, 12 types of binders inhibited the binding capability to recombinant KIR2DS1, 2, or 4 but showed the binding capability to recombinant KIR2DL2 and KIR2DL3.

Example 5: Evaluation of agonist activity of KIR2DL2- and/or KIR2DL3-specific binders (initial evaluation)

The binders that were verified to have specificity in Example 4 were evaluated in terms of the agonist activity.

These binders were inserted into pLVSIN EF1$\alpha$ in accordance with the method described in the [Preparation of lentivirus] section as the genes encoding the polypeptides comprising membrane-type scFv and Luciferase linked thereto in the construct described below to prepare the lentivirus.

The sequence identification numbers of the amino acid sequences of the heavy chain variable regions and the light chain variable regions of the binders are shown in Table 8. In order to express a membrane protein comprising a binder in an extracellular domain in a target cell, a gene encoding a polypeptide comprising a self-cleaving peptide T2A (SEQ ID NO: 20) and a firefly luciferase (SEQ ID NO: 17) linked in that order to membrane-type scFv comprising a human CD8-derived signal peptide (SEQ ID NO: 23), a Flag tag (SEQ ID NO: 2), a linker (GSG), a variable region (VH) shown in Table 8, a GS(G4S) linker (SEQ ID NO: 25), a variable region (VL) shown in Table 8, a CD8-derived hinge region (SEQ ID NO: 26), a CD8-derived cell membrane domain (SEQ ID NO: 27), and a CD8-derived intracellular domain (SEQ ID NO: 28) from the N terminus was inserted into pLVSIN EF1α in accordance with the method described in the [Preparation of lentivirus] section above to prepare the lentivirus. In Table 9, "a2DL2/3" indicates that a binder binds to 2DL2 and/or 2DL3.

[Table 9]

| Binder | Variable region | CDR1 | CDR2 | CDR3 |
|---|---|---|---|---|
| a2DL2/3-DS1 | VH (SEQ ID NO: 33) | 26-33 | 51-58 | 97-109 |
| | VL (SEQ ID NO: 34) | 27-34 | 52-54 | 91-101 |
| a2DL2/3-DS2 | VH (SEQ ID NO: 35) | 26-33 | 51-58 | 97-109 |
| | VL (SEQ ID NO: 36) | 26-34 | 52-54 | 91-102 |
| a2DL2/3-DS3 | VH (SEQ ID NO: 37) | 26-33 | 51-58 | 97-113 |
| | VL (SEQ ID NO: 38) | 26-34 | 52-54 | 91-101 |
| a2DL2/3-DS4 | VH (SEQ ID NO: 39) | 26-33 | 51-58 | 97-105 |
| | VL (SEQ ID NO: 40) | 27-32 | 50-52 | 89-97 |
| a2DL2/3-DS5 | VH (SEQ ID NO: 41) | 26-33 | 51-58 | 97-110 |
| | VL (SEQ ID NO: 42) | 27-32 | 50-52 | 89-97 |
| a2DL2/3-DS6 | VH (SEQ ID NO: 43) | 26-33 | 51-58 | 97-113 |
| | VL (SEQ ID NO: 44) | 27-33 | 51-53 | 90-98 |
| a2DL2/3-DS7 | VH (SEQ ID NO: 45) | 26-33 | 51-58 | 97-114 |
| | VL (SEQ ID NO: 46) | 27-32 | 50-52 | 89-97 |
| a2DL2/3-DS8 | VH (SEQ ID NO: 47) | 26-33 | 51-58 | 97-111 |
| | VL (SEQ ID NO: 48) | 27-33 | 51-53 | 90-98 |
| a2DL2/3-DS9 | VH (SEQ ID NO: 49) | 26-33 | 51-58 | 97-111 |
| | VL (SEQ ID NO: 50) | 27-33 | 51-53 | 90-98 |
| a2DL2/3-DS10 | VH (SEQ ID NO: 51) | 27-33 | 51-58 | 97-112 |
| | VL (SEQ ID NO: 52) | 27-32 | 50-52 | 89-97 |
| a2DL2/3-DS11 | VH (SEQ ID NO: 61) | 26-33 | 51-58 | 97-110 |
| | VL (SEQ ID NO: 62) | 26-33 | 51-53 | 90-100 |
| a2DL2/3-DS12 | VH (SEQ ID NO: 63) | 26-33 | 51-58 | 97-110 |
| | VL (SEQ ID NO: 64) | 26-33 | 51-53 | 90-100 |
| * In the table, CDR1, CDR2, and CDR3 are each indicated with the amino acid numbers in the amino acid sequence of relevant variable region. The amino acid numbers are specified in accordance with the IMGT definitions. | | | | |

Under the same evaluation conditions as employed in Example 2 and Example 4, the susceptibility to cytocidal activity of membrane-type scFv-K562 of each binder was evaluated using NK92 cell lines expressing various types of KIRs. In order to evaluate the binder-dependent activity, the activity ratio was calculated in accordance with the following equation. In order to further calculate a binder-dependent activity ratio, the activity ratio was calculated while designating the activity of the Luc-K-562 cell line to be 1.

$$\text{KIR-dependent cytocidal activity ratio} = A^{\text{binder-scFv-K562}}/A^{\text{K562}}$$

[A = cytocidal activity (%) to K562 = (luciferase activity of target cell not supplemented with effector cell - luciferase activity of target cell supplemented with effector cell) / luciferase activity of target cell not supplemented with effector cell × 100]

The results are shown in Figure 5. All the binders exerted the agonist activity on the KIR2DL2 and KIR2DL3-NK92 cell lines, and the binders did not activate the KIR2DS1/2/4-NK92 cell line, unlike Lirilumab or Pan2D. Thus, agonist binders specific to inhibitory KIR having the profile as intended were obtained. Since such KIRs are expressed at high frequency on the Allele Frequency Net Database, the specificity and the agonist activity thereof may be applicable to many human subjects.

Example 6: Evaluation of agonist activity of KIR2DL2- and/or KIR2DL3-specific binders using primary NK cells

NK92 cells carrying inhibitory KIR and activating KIR had been used as effector cells. In order to inspect as to whether or not the binders identified in Example 4 would exert the agonist activity on KIRs expressed on the NK cells *in vivo,* the agonist activity was evaluated using NK cells sampled from human peripheral blood mononuclear cells (PBMCs).

An NK cell has a function of missing-self recognition by which the NK cell recognizes its own HLA type (C1, C2, or Bw4) and exerts cytocidal activity on a target cell. For example, an NK cell sampled from a HLA-C1/C1 homozygous human subject recognizes C1 as the self. When a target cell does not have the HLA-C1 type, accordingly, a signal is introduced into an NK cell through an inhibitory receptor, and it exerts the cytocidal activity. In contrast, an NK cell does not exert the cytocidal activity on the target cell of the C1 type. When an agonist binder that can serve as an alternative to the C1 type is expressed, the cytocidal activity can be reduced.

The same applies to HLA-C2 homozygous NK or HLA-Bw4+ NK and different types of inhibitory KIR serve as corresponding to each ligand HLA. KIR2DL2 and KIR2DL3 can serve as missing-self inhibitory receptors on the HLA-C1 homozygous NK cell, KIR2DL1 can serve as a missing-self inhibitory receptor on the HLA-C2 homozygous NK cell, and KIR3DL1 can serve as a missing-self inhibitory receptor on the HLA-Bw4+ NK cell. Thus, the agonist activity on such KIRs can be evaluated.

[Preparation of PBMC-derived NK cell]

As effector cells, 2 types of HLA-C1/C1/Bw4- PBMCs were prepared (referred to as "PBMC-A" and "PBMC-B"). On the basis of the KIR haplotype, PBMC-A was found to have the KIR2DL2, KIR2DL3, KIR2DS1, and KIR2DS2 genes and PBMC-B was found to have the KIR2DL3 gene.

The anti-CD16 antibody (302050, Biolegend) was diluted to 5 μg/ml in PBS and added to the non-treatment dish. The dish was allowed to stand at room temperature for 2 hours or at 4°C overnight and washed with PBS. To the MyeloCult H5100 medium supplemented with IL-2 and IL-18 (9124-IL, R&D systems) at 1000 U/ml and 100 ng/ml, respectively (hereafter referred to as "NK medium"), a suspension of human-derived peripheral blood mononuclear cells (PBMCs, CTL-UP1, Cellular Technology Limited) was added. The cells were collected 7 to 10 days later, and CD56+ cells (NK cells) were purified using CD56 MicroBeads (130-050-401, Miltenyi Biotec). Thereafter, culture was continued in Myelocult containing IL-2/18 again.

[Preparation of T cell expressing target gene]

As target cells, HLA-C1/C1/Bw4-PBMCs (PBMC-A, the same as the effector cells) and HLA-C2/C2/Bw4- PBMCs (PBMC-C) were prepared.

The anti-CD3 antibody (317347, Biolegend) and RetroNectin (T100B, Takara Bio) were diluted to 5 to 10 μg/ml and 20 to 100 μg/ml, respectively, in phosphate buffered saline (PBS) and added to the non-treatment dish. The dish was allowed to stand at room temperature for 2 hours or at 4°C overnight and washed with PBS to prepare a coated plate (hereafter, referred to as the "CD3 Ab/RetroNectin-coated plate").

Solutions of lentiviruses encoding membrane-type scFv of the 10 binders used in Example 5 were prepared. As a control, an artificial gene comprising a polypeptide comprising the human CD19 polypeptide (SEQ ID NO: 53), a self-cleaving peptide T2A (SEQ ID NO: 20), and the firefly luciferase (SEQ ID NO: 17) linked to each other was introduced into pLVSIN EF1α to prepare a lentivirus vector.

The virus concentrate was added to the plate, and the plate was subjected to centrifugation at 2000× g for 2 hours to prepare a virus-bound plate. Human-derived peripheral blood mononuclear cells (PBMCs, CTL-UP1, Cellular Technology Limited) were suspended at 1 to 2 × 10^6 cells/ml in the basal medium supplemented with IL-2 (87-890, Nipro or 58697900, Kyowa Pharmaceutical Industry Co., Ltd.) at the final concentration of 100 U/ml (hereafter, referred to as the "IL-2 medium"), the suspension was added to the virus-bound plate, and the plate was subjected to centrifugation at 300× g for 3 to 5 minutes.

PBMC-A was infected with the human CD19-luciferase, and PBMC-C was infected with the human CD19-luciferase or the lentivirus of the membrane-type scFv-luciferase of the 10 types of binders. The total volume of the medium was exchanged with the IL-2 medium every 1 to 3 days from 3 days after the culture, and cells were selected with the use of the APC-labeled anti-CD19 antibody, the APC-labeled anti-Flag antibody, and the anti-APC Microbeads (130-090-855, Miltenyi) a week after the culture to concentrate target protein positive cells.

[Evaluation of cytocidal activity using PBMC-derived NK cell]

The PBMC-NK cells for using in the cytotoxic assay ($1 \times 10^5$ cells) were added to a 96-well plate, T cells expressing the target gene in an amount one tenth the amount of the PBMC-NK cells (i.e., $1 \times 10^4$ cells) were added to individual wells, and culture was further conducted. All culture procedures were performed using the basal medium. The cytotoxic activity was determined in terms of the number of remaining target cells by measuring the luciferase activity of the T cells expressing the target gene 4 hours later, relative to the number of target cells in the wells not supplemented with the PBMC-NK cells designated to be 100%.

The results are shown in Figure 6.

Because the effector cells are C1/C1/Bw4- cells, apparent cytocidal activity is observed against C2/C2/Bw4- T cells when targeting C2/C2/Bw4- cells compared to C1/C1/Bw4- cells. This is considered to be missing-self recognition.

When the KIR2DL2/3-specific binder was expressed in the C2/C2/Bw4- T cells, in contrast, the cytocidal activity was attenuated, compared with the cytocidal activity when the CD19-luciferase was expressed in the C2/C2/Bw4- T cells. Such results were observed in PBMC-A-derived NK cells and the PBMC-B-derived NK cells, and the KIR2DL2/3-specific binder obtained in Example 5 was found to exert the agonist activity on primary NK cells.

Example 7: Evaluation of binding capability of KIR2DL2- and/or KIR2DL3-specific binders

The binder, the agonist activity of which was examined in Example 6, was evaluated in terms of the binding capability to inhibitory molecules and to activating molecules on the NK cell surface. As a control, Pan2D was used.

The K562 cells expressing the membrane-type a2DL2/3-DS4 scFv or membrane-type a2DL2/3-DS10 scFv prepared in Example 5 or the K562 cells expressing the membrane-type scFv of Pan2D prepared in Example 3 ($1 \times 10^4$ cells) were supplemented with the Fc- or His-tagged recombinant protein at 0.1 μM, and the reaction was allowed to proceed at 4°C for 90 minutes. The reaction product was washed with PBS, the APC-labeled anti-human IgG Fc antibody (409306, BIOLEGEND) or the APC-labeled anti-His tag antibody (362605, BIOLEGEND) was added, and the reaction was allowed to proceed at 4°C for 30 minutes. The reaction product was washed two times with PBS and subjected to flow cytometry to evaluate the binding capability. The binding capability to the K562 cell line (the median fluorescence intensity derived from APC) was designated to be 1, and the ratio relative thereto was calculated.

The recombinant proteins shown in Table 10 were used. As the recombinant proteins indicated as "In house," the same recombinant proteins as shown in Table 7 were used. As Fc-His-Avi, a recombinant KIR protein comprising the IgG Fc domain (SEQ ID NO: 98), the GAA linker, and the Avi tag (SEQ ID NO: 99) linked from the N terminus was prepared.

[Table 10]

| No. | Protein | Addition or tag | Manufacturer | Catalog No. |
|-----|---------|-----------------|--------------|-------------|
| 1 | Fc-His-Avi | Fc | In house | Prepared in-house |
| 2 | KIR2DL1 | Fc | R&D system | 1844-KR-050 |
| 3 | KIR2DL2 | Fc | In house | Prepared in-house |
| 4 | KIR2DL3 | Fc | R&D system | 2014-KR-050 |
| 5 | KIR2DL4 | Fc | R&D system | 2238-KR-050 |
| 6 | KIR2DL5 | Fc | R&D system | 6634-KR-050 |
| 7 | KIR2DS1 | Fc | In house | Prepared in-house |
| 8 | KIR2DS2 | Fc | In house | Prepared in-house |
| 9 | KIR2DS3 | Fc | In house | Prepared in-house |
| 10 | KIR2DS4 | Fc | In house | Prepared in-house |
| 11 | KIR3DL1 | Fc | R&D system | 1225-KR-050 |
| 12 | KIR3DL3 | Fc | R&D system | 10104-KR-050 |

(continued)

| No. | Protein | Addition or tag | Manufacturer | Catalog No. |
|-----|---------|-----------------|--------------|-------------|
| 13 | KIR3DS1 | Fc | R&D system | 4136-KR-050 |
| 14 | NKG2A | Fc | R&D system | 10511-NK-050 |
| 15 | NKG2C | Fc | R&D system | 138-NK-050 |
| 16 | NKG2D | Fc | R&D system | 1299-NK-050 |
| 17 | NKG2E | Fc | R&D system | 9759-NK-050 |
| 18 | NKp30 | Fc | R&D system | 1849-NK-025 |
| 19 | NKp44 | Fc | R&D system | 2249-NK-050 |
| 20 | NKp46 | Fc | R&D system | 1850-NK-025 |
| 21 | CD16a | Fc | R&D system | 4325-FC-050 |
| 22 | CD16b | Fc | R&D system | 1597-FC-050 |
| 23 | CD32a | Fc | R&D system | 1330-CD-050 |
| 24 | CD32b/c | Fc | R&D system | 1875-CD-050 |
| 25 | CD64 | Fc | R&D system | 1257-FC-050 |
| 26 | LILRB1 | Fc | R&D system | 2017-T2-050 |
| 27 | LILRB2 | His | R&D system | 8429-T4-050 |
| 28 | TIGIT | Fc | R&D system | 9464-TG-050 |
| 29 | CD161 | Fc | R&D system | 7448-CD-050 |

The results are shown in Figure 7.

While Pan2D bound to KIR2DS1, 2, and 4, the binder (a2DL2/3-DS4) and the binder (a2DL2/3-DS10) did not bind to KIR2DS1, 2, and 4, and such binders did not bind to other inhibitory receptor or activating receptor.

The results indicate that a binder does not bind to another NK cell receptor to act thereon but binds to KIR2DL2 and/or KIR2DL3 and inhibits activation of an NK cell.

Example 8: Evaluation of agonist activity of KIR2DL1 binder and KIR3DL1 binder

Whether or not a binder that would bind to KIR2DL1 and KIR3DL1 would exert the agonist activity on NK cells expressing various receptors was evaluated.

[Gene introduction into K-562 cell and preparation]

In order to establish the human HLA-C2-expressing K562 cell line (HLA-Cw4-Luc-K562 cell line), an artificial gene encoding a polypeptide comprising a signal peptide shown in Table 11, a Flag tag (SEQ ID NO: 2), and an HLA peptide shown in Table 10 linked in that order from the N terminus and an artificial gene encoding a polypeptide of luciferase (SEQ ID NO: 17) downstream of the internal ribosome entry site (IRES) were inserted into pLVSIN EF1α in accordance with the method described in the [Preparation of lentivirus] section above to prepare the lentivirus.

[Table 11]

| Target HLA | IPD Accession No. | Cell line | Signal peptide sequence | HLA sequence |
|------------|-------------------|-----------|-------------------------|--------------|
| Human HLA-C0401 | HLA00420 | HLA-Cw4-K562 cell line | SEQ ID NO: 21 | SEQ ID NO: 54 |

The sequence of the anti-3DL1 antibody was quoted from WO 2018/148223 A1, a polypeptide was linked in the construct described below, and the resultant was inserted into pLVSIN EF1α in accordance with the method described in the [Preparation of lentivirus] section above to prepare the lentivirus.

Table 12 shows the sequence identification numbers of the amino acid sequences of the heavy chain variable regions and the light chain variable regions of the binders. In order to express a membrane protein comprising a binder in the extracellular domain in a target cell, a gene encoding a polypeptide comprising a self-cleaving peptide T2A(SEQ ID NO: 20) and the firefly luciferase (SEQ ID NO: 17) linked in that order to the C terminus of the membrane-type scFv comprising the human CD8-derived signal peptide (SEQ ID NO: 23), the Flag tag (SEQ ID NO: 2), the linker (GSG), a variable region (VL) shown in Table 12, the GS(G4S) linker (SEQ ID NO: 25), variable region (VH) shown in Table 12, the sequence of the CD8-derived hinge region (66 amino acids) (SEQ ID NO: 24), the CD8-derived cell membrane domain (SEQ ID NO: 27), and the CD8-derived intracellular domain (SEQ ID NO: 28) from the N terminus was inserted into pLVSIN EF1α in accordance with the method described in the [Preparation of lentivirus] section above to prepare the lentivirus.

[Table 12]

| Binder | Variable region | CDR1 | CDR2 | CDR3 |
|---|---|---|---|---|
| a3DL1-DS1 | VL (SEQ ID NO: 55) | 26-33 | 49-51 | 88-98 |
| | VH (SEQ ID NO: 56) | 26-33 | 51-58 | 97-109 |
| * In the table, CDR1, CDR2, and CDR3 are each indicated with the amino acid numbers in the amino acid sequence of relevant variable region. The amino acid numbers are specified in accordance with the IMGT definitions. | | | | |

RetroNectin (T100B, Takara Bio) was diluted to 20 to 100 μg/ml in phosphate buffered saline (PBS) and added to the non-treatment dish. The dish was allowed to stand at room temperature for 2 hours or at 4°C overnight and washed with PBS to prepare a coated plate.

K562 cells were suspended at 1 to $2\times10^6$ cells/ml in PRMI medium. The lentivirus concentrate encoding HLA or the membrane-type scFv of a binder was mixed with a suspension of NK cells on a RetroNectin-coated plate, and the mixture was centrifuged at $1000\times$ g and 32°C for 1 hour. Thereafter, culture was conducted at 37°C in the presence of 5% $CO_2$. The total volume of the medium was exchanged with the RPMI medium every 1 to 3 days from 3 days after the culture, and positive cells were selected with the use of the APC-labeled anti-Flag antibody and the anti-APC Microbeads (130-090-855, Miltenyi Biotec) 1 week after the culture.

According to need, the collected cells were suspended at 0.5 to 1 $\times$ $10^7$ cells/ml in CELLBANKER 1 (CB011, Takara Bio) and cryopreserved at -80°C or -150°C. When frozen cells were used in experimentation, the cells were thawed and cultured in the RPMI medium before use.

Concerning the anti-2DL1 antibody, Pan2D binding to KIR2DL1 was used. Specifically, the Pan2D-scFv-K562 cell line of Example 2 was used.

Under the same evaluation conditions as employed in Example 2 and Example 4, the susceptibility to cytocidal activity of membrane-type scFv-K562 of each binder was evaluated using NK92 cell lines expressing various types of KIRs, and the activity was calculated in accordance with the following equation.

Cytocidal activity (%) to K562 = (luciferase activity of target cell not supplemented with effector cell - luciferase activity of target cell supplemented with effector cell) / luciferase activity of target cell not supplemented with effector cell $\times$ 100

The results are shown in Figure 8.

HLA-Cw4, which is a positive control, exerted the agonist activity on the KIR2DL1-NK92 cell line. In this system, Pan2D exerted the agonist activity on KIR2DL1, and a3DL1-DS1 exerted the agonist activity on KIR3DL1.

Thus, a binder exerting the agonist activity on KIR2DL1 and a binder exerting the agonist activity on KIR3DL1 were identified.

Example 9: Evaluation of influence of agonist activity of KIR2DL2- and/or KIR2DL3-specific binders on drug efficacy of CAR-T cell using primary NK cell

With the use of the PBMC-derived NK cells as primary NK cells, CAR-T cells prepared from the same PBMCs as the PBMC-derived NK cells or CAR-T cells prepared from PBMCs derived from a different donor were evaluated in terms of the cytocidal activity thereof on the target cells in the presence of PBMC-derived NK cells.

In the presence of primary NK cells, CAR-T cells derived from a different donor in which HLA matching the inhibitory KIR of primary NK cells is not expressed are attacked by NK cells through the mechanism of missing-self recognition.

In Example 6, the binders identified in Example 4 were found to exert the agonist activity on primary NK cells and inhibit the cytocidal activity of the primary NK cells. Whether or not inhibition of the cytocidal activity of the primary NK cells would play a key role in maintenance of the cytocidal activity of the CAR-T cells was examined using PBMC-derived NK cells as the primary NK cells, mixing 3 types of cells; i.e., PBMC-derived NK cells, CAR-T cells derived from a donor different from the donor of the PBMC-derived NK cells or CAR-T cells derived from a donor different from the donor of the PBMC-derived NK cells expressing the binder identified in Example 4, and cells on which the CAR-T cells exert the CAR-mediated cytocidal activity, and evaluating the cytocidal activity of the CAR-T cells on the target cells.

The PBMC-A-derived NK cells prepared in accordance with the method described in the [Preparation of PBMC-derived NK cell] section were suspended in CELLBANKER 1 (CB011, Takara Bio), the suspension was cryopreserved at -80°C or -150°C, the cryopreserved product was thawed 2 days before the test, and the resultant was then cultured in the MyeloCult H5100 medium supplemented with IL-2 and IL-18. On the day before the test, the medium for the PBMC-A-derived NK cells was exchanged with the IL-2- or IL-18-free MyeloCult H5100 medium, and culture was then conducted.

[Preparation of T cell expressing CAR and KIR2DL2/-specific binder]

In order to prepare CAR-expressing T cells, HLA-C2/C2/Bw4- PBMCs (PBMC-C) were used.

In order to express anti-CD19 CAR (CAR described in the report titled that T-cell clones can be rendered specific for CD19: Toward the selective augmentation of the graft-versus-B-lineage leukemia effect, L. J. Cooper et al., Blood, Feb 15 2003; 101 (4): 1637-44) (hereafter, referred to as "CD19 CAR"), an artificial gene encoding a CD8-derived signal peptide (SEQ ID NO: 23), CD19 CAR VL (SEQ ID NO: 100), a GS(G4S) linker (SEQ ID NO: 25), CD19 CAR VH (SEQ ID NO: 101), the CD8-derived hinge region (SEQ ID NO: 26), a CD8-derived cell membrane domain for CAR (SEQ ID NO: 102), an intracellular costimulatory molecule (SEQ ID NO: 103), a linker (GSG), a self-cleaving peptide T2A (SEQ ID NO: 20), and a blue fluorescent protein (BFP) (SEQ ID NO: 104) from the N terminus was inserted into pLVSIN EF1$\alpha$, and a lentivirus concentrate comprising anti-CD19 CAR was prepared by the method described in the [Preparation of lentivirus] section. In order to express membrane-type a2DL2/3-DS10 scFv, a lentivirus concentrate encoding membrane-type a2DL2/3-DS10 scFv was prepared using the plasmid prepared in Example 4 by the method described in the [Preparation of lentivirus] section. As a control artificial gene, a gene encoding a polypeptide comprising a NGFR peptide (SEQ ID NO: 58), a self-cleaving peptide T2A (SEQ ID NO: 20), and a firefly luciferase (SEQ ID NO: 17) positioned in that order from the N terminus (hereafter, referred to as "NGFR-Luc") was employed, and a lentivirus concentrate comprising NGFR-Luc was prepared in the same manner.

PBMC-C was suspended in the AIM V medium (12055-083, Thermo Fisher Scientific) supplemented with IL-2 (87-890, Nipro) and FCS (10270-106, Thermo Fisher Scientific) (5(v/v)%) and streptomycin/penicillin (15240-062, Thermo Fisher Scientific) (hereafter, referred to as the "IL-2 medium"), the suspension was mixed with the prepared lentivirus concentrate (the lentivirus encoding NGFR-Luc, the lentivirus encoding membrane-type a2DL2/3-DS10 scFv, the lentivirus encoding CD19 CAR, or a mixture of the lentivirus encoding CD19 CAR and the lentivirus encoding membrane-type a2DL2/3-DS10 scFv), and the mixture was added to the CD3 Ab/RetroNectin-coated plate described in the [Preparation of T cell expressing target gene] section. The resultant was centrifuged using a centrifuge for 1 hour. On the following day, the virus concentrate as used on the previous day was added to the wells, and centrifugation was performed using a centrifuge for 1 hour.

The total volume of the medium was adequately exchanged with the IL-2 medium, culture was conducted, PBMC-C infected with the lentivirus encoding membrane-type a2DL2/3-DS10 scFv or the mixture of the lentivirus encoding CD19 CAR and the lentivirus encoding membrane-type a2DL2/3-DS10 scFv was subjected to concentration of membrane-type a2DL2/3-DS10 scFv$^+$ cells using the APC-labeled anti-Flag antibody and anti-APC Microbeads (130-090-855, Miltenyi Biotec). Also, PBMC-C infected with the lentivirus encoding NGFR-Luc was subjected to concentration of NGFR$^+$ cells using anti-LNGFR Microbeads (130-099-023, Miltenyi Biotec). As a result, PBMC-C-derived T cells expressing membrane-type a2DL2/3-DS10 scFv were obtained. The PBMC-C-derived T cells infected with the mixture of the lentivirus encoding CAR and the lentivirus encoding membrane-type a2DL2/3-DS10 scFv are classified as cells expressing both CAR and a2DL2/3-DS10 scFv, cells expressing either CAR and a2DL2/3-DS10 scFv, or cells expressing neither thereof. As a result of concentration, a mixture of PBMC-C-derived T cells expressing both of CD19 CAR and membrane-type a2DL2/3-DS10 scFv and PBMC-C-derived T cells selectively expressing membrane-type a2DL2/3-DS10 scFv was obtained. PBMC-C-derived T cells expressing NGFR-Luc were also obtained.

NGFR-Luc-expressing PBMC-C-derived T cells and membrane-type a2DL2/3-DS10 scFv-expressing PBMC-C-derived T cells were concentrated, the total volume of the medium was adequately exchanged with the IL-2 medium, culture was conducted, the culture product was suspended in CELLBANKER 1 (CB011, Takara Bio), and the suspension was cryopreserved at -80°C or -150°C.

PBMC-C selectively infected with the lentivirus encoding CD19 CAR and the mixture of the PBMC-C-derived T cells expressing CD19 CAR and membrane-type a2DL2/3-DS10 scFv and the PBMC-C-derived T cells selectively expressing membrane-type a2DL2/3-DS10 scFv obtained as a result of the concentration were cultured, the total volume of the culture product was suspended in the IL-2 medium, and the suspension was added to the CD19-coated plate coated with the CD19 human recombinant protein (TP302922, Origene). After the culture, concentration of the PBMC-C-derived T cells expressing CD19 CAR or PBMC-C-derived T cells expressing CD19 CAR and membrane-type a2DL2/3-DS10 scFv was confirmed, the cells were suspended in CELLBANKER 1 (CB011, Takara Bio), and the suspension was cryopreserved at -80°C or -150°C.

The PBMC-C-derived T cells were thawed on the day before the test and culture in the IL-2 medium.

[Evaluation of cytocidal activity of CAR-T cell on target cell in the presence of PBMC-derived NK cell]

Raji cells constitutively expressing CD19 with virally introduced GFP (hereafter referred to as "Raji-GFP") were used as targets for CD19 CAR-T cells. Samples containing Raji-GFP cells and PBMC-A-derived NK cells and samples containing only Raji-GFP cells were prepared. Then, PBMC-C-derived T cells expressing NGFR-Luc, PBMC-C-derived T cells selectively expressing CD19 CAR, PBMC-C-derived T cells selectively expressing membrane-type a2DL2/3-DS10, or PBMC-C-derived T cells expressing CD19 CAR and membrane-type a2DL2/3-DS10 scFv were added at a ratio of 1:1, 1:0.5, or 1:0.1, relative to the amount of the Raji-GFP cells, and culture was conducted on a 96-well plate. With the use of the MyeloCult H5100 medium, the AIM V medium supplemented with FCS at 5(v/v)%, the RPMI 1640 medium supplemented with FCS at 10(v/v)%, and GlutaMAX (all the media are supplemented with streptomycin/penicillin), cell suspensions were mixed to contain equivalent amounts of the media.

The cell count in each well was measured by the method described in the [Flow cytometry and antibody to be added] section 4 days after the initiation of culture. As the antibody to detect PBMC-C-derived T cells, the APC/Fire 750 anti-human CD3 antibody (300470, BIOLEGEND) was used. GFP was used to detect the Raji-GFP cells.

Figure 27-1 shows the results of plotting, on the vertical axis, the ratio of the viable Raji cell count in each well and, on the horizontal axis, the ratio of the T cells relative to the Raji-GFP cells in each well at the beginning of the culture. The number of viable Raji cells in the wells containing only the Raji-GFP cells is designated to be 100%.

In wells where PBMC-A-derived NK cells were not cocultured, the cytotoxic activity of the PBMC-C-derived T cells only expressing CD19 CARs on the Raji-GFP cells was equivalent to that of PBMC-C-derived T cells expressing CD19 CAR and membrane-type a2DL2/3-DS10 scFv on the Raji-GFP cells. In wells where PBMC-A-derived NK cell were cocultured, in contrast, the cytotoxic activity of the PBMC-C-derived T cells only expressing CD19 CARs on the Raji-GFP cells was attenuated, compared to that of the PBMC-C-derived T cells expressing CD19 CAR and membrane-type a2DL2/3-DS10 scFv on the Raji-GFP cells.

In wells where PBMC-A-derived NK cells were cocultured, as shown in Figure 27-2, the number of PBMC-C-derived T cells only expressing CD19 CARs is decreased, compared with the number of PBMC-C-derived T cells expressing CD19 CAR and membrane-type a2DL2/3-DS10 scFv. It seemed that the number of the PBMC-C-derived T cells only expressing CD19 CARs was decreased because they were attacked by the PBMC-A-derived NK cells through the mechanism of missing-self recognition. In contrast, the number of the PBMC-C-derived T cells expressing CD19 CAR and membrane-type a2DL2/3-DS10 scFv was not decreased because such T cells exerted the agonist activity on inhibitory KIR mediated by 2DL2/3-DS10 on the PBMC-A-derived NK cells and avoided the attack by the PBMC-A-derived NK cells.

This indicates that CAR-T cells expressing the binders identified in Example 4 would inhibit the cytocidal activity of primary NK cells and maintain the cytocidal activity of the CAR-T cells on the target cells more efficiently, compared with the CAR-T cells not expressing the binder identified in Example 4 in the presence of primary NK cells.

Example 10: Evaluation of influence of modification of CDR in KIR2DL2- and/or KIR2DL3-specific binders on agonist activity

The influence of substitution of a particular amino acid residue with alanine in a variable region (VH) CDR3 of a2DL2/3-DS10 on the agonist activity on inhibitory KIR was evaluated.

In the membrane-type a2DL2/3-DS10 scFv, each amino acid residue was substituted with alanine in CDR3 (amino acids 97 to 112 in SEQ ID NO: 51) of a heavy chain variable region (VH) of a2DL2/3-DS10 to prepare membrane-type a2DL2/3-DS10 scFv variants, and an artificial gene comprising the membrane-type a2DL2/3-DS10 scFv variants integrated into the membrane-type scFv expression format in the order VH-VL was inserted into pLVSIN EF1α. Table 13 shows the variant name and the amino acid substituted with alanine.

[Table 13]

| Variant | VH CDR3 sequence |
|---------|------------------|
| R98A | SEQ ID NO: 67 |
| V99A | SEQ ID NO: 68 |
| S100A | SEQ ID NO: 69 |
| G101A | SEQ ID NO: 70 |

(continued)

| Variant | VH CDR3 sequence |
|---------|------------------|
| T102A | SEQ ID NO: 71 |
| T103A | SEQ ID NO: 72 |
| G104A | SEQ ID NO: 73 |
| G105A | SEQ ID NO: 74 |
| Y106A | SEQ ID NO: 75 |
| Y107A | SEQ ID NO: 76 |
| Y108A | SEQ ID NO: 77 |
| G109A | SEQ ID NO: 78 |
| M110A | SEQ ID NO: 79 |
| D111A | SEQ ID NO: 80 |
| V112A | SEQ ID NO: 81 |

With the use of pLVSIN EF1$\alpha$ encoding a membrane-type scFv variant, the lentivirus was prepared by the method described in the [Preparation of lentivirus] section. With the use of the lentivirus prepared, K562 cell lines expressing membrane-type scFv variants were prepared by the method described in the [Introduction of scFv into K-562 cell and preparation] section.

With the use of the NK92 cell line, the KIR2DL3-NK92 cell line, and the KIR2DS2-NK92 cell line used in Example 5, the cytocidal activity of each NK92 cell line on the K562 cell lines expressing the membrane-type scFv variant was evaluated by the method described in the [Evaluation of cytocidal activity of KIR2DL2-expressing NK92 on Lirilumab-scFv/Pan2D-scFv-K562] section.

The results are shown in Figure 28. In the variants R98A, S100A, G101A, T102A, G105A, Y106A, and Y107A, the agonist activity on KIR2DL3 was observed to be attenuated or lost, in comparison with the intact membrane-type a2DL2/3-DS10 WT scFv. Meanwhile, variants other than those indicated above exerted the agonist activity equivalent to that of the membrane-type a2DL2/3-DS10 WT scFv. This indicates that the rest of the amino acids, except for the 7 amino acids mentioned above, whose changes result in the loss of inhibition, are not critical for the agonist activity. In addition, these variants did not exert the agonist activity on KIR2DS2, which is activating KIR exhibiting the highest homology to KIR2DL2/3.

The above results indicate that modification of an amino acid sequence in CDR of a binder having the agonist activity specific to inhibitory KIR may lead to production of a binder having equivalent properties. The results also indicate that amino acids R98, S100, G101, T102, G105, Y106, and Y107 in CDR3 of a2DL2/3-DS10 play a key role in the agonist activity specific to inhibitory KIR.

Example 11: Evaluation concerning CDR of KIR2DL2- and/or KIR2DL3-specific binders as to whether or not CDR grafted into constant region of different scFv maintains KIR2DL2- and/or KIR2DL3-specific agonist activity

In order to confirm that the specificity to KIR2DL2 and/or KIR2DL3 would be maintained if only CDRs of the KIR2DL2- and/or KIR2DL3-specific binders were grafted, CDRs in heavy and light chains of a2DL2/3-DS1, a2DL2/3-DS2, or a2DL2/3-DS3 were grafted into constant regions of a2DL2/3-DS4 excluding CDR (hereafter, referred to as "a2DL2/3-DS1-2" "a2DL2/3-DS2-2," or "a2DL2/3-DS3-2"). They were expressed as a membrane-type scFv in K562 cells and the agonist activity on inhibitory KIR was evaluated.

An artificial gene comprising a2DL2/3-DS1-2, a2DL2/3-DS2-2, or a2DL2/3-DS3-2 integrated into a membrane-type scFv expression format was inserted into pLVSIN EF1$\alpha$. Table 14 shows the amino acid sequences of CDR-grafted constructs.

[Table 14]

| CDR-grafted body | VH amino acid sequence | VL amino acid sequence |
|------------------|------------------------|------------------------|
| a2DL2/3-DS1-2 | SEQ ID NO: 82 | SEQ ID NO: 83 |
| a2DL2/3-DS2-2 | SEQ ID NO: 84 | SEQ ID NO: 85 |

(continued)

| CDR-grafted body | VH amino acid sequence | VL amino acid sequence |
|---|---|---|
| a2DL2/3-DS3-2 | SEQ ID NO: 86 | SEQ ID NO: 87 |

With the use of pLVSIN EF1α encoding CDR-grafted membrane-type scFv, the lentivirus was prepared by the method described in the [Preparation of lentivirus] section. With the use of the lentivirus prepared, K562 cell lines expressing various CDR-grafted membrane-type scFv were prepared by the method described in the [Introduction of scFv gene into K-562 cell and preparation] section. K562 cells expressing other target genes as used in Example 5 were used herein.

With the use of the NK92 cell line, the KIR2DL2-NK92 cell line, the KIR2DL3-NK92 cell line, the KIR2DS1-NK92 cell line, the KIR2DS2-NK92 cell line, and the KIR2DS4-NK92 cell line used in Example 5, the cytocidal activity of each NK92 cell line on a K562 cell line expressing each CDR-grafted membrane-type scFv was evaluated by the method described in the [Evaluation of cytocidal activity of KIR2DL2-expressing NK92 on Lirilumab-scFv/Pan2D-scFv-K562] section.

The results are shown in Figure 29. A K562 cell line expressing a CDR-grafted membrane-type scFv was found to exert the agonist activity on KIR2DL2 and KIR2DL3 equivalent to the agonist activity of the K562 cell line expressing a CDR-non-grafted membrane-type a2DL2/3-DS1, membrane-type a2DL2/3-DS2, or membrane-type a2DL2/3-DS3. However, a CDR-grafted membrane-type scFv did not exert the agonist activity on KIR2DS1, KIR2DS2, or KIR2DS4, unlike scFv of Pan2D.

The results indicate that CDR of KIR2DL2- and/or KIR2DL3-specific binders grafted into a constant region of a different scFv maintains KIR2DL2- and/or KIR2DL3-specific agonist activity. This indicates that a CDR sequence is important for the KIR2DL2- and/or KIR2DL3-specific agonist activity and such specificity is maintained even when it is grafted into a different constant region.

Example 12: Evaluation of additive inhibitory activity on cytocidal activity of NK cell upon coexpression of KIR2DL2- and/or KIR2DL3-specific binders and HLA-E

In order to examine as to whether or not coexpression of membrane-type a2DL2/3-DS4 scFv or membrane-type a2DL2/3-DS10scFv and HLA-E would lead to higher inhibitory activity on the cytocidal activity of NK cells coexpressing KIR2DL2 or KIR2DL3 and NKG2A, the cytocidal activity of NK92 cells coexpressing KIR2DL2 or KIR2DL3 and NKG2A on K562 cells coexpressing membrane-type a2DL2/3-DS4 scFv or membrane-type a2DL2/3-DS10 scFv and HLA-E was evaluated.

In order to prepare a lentivirus to transduce HLA-E, an artificial gene encoding a polypeptide comprising a human B2M-derived signal peptide (SEQ ID NO: 18), a HLA-G signal peptide-derived peptide (SEQ ID NO: 88), a GS(G4S) linker (SEQ ID NO: 25), a human B2M peptide (SEQ ID NO: 19), a GS(G4S)4 linker (SEQ ID NO: 89), and a HLA-E peptide (SEQ ID NO: 90) linked in that order from the N terminus and comprising a self-cleaving peptide T2A (SEQ ID NO: 20) and a firefly luciferase (SEQ ID NO: 17) linked in that order to the C terminus was inserted into pLVSIN EF1α.

With the use of pLVSIN EF1α encoding HLA-E, a lentivirus was prepared in accordance with the method described in the [Preparation of lentivirus] section. With the use of the lentivirus prepared, the K562 cells expressing the membrane-type a2DL2/3-DS4 scFv or membrane-type a2DL2/3-DS10 scFv prepared in Example 4 were infected with the lentivirus by the method described in the [Introduction of scFv gene into K-562 cell and preparation] section to prepare the K562 cell line expressing a HLA-E trimer comprising a HLA-G signal peptide, B2M, and HLA-E linked to each other via linkers (hereafter, referred to as "tHLA-E") and the membrane-type a2DL2/3-DS4 scFv or membrane-type a2DL2/3-DS10 scFv.

NK92 cells constitutively express NKG2A. With the use of the NK92 cell line, the KIR2DL2-NK92 cell line, and the KIR2DL3-NK92 cell line used in Example 5, the cytocidal activity of the NK92 cell line on the K562 cell line expressing tHLA-E and membrane-type a2DL2/3-DS4 scFv or membrane-type a2DL2/3-DS10 scFv was evaluated by the method described in the [Evaluation of cytocidal activity of KIR2DL2-expressing NK92 on Lirilumab-scFv/Pan2D-scFv-K562] section.

The results are shown in Figure 30. The K562 cell line expressing tHLA-E and the membrane-type a2DL2/3-DS4 scFv or membrane-type a2DL2/3-DS10 scFv exerted the higher inhibitory activity on the cytocidal activity on the KIR2DL2-NK92 cell line and the KIR2DL3-NK92 cell line, compared with that of the K562 cell line expressing only tHLA-E or the K562 cell line expressing only the membrane-type a2DL2/3-DS4 scFv or membrane-type a2DL2/3-DS10 scFv.

The results demonstrate that coexpression of KIR2DL2- and/or KIR2DL3-specific binders and HLA-E would exert the synergistic inhibitory activity on the cytocidal activity on NK cells coexpressing KIR2DL2 and/or KIR2DL3 and NKG2A.

Example 13: Evaluation of inhibition of cytocidal activity of NK cell on iPS cell not expressing class I HLA but expressing KIR2DL2- and/or KIR2DL3-specific binders

iPS cells that do not express class I HLA are attacked by NK cells through the mechanism of missing-self recognition. In order to evaluate as to whether or not iPS cells that do not express class I HLA but express the KIR2DL2- and/or KIR2DL3-specific binders would inhibit the cytocidal activity of NK cells, the KIR2DL2- and/or KIR2DL3-specific binders were introduced into the iPS cells in which class I HLA expression was lacked due to deletion of B2M by gene editing. The iPS cells were cocultured with the PBMC-derived NK cells, and the cytocidal activity of the PBMC-derived NK cells on the iPS cells was evaluated.

In order to prepare iPS cells that do not express class I HLA but express the KIR2DL2- and/or KIR2DL3-specific binders, the membrane-type a2DL2/3-DS10 scFv was introduced into iPS cells genetically modified to delete B2M using CRISPR/CAS9 (hereafter, referred to as "B2M KO iPS cells"). As a control, NGFR-Luc was introduced into wild-type iPS cells or B2M KO iPS cells. A lentivirus concentrate of the membrane-type a2DL2/3-DS10 scFv and that of NGFR-Luc were prepared with the use of the plasmid as used in Example 4 and the plasmid as used in Example 9, respectively, by the method described in the [Preparation of lentivirus] section.

The mTeSR Plus cGMP medium (100-0276, VERITAS) (hereafter, referred to as the "mTeSR medium") was supplemented with iMatrix-511 silk (892021, Nippi), and the resultant was supplemented with CultureSureR Y-27632 (034-24024, WAKO). The iPS cells detached with the use of Accutase (ICT) (AT104, Funakoshi) were suspended in the medium. The prepared lentivirus concentrate and the iPS cell suspension were added to a RetroNectin-coated plate and centrifuged using a centrifuge. On the following day, the medium was exchanged with the mTeSR medium, culture was conducted, and positive cells were then selected using the APC-labeled anti-Flag antibody and anti-APC Microbeads.

Concerning the iPS cells used for cytocidal activity evaluation, the total volume of the medium was exchanged with the mTeSR medium containing Recombinant Human IFN-γ (300-02, PEPROTECH) 3 days, 2 days, and a day before the test.

The PBMC-A-derived NK cells prepared in accordance with the method described in the [Preparation of PBMC-derived NK cell] section were suspended in CELLBANKER 1 (CB011, Takara Bio), the suspension was cryopreserved at -80°C or -150°C, the cryopreserved product was thawed 2 days before the test, and the resultant was then cultured in the MyeloCult H5100 medium supplemented with IL-2 and IL-18 (9124-IL, R&D systems). On the day before the test, the medium for the PBMC-A-derived NK cells was exchanged with the IL-2- or IL-18-free MyeloCult H5100 medium, and culture was then conducted.

The PBMC-A-derived NK cells were mixed with wild-type iPS cells, B2M KO iPS cells, or B2M KO iPS cells expressing membrane-type a2DL2/3-DS10 scFv, and culture was conducted on a 96-well plate. With the use of the MyeloCult H5100 medium and the mTeSR medium supplemented with CultureSureR Y-27632, cell suspensions were mixed to contain equivalent amounts of the media. The luciferase activity in each well was evaluated using ONE-Glo™ Luciferase Assay System 2 hours after the initiation of culture. The value obtained by subtracting the luciferase activity in the wells supplemented with the PBMC-A-derived NK cells from the luciferase activity in the wells not supplemented with the PBMC-A-derived NK cells was calculated as the cytocidal activity, relative to the luciferase activity in the wells not supplemented with the PBMC-A-derived NK cells, designated to be 100%.

The results are shown in Figure 31. The B2M KO iPS cells cannot inhibit the cytocidal activity of the PBMC-derived NK cells compared to the wild-type iPS cells. In contrast, the B2M KO iPS cells expressing membrane-type a2DL2/3-DS10 scFv exerted the effects of inhibiting the cytocidal activity of the PBMC-derived NK cells equivalent to those of the wild-type iPS cells.

The results demonstrate that iPS cells not expressing class I HLA are attacked by NK cells through the mechanism of missing-self recognition; however, the cytocidal activity of NK cells is inhibited as a result of expression of the KIR2DL2- and/or KIR2DL3-specific binders in the iPS cells not expressing class I HLA. In addition, the case of iPS cells demonstrate that inhibition of NK cells by KIR of the KIR2DL2- and/or KIR2DL3-specific binders are effective in cells which lack class I HLA expression by gene editing, in addition to the cell lines not expressing class I HLA without gene editing as in the case of K562.

Example 14: Identification of epitope of KIR2DL2- and/or KIR2DL3-specific binder to KIR2DL2 by X-ray crystallography

In order to identify the epitope of the KIR2DL2- and/or KIR2DL3-specific binders to KIR2DL2, the complex of the Fab fragment of the KIR2DL2- and/or KIR2DL3-specific binders and the recombinant KIR2DL2 was crystallized and subjected to X-ray crystallography.

As the Fab fragment of the KIR2DL2- and/or KIR2DL3-specific binders, the Fab fragment of a2DL2/3-DS10 was used (the amino acid sequence thereof is shown in Table 15). As the recombinant KIR2DL2, a recombinant protein comprising the IL-2-derived signal peptide (SEQ ID NO: 91), KIR2DL2 (22-225) (amino acids 1 to 204 in SEQ ID NO: 6), and the His tag (SEQ ID NO: 92) linked in that order from the N terminus was used.

Protein Deglycosylation Mix II (340 μl) (P6044S, NEW ENGLAND BioLabs) was added to 9 mg of KIR2DL2 (22-225)-His, and the reaction was allowed to proceed at 4°C overnight. The reaction solution was added to Ni Sepharose 6 Fast Flow (17-5318-03, Cytiva) equilibrated with 50 mM HEPES (pH 8), 500 mM NaCl, 20 mM imidazole, and 5%(v/v) glycerol, and the column was then washed with 50 mM HEPES (pH 8), 500 mM NaCl, 250 mM imidazole, and 5%(v/v) glycerol. Subsequently, elution was performed with 50 mM HEPES (pH 8), 500 mM NaCl, 500 mM imidazole, and 5%(v/v) glycerol. The collected deglycosylated KIR2DL2(22-225)-His fraction was purified using HiLoad 16/600 Superdex 200 pg (28989336, Cytiva) equilibrated with PBS(-).

The deglycosylated KIR2DL2(22-225)-His fraction was mixed with the Fab fragment of a2DL2/3-DS10 to bring the ratio thereof to 1.2:1 by mole, and the mixture was incubated at 4°C for 1 hour. The mixture was added to HiLoad 16/600 Superdex 200 pg (Cytiva) equilibrated with 20 mM Tris-HCl (pH 7.5) and 150 mM NaCl, and a complex of deglycosylated KIR2DL2(22-225)-His and 61_3092_02_F10_Fab was purified.

The resulting a2DL2/3-DS10 Fab-KIR2DL2 complex was concentrated to 19 mg/ml and subjected to crystallization. Crystallization was performed by vapor diffusion. To 0.1 to 0.2 μl of the protein solution, equivalent amounts of a precipitant solution (10% (w/v) Polyethylene glycol 3350 (HR2-527, Hampton Research), 0.2 M calcium acetate (HR2-567, Hampton Research), and 0.1 M imidazole-hydrochloric acid (pH 7.5, 19004-35, Nacalai Tesque) were added to prepare a solution, the resulting solution was accommodated in a sealed container containing 0.05 ml of a precipitant solution while avoiding the contact therebetween, and the container was allowed to stand at 20°C. A single crystal (0.2 mm × 0.1 mm × 0.05 mm) was obtained 3 days later.

The resulting crystal was soaked in a precipitant solution supplemented with 35%(v/v) Polyethylene glycol 3350 and then frozen in liquid nitrogen. The X-ray diffraction data were collected under a 95 K nitrogen stream on beamline BL-17A of the Photon Factory, the High Energy Accelerator Research Organization. From the diffraction image obtained, the diffraction intensity was digitized using the XDS software (produced by Wolfgang Kabsch, Max Planck Institute for Medical Research) to determine the crystal structure factors. The crystal was found to belong to the monoclinic crystal system and the space group C121 and have unit lattice constants of a = 170.11 Å, b = 79.24 Å, c = 64.36 Å, and β = 92.39°.

Structural analysis was performed using a software package Phenix (Python-Based Hierarchical Environment for Integrated Xtallography). With the use of the software Phaser, molecular replacement was performed using the tertiary structure coordinates of the structural factors, KIR2DL2 (PDB code: 2DL2), and Fab (PDB code: a similar part is extracted from 1MIM) to determine the phase. The asymmetric unit of the crystal contained a complex.

Structure refinement was performed with the use of a software phenix.refine, and model modification was performed with the use of the software Coot. This procedure was repeated to obtain the final R-value of 23.4% and the free R-value of 27.5% at 2.5 Å resolution. The model is composed of a complex comprising amino acids 1 to 210 of the VL chain and amino acids 1 to 224 of the H chain of a2DL2/3-DS10 Fab, amino acids 25 to 221 of KIR2DL2, 2 N-acetylglucosamine residues, and 14 water molecules. The current density of each of the 2 C-terminal residues of the L chain and the 6 C-terminal residues and 8 residues from Ser137 to Glu144 of the H chain of a2DL2/3-DS10 Fab and the C-terminal 10 residues including the His tag of KIR2DL2 was not clear. Accordingly, models thereof were not constructed.

Figure 32-1 shows a ribbon model of the entire complex. Amino acid residues of KIR2DL2 located within 4Å distance from the a2DL2/3-DS10 Fab are as indicated below: Glu42, His61, Lys65, Phe66, Lys67, Asp68, Thr69, Leu70, Ser86, Ile87, Gly88, Pro89, Met91, Gln92, Asp93, Leu94, Tyr101, and Leu111. Figure 32-2 shows the amino acids constituting the epitope of KIR2DL2 of a2DL2/3-DS10 highlighted on the amino acid sequence of KIR2DL2 in comparison with the amino acid sequence of activating KIR. As the amino acid sequences of each KIR, the signal peptide and receptor sequences shown in Table 5 were used. The amino acid number of each KIR was determined by binding the signal peptide and the receptor sequence shown in Table 4 and assigning a number from 1. Specifically, the number of each amino acid in the epitope of KIR2DL2 was determined by adding 21 (i.e., the amino acid length of the signal peptide) to the number of the corresponding amino acid in the amino acid sequence as shown in SEQ ID NO: 6. Also, the number of each amino acid in corresponding KIR2DS1/2/4 was determined by adding 21 to the number of the corresponding amino acid in the amino acid sequence as shown in SEQ ID NO: 10, 12, or 14, respectively.

With the use of the software Maestro (SCHRODINGER), amino acid residues that play a key role in a2DL2/3-DS10 selectivity were analyzed. As a result of analysis, an amino acid corresponding to the amino acid Gly88 of KIR2DL2 was found to be the amino acid Ser88 in KIR2DS1. Occurrence of steric hindrance with the amino acid Gly104 of a2DL2/3-DS10 VH CDR3 indicates that the amino acid Gly104 that plays a key role in KIR2DL2 selectivity and KIR2DS1 selectivity. An amino acid corresponding to the amino acid Pro89 on KIR2DL2 is the amino acid Arg89 of KIR2DS1, but a hydrogen bond with the amino acid Tyr106 of a2DL2/3-DS10 VH CDR3 is not formed or weak. This indicates that the amino acid Tyr106 plays a key role in KIR2DL2 selectivity and KIR2DS1 selectivity.

An amino acid corresponding to the amino acid Phe66 of KIR2DL2 is the amino acid Tyr66 of KIR2DS2. Occurrence of steric hindrance with the amino acid Tyr107 of a2DL2/3-DS10 VH CDR3 indicates that the amino acid Tyr107 plays a key role in KIR2DL2 selectivity and KIR2DS2 selectivity.

An amino acid corresponding to the amino acid Asp68 of KIR2DL2 is the amino acid Asn68 of KIR2DS4, but a hydrogen bond with the amino acid Tyr107 of a2DL2/3-DS10 VH CDR3 is not formed or weak. This indicates that the amino acid Tyr107 plays a key role in KIR2DL2 selectivity and KIR2DS4 selectivity.

As a result of the structural analysis, threonine, which is the amino acid 94 of the VL chain of a2DL2/3-DS10 (the amino acid 94 in SEQ ID NO: 52; hereafter, referred to as "DS10-VL-T94"), was found to have formed a hydrogen bond with the amino acid Asp93 of KIR2DL2. Upon substitution with the amino acid Val93 in KIR2DS4, a hydrogen bond with an NH group in the DS10-VL-Thr94 main chain is not formed or weak. This indicates that DS10-VL-Thr94 plays a key role in KIR2DL2 selectivity and KIR2DS4 selectivity of a2DL2/3-DS10. DS10-VL-T94 was analyzed with the use of the software BIOLUMINATE (SCHRODINGER). As a result, in the a2DL2/3-DS10 variant resulting from substitution of the amino acid indicated above with the amino acid shown in Table 15, formation of a hydrogen bond with Asp93 of KIR2DL2 was maintained in the NH group in the main chain, but formation of a hydrogen bond with Val93 of KIR2DS4 was not maintained.

Substitution products with these amino acids are deduced to maintain the specificity such that they would bind to inhibitory KIR but would not bind to activating KIR.

[Table 15]

| Amino acid after substitution |
| --- |
| Alanine |
| Asparagine |
| Glycine |
| Histidine |
| Isoleucine |
| Serine |
| Valine |

Example 15: Evaluation of epitope site by competition of a2DL2/3-DS10 with multiple KIR2DL2/3-specific binders in antigen-binding test on KIR2DL2- and/or KIR2DL3-specific binders

Fab fragments of KIR2DL2- and/or KIR2DL3-specific binders were prepared. To the K562 cell line expressing membrane-type a2DL2/3-DS10 scFv, the Fab fragments and recombinant KIR2DL2 were added, whether or not the binding of the K562 cell line expressing membrane-type a2DL2/3-DS10 scFv to recombinant KIR2DL2 would be inhibited by competition was examined, and the epitope sites of the binders were evaluated using the results as the indicator.

For the Fab fragments of various KIR2DL2- and/or KIR2DL3-specific binders, recombinant proteins each comprising a VH-derived signal peptide (SEQ ID NO: 92), a VH chain variable region shown in Table 15, and a VH-chain constant region of a Fab fragment (SEQ ID NO: 95) linked in that order from the N terminus and recombinant proteins each comprising a VL-derived signal peptide (SEQ ID NO: 95), a VL-chain variable region shown in Table 16, and a VL-chain constant region of a Fab fragment (SEQ ID NO: 96) linked in that order from the N terminus were prepared and aggregated to each other to prepare Fab fragments. Table 15 shows the amino acid sequences of variable regions of these binders. As recombinant KIR2DL2, human KIR2DL2(22-245)-Fc-Avi used in Example 4 was biotinylated and used (hereafter, referred to as "human KIR2DL2(22-245)-Fc-Biotin"). As the K562 cell line expressing membrane-type a2DL2/3-DS10 scFv, the K562 cell line prepared in Example 4 was used.

[Table 16]

| Binder | Variable region |
| --- | --- |
| a2DL2/3-DS1 VH chain | (SEQ ID NO: 33) |
| a2DL2/3-DS1 VL chain | (SEQ ID NO: 34) |
| a2DL2/3-DS2 VH chain | (SEQ ID NO: 35) |
| a2DL2/3-DS2 VL chain | (SEQ ID NO: 36) |
| a2DL2/3-DS3 VH chain | (SEQ ID NO: 37) |
| a2DL2/3-DS3 VL chain | (SEQ ID NO: 38) |
| a2DL2/3-DS4 VH chain | (SEQ ID NO: 39) |
| a2DL2/3-DS4 VL chain | (SEQ ID NO: 40) |

(continued)

| Binder | Variable region |
|---|---|
| a2DL2/3-DS5 VH chain | (SEQ ID NO: 41) |
| a2DL2/3-DS5 VL chain | (SEQ ID NO: 42) |
| a2DL2/3-DS6 VH chain | (SEQ ID NO: 43) |
| a2DL2/3-DS6 VL chain | (SEQ ID NO: 44) |
| a2DL2/3-DS7 VH chain | (SEQ ID NO: 45) |
| a2DL2/3-DS7 VL chain | (SEQ ID NO: 46) |
| a2DL2/3-DS9 VH chain | (SEQ ID NO: 49) |
| a2DL2/3-DS9 VL chain | (SEQ ID NO: 50) |
| a2DL2/3-DS10 VH chain | (SEQ ID NO: 51) |
| a2DL2/3-DS10 VL chain | (SEQ ID NO: 52) |
| a2DL2/3-DS11 VH chain | (SEQ ID NO: 61) |
| a2DL2/3-DS11 VL chain | (SEQ ID NO: 62) |
| a2DL2/3-DS12 VH chain | (SEQ ID NO: 63) |
| a2DL2/3-DS12 VL chain | (SEQ ID NO: 64) |

The K562 cell line or K562 cell line expressing membrane-type a2DL2/3-DS10 scFv was suspended in autoMACS Running Buffer-MACS Separation Buffer, Fab fragments of the binders were added to the final concentration of 1.5 μM, 0.5 μM, 0.17 μM, 0.06 μM, 0.02 μM, or 0.01μM, and the resultant was then allowed to stand. Human KIR2DL2(22-245)-Fc-Biotin was added to the final concentration of 0.05 μM thereto, and the resultant was then allowed to stand.

The resultant was washed with autoMACS Running Buffer-MACS Separation Buffer, the APC-labeled anti-human IgG Fc antibody was added thereto, and the resultant was then allowed to stand. The resultant was washed with autoMACS Running Buffer-MACS Separation Buffer, and binding to human KIR2DL2(22-245)-Fc-Biotin was examined by flow cytometry based on the median of the APC-derived fluorescence intensity.

The results are shown in Figure 33. Except for a2DL2/3-DS5,7, the Fab fragments of the KIR2DL2- and/or KIR2DL3-specific binders added at 1.5 μM were found to inhibit the binding of the K562 cell line expressing membrane-type a2DL2/3-DS10 scFv to human KIR2DL2(22-245)-Fc-Biotin by 30% or more, compared with the control K562 cell. This indicates that a2DL2/3-DS10 and many KIR2DL2- and/or KIR2DL3-specific binders compete with KIR2DL2.

The results demonstrate that epitopes where a2DL2/3-DS10 binds to are positioned adjacent to those of major KIR2DL2- and/or KIR2DL3-specific binders. The epitope of a2DL2/3-DS10 identified by X-ray crystallography may be important for KIR2DL2- and/or KIR2DL3-specific agonist activity.

Example 16: Acquisition of KIR2DL1 or KIR3DL1-specific binder by phage panning

In the examples above, the binders having KIR2DL2- and/or KIR2DL3-specific agonist activity were found to inhibit the cytocidal activity exerted on the cells that were recognized as "missing-self" cells by the NK cells expressing KIR2DL2 and/or KIR2DL3. In order to achieve equivalent effects of inhibiting the cytocidal activity on the NK cells expressing KIR2DL1 or KIR3DL1, accordingly, phage panning was performed by the same method as described in the [Acquisition of inhibitory KIR-specific binder by phage panning] section in Example 4, so as to obtain a novel binder that would bind specifically to KIR2DL1 or KIR3DL1 and exert the agonist activity.

In order to perform phage panning, as soluble KIRs, recombinant proteins each comprising an IL-2-derived signal peptide (SEQ ID NO: 91), a KIR protein extracellular domain shown in Table 17, a GGGS linker (SEQ ID NO: 97), an IgG Fc domain (SEQ ID NO: 98), a GAA linker, and the Avi tag (SEQ ID NO: 99) linked from the N terminus were prepared. Table 17 shows the sequences of extracellular domains of KIRs adopted for soluble KIRs. As soluble KIR3DS1, the recombinant human KIR3DS1/CD158e2 Fc chimera protein (4136-KR-050, R&D Systems) was used. Phage panning was performed with the use of soluble KIR subjected to biotinylation or soluble KIR not subjected to biotinylation.

[Table 17]

| Recombinant protein | Extracellular domain |
|---|---|
| KIR2DL1 | Amino acids 1 to 224 in SEQ ID NO: 4 |
| KIR2DL2 | Amino acids 1 to 224 in SEQ ID NO: 6 |
| KIR2DL3 | Amino acids 1 to 224 in SEQ ID NO: 8 |
| KIR2DS1 | Amino acids 1 to 224 in SEQ ID NO: 10 |
| KIR2DS2 | Amino acids 1 to 224 in SEQ ID NO: 12 |
| KIR2DS4 | Amino acids 1 to 224 in SEQ ID NO: 14 |
| KIR3DL1 | Amino acids 1 to 319 in SEQ ID NO: 16 |

During each round of phage panning, inhibitory KIR was subjected to positive selection, and activating KIR was subjected to both or either of depletion and deselection. After the second round, a polyclonal phage library with the enriched phages expressing binders binding specifically to either KIR2DL1 or KIR3DL1 was obtained.

ELISA was performed using soluble KIR of the target KIR (KIR2DL1 or KIR3DL1) to identify the scFv-expressing phage binding to KIR2DL1 or KIR3DL1 from the polyclonal phage library. From the phagemids of the identified scFv-expressing phages, the binder sequences were transferred to an *E. coli* recombinant protein expression vector to integrate the binder sequences into *E. coli,* and the recombinant proteins of the binders were obtained from the culture supernatant. The binders were subjected to ELISA using the soluble KIRs corresponding to the KIRs shown in Table 16, and information on the binding capability of the binders to KIRs was obtained.

A variable region of a binder that would bind to KIR2DL1 or KIR3DL1 but would not bind to KIR2DS1, KIR2DS2, KIR2DS4, or KIR3DS1 was selected therefrom, introduced into the membrane-type scFv expression format described in the [Introduction of scFv gene into K-562 cell and preparation] section above, and then incorporated into pPB[Exp]-EF1A (VectorBuilder).

Table 18 shows the sequence identification numbers of the amino acid sequences of the heavy chain variable regions and the light chain variable regions of the KIR2DL1-specific binders. Table 19 shows the sequence identification numbers of the amino acid sequences of the heavy chain variable regions and the light chain variable regions of the KIR3DL1-specific binders.

[Table 18]

| Binder | Variable region | CDR1 | CDR2 | CDR3 |
|---|---|---|---|---|
| a2DL1-DS1 | VH (SEQ ID NO: 107) | 26-35 | 53-61 | 100-113 |
|  | VL (SEQ ID NO: 108) | 27-38 | 56-58 | 95-103 |
| a2DL1-DS2 | VH (SEQ ID NO: 109) | 26-33 | 51-58 | 97-113 |
|  | VL (SEQ ID NO: 110) | 27-32 | 50-52 | 89-97 |
| a2DL1-DS3 | VH (SEQ ID NO: 111) | 26-35 | 53-61 | 100-111 |
|  | VL (SEQ ID NO: 112) | 27-38 | 56-58 | 95-103 |
| a2DL1-DS4 | VH (SEQ ID NO: 113) | 26-33 | 51-58 | 97-105 |
|  | VL (SEQ ID NO: 114) | 27-33 | 51-53 | 90-98 |
| a2DL1-DS5 | VH (SEQ ID NO: 115) | 26-33 | 51-58 | 97-114 |
|  | VL (SEQ ID NO: 116) | 27-32 | 50-52 | 89-97 |
| a2DL1-DS6 | VH (SEQ ID NO: 117) | 26-33 | 51-58 | 97-106 |
|  | VL (SEQ ID NO: 118) | 27-32 | 50-52 | 89-98 |
| a2DL1-DS7 | VH (SEQ ID NO: 119) | 26-33 | 51-58 | 97-112 |
|  | VL (SEQ ID NO: 120) | 26-34 | 52-54 | 91-100 |
| a2DL1-DS8 | VH (SEQ ID NO: 121) | 26-33 | 51-58 | 97-107 |
|  | VL (SEQ ID NO: 122) | 26-33 | 51-53 | 90-100 |
| a2DL1-DS9 | VH (SEQ ID NO: 123) | 26-33 | 51-58 | 97-106 |

66

(continued)

| Binder | Variable region | CDR1 | CDR2 | CDR3 |
|---|---|---|---|---|
| | VL (SEQ ID NO: 124) | 26-33 | 51-53 | 90-100 |
| a2DL1-DS10 | VH (SEQ ID NO: 125) | 26-33 | 51-58 | 97-112 |
| | VL (SEQ ID NO: 126) | 26-33 | 51-53 | 90-100 |
| a2DL1-DS11 | VH (SEQ ID NO: 127) | 26-33 | 51-58 | 97-105 |
| | VL (SEQ ID NO: 128) | 26-33 | 51-53 | 90-100 |
| a2DL1-DS12 | VH (SEQ ID NO: 129) | 26-33 | 51-58 | 97-111 |
| | VL (SEQ ID NO: 130) | 26-34 | 52-54 | 91-99 |
| a2DL1-DS13 | VH (SEQ ID NO: 131) | 26-33 | 51-58 | 97-104 |
| | VL (SEQ ID NO: 132) | 26-33 | 51-53 | 90-100 |
| a2DL1-DS14 | VH (SEQ ID NO: 133) | 26-35 | 53-61 | 100-114 |
| | VL (SEQ ID NO: 134) | 26-34 | 52-54 | 91-101 |
| a2DL1-DS15 | VH (SEQ ID NO: 135) | 26-33 | 51-58 | 97-115 |
| | VL (SEQ ID NO: 136) | 26-34 | 52-54 | 91-102 |

[Table 19]

| Binder | Variable region | CDR1 | CDR2 | CDR3 |
|---|---|---|---|---|
| a3DL1-DS2 | VH (SEQ ID NO: 137) | 26-35 | 53-61 | 100-111 |
| | VL (SEQ ID NO: 138) | 27-32 | 50-52 | 89-97 |
| a3DL1-DS3 | VH (SEQ ID NO: 139) | 26-35 | 53-61 | 100-111 |
| | VL (SEQ ID NO: 140) | 27-32 | 50-52 | 89-98 |
| a3DL1-DS4 | VH (SEQ ID NO: 141) | 26-33 | 51-58 | 97-115 |
| | VL (SEQ ID NO: 142) | 27-33 | 51-53 | 90-98 |
| a3DL1-DS5 | VH (SEQ ID NO: 143) | 26-35 | 53-61 | 100-111 |
| | VL (SEQ ID NO: 144) | 27-32 | 50-52 | 89-97 |
| a3DL1-DS6 | VH (SEQ ID NO: 145) | 26-33 | 51-58 | 97-109 |
| | VL (SEQ ID NO: 146) | 26-34 | 52-54 | 91-101 |

Gene introduction into K562 cells was performed by electroporation. At the time of electroporation, pPB [Exp]-EF1A encoding membrane-type scFv and the pRP[Exp]-mCherry-CAG>hyPBase (pDNA (VB010000-9365tax)-P, VectorBuilder) were suspended together with the K562 cells. After electroporation, the K562 cells were cultured in RPMI medium, and membrane-type scFv-positive cells were selected with the use of magnetic beads.

According to need, the collected cells were suspended at 0.5 to $1 \times 10^7$ cells/ml in CELLBANKER 1 and cryopreserved at -80°C or -150°C. When frozen cells were used in experimentation, the cells were thawed and cultured in the RPMI medium before use.

The recombinant KIR protein used in phage panning or the recombinant human KIR3DS1/CD158e2 Fc chimera protein was diluted to 0.1 μM in the autoMACS Running Buffer-MACS Separation Buffer, the resultant was added to the target cells, and the reaction was allowed to proceed at 4°C for 45 to 90 minutes. The K562 cells expressing membrane-type a3DL1-DS1 scFv as used in Example 8 were used.

After the reaction, the target cells were washed with the autoMACS Running Buffer-MACS Separation Buffer, the APC-labeled anti-human IgG Fc antibody or the PE-labeled anti-human IgG Fc antibody (409304, BIOLEGEND) was added thereto, and the reaction was allowed to proceed at 4°C for 30 minutes. The reaction product was washed with the autoMACS Running Buffer-MACS Separation Buffer and subjected to flow cytometry to examine the binding capability. The binding capability to the wild-type K562 cell line (i.e., the median fluorescence intensity derived from APC or PE) was designated to be 1, and the binding capability of each recombinant KIR protein to the K562 cell line expressing membrane-type scFv of each binder was calculated relative thereto.

The results are shown in Figure 34. The K562 cell expressing membrane-type scFv of a novel KIR2DL1-specific binder exerted a low binding capability to recombinant KIR2DS1, recombinant KIR2DS2, or recombinant KIR2DS4 and exerted a specific binding capability to the target recombinant KIR2DL1. The K562 cell expressing membrane-type scFv of a novel KIR3DL1-specific binder exerted a low binding capability to KIR3DS1 and exerted a specific binding capability to the target recombinant KIR3DL1.

Example 17: Evaluation of agonist activity of KIR2DL1-specific binder

The KIR2DL1-specific binder that was verified to have specificity in Example 16 was evaluated in terms of the agonist activity on inhibitory KIR and activating KIR with the use of the K562 cell line expressing membrane-type scFv of each binder and the NK92 cell line expressing each KIR.

The K562 cell line expressing KIR2DL1-specific membrane-type scFv as used in Example 16 was used. The NK92 cell lines expressing inhibitory or activating KIRs prepared by the method described in the [Introduction of KIR gene into NK92 cell and preparation] section were used herein.

By the method described in the [Evaluation of cytocidal activity of KIR2DL2-expressing NK92 on Lirilumab-scFv/Pan2D-scFv-K562] section, the cytocidal activity of the NK92 cells on the K562 cells expressing KIR2DL1-specific membrane-type scFv was evaluated.

The results are shown in Figure 35. The results demonstrate that all the binders lowered the cytocidal activity of the NK92 cell line expressing the target KIR2DL1 and exerted the agonist activity on KIR2DL1. In contrast, the NK92 cell lines expressing KIR2DS1, KIR2DS2, or KIR2DS4 were not activated. The results demonstrate that a binder having the profile as intended; i.e., a binder that binds specifically to inhibitory KIR and has the agonist activity on inhibitory KIR, was obtained.

Example 18: Evaluation of agonist activity of KIR3DL1-specific binder

The KIR3DL1 binder that was verified to have specificity in Example 16 was evaluated in terms of the agonist activity on inhibitory KIR and activating KIR with the use of the K562 cell line expressing membrane-type scFv of each binder and the NK92 cell line expressing each KIR.

The K562 cell line expressing KIR3DL1-specific membrane-type scFv as used in Example 16 was used. The NK92 cell lines expressing inhibitory KIRs other than KIR3DS1 prepared by the method described in the [Introduction of KIR gene into NK92 cell and preparation] section were used herein. The K562 cell line expressing membrane-type a3DL1-DS1 scFv as used in Example 8 was used.

The NK92 cell line expressing KIR3DS1 was not obtained by gene introduction into the NK92 cell line with the use of the lentivirus. In order to evaluate the cytocidal activity of the NK cell line expressing KIR3DS1, PBMC-derived NK cells were cultured, and the NK cells expressing KIR3DS1 were concentrated using a cell sorter.

As effector cells, HLA-C1/C1/Bw4$^+$ PBMCs or HLA-C2/C2/Bw4$^+$ PBMCs were prepared (referred to as PBMC-D and PBMC-E).

As feeder cells, the K562 cells expressing membrane-type IL-21 and tHLA-E (hereafter, referred to as "K562 feeder cells") were used.

Concerning membrane-type IL-21, a gene encoding the IL-2-derived signal peptide (SEQ ID NO: 91), the IL-21 fragment (SEQ ID NO: 105), the CD8-derived hinge region (62 amino acids) (SEQ ID NO: 106), the CD8-derived cell membrane domain and intracellular domain (SEQ ID NO: 102), the IRES sequence, and the blue fluorescent protein (BFP) (SEQ ID NO: 104) from the N terminus was inserted into pLVSIN EF1$\alpha$. A lentivirus concentrate was prepared by the method described in the [Preparation of lentivirus] section. With the use of the lentivirus prepared, the K562 cells expressing tHLA-E used in Example 12 were infected with the virus vector by the method described in the [scFv introduction into K-562 cell and preparation] section, BFP-expressing clones were selected, and the K562 feeder cell line expressing membrane-type IL-21 and tHLA-E were prepared.

K562 feeder cells were suspended in the RPMI medium supplemented with the Mitomycin C Solution (20898-21, Nacalai Tesque) at 10 $\mu$g/ml and incubated therein for 3 hours. The K562 feeder cells were washed in the RPMI medium, suspended in the MyeloCult H5100 medium, and cultured with PBMC-D or PBMC-E suspended in the MyeloCult H5100 medium supplemented with IL-2 at 200 U/ml. The cells were collected 7 to 10 days later, suspended in the MACSQuant Tyto Running Buffer (130-107-206, Miltenyi Biotech) to adjust the concentration of the antibodies shown in Table 20 to 1 to 10 $\mu$g/ml, and added to the cells. The reaction was then allowed to proceed at 4°C for 30 minutes.

[Table 20]

| Antibody | Manufacturer | Catalog No. | Label |
|---|---|---|---|
| Anti-CD56 antibody | BIOLEGEND | 362534 | BV510 |

(continued)

| Antibody | Manufacturer | Catalog No. | Label |
|---|---|---|---|
| Anti-CD3 antibody | BIOLEGEND | 300434 | BV421 |
| Anti-KIR3DL1 antibody | BIOLEGEND | 312706 | FITC |
| Anti-KIR3DL1/3DS1 antibody | Beckman Coulter | A60795 | APC |

The antibody-stained cells were washed 2 times with the MACSQuant Tyto Running Buffer, suspended again to the concentration of $50 \times 10^6$ cells/ml or lower, and filled in the MACSQuant Tyto Cartridges (130-106-088) to sort the NK cells expressing KIR3DS1 using the MACSQuant Tyto Cell Sorter (130-103-931). From among the CD3⁻ CD56⁺ NK cells, the anti-KIR3DL1 antibody⁻ anti-KIR3DL1/3DS1 antibody⁺ cell fraction was sorted as the NK cells expressing KIR3DS1. The anti-KIR3DL1/3DS1 antibody⁻ cell fraction was sorted as the control NK cells. Thereafter, culture was continued in the MyeloCult H5100 medium supplemented with IL-2 and IL-18 at 1000 U/ml and 100 ng/ml, respectively.

The cytocidal activity of the NK92 cell lines or PBMC-derived NK cells expressing KIR3DS1 on the K562 cell lines expressing KIR3DL1-specific membrane-type scFv was evaluated by the method described in the [Evaluation of cytocidal activity of KIR2DL2-expressing NK92 on Lirilumab-scFv/Pan2D-scFv-K562] section. The K562 cells were mixed with the PBMC-derived NK cells at a ratio (cell count) of 1:3.

The results are shown in Figure 36. The results demonstrate that all the binders lowered the cytocidal activity of the NK92 cell line expressing the target KIR3DL1 and exerted the agonist activity on KIR3DL1. In contrast, the PBMC-derived NK cells expressing KIR3DS1 were not activated. While the cytocidal activity of the PBMC-NK cells expressing KIR3DS1 on some K562 cells expressing KIR3DL1-specific membrane-type scFv was higher than that on the control K562 cells, the K562 cells expressing KIR3DL1-specific membrane-type scFv did not bind to the recombinant KIR3DS1 in Example 16. This indicates that such increase in the cytocidal activity falls within the scope of experimental error. The results demonstrate that a binder having the profile as intended; i.e., a binder that binds specifically to inhibitory KIR and has the agonist activity on inhibitory KIR, was obtained.

Industrial Applicability

The substance having activity of binding to inhibitory KIR2DL2 and/or KIR2DL3 and inhibiting the cytocidal activity of an NK cell of the present invention can be used as a composition that reduces a risk of immune rejection at the time of transplantation.

Sequence Listing Free Text

SEQ ID NO: 1: the amino acid sequence of the KIR2DL1 signal peptide
SEQ ID NO: 2: the amino acid sequence of the Flag tag
SEQ ID NO: 3: the amino acid sequence of the GSGS linker
SEQ ID NO: 4: the amino acid sequence of KIR2DL1*003
SEQ ID NO: 5: the amino acid sequence of the KIR2DL2 signal peptide
SEQ ID NO: 6: the amino acid sequence of KIR2DL2*001
SEQ ID NO: 7: the amino acid sequence of the KIR2DL3 signal peptide
SEQ ID NO: 8: the amino acid sequence of KIR2DL3*001
SEQ ID NO: 9: the amino acid sequence of the KIR2DS1 signal peptide
SEQ ID NO: 10: the amino acid sequence of KIR2DS1*002
SEQ ID NO: 11: the amino acid sequence of the KIR2DS2 signal peptide
SEQ ID NO: 12: the amino acid sequence of KIR2DS2*001
SEQ ID NO: 13: the amino acid sequence of the KIR2DS4 signal peptide
SEQ ID NO: 14: the amino acid sequence of KIR2DS4*001
SEQ ID NO: 15: the amino acid sequence of the KIR3DL1 signal peptide
SEQ ID NO: 16: the amino acid sequence of KIR3DL1*015
SEQ ID NO: 17: the amino acid sequence of firefly luciferase
SEQ ID NO: 18: the amino acid sequence of the B2M signal peptide
SEQ ID NO: 19: the amino acid sequence of B2M
SEQ ID NO: 20: the amino acid sequence of T2A
SEQ ID NO: 21: the amino acid sequence of the HLA-Cw3 signal peptide

SEQ ID NO: 22: the amino acid sequence of HLA-Cw3

SEQ ID NO: 23: the amino acid sequence of the CD8-derived signal peptide

SEQ ID NO: 24: the amino acid sequence of the CD8-derived hinge (66 amino acids)

SEQ ID NO: 25: the amino acid sequence of the G4S linker

SEQ ID NO: 26: the amino acid sequence of the CD8-derived hinge (45 amino acids)

SEQ ID NO: 27: the amino acid sequence of the CD8-derived cell membrane domain

SEQ ID NO: 28: the amino acid sequence of the CD8-derived intracellular domain

SEQ ID NO: 29: the VH amino acid sequence of Lirilumab (1-7F9)

SEQ ID NO: 30: the VL amino acid sequence of Lirilumab (1-7F9)

SEQ ID NO: 31: the VH amino acid sequence of Pan2D

SEQ ID NO: 32: the VL amino acid sequence of Pan2D

SEQ ID NO: 33: the VH amino acid sequence of a2DL2/3-DS1

SEQ ID NO: 34: the VL amino acid sequence of a2DL2/3-DS1

SEQ ID NO: 35: the VH amino acid sequence of a2DL2/3-DS2

SEQ ID NO: 36: the VL amino acid sequence of a2DL2/3-DS2

SEQ ID NO: 37: the VH amino acid sequence of a2DL2/3-DS3

SEQ ID NO: 38: the VL amino acid sequence of a2DL2/3-DS3

SEQ ID NO: 39: the VH amino acid sequence of a2DL2/3-DS4

SEQ ID NO: 40: the VL amino acid sequence of a2DL2/3-DS4

SEQ ID NO: 41: the VH amino acid sequence of a2DL2/3-DS5

SEQ ID NO: 42: the VL amino acid sequence of a2DL2/3-DS5

SEQ ID NO: 43: the VH amino acid sequence of a2DL2/3-DS6

SEQ ID NO: 44: the VL amino acid sequence of a2DL2/3-DS6

SEQ ID NO: 45: the VH amino acid sequence of a2DL2/3-DS7

SEQ ID NO: 46: the VL amino acid sequence of a2DL2/3-DS7

SEQ ID NO: 47: the VH amino acid sequence of a2DL2/3-DS8

SEQ ID NO: 48: the VL amino acid sequence of a2DL2/3-DS8

SEQ ID NO: 49: the VH amino acid sequence of a2DL2/3-DS9

SEQ ID NO: 50: the VL amino acid sequence of a2DL2/3-DS9

SEQ ID NO: 51: the VH amino acid sequence of a2DL2/3-DS10

SEQ ID NO: 52: the VL amino acid sequence of a2DL2/3-DS10

SEQ ID NO: 53: the amino acid sequence of truncated CD19

SEQ ID NO: 54: the amino acid sequence of HLA-Cw4

SEQ ID NO: 55: the VL amino acid sequence of a3DL1-DS1

SEQ ID NO: 56: the VH amino acid sequence of a3DL1-DS1

SEQ ID NO: 57: the amino acid sequence of KIR2DL2*003

SEQ ID NO: 58: the amino acid sequence of truncated NGFR

SEQ ID NO: 59: the amino acid sequence of KIR3DL2*002

SEQ ID NO: 60: the amino acid sequence of KIR3DS1*013

SEQ ID NO: 61: the VH amino acid sequence of a2DL2/3-DS11

SEQ ID NO: 62: the VL amino acid sequence of a2DL2/3-DS11

SEQ ID NO: 63: the VH amino acid sequence of a2DL2/3-DS12

SEQ ID NO: 64: the VL amino acid sequence of a2DL2/3-DS12

SEQ ID NO: 65: the amino acid sequence of the 3DL2 signal peptide

SEQ ID NO: 66: the amino acid sequence of the 3DS1 signal peptide

SEQ ID NO: 67: the amino acid sequence of R99A CDR3

SEQ ID NO: 68: the amino acid sequence of V99A CDR3

SEQ ID NO: 69: the amino acid sequence of S100A CDR3

SEQ ID NO: 70: the amino acid sequence of G101A CDR3

SEQ ID NO: 71: the amino acid sequence of T102A CDR3

SEQ ID NO: 72: the amino acid sequence of T103A CDR3

SEQ ID NO: 73: the amino acid sequence of G104A CDR3

SEQ ID NO: 74: the amino acid sequence of G105A CDR3

SEQ ID NO: 75: the amino acid sequence of Y106A CDR3

SEQ ID NO: 76: the amino acid sequence of Y107A CDR3

SEQ ID NO: 77: the amino acid sequence of T108A CDR3

SEQ ID NO: 78: the amino acid sequence of G109A CDR3

SEQ ID NO: 79: the amino acid sequence of M110A CDR3

SEQ ID NO: 80:the amino acid sequence of D111A CDR3

SEQ ID NO: 81: the amino acid sequence of V112A CDR3

SEQ ID NO: 82: the VH amino acid sequence of a2DL2/3-DS1-2

SEQ ID NO: 83: the VL amino acid sequence of a2DL2/3-DS1-2

SEQ ID NO: 84: the VH amino acid sequence of a2DL2/3-DS2-2

SEQ ID NO: 85: the VL amino acid sequence of a2DL2/3-DS2-2

SEQ ID NO: 86: the VH amino acid sequence of a2DL2/3-DS3-2

SEQ ID NO: 87: the VL amino acid sequence of a2DL2/3-DS3-2

SEQ ID NO: 88: the amino acid sequence of the HLA-G signal peptide-derived peptide

SEQ ID NO: 89: the amino acid sequence of the G4S linker

SEQ ID NO: 90: the amino acid sequence of HLA-E

SEQ ID NO: 91: the amino acid sequence of the IL-2 signal peptide

SEQ ID NO: 92: the amino acid sequence of the His tag

SEQ ID NO: 93: the amino acid sequence of the VH signal peptide

SEQ ID NO: 94: the amino acid sequence of the Fab fragment H-chain constant region

SEQ ID NO: 95: the amino acid sequence of the Vl signal peptide

SEQ ID NO: 96: the amino acid sequence of the Fab fragment L-chain constant region

SEQ ID NO: 97: the amino acid sequence of the GGGS linker

SEQ ID NO: 98: the amino acid sequence of the IgG Fc domain

SEQ ID NO: 99: the amino acid sequence of the Avi tag

SEQ ID NO: 100: the VL amino acid sequence of CA19CAR

SEQ ID NO: 101: the VH amino acid sequence of CA19CAR

SEQ ID NO: 102: the amino acid sequence of the CD8-derived cell membrane domain and intracellular domain

SEQ ID NO: 103: the amino acid sequence of the intracellular costimulatory molecule

SEQ ID NO: 104: the amino acid sequence of the blue fluorescent protein

SEQ ID NO: 105: the amino acid sequence of the IL-21 fragment

SEQ ID NO: 106: the amino acid sequence of the CD8 hinge (62 amino acids)

SEQ ID NO: 107: the VH amino acid sequence of a2DL1-DS1

SEQ ID NO: 108: the VL amino acid sequence of a2DL1-DS1

SEQ ID NO: 109: the VH amino acid sequence of a2DL1-DS2

SEQ ID NO: 110: the VL amino acid sequence of a2DL1-DS2

SEQ ID NO: 111: the VH amino acid sequence of a2DL1-DS3

SEQ ID NO: 112: the VL amino acid sequence of a2DL1-DS3

SEQ ID NO: 113: the VH amino acid sequence of a2DL1-DS4

SEQ ID NO: 114: the VL amino acid sequence of a2DL1-DS4

SEQ ID NO: 115: the VH amino acid sequence of a2DL1-DS5

SEQ ID NO: 116: the VL amino acid sequence of a2DL1-DS5

SEQ ID NO: 117: the VH amino acid sequence of a2DL1-DS6

SEQ ID NO: 118: the VL amino acid sequence of a2DL1-DS6

SEQ ID NO: 119: the VH amino acid sequence of a2DL1-DS7

SEQ ID NO: 120: the VL amino acid sequence of a2DL1-DS7

SEQ ID NO: 121: the VH amino acid sequence of a2DL1-DS8

SEQ ID NO: 122: the VL amino acid sequence of a2DL1-DS8

SEQ ID NO: 123: the VH amino acid sequence of a2DL1-DS9

SEQ ID NO: 124: the VL amino acid sequence of a2DL1-DS9

SEQ ID NO: 125: the VH amino acid sequence of a2DL1-DS10

SEQ ID NO: 126: the VL amino acid sequence of a2DL1-DS10

SEQ ID NO: 127: the VH amino acid sequence of a2DL1-DS11

SEQ ID NO: 128: the VL amino acid sequence of a2DL1-DS11

SEQ ID NO: 129: the VH amino acid sequence of a2DL1-DS12

SEQ ID NO: 130: the VL amino acid sequence of a2DL1-DS12

SEQ ID NO: 131: the VH amino acid sequence of a2DL1-DS13

SEQ ID NO: 132: the VL amino acid sequence of a2DL1-DS13

SEQ ID NO: 133: the VH amino acid sequence of a2DL1-DS14

SEQ ID NO: 134: the VL amino acid sequence of a2DL1-DS14

SEQ ID NO: 135: the VH amino acid sequence of a2DL1-DS15

SEQ ID NO: 136: the VL amino acid sequence of a2DL1-DS15

SEQ ID NO: 137: the VH amino acid sequence of a3DL1-DS2

SEQ ID NO: 138: the VL amino acid sequence of a3DL1-DS2
SEQ ID NO: 139: the VH amino acid sequence of a3DL1-DS3
SEQ ID NO: 140: the VL amino acid sequence of a3DL1-DS3
SEQ ID NO: 141: the VH amino acid sequence of a3DL1-DS4
SEQ ID NO: 142: the VL amino acid sequence of a3DL1-DS4
SEQ ID NO: 143: the VH amino acid sequence of a3DL1-DS5
SEQ ID NO: 144: the VL amino acid sequence of a3DL1-DS5
SEQ ID NO: 145: the VH amino acid sequence of a3DL1-DS6
SEQ ID NO: 146: the VL amino acid sequence of a3DL1-DS6

All publications, patents, and patent applications cited herein are incorporated herein by reference in their entirety.

**Claims**

1. A substance having activity of binding to human inhibitory KIR and inhibiting an NK cell.

2. The substance according to claim 1, which is a polypeptide comprising a site binding to human inhibitory KIR.

3. The substance according to claim 1, wherein the site binding to human inhibitory KIR comprises an amino acid sequence derived from one or two variable regions of a monoclonal antibody binding to human inhibitory KIR or an antigen-binding fragment thereof.

4. The substance according to claim 1, wherein the activity of binding to human inhibitory KIR is higher than the activity of binding to human activating KIR corresponding thereto.

5. The substance according to claim 1, wherein the agonist activity on human inhibitory KIR is higher than the agonist activity on human activating KIR corresponding thereto.

6. The substance according to claim 5, which exerts substantially no agonist activity on the corresponding human activating KIR.

7. The substance according to claim 2, wherein the polypeptide is any of (1) to (3) below:

   (1) a polypeptide in which the site binding to KIR2DL2 and/or KIR2DL3 comprise a C1 epitope motif sequence contained in HLA-Cw1, Cw3, Cw7, Cw8, Cw9, Cw10, Cw12, Cw14, or Cw16 that belongs to the HLA-C1 type or HLA-B46 or B73 (in which amino acid 77 is S and amino acid 80 is N) and a TCR-binding capability is lost or attenuated;
   (2) a polypeptide in which the site binding to KIR2DL1 comprise a C2 epitope motif sequence contained in HLA-Cw2, Cw4, Cw5, Cw6, Cw15, or Cw17 that belongs to the HLA-C2 type (in which amino acid 77 is N and amino acid 80 is K) and a TCR-binding capability is lost or attenuated; and
   (3) a polypeptide in which the site binding to KIR3DL1 comprise a Bw4 epitope motif sequence contained in HLA-B5, B27, B37, B38, B44, B49, B51, B52, B53, B57, B58, B59, B77, A23, A24, A25, or A32 that belongs to the HLA-Bw4 type (in which amino acid 77 is N, amino acid 80 is I or T, and amino acid 83 is R) and a TCR-binding capability is lost or attenuated.

8. The substance according to claim 3, wherein the human inhibitory KIR is KIR2DL2 and/or KIR2DL3 and the binding site comprises an amino acid sequence derived from one or two variable regions of a monoclonal antibody binding to KIR2DL2 and/or KIR2DL3 or an antigen-binding fragment thereof.

9. The substance according to claim 8, wherein the monoclonal antibody binding to KIR2DL2 and/or KIR2DL3 or an antigen-binding fragment thereof competes with Lirilumab, Pan2D, or scFv consisting of an amino acid sequence according to any of (i) to (xii) below when binding to KIR2DL2 and/or KIR2DL3:

   (i) an amino acid sequence comprising the amino acid sequence of a heavy chain variable region shown in SEQ ID NO: 33 in combination with the amino acid sequence of a light chain variable region shown in SEQ ID NO: 34;
   (ii) an amino acid sequence comprising the amino acid sequence of a heavy chain variable region shown in SEQ ID NO: 35 in combination with the amino acid sequence of a light chain variable region shown in SEQ ID NO: 36;

(iii) an amino acid sequence comprising the amino acid sequence of a heavy chain variable region shown in SEQ ID NO: 37 in combination with the amino acid sequence of a light chain variable region shown in SEQ ID NO: 38;

(iv) an amino acid sequence comprising the amino acid sequence of a heavy chain variable region shown in SEQ ID NO: 39 in combination with the amino acid sequence of a light chain variable region shown in SEQ ID NO: 40;

(v) an amino acid sequence comprising the amino acid sequence of a heavy chain variable region shown in SEQ ID NO: 41 in combination with the amino acid sequence of a light chain variable region shown in SEQ ID NO: 42;

(vi) an amino acid sequence comprising the amino acid sequence of a heavy chain variable region shown in SEQ ID NO: 43 in combination with the amino acid sequence of a light chain variable region shown in SEQ ID NO: 44;

(vii) an amino acid sequence comprising the amino acid sequence of a heavy chain variable region shown in SEQ ID NO: 45 in combination with the amino acid sequence of a light chain variable region shown in SEQ ID NO: 46;

(viii) an amino acid sequence comprising the amino acid sequence of a heavy chain variable region shown in SEQ ID NO: 47 in combination with the amino acid sequence of a light chain variable region shown in SEQ ID NO: 48;

(ix) an amino acid sequence comprising the amino acid sequence of a heavy chain variable region shown in SEQ ID NO: 49 in combination with the amino acid sequence of a light chain variable region shown in SEQ ID NO: 50;

(x) an amino acid sequence comprising the amino acid sequence of a heavy chain variable region shown in SEQ ID NO: 51 in combination with the amino acid sequence of a light chain variable region shown in SEQ ID NO: 52;

(xi) an amino acid sequence comprising the amino acid sequence of a heavy chain variable region shown in SEQ ID NO: 61 in combination with the amino acid sequence of a light chain variable region shown in SEQ ID NO: 62; and

(xii) an amino acid sequence comprising the amino acid sequence of a heavy chain variable region shown in SEQ ID NO: 63 in combination with the amino acid sequence of a light chain variable region shown in SEQ ID NO: 64.

10. The substance according to claim 8, wherein the site binding to KIR2DL2 and/or KIR2DL3 comprises a variable region comprising amino acids located adjacent to 1 to 6 amino acids including Asp-47 in a region of amino acids 44 to 49, 2 to 4 amino acids including Gly-67 and Pro-68 in a region of amino acids 65 to 68, and 1 to 4 amino acids including Asp-72 in a region of amino acids 70 to 73 in the amino acid sequence of KIR2DL2 of SEQ ID NO: 6.

11. The substance according to claim 10, wherein, in the site binding to KIR2DL2 and/or KIR2DL3, an amino acid interacting with Asp-47 of SEQ ID NO: 6 is Tyr, an amino acid interacting with Gly-67 of SEQ ID NO: 6 is Gly, an amino acid interacting with Pro-68 of SEQ ID NO: 6 is Tyr, and an amino acid interacting with Asp-72 of SEQ ID NO: 6 is Thr, Asn, Gly, His, Ile, Ser, Val, or Ala.

12. The substance according to claim 11, wherein the amino acid included in the variable region identified in claim 11 belongs to CDR3.

13. The substance according to claim 8, which is a polypeptide comprising a binding site according to any of (I) to (XII) below as the site binding to KIR2DL2 and/or KIR2DL3 and optionally comprising mutation of 1 or 2 amino acids in each CDR:

(I) a binding site comprising a first variable region comprising the amino acid sequence derived from the amino acid sequence of CDR1, CDR2, and CDR3 shown in SEQ ID NO: 33 in combination with a second variable region comprising the amino acid sequence derived from the amino acid sequence of CDR1, CDR2, and CDR3 shown in SEQ ID NO: 34;

(II) a binding site comprising a first variable region comprising the amino acid sequence derived from the amino acid sequence of CDR1, CDR2, and CDR3 shown in SEQ ID NO: 35 in combination with a second variable region comprising the amino acid sequence derived from the amino acid sequence of CDR1, CDR2, and CDR3 shown in SEQ ID NO: 36;

(III) a binding site comprising a first variable region comprising the amino acid sequence derived from the amino acid sequence of CDR1, CDR2, and CDR3 shown in SEQ ID NO: 37 in combination with a second variable region comprising the amino acid sequence derived from the amino acid sequence of CDR1, CDR2, and CDR3 shown in SEQ ID NO: 38;

(IV) a binding site comprising a first variable region comprising the amino acid sequence derived from the amino acid sequence of CDR1, CDR2, and CDR3 shown in SEQ ID NO: 39 in combination with a second variable region comprising the amino acid sequence derived from the amino acid sequence of CDR1, CDR2, and CDR3 shown in SEQ ID NO: 40;

(V) a binding site comprising a first variable region comprising the amino acid sequence derived from the amino acid sequence of CDR1, CDR2, and CDR3 shown in SEQ ID NO: 41 in combination with a second variable region comprising the amino acid sequence derived from the amino acid sequence of CDR1, CDR2, and CDR3 shown in SEQ ID NO: 42;

(VI) a binding site comprising a first variable region comprising the amino acid sequence derived from the amino acid sequence of CDR1, CDR2, and CDR3 shown in SEQ ID NO: 43 in combination with a second variable region comprising the amino acid sequence derived from the amino acid sequence of CDR1, CDR2, and CDR3 shown in SEQ ID NO: 44;

(VII) a binding site comprising a first variable region comprising the amino acid sequence derived from the amino acid sequence of CDR1, CDR2, and CDR3 shown in SEQ ID NO: 45 in combination with a second variable region comprising the amino acid sequence derived from the amino acid sequence of CDR1, CDR2, and CDR3 shown in SEQ ID NO: 46;

(VIII) a binding site comprising a first variable region comprising the amino acid sequence derived from the amino acid sequence of CDR1, CDR2, and CDR3 shown in SEQ ID NO: 47 in combination with a second variable region comprising the amino acid sequence derived from the amino acid sequence of CDR1, CDR2, and CDR3 shown in SEQ ID NO: 48;

(IX) a binding site comprising a first variable region comprising the amino acid sequence derived from the amino acid sequence of CDR1, CDR2, and CDR3 shown in SEQ ID NO: 49 in combination with a second variable region comprising the amino acid sequence derived from the amino acid sequence of CDR1, CDR2, and CDR3 shown in SEQ ID NO: 50;

(X) a binding site comprising a first variable region comprising the amino acid sequence derived from the amino acid sequence of CDR1, CDR2, and CDR3 shown in SEQ ID NO: 51 in combination with a second variable region comprising the amino acid sequence derived from the amino acid sequence of CDR1, CDR2, and CDR3 shown in SEQ ID NO: 52;

(XI) a binding site comprising a first variable region comprising the amino acid sequence derived from the amino acid sequence of CDR1, CDR2, and CDR3 shown in SEQ ID NO: 61 in combination with a second variable region comprising the amino acid sequence derived from the amino acid sequence of CDR1, CDR2, and CDR3 shown in SEQ ID NO: 62; and

(XII) a binding site comprising a first variable region comprising the amino acid sequence derived from the amino acid sequence of CDR1, CDR2, and CDR3 shown in SEQ ID NO: 63 in combination with a second variable region comprising the amino acid sequence derived from the amino acid sequence of CDR1, CDR2, and CDR3 shown in SEQ ID NO: 64.

14. The substance according to claim 13, which optionally comprises mutation of 1 or 2 amino acids on the C and/or N terminal sides of each CDR.

15. The substance according to claim 13, which is a polypeptide comprising a binding site according to any of (I) to (XII) below as the site binding to KIR2DL2 and/or KIR2DL3:

(I) a binding site comprising a first variable region comprising the amino acid sequence shown in SEQ ID NO: 33 in combination with a second variable region comprising the amino acid sequence shown in SEQ ID NO: 34;

(II) a binding site comprising a first variable region comprising the amino acid sequence shown in SEQ ID NO: 35 in combination with a second variable region comprising the amino acid sequence shown in SEQ ID NO: 36;

(III) a binding site comprising a first variable region comprising the amino acid sequence shown in SEQ ID NO: 37 in combination with a second variable region comprising the amino acid sequence shown in SEQ ID NO: 38;

(IV) a binding site comprising a first variable region comprising the amino acid sequence shown in SEQ ID NO: 39 in combination with a second variable region comprising the amino acid sequence shown in SEQ ID NO: 40;

(V) a binding site comprising a first variable region comprising the amino acid sequence shown in SEQ ID NO: 41 in combination with a second variable region comprising the amino acid sequence shown in SEQ ID NO: 42;

(VI) a binding site comprising a first variable region comprising the amino acid sequence shown in SEQ ID NO: 43 in combination with a second variable region comprising the amino acid sequence shown in SEQ ID NO: 44;

(VII) a binding site comprising a first variable region comprising the amino acid sequence shown in SEQ ID NO: 45 in combination with a second variable region comprising the amino acid sequence shown in SEQ ID NO: 46;

(VIII) a binding site comprising a first variable region comprising the amino acid sequence shown in SEQ ID NO: 47 in combination with a second variable region comprising the amino acid sequence shown in SEQ ID NO: 48;

(IX) a binding site comprising a first variable region comprising the amino acid sequence shown in SEQ ID NO: 49 in combination with a second variable region comprising the amino acid sequence shown in SEQ ID NO: 50;

(X) a binding site comprising a first variable region comprising the amino acid sequence shown in SEQ ID NO: 51 in combination with a second variable region comprising the amino acid sequence shown in SEQ ID NO: 52;

(XI) a binding site comprising a first variable region comprising the amino acid sequence shown in SEQ ID NO: 61 in combination with a second variable region comprising the amino acid sequence shown in SEQ ID NO: 62; and
(XII) a binding site comprising a first variable region comprising the amino acid sequence shown in SEQ ID NO: 63 in combination with a second variable region comprising the amino acid sequence shown in SEQ ID NO: 64.

16. The substance according to claim 8, wherein the activity of binding to KIR2DL2 and/or KIR2DL3 is higher than the activity of binding to KIR2DS1, KIR2DS2, and/or KIR2DS4.

17. The substance according to claim 8, wherein the agonist activity on KIR2DL2 and/or KIR2DL3 is higher than the agonist activity on KIR2DS1, KIR2DS2, and/or KIR2DS4.

18. The substance according to claim 17, which exerts substantially no agonist activity on KIR2DS1, KIR2DS2, and/or KIR2DS4.

19. The substance according to claim 17, which does not substantially bind to KIR2DS1, KIR2DS2, and KIR2DS4.

20. The substance according to claim 19, which comprises an antigen-binding fragment obtained by the method comprising:

   step A: a step of obtaining a population of display bodies displaying an antigen-binding site binding to KIR2DL2 or KIR2DL3 comprising mixing a labeled soluble protein comprising an extracellular domain of KIR2DL2 or KIR2DL3 in a suspension containing a population of polyclonal display bodies including display bodies displaying the antigen-binding site binding to KIR2DL2 or KIR2DL3 on the surface, bringing the suspension into contact with a carrier comprising, immobilized thereon, a substance binding specifically to the label part of said labeled soluble protein, and collecting the display bodies bound to the carrier;
   step B: a step of removing display bodies displaying the antigen-binding site binding to KIR2DS1, KIR2DS2, and KIR2DS4 from the population of display bodies obtained in Step A, comprising mixing a labeled soluble protein comprising extracellular domains of KIR2DS1, KIR2DS2, and KIR2DS4 in the suspension containing the population of display bodies obtained in step A, bringing the suspension into contact with a carrier comprising, immobilized thereon, a substance binding to the label part of said labeled foluble protein, and collecting the display bodies not bound to the carrier;
   step C: a step of identifying a sequence of a gene encoding the antigen-binding site by performing genetic analysis of the display bodies obtained the step B; and
   step D: a step of expressing the identified gene comprising the antigen-binding site in a cell.

21. The substance according to claim 19, which comprises an antigen-binding fragment obtained by the method comprising:

   step A: a step of obtaining a population of display bodies displaying an antigen-binding site binding specifically to KIR2DL2 or KIR2DL3 comprising mixing a labeled soluble protein comprising an extracellular domain of KIR2DL2 or KIR2DL3 and an unlabeled soluble protein comprising extracellular domains of KIR2DS1, KIR2DS2, and KIR2DS4 in a suspension containing a population of polyclonal display bodies including display bodies displaying the antigen-binding site binding to KIR2DL2 or KIR2DL3 on the surface, bringing the suspension into contact with a carrier comprising, immobilized thereon, a substance binding specifically to the label part of said labeled soluble protein, and collecting the display bodies bound to the carrier;
   step B: a step of identifying a sequence of a gene encoding the antigen-binding site by performing genetic analysis of the display bodies obtained in the step A; and
   step C: a step of expressing the identified gene comprising the antigen-binding site in a cell.

22. The substance according to claim 20 or 21, wherein a combination of a label and a label-binding substance is a combination of biotin and avidin.

23. The substance according to claim 20 or 21, which is obtained by a method further comprising a step of selecting a substance having an agonist activity on KIR2DL2 or KIR2DL3.

24. The substance according to any one of claims 8 to 23, wherein the polypeptide is a monoclonal antibody or an antigen-binding fragment.

25. The substance according to claim 24, which is a single-stranded antibody or scFv comprising a first variable region linked to a second variable region via a GS linker or a G4S linker.

26. The substance according to any one of claims 8 to 23, wherein the polypeptide is a membrane protein having an extracellular domain comprising the site binding to KIR2DL2 and/or KIR2DL3 and a transmembrane domain.

27. The substance according to claim 3, wherein the inhibitory KIR is KIR3DL1 and the binding site comprises an amino acid sequence derived from a heavy chain variable region and a light chain variable region of the monoclonal antibody binding to KIR3DL1 or an antigen-binding fragment.

28. The substance according to claim 27, wherein the monoclonal antibody binding to KIR3DL1 or an antigen-binding fragment thereof competes with scFv consisting of an amino acid sequence according to any of (xiii) to (xviii) below when binding to KIR3DL1:

   (xiii) an amino acid sequence comprising the amino acid sequence of a heavy chain variable region shown in SEQ ID NO: 56 in combination with the amino acid sequence of a light chain variable region shown in SEQ ID NO: 55;
   (xiv) an amino acid sequence comprising the amino acid sequence of a heavy chain variable region shown in SEQ ID NO: 137 in combination with the amino acid sequence of a light chain variable region shown in SEQ ID NO: 138;
   (xv) an amino acid sequence comprising the amino acid sequence of a heavy chain variable region shown in SEQ ID NO: 139 in combination with the amino acid sequence of a light chain variable region shown in SEQ ID NO: 140;
   (xvi) an amino acid sequence comprising the amino acid sequence of a heavy chain variable region shown in SEQ ID NO: 141 in combination with the amino acid sequence of a light chain variable region shown in SEQ ID NO: 142;
   (xvii) an amino acid sequence comprising the amino acid sequence of a heavy chain variable region shown in SEQ ID NO: 143 in combination with the amino acid sequence of a light chain variable region shown in SEQ ID NO: 144; and
   (xviii) an amino acid sequence comprising the amino acid sequence of a heavy chain variable region shown in SEQ ID NO: 145 in combination with the amino acid sequence of a light chain variable region shown in SEQ ID NO: 146.

29. The substance according to claim 27, which is a polypeptide comprising a binding site according to any of (XIII) to (XVIII) below as the site binding to KIR3DL1 and optionally comprising mutation of 1 or 2 amino acids in each CDR:

   (XIII) a binding site comprising a first variable region comprising the amino acid sequence derived from the amino acid sequence of CDR1, CDR2, and CDR3 shown in SEQ ID NO: 56 in combination with a second variable region comprising the amino acid sequence derived from the amino acid sequence of CDR1, CDR2, and CDR3 shown in SEQ ID NO: 55;
   (XIV) a binding site comprising a first variable region comprising the amino acid sequence derived from the amino acid sequence of CDR1, CDR2, and CDR3 shown in SEQ ID NO: 137 in combination with a second variable region comprising the amino acid sequence derived from the amino acid sequence of CDR1, CDR2, and CDR3 shown in SEQ ID NO: 138;
   (XV) a binding site comprising a first variable region comprising the amino acid sequence derived from the amino acid sequence of CDR1, CDR2, and CDR3 shown in SEQ ID NO: 139 in combination with a second variable region comprising the amino acid sequence derived from the amino acid sequence of CDR1, CDR2, and CDR3 shown in SEQ ID NO: 140;
   (XVI) a binding site comprising a first variable region comprising the amino acid sequence derived from the amino acid sequence of CDR1, CDR2, and CDR3 shown in SEQ ID NO: 141 in combination with a second variable region comprising the amino acid sequence derived from the amino acid sequence of CDR1, CDR2, and CDR3 shown in SEQ ID NO: 142;
   (XVII) a binding site comprising a first variable region comprising the amino acid sequence derived from the amino acid sequence of CDR1, CDR2, and CDR3 shown in SEQ ID NO: 143 in combination with a second variable region comprising the amino acid sequence derived from the amino acid sequence of CDR1, CDR2, and CDR3 shown in SEQ ID NO: 144; and
   (XVIII) a binding site comprising a first variable region comprising the amino acid sequence derived from the amino acid sequence of CDR1, CDR2, and CDR3 shown in SEQ ID NO: 145 in combination with a second variable region comprising the amino acid sequence derived from the amino acid sequence of CDR1, CDR2, and CDR3 shown in SEQ ID NO: 146.

30. The substance according to claim 29, which optionally comprises mutation of 1 or 2 amino acids on the C and/or N terminal sides of each CDR.

31. The substance according to claim 27, which is a polypeptide comprising a binding site according to any of (XIII) to (XVIII) below as the site binding to KIR3DL1;

(XIII) a binding site comprising a first variable region comprising the amino acid sequence shown in SEQ ID NO: 56 in combination with a second variable region comprising the amino acid sequence shown in SEQ ID NO: 55;
(XIIII) a binding site comprising a first variable region comprising the amino acid sequence shown in SEQ ID NO: 137 in combination with a second variable region comprising the amino acid sequence shown in SEQ ID NO: 138;
(XV) a binding site comprising a first variable region comprising the amino acid sequence shown in SEQ ID NO: 139 in combination with a second variable region comprising the amino acid sequence shown in SEQ ID NO: 140;
(XVI) a binding site comprising a first variable region comprising the amino acid sequence shown in SEQ ID NO: 141 in combination with a second variable region comprising the amino acid sequence shown in SEQ ID NO: 142;
(XVII) a binding site comprising a first variable region comprising the amino acid sequence shown in SEQ ID NO: 143 in combination with a second variable region comprising the amino acid sequence shown in SEQ ID NO: 144; and
(XVIII) a binding site comprising a first variable region comprising the amino acid sequence shown in SEQ ID NO: 145 in combination with a second variable region comprising the amino acid sequence shown in SEQ ID NO: 146.

32. The substance according to claim 27, wherein the activity of binding to KIR3DL1 is higher than the activity of binding to KIR3DS1.

33. The substance according to claim 27, wherein the agonist activity on KIR3DL1 is higher than the agonist activity on KIR3DS1.

34. The substance according to claim 33, which exerts substantially no agonist activity on KIR3DS1.

35. The substance according to claim 34, which does not substantially bind to KIR3DS1.

36. The substance according to claim 35, which comprises an antigen-binding fragment obtained by the method comprising:

step A: a step of obtaining a population of display bodies displaying an antigen-binding site binding to KIR3DL1 comprising mixing a labeled soluble protein comprising an extracellular domain of KIR3DL1 in a suspension containing a population of polyclonal display bodies including display bodies displaying, on the surface, the antigen-binding site binding to KIR3DL1, bringing the suspension into contact with a carrier comprising, immobilized thereon, a substance binding specifically to the label part of said labeled soluble protein, and collecting the display bodies bound to the carrier;
step B: a step of removing display bodies displaying the antigen-binding site binding to KIR3DS1 from the population of display bodies obtained in Step A, comprising mixing a labeled soluble protein comprising an extracellular domain of KIR3DS1 in the suspension containing the population of display bodies obtained in step A, bringing the suspension into contact with a carrier comprising, immobilized thereon, a substance binding to the label part of said labeled soluble protein, and collecting the display bodies not bound to the carrier;
step C: a step of identifying a sequence of a gene encoding the antigen-binding site by performing genetic analysis of the display bodies obtained in the step B; and
step D: a step of expressing the identified gene comprising the antigen-binding site in a cell.

37. The substance according to claim 36, which comprises an antigen-binding fragment obtained by the method comprising:

step A: a step of obtaining a population of display bodies displaying an antigen-binding site binding specifically to KIR3DL1 comprising mixing a labeled soluble protein comprising an extracellular domain of KIR3DL1 and an unlabeled soluble protein comprising an extracellular domain of KIR3DS1 in a suspension containing a population of polyclonal display bodies including display bodies displaying, on the surface, the antigen-binding site binding to KIR3DL1, bringing the suspension into contact with a carrier comprising, immobilized thereon, a substance binding specifically to the label part of said labeled soluble protein, and collecting the display bodies bound to the carrier;
step B: a step of identifying a sequence of a gene encoding the antigen-binding site by performing genetic analysis of the display bodies obtained in the step A; and
step C: a step of expressing the identified gene comprising the antigen-binding site in a cell.

**38.** The substance according to claim 36 or 37, wherein a combination of a label and a label-binding substance is a combination of biotin and avidin.

**39.** The substance according to any one of claims 36 to 38, which is obtained by a method further comprising a step of selecting a substance having an agonist activity on KIR3DL1.

**40.** The substance according to any one of claims 27 to 39, wherein the polypeptide is a monoclonal antibody or an antigen-binding fragment.

**41.** The substance according to claim 40, which is a single-stranded antibody or scFv comprising a first variable region linked to a second variable region via a GS linker or a G4S linker.

**42.** The substance according to any one of claims 27 to 34, wherein the polypeptide is a membrane protein having an extracellular domain comprising the site binding to KIR3DL1 and a transmembrane domain.

**43.** The substance according to claim 3, wherein the inhibitory KIR is KIR2DL1 and the binding site comprises an amino acid sequence derived from a heavy chain variable region and a light chain variable region of a monoclonal antibody binding to KIR2DL1 or an antigen-binding fragment.

**44.** The substance according to claim 43, wherein the monoclonal antibody binding to KIR2DL1 or an antigen-binding fragment competes with Pan2D when binding to KIR2DL1.

**45.** The substance according to claim 43, wherein the monoclonal antibody binding to KIR2DL1 or an antigen-binding fragment thereof competes with scFv consisting of an amino acid sequence according to any of (ci) to (cxv) below when binding to KIR2DL1:

(ci) an amino acid sequence comprising the amino acid sequence of a heavy chain variable region shown in SEQ ID NO: 107 in combination with the amino acid sequence of a light chain variable region shown in SEQ ID NO: 108;
(cii) an amino acid sequence comprising the amino acid sequence of a heavy chain variable region shown in SEQ ID NO: 109 in combination with the amino acid sequence of a light chain variable region shown in SEQ ID NO: 110;
(ciii) an amino acid sequence comprising the amino acid sequence of a heavy chain variable region shown in SEQ ID NO: 111 in combination with the amino acid sequence of a light chain variable region shown in SEQ ID NO: 112;
(civ) an amino acid sequence comprising the amino acid sequence of a heavy chain variable region shown in SEQ ID NO: 113 in combination with the amino acid sequence of a light chain variable region shown in SEQ ID NO: 114;
(cv) an amino acid sequence comprising the amino acid sequence of a heavy chain variable region shown in SEQ ID NO: 115 in combination with the amino acid sequence of a light chain variable region shown in SEQ ID NO: 116;
(cvi) an amino acid sequence comprising the amino acid sequence of a heavy chain variable region shown in SEQ ID NO: 117 in combination with the amino acid sequence of a light chain variable region shown in SEQ ID NO: 118;
(cvii) an amino acid sequence comprising the amino acid sequence of a heavy chain variable region shown in SEQ ID NO: 119 in combination with the amino acid sequence of a light chain variable region shown in SEQ ID NO: 120;
(cviii) an amino acid sequence comprising the amino acid sequence of a heavy chain variable region shown in SEQ ID NO: 121 in combination with the amino acid sequence of a light chain variable region shown in SEQ ID NO: 122;
(cviiii) an amino acid sequence comprising the amino acid sequence of a heavy chain variable region shown in SEQ ID NO: 123 in combination with the amino acid sequence of a light chain variable region shown in SEQ ID NO: 124;
(cx) an amino acid sequence comprising the amino acid sequence of a heavy chain variable region shown in SEQ ID NO: 125 in combination with the amino acid sequence of a light chain variable region shown in SEQ ID NO: 126;
(cxi) an amino acid sequence comprising the amino acid sequence of a heavy chain variable region shown in SEQ ID NO: 127 in combination with the amino acid sequence of a light chain variable region shown in SEQ ID NO: 128;
(cxii) an amino acid sequence comprising the amino acid sequence of a heavy chain variable region shown in SEQ ID NO: 129 in combination with the amino acid sequence of a light chain variable region shown in SEQ ID NO: 130;
(cxiii) an amino acid sequence comprising the amino acid sequence of a heavy chain variable region shown in SEQ ID NO: 131 in combination with the amino acid sequence of a light chain variable region shown in SEQ ID NO: 132;
(cxiv) an amino acid sequence comprising the amino acid sequence of a heavy chain variable region shown in SEQ ID NO: 133 in combination with the amino acid sequence of a light chain variable region shown in SEQ ID NO: 134; and

(cxv) an amino acid sequence comprising the amino acid sequence of a heavy chain variable region shown in SEQ ID NO: 135 in combination with the amino acid sequence of a light chain variable region shown in SEQ ID NO: 136.

46. The substance according to claim 43, which is a polypeptide comprising a binding site according to any of (CI) to (CXV) below as the site binding to KIR2DL1 and optionally comprising mutation of 1 or 2 amino acids in each CDR:

(CI) a binding site comprising a first variable region comprising the amino acid sequence derived from the amino acid sequence of CDR1, CDR2, and CDR3 shown in SEQ ID NO: 107 in combination with a second variable region comprising the amino acid sequence derived from the amino acid sequence of CDR1, CDR2, and CDR3 shown in SEQ ID NO: 108;

(CII) a binding site comprising a first variable region comprising the amino acid sequence derived from the amino acid sequence of CDR1, CDR2, and CDR3 shown in SEQ ID NO: 109 in combination with a second variable region comprising the amino acid sequence derived from the amino acid sequence of CDR1, CDR2, and CDR3 shown in SEQ ID NO: 110;

(CIII) a binding site comprising a first variable region comprising the amino acid sequence derived from the amino acid sequence of CDR1, CDR2, and CDR3 shown in SEQ ID NO: 111 in combination with a second variable region comprising the amino acid sequence derived from the amino acid sequence of CDR1, CDR2, and CDR3 shown in SEQ ID NO: 112;

(CIV) a binding site comprising a first variable region comprising the amino acid sequence derived from the amino acid sequence of CDR1, CDR2, and CDR3 shown in SEQ ID NO: 113 in combination with a second variable region comprising the amino acid sequence derived from the amino acid sequence of CDR1, CDR2, and CDR3 shown in SEQ ID NO: 114 (preferably, a variable region comprising the amino acid sequence shown in SEQ ID NO: 114);

(CV) a binding site comprising a first variable region comprising the amino acid sequence derived from the amino acid sequence of CDR1, CDR2, and CDR3 shown in SEQ ID NO: 115 in combination with a second variable region comprising the amino acid sequence derived from the amino acid sequence of CDR1, CDR2, and CDR3 shown in SEQ ID NO: 116;

(CVI) a binding site comprising a first variable region comprising the amino acid sequence derived from the amino acid sequence of CDR1, CDR2, and CDR3 shown in SEQ ID NO: 117 in combination with a second variable region comprising the amino acid sequence derived from the amino acid sequence of CDR1, CDR2, and CDR3 shown in SEQ ID NO: 118;

(CVII) a binding site comprising a first variable region comprising the amino acid sequence derived from the amino acid sequence of CDR1, CDR2, and CDR3 shown in SEQ ID NO: 119 in combination with a second variable region comprising the amino acid sequence derived from the amino acid sequence of CDR1, CDR2, and CDR3 shown in SEQ ID NO: 120;

(CVIII) a binding site comprising a first variable region comprising the amino acid sequence derived from the amino acid sequence of CDR1, CDR2, and CDR3 shown in SEQ ID NO: 121 in combination with a second variable region comprising the amino acid sequence derived from the amino acid sequence of CDR1, CDR2, and CDR3 shown in SEQ ID NO: 122;

(CVIIII) a binding site comprising a first variable region comprising the amino acid sequence derived from the amino acid sequence of CDR1, CDR2, and CDR3 shown in SEQ ID NO: 123 in combination with a second variable region comprising the amino acid sequence derived from the amino acid sequence of CDR1, CDR2, and CDR3 shown in SEQ ID NO: 124;

(CX) a binding site comprising a first variable region comprising the amino acid sequence derived from the amino acid sequence of CDR1, CDR2, and CDR3 shown in SEQ ID NO: 125 in combination with a second variable region comprising the amino acid sequence derived from the amino acid sequence of CDR1, CDR2, and CDR3 shown in SEQ ID NO: 126;

(CXI) a binding site comprising a first variable region comprising the amino acid sequence derived from the amino acid sequence of CDR1, CDR2, and CDR3 shown in SEQ ID NO: 127 in combination with a second variable region comprising the amino acid sequence derived from the amino acid sequence of CDR1, CDR2, and CDR3 shown in SEQ ID NO: 128;

(CXII) a binding site comprising a first variable region comprising the amino acid sequence derived from the amino acid sequence of CDR1, CDR2, and CDR3 shown in SEQ ID NO: 129 in combination with a second variable region comprising the amino acid sequence derived from the amino acid sequence of CDR1, CDR2, and CDR3 shown in SEQ ID NO: 130;

(CXIII) a binding site comprising a first variable region comprising the amino acid sequence derived from the amino acid sequence of CDR1, CDR2, and CDR3 shown in SEQ ID NO: 131 in combination with a second variable region comprising the amino acid sequence derived from the amino acid sequence of CDR1, CDR2, and CDR3 shown in SEQ ID NO: 132;

(CXIV) a binding site comprising a first variable region comprising the amino acid sequence derived from the amino acid sequence of CDR1, CDR2, and CDR3 shown in SEQ ID NO: 133 in combination with a second variable region comprising the amino acid sequence derived from the amino acid sequence of CDR1, CDR2, and CDR3 shown in SEQ ID NO: 134; and

(CXV) a binding site comprising a first variable region comprising the amino acid sequence derived from the amino acid sequence of CDR1, CDR2, and CDR3 shown in SEQ ID NO: 135 in combination with a second variable region comprising the amino acid sequence derived from the amino acid sequence of CDR1, CDR2, and CDR3 shown in SEQ ID NO: 136.

47. The substance according to claim 46, which optionally comprises mutation of 1 or 2 amino acids on the C and/or N terminal sides of each CDR.

48. The substance according to claim 46, which is a polypeptide comprising a binding site according to any of (CI) to (CXV) below as the site binding to KIR2DL1:

(CI) a binding site comprising a first variable region comprising the amino acid sequence shown in SEQ ID NO: 107 in combination with a second variable region comprising the amino acid sequence shown in SEQ ID NO: 108;

(CII) a binding site comprising a first variable region comprising the amino acid sequence shown in SEQ ID NO: 109 in combination with a second variable region comprising the amino acid sequence shown in SEQ ID NO: 110;

(CIII) a binding site comprising a first variable region comprising the amino acid sequence shown in SEQ ID NO: 111 in combination with a second variable region comprising the amino acid sequence shown in SEQ ID NO: 112;

(CIV) a binding site comprising a first variable region comprising the amino acid sequence shown in SEQ ID NO: 113 in combination with a second variable region comprising the amino acid sequence shown in SEQ ID NO: 114 (preferably, a variable region comprising the amino acid sequence shown in SEQ ID NO: 114);

(CV) a binding site comprising a first variable region comprising the amino acid sequence shown in SEQ ID NO: 115 in combination with a second variable region comprising the amino acid sequence shown in SEQ ID NO: 116;

(CVI) a binding site comprising a first variable region comprising the amino acid sequence shown in SEQ ID NO: 117 in combination with a second variable region comprising the amino acid sequence shown in SEQ ID NO: 118;

(CVII) a binding site comprising a first variable region comprising the amino acid sequence shown in SEQ ID NO: 119 in combination with a second variable region comprising the amino acid sequence shown in SEQ ID NO: 120;

(CVIII) a binding site comprising a first variable region comprising the amino acid sequence shown in SEQ ID NO: 121 in combination with a second variable region comprising the amino acid sequence shown in SEQ ID NO: 122;

(CVIIII) a binding site comprising a first variable region comprising the amino acid sequence shown in SEQ ID NO: 123 in combination with a second variable region comprising the amino acid sequence shown in SEQ ID NO: 124;

(CX) a binding site comprising a first variable region comprising the amino acid sequence shown in SEQ ID NO: 125 in combination with a second variable region comprising the amino acid sequence shown in SEQ ID NO: 126;

(CXI) a binding site comprising a first variable region comprising the amino acid sequence shown in SEQ ID NO: 127 in combination with a second variable region comprising the amino acid sequence shown in SEQ ID NO: 128;

(CXII) a binding site comprising a first variable region comprising the amino acid sequence shown in SEQ ID NO: 129 in combination with a second variable region comprising the amino acid sequence shown in SEQ ID NO: 130;

(CXIII) a binding site comprising a first variable region comprising the amino acid sequence shown in SEQ ID NO: 131 in combination with a second variable region comprising the amino acid sequence shown in SEQ ID NO: 132;

(CXIV) a binding site comprising a first variable region comprising the amino acid sequence shown in SEQ ID NO: 133 in combination with a second variable region comprising the amino acid sequence shown in SEQ ID NO: 134; and

(CXV) a binding site comprising a first variable region comprising the amino acid sequence shown in SEQ ID NO: 135 in combination with a second variable region comprising the amino acid sequence shown in SEQ ID NO: 136.

49. The substance according to claim 43, wherein the activity of binding to KIR2DL1 is higher than the activity of binding to KIR2DS1, KIR2DS2, and KIR2DS4.

50. The substance according to claim 43, wherein the agonist activity on KIR2DL1 is higher than the agonist activity on KIR2DS1, KIR2DS2, and KIR2DS4.

51. The substance according to claim 50, which exerts substantially no agonist activity on KIR2DS1, KIR2DS2, and KIR2DS4.

52. The substance according to claim 51, which does not substantially bind to KIR2DS1, KIR2DS2, and KIR2DS4.

53. The substance according to claim 52, which comprises an antigen-binding fragment obtained by the method comprising:

step A: a step of obtaining a population of display bodies displaying an antigen-binding site binding to KIR2DL1 comprising mixing a labeled soluble protein comprising an extracellular domain of KIR2DL1 in a suspension containing a population of polyclonal display bodies including display bodies displaying, on the surface, the antigen-binding site binding to KIR2DL1, bringing the suspension into contact with a carrier comprising, immobilized thereon, a substance binding specifically to the label part of said labeled soluble protein, and collecting the display bodies bound to the carrier;
step B: a step of removing display bodies displaying the antigen-binding site binding to KIR2DS1, KIR2DS2, and KIR2DS4 from the population of display bodies obtained in Step A, comprising mixing a labeled soluble protein comprising extracellular domains of KIR2DS1, KIR2DS2, and KIR2DS4 in the suspension containing the population of display bodies obtained in step A, bringing the suspension into contact with a carrier comprising, immobilized thereon, a substance binding to the label part of said labeled soluble protein, and collecting the display bodies not bound to the carrier;
step C: a step of identifying a sequence of a gene encoding the antigen-binding site by performing genetic analysis of the display bodies obtained in Step B; and
step D: a step of expressing the identified gene comprising the antigen-binding site in a cell.

54. The substance according to claim 52, which comprises an antigen-binding fragment obtained by the method comprising:

step A: a step of obtaining a population of display bodies displaying an antigen-binding site binding specifically to KIR2DL1 comprising mixing a labeled soluble protein comprising an extracellular domain of KIR2DL1 and an unlabeled soluble protein comprising extracellular domains of KIR2DS1, KIR2DS2, and KIR2DS4 in a suspension containing a population of polyclonal display bodies including display bodies displaying, on the surface, the antigen-binding site binding to KIR2DL1, bringing the suspension into contact with a carrier comprising, immobilized thereon, a substance binding specifically to the label part of said labeled soluble protein, and collecting the display bodies bound to the carrier;
step B: a step of identifying a sequence of a gene encoding the antigen-binding site by performing genetic analysis of the display bodies obtained in Step A; and
step C: a step of expressing the identified gene comprising the antigen-binding site in a cell.

55. The substance according to claim 53 or 54, wherein a combination of a label and a label-binding substance is a combination of biotin and avidin.

56. The substance according to any one of claims 53 to 55, which is obtained by a method further comprising a step of selecting a substance having an agonist activity on KIR3DL1.

57. The substance according to any one of claims 43 to 56, wherein the polypeptide is a monoclonal antibody or an antigen-binding fragment.

58. The substance according to claim 57, which is a single-stranded antibody or scFv comprising a first variable region linked to a second variable region via a GS linker.

59. The substance according to any one of claims 43 to 56, wherein the polypeptide is a membrane protein having an extracellular domain comprising the site binding to KIR2DL1 and a transmembrane domain.

60. A pharmaceutical composition comprising, as an active ingredient, the substance according to any one of claims 1 to 59.

61. The pharmaceutical composition according to claim 60, which is an immune rejection inhibitor.

**62.** A cell comprising a substance having activity of binding to the inhibitory KIR according to any one of claims 1 to 59 and inhibiting the cytocidal activity of an NK cell expressed on the surface.

**63.** The cell according to claim 62, which further comprises a ligand for NKG2A expressed on the cell surface.

**64.** The cell according to claim 63, wherein the ligand for NKG2A is HLA-E.

**65.** The cell according to claim 62, wherein the expression of at least one class I HLA is quenched or attenuated on the cell surface.

**66.** The cell according to claim 65, wherein the expression of at least one class I HLA is quenched or attenuated on the cell surface by modification of class I HLA, B2M, TAP1, TAP2, and TAPBP regions on the genome by gene editing.

**67.** The cell according to claim 65, which is a cell line established from an individual in which HLA expression is genetically quenched.

**68.** The cell according to claim 62, which further comprises a ligand for NKG2A expressed on the cell surface and shows quenched or attenuated expression of at least one class I HLA on the cell surface.

**69.** The cell according to claim 62, which is a pluripotent stem cell, T cell, NK cell, peripheral blood mononuclear cell (PBMC), mesenchymal stem cell, myocardial cell, cartilage cell, retinal cell, hepatic cell, kidney cell, or pancreatic cell.

**70.** The cell according to claim 69, wherein the pluripotent stem cell is an iPS cell or ES cell.

**71.** The cell according to any one of claims 62 to 70, which further comprises a chimeric receptor expressed on the cell surface.

**72.** The cell according to claim 62, wherein the inhibitory KIR is KIR2DL2 and/or KIR2DL3 and the substance having activity of binding to KIR2DL2 and/or KIR2DL3 and inhibiting the cytocidal activity of an NK cell is the membrane protein according to claim 26.

**73.** The cell according to claim 72, wherein the membrane protein binding to KIR2DL2 and/or KIR2DL3 comprises a binding site competing with Lirilumab, Pan2D, or scFv consisting of an amino acid sequence according to any of (i) to (xii) below when binding to KIR2DL2 and/or KIR2DL3:

(i) an amino acid sequence comprising the amino acid sequence of a heavy chain variable region shown in SEQ ID NO: 33 in combination with the amino acid sequence of a light chain variable region shown in SEQ ID NO: 34;
(ii) an amino acid sequence comprising the amino acid sequence of a heavy chain variable region shown in SEQ ID NO: 35 in combination with the amino acid sequence of a light chain variable region shown in SEQ ID NO: 36;
(iii) an amino acid sequence comprising the amino acid sequence of a heavy chain variable region shown in SEQ ID NO: 37 in combination with the amino acid sequence of a light chain variable region shown in SEQ ID NO: 38;
(iv) an amino acid sequence comprising the amino acid sequence of a heavy chain variable region shown in SEQ ID NO: 39 in combination with the amino acid sequence of a light chain variable region shown in SEQ ID NO: 40;
(v) an amino acid sequence comprising the amino acid sequence of a heavy chain variable region shown in SEQ ID NO: 41 in combination with the amino acid sequence of a light chain variable region shown in SEQ ID NO: 42;
(vi) an amino acid sequence comprising the amino acid sequence of a heavy chain variable region shown in SEQ ID NO: 43 in combination with the amino acid sequence of a light chain variable region shown in SEQ ID NO: 44;
(vii) an amino acid sequence comprising the amino acid sequence of a heavy chain variable region shown in SEQ ID NO: 45 in combination with the amino acid sequence of a light chain variable region shown in SEQ ID NO: 46;
(viii) an amino acid sequence comprising the amino acid sequence of a heavy chain variable region shown in SEQ ID NO: 47 in combination with the amino acid sequence of a light chain variable region shown in SEQ ID NO: 48;
(ix) an amino acid sequence comprising the amino acid sequence of a heavy chain variable region shown in SEQ ID NO: 49 in combination with the amino acid sequence of a light chain variable region shown in SEQ ID NO: 50;
(x) an amino acid sequence comprising the amino acid sequence of a heavy chain variable region shown in SEQ ID NO: 51 in combination with the amino acid sequence of a light chain variable region shown in SEQ ID NO: 52;
(xi) an amino acid sequence comprising the amino acid sequence of a heavy chain variable region shown in SEQ ID NO: 61 in combination with the amino acid sequence of a light chain variable region shown in SEQ ID NO: 62; and

(xii) an amino acid sequence comprising the amino acid sequence of a heavy chain variable region shown in SEQ ID NO: 63 in combination with the amino acid sequence of a light chain variable region shown in SEQ ID NO: 64.

74. The cell according to claim 72, which comprises, as the site binding to KIR2DL2 and/or KIR2DL3, a membrane protein comprising a binding site according to any of (I) to (XII) below and optionally comprising mutation of 1 or 2 amino acids in each CDR expressed on the cell surface:

(I) a binding site comprising a first variable region comprising the amino acid sequence derived from the amino acid sequence of CDR1, CDR2, and CDR3 shown in SEQ ID NO: 33 in combination with a second variable region comprising the amino acid sequence derived from the amino acid sequence of CDR1, CDR2, and CDR3 shown in SEQ ID NO: 34;

(II) a binding site comprising a first variable region comprising the amino acid sequence derived from the amino acid sequence of CDR1, CDR2, and CDR3 shown in SEQ ID NO: 33 in combination with a second variable region comprising the amino acid sequence derived from the amino acid sequence of CDR1, CDR2, and CDR3 shown in SEQ ID NO: 36;

(III) a binding site comprising a first variable region comprising the amino acid sequence derived from the amino acid sequence of CDR1, CDR2, and CDR3 shown in SEQ ID NO: 37 in combination with a second variable region comprising the amino acid sequence derived from the amino acid sequence of CDR1, CDR2, and CDR3 shown in SEQ ID NO: 38;

(IV) a binding site comprising a first variable region comprising the amino acid sequence derived from the amino acid sequence of CDR1, CDR2, and CDR3 shown in SEQ ID NO: 39 in combination with a second variable region comprising the amino acid sequence derived from the amino acid sequence of CDR1, CDR2, and CDR3 shown in SEQ ID NO: 40;

(V) a binding site comprising a first variable region comprising the amino acid sequence derived from the amino acid sequence of CDR1, CDR2, and CDR3 shown in SEQ ID NO: 41 in combination with a second variable region comprising the amino acid sequence derived from the amino acid sequence of CDR1, CDR2, and CDR3 shown in SEQ ID NO: 42;

(VI) a binding site comprising a first variable region comprising the amino acid sequence derived from the amino acid sequence of CDR1, CDR2, and CDR3 shown in SEQ ID NO: 43 in combination with a second variable region comprising the amino acid sequence derived from the amino acid sequence of CDR1, CDR2, and CDR3 shown in SEQ ID NO: 44;

(VII) a binding site comprising a first variable region comprising the amino acid sequence derived from the amino acid sequence of CDR1, CDR2, and CDR3 shown in SEQ ID NO: 45 in combination with a second variable region comprising the amino acid sequence derived from the amino acid sequence of CDR1, CDR2, and CDR3 shown in SEQ ID NO: 46;

(VIII) a binding site comprising a first variable region comprising the amino acid sequence derived from the amino acid sequence of CDR1, CDR2, and CDR3 shown in SEQ ID NO: 47 in combination with a second variable region comprising the amino acid sequence derived from the amino acid sequence of CDR1, CDR2, and CDR3 shown in SEQ ID NO: 48;

(IX) a binding site comprising a first variable region comprising the amino acid sequence derived from the amino acid sequence of CDR1, CDR2, and CDR3 shown in SEQ ID NO: 49 in combination with a second variable region comprising the amino acid sequence derived from the amino acid sequence of CDR1, CDR2, and CDR3 shown in SEQ ID NO: 50;

(X) a binding site comprising a first variable region comprising the amino acid sequence derived from the amino acid sequence of CDR1, CDR2, and CDR3 shown in SEQ ID NO: 51 in combination with a second variable region comprising the amino acid sequence derived from the amino acid sequence of CDR1, CDR2, and CDR3 shown in SEQ ID NO: 52;

(XI) a binding site comprising a first variable region comprising the amino acid sequence derived from the amino acid sequence of CDR1, CDR2, and CDR3 shown in SEQ ID NO: 61 in combination with a second variable region comprising the amino acid sequence derived from the amino acid sequence of CDR1, CDR2, and CDR3 shown in SEQ ID NO: 62; and

(XII) a binding site comprising a first variable region comprising the amino acid sequence derived from the amino acid sequence of CDR1, CDR2, and CDR3 shown in SEQ ID NO: 63 in combination with a second variable region comprising the amino acid sequence derived from the amino acid sequence of CDR1, CDR2, and CDR3 shown in SEQ ID NO: 64.

75. The cell according to claim 74, which optionally comprises mutation of 1 or 2 amino acids on the C and/or N terminal sides of each CDR.

**76.** The cell according to claim 62, wherein the inhibitory KIR is KIR3DL1 and the substance having activity of binding to KIR3DL1 and inhibiting the cytocidal activity of an NK cell is the membrane protein according to claim 42.

**77.** The cell according to claim 76, wherein the membrane protein binding to KIR3DL1 comprises a binding site competing with scFv consisting of an amino acid sequence according to any of (xiii) to (xviii) below when binding to KIR3DL1:

(xiii) an amino acid sequence comprising the amino acid sequence of a heavy chain variable region shown in SEQ ID NO: 56 in combination with the amino acid sequence of a light chain variable region shown in SEQ ID NO: 55;
(xiv) an amino acid sequence comprising the amino acid sequence of a heavy chain variable region shown in SEQ ID NO: 137 in combination with the amino acid sequence of a light chain variable region shown in SEQ ID NO: 138;
(xv) an amino acid sequence comprising the amino acid sequence of a heavy chain variable region shown in SEQ ID NO: 139 in combination with the amino acid sequence of a light chain variable region shown in SEQ ID NO: 140;
(xvi) an amino acid sequence comprising the amino acid sequence of a heavy chain variable region shown in SEQ ID NO: 141 in combination with the amino acid sequence of a light chain variable region shown in SEQ ID NO: 142;
(xvii) an amino acid sequence comprising the amino acid sequence of a heavy chain variable region shown in SEQ ID NO: 143 in combination with the amino acid sequence of a light chain variable region shown in SEQ ID NO: 144; and
(xviii) an amino acid sequence comprising the amino acid sequence of a heavy chain variable region shown in SEQ ID NO: 145 in combination with the amino acid sequence of a light chain variable region shown in SEQ ID NO: 146.

**78.** The cell according to claim 75, which comprises, as the site binding to KIR3DL1, a membrane protein comprising a binding site according to any of (XIII) to (XVIII) below and optionally comprising mutation of 1 or 2 amino acids in each CDR expressed on the cell surface:

(XIII) a binding site comprising a first variable region comprising the amino acid sequence derived from the amino acid sequence of CDR1, CDR2, and CDR3 shown in SEQ ID NO: 56 in combination with a second variable region comprising the amino acid sequence derived from the amino acid sequence of CDR1, CDR2, and CDR3 shown in SEQ ID NO: 55;
(XIV) a binding site comprising a first variable region comprising the amino acid sequence derived from the amino acid sequence of CDR1, CDR2, and CDR3 shown in SEQ ID NO: 137 in combination with a second variable region comprising the amino acid sequence derived from the amino acid sequence of CDR1, CDR2, and CDR3 shown in SEQ ID NO: 138 (preferably, a variable region comprising the amino acid sequence shown in SEQ ID NO: 138);
(XV) a binding site comprising a first variable region comprising the amino acid sequence derived from the amino acid sequence of CDR1, CDR2, and CDR3 shown in SEQ ID NO: 139 in combination with a second variable region comprising the amino acid sequence derived from the amino acid sequence of CDR1, CDR2, and CDR3 shown in SEQ ID NO: 140;
(XVI) a binding site comprising a first variable region comprising the amino acid sequence derived from the amino acid sequence of CDR1, CDR2, and CDR3 shown in SEQ ID NO: 141 in combination with a second variable region comprising the amino acid sequence derived from the amino acid sequence of CDR1, CDR2, and CDR3 shown in SEQ ID NO: 142;
(XVII) a binding site comprising a first variable region comprising the amino acid sequence derived from the amino acid sequence of CDR1, CDR2, and CDR3 shown in SEQ ID NO: 143 in combination with a second variable region comprising the amino acid sequence derived from the amino acid sequence of CDR1, CDR2, and CDR3 shown in SEQ ID NO: 144; and
(XVIII) a binding site comprising a first variable region comprising the amino acid sequence derived from the amino acid sequence of CDR1, CDR2, and CDR3 shown in SEQ ID NO: 145 in combination with a second variable region comprising the amino acid sequence derived from the amino acid sequence of CDR1, CDR2, and CDR3 shown in SEQ ID NO: 146.

**79.** The cell according to claim 78, which optionally comprises mutation of 1 or 2 amino acids on the C and/or N terminal sides of each CDR.

**80.** The cell according to claim 62, wherein the inhibitory KIR is KIR2DL 1 and the substance having activity of binding to KIR2DL1 and inhibiting the cytocidal activity of an NK cell is the membrane protein according to claim 59.

**81.** The cell according to claim 80, wherein the membrane protein binding to KIR2DL1 comprises a binding site competing with Pan2D when binding to KIR2DL1.

82. The cell according to claim 78, which comprises, as the site binding to KIR2DL1, a membrane protein comprising the binding site (P) below and optionally comprising mutation of 1 or 2 amino acids in each CDR expressed on the cell surface:

(P) a binding site comprising a first variable region comprising the amino acid sequence derived from the amino acid sequence of CDR1, CDR2, and CDR3 shown in SEQ ID NO: 31 in combination with a second variable region comprising the amino acid sequence derived from the amino acid sequence of CDR1, CDR2, and CDR3 shown in SEQ ID NO: 32.

83. The cell according to claim 82, which optionally comprises mutation of 1 or 2 amino acids on the C and/or N terminal sides of each CDR.

84. The cell according to claim 80, wherein the membrane protein binding to KIR2DL1 comprises a binding site competing with scFv consisting of an amino acid sequence according to any of (ci) to (cxv) below when binding to KIR2DL1:

(ci) an amino acid sequence comprising the amino acid sequence of a heavy chain variable region shown in SEQ ID NO: 107 in combination with the amino acid sequence of a light chain variable region shown in SEQ ID NO: 108;
(cii) an amino acid sequence comprising the amino acid sequence of a heavy chain variable region shown in SEQ ID NO: 109 in combination with the amino acid sequence of a light chain variable region shown in SEQ ID NO: 110;
(ciii) an amino acid sequence comprising the amino acid sequence of a heavy chain variable region shown in SEQ ID NO: 111 in combination with the amino acid sequence of a light chain variable region shown in SEQ ID NO: 112;
(civ) an amino acid sequence comprising the amino acid sequence of a heavy chain variable region shown in SEQ ID NO: 113 in combination with the amino acid sequence of a light chain variable region shown in SEQ ID NO: 114;
(cv) an amino acid sequence comprising the amino acid sequence of a heavy chain variable region shown in SEQ ID NO: 115 in combination with the amino acid sequence of a light chain variable region shown in SEQ ID NO: 116;
(cvi) an amino acid sequence comprising the amino acid sequence of a heavy chain variable region shown in SEQ ID NO: 117 in combination with the amino acid sequence of a light chain variable region shown in SEQ ID NO: 118;
(cvii) an amino acid sequence comprising the amino acid sequence of a heavy chain variable region shown in SEQ ID NO: 119 in combination with the amino acid sequence of a light chain variable region shown in SEQ ID NO: 120;
(cviii) an amino acid sequence comprising the amino acid sequence of a heavy chain variable region shown in SEQ ID NO: 121 in combination with the amino acid sequence of a light chain variable region shown in SEQ ID NO: 122;
(cviiii) an amino acid sequence comprising the amino acid sequence of a heavy chain variable region shown in SEQ ID NO: 123 in combination with the amino acid sequence of a light chain variable region shown in SEQ ID NO: 124;
(cx) an amino acid sequence comprising the amino acid sequence of a heavy chain variable region shown in SEQ ID NO: 125 in combination with the amino acid sequence of a light chain variable region shown in SEQ ID NO: 126;
(cxi) an amino acid sequence comprising the amino acid sequence of a heavy chain variable region shown in SEQ ID NO: 127 in combination with the amino acid sequence of a light chain variable region shown in SEQ ID NO: 128;
(cxii) an amino acid sequence comprising the amino acid sequence of a heavy chain variable region shown in SEQ ID NO: 129 in combination with the amino acid sequence of a light chain variable region shown in SEQ ID NO: 130;
(cxiii) an amino acid sequence comprising the amino acid sequence of a heavy chain variable region shown in SEQ ID NO: 131 in combination with the amino acid sequence of a light chain variable region shown in SEQ ID NO: 132;
(cxiv) an amino acid sequence comprising the amino acid sequence of a heavy chain variable region shown in SEQ ID NO: 133 in combination with the amino acid sequence of a light chain variable region shown in SEQ ID NO: 134; and
(cxv) an amino acid sequence comprising the amino acid sequence of a heavy chain variable region shown in SEQ ID NO: 135 in combination with the amino acid sequence of a light chain variable region shown in SEQ ID NO: 136.

85. The cell according to claim 80, which comprises, as the site binding to KIR2DL1, a membrane protein comprising a binding site according to any of (CI) to (CXV) below and optionally comprising mutation of 1 or 2 amino acids in each CDR expressed on the cell surface:

(CI) a binding site comprising a first variable region comprising the amino acid sequence derived from the amino acid sequence of CDR1, CDR2, and CDR3 shown in SEQ ID NO: 107 in combination with a second variable region comprising the amino acid sequence derived from the amino acid sequence of CDR1, CDR2, and CDR3 shown in SEQ ID NO: 108;

(CII) a binding site comprising a first variable region comprising the amino acid sequence derived from the amino acid sequence of CDR1, CDR2, and CDR3 shown in SEQ ID NO: 109 in combination with a second variable region comprising the amino acid sequence derived from the amino acid sequence of CDR1, CDR2, and CDR3 shown in SEQ ID NO: 110;

(CIII) a binding site comprising a first variable region comprising the amino acid sequence derived from the amino acid sequence of CDR1, CDR2, and CDR3 shown in SEQ ID NO: 111 in combination with a second variable region comprising the amino acid sequence derived from the amino acid sequence of CDR1, CDR2, and CDR3 shown in SEQ ID NO: 112;

(CIV) a binding site comprising a first variable region comprising the amino acid sequence derived from the amino acid sequence of CDR1, CDR2, and CDR3 shown in SEQ ID NO: 113 in combination with a second variable region comprising the amino acid sequence derived from the amino acid sequence of CDR1, CDR2, and CDR3 shown in SEQ ID NO: 114 (preferably, a variable region comprising the amino acid sequence shown in SEQ ID NO: 114);

(CV) a binding site comprising a first variable region comprising the amino acid sequence derived from the amino acid sequence of CDR1, CDR2, and CDR3 shown in SEQ ID NO: 115 in combination with a second variable region comprising the amino acid sequence derived from the amino acid sequence of CDR1, CDR2, and CDR3 shown in SEQ ID NO: 116;

(CVI) a binding site comprising a first variable region comprising the amino acid sequence derived from the amino acid sequence of CDR1, CDR2, and CDR3 shown in SEQ ID NO: 117 in combination with a second variable region comprising the amino acid sequence derived from the amino acid sequence of CDR1, CDR2, and CDR3 shown in SEQ ID NO: 118;

(CVII) a binding site comprising a first variable region comprising the amino acid sequence derived from the amino acid sequence of CDR1, CDR2, and CDR3 shown in SEQ ID NO: 119 in combination with a second variable region comprising the amino acid sequence derived from the amino acid sequence of CDR1, CDR2, and CDR3 shown in SEQ ID NO: 120;

(CVIII) a binding site comprising a first variable region comprising the amino acid sequence derived from the amino acid sequence of CDR1, CDR2, and CDR3 shown in SEQ ID NO: 121 in combination with a second variable region comprising the amino acid sequence derived from the amino acid sequence of CDR1, CDR2, and CDR3 shown in SEQ ID NO: 122;

(CVIIII) a binding site comprising a first variable region comprising the amino acid sequence derived from the amino acid sequence of CDR1, CDR2, and CDR3 shown in SEQ ID NO: 123 in combination with a second variable region comprising the amino acid sequence derived from the amino acid sequence of CDR1, CDR2, and CDR3 shown in SEQ ID NO: 124;

(CX) a binding site comprising a first variable region comprising the amino acid sequence derived from the amino acid sequence of CDR1, CDR2, and CDR3 shown in SEQ ID NO: 125 in combination with a second variable region comprising the amino acid sequence derived from the amino acid sequence of CDR1, CDR2, and CDR3 shown in SEQ ID NO: 126;

(CXI) a binding site comprising a first variable region comprising the amino acid sequence derived from the amino acid sequence of CDR1, CDR2, and CDR3 shown in SEQ ID NO: 127 in combination with a second variable region comprising the amino acid sequence derived from the amino acid sequence of CDR1, CDR2, and CDR3 shown in SEQ ID NO: 128;

(CXII) a binding site comprising a first variable region comprising the amino acid sequence derived from the amino acid sequence of CDR1, CDR2, and CDR3 shown in SEQ ID NO: 129 in combination with a second variable region comprising the amino acid sequence derived from the amino acid sequence of CDR1, CDR2, and CDR3 shown in SEQ ID NO: 130;

(CXIII) a binding site comprising a first variable region comprising the amino acid sequence derived from the amino acid sequence of CDR1, CDR2, and CDR3 shown in SEQ ID NO: 131 in combination with a second variable region comprising the amino acid sequence derived from the amino acid sequence of CDR1, CDR2, and CDR3 shown in SEQ ID NO: 132;

(CXIV) a binding site comprising a first variable region comprising the amino acid sequence derived from the amino acid sequence of CDR1, CDR2, and CDR3 shown in SEQ ID NO: 133 in combination with a second variable region comprising the amino acid sequence derived from the amino acid sequence of CDR1, CDR2, and CDR3 shown in SEQ ID NO: 134; and

(CXV) a binding site comprising a first variable region comprising the amino acid sequence derived from the amino acid sequence of CDR1, CDR2, and CDR3 shown in SEQ ID NO: 135 in combination with a second variable region comprising the amino acid sequence derived from the amino acid sequence of CDR1, CDR2, and CDR3 shown in SEQ ID NO: 136.

86. The cell according to claim 85, which optionally comprises mutation of 1 or 2 amino acids on the C and/or N terminal sides of each CDR.

87. A pharmaceutical composition comprising, as an active ingredient, the cell according to any one of claims 62 to 86 and exhibiting a lower risk of immune rejection in a target of administration or transplantation.

88. The pharmaceutical composition according to claim 87, which is an immune rejection inhibitor.

89. A method for lowering or inhibiting immune rejection against a therapeutic cell by a subject comprising a step of expressing the substance according to any one of claims 1 to 59 on the surface of the therapeutic cell.

90. A method for producing a therapeutic cell exhibiting a lowered or inhibited risk of immune rejection by a subject, which comprises a step of expressing the substance according to any one of claims 1 to 59 on the surface of the therapeutic cell.

91. A method for evaluating activity of a test substance for avoiding immune rejection caused by the agonist activity on inhibitory KIR2DL2 or KIR2DL3 comprising:

   (A) a step of bringing NK cells derived from a HLA-C1/C1 homozygous subject into contact with target cells derived from a HLA-C2/C2 homozygous subject *ex vivo* in the presence of a test substance binding to KIR2DL2 or KIR2DL3 (wherein the Bw4 motif-containing HLA phenotype of the donor of the NK cells is identical to that of the donor of the target cells); and
   (B) a subsequent step of measuring the viable target cell count.

92. A method for evaluating activity of a test substance for avoiding immune rejection caused by the agonist activity on inhibitory KIR2DL1 comprising:

   (A) a step of bringing NK cells derived from a HLA-C2/C2 homozygous subject into contact with target cells derived from a HLA-C1/C1 homozygous subject *ex vivo* in the presence of a test substance binding to KIR2DL1 (wherein the Bw4 motif-containing HLA phenotype of the donor of the NK cells is identical to that of the donor of the target cells); and
   (B) a subsequent step of measuring the viable target cell count.

93. A method for evaluating activity of a test substance for avoiding immune rejection caused by the agonist activity on inhibitory KIR3DL1 comprising:

   (A) a step of bringing NK cells derived from a HLA-Bw4$^+$/Bw4$^+$ homozygous subject or a Bw4$^+$/Bw4$^-$ heterozygous subject into contact with target cells derived from a HLA-Bw4$^-$/Bw4$^-$ homozygous subject *ex vivo* in the presence of a test substance binding to KIR3DL1 (wherein the HLA-C phenotype of the donor of the NK cells is identical to that of the donor of the target cells); and
   (B) a subsequent step of measuring the viable target cell count.

94. A method for screening for a substance having activity of avoiding immune rejection comprising a step of selecting a test substance exhibiting a high viable target cell count in the step B as a substance having activity of avoiding immune rejection by the method according to any one of claims 91 to 93.

95. The method according to any one of claims 91 to 93, wherein, in step (A), the test substance is expressed as a membrane protein on the surface of the target cells.

96. A method for producing a substance having activity of binding to human inhibitory KIR and inhibiting NK cells comprising:

   step A: a step of obtaining a population of display bodies displaying an antigen-binding site binding to inhibitory KIR comprising mixing a labeled soluble protein comprising an extracellular domain of inhibitory KIR in a suspension containing a population of polyclonal display bodies including display bodies displaying an antigen-binding site binding to inhibitory KIR on the surface, bringing the suspension into contact with a carrier comprising, immobilized thereon, a substance binding specifically to the label part of said labeled soluble protain, and collecting the display bodies bound to the carrier;

step B: a step of removing display bodies displaying the antigen-binding site binding to the activating KIR from the population of display bodies obtained in Step A, comprising mixing a labeled soluble protein comprising an extracellular domain of activating KIR corresponding to the inhibitory KIR in the suspension containing the population of display bodies obtained in step A, bringing the suspension into contact with a carrier comprising, immobilized thereon, a substance binding to the label part of said labeled soluble proteein, and collecting the display bodies not bound to the carrier;

step C: a step of identifying a sequence of a gene encoding the antigen-binding site by performing genetic analysis of the display bodies obtained in Step B; and

step D: a step of expressing the identified gene comprising the antigen-binding site in a cell.

97. A method for producing a substance having activity of binding to human inhibitory KIR and inhibiting NK cells comprising:

step A: a step of obtaining a population of display bodies displaying an antigen-binding site binding specifically to inhibitory KIR comprising mixing a labeled soluble protein comprising an extracellular domain of inhibitory KIR and an unlabeled soluble protein comprising an extracellular domain of activating KIR corresponding to the inhibitory KIR in a suspension containing a population of polyclonal display bodies including display bodies displaying the antigen-binding site binding to the inhibitory KIR on the surface, bringing the suspension into contact with a carrier comprising, immobilized thereon, a substance binding specifically to the label part of said labeled soluble proteinn and collecting the display bodies bound to the carrier;

step B: a step of identifying a sequence of a gene encoding the antigen-binding site by performing genetic analysis of the display bodies obtained in Step A; and

step C: a step of expressing the identified gene comprising the antigen-binding site in a cell.

98. The method according to claim 96 or 97, wherein a combination of a label and a label-binding substance is a combination of biotin and avidin.

99. The method according to any one of claims 96 to 98, which further comprises a step of selecting a substance having an agonist activity on the inhibitory KIR.

100.
The method according to any one of claims 96 to 99, wherein the inhibitory KIR is KIR2DL2 or KIR2DL3 and activating KIR corresponding thereto is KIR2DS1, KIR2DS2, or KIR2DS4.

101.
The method according to any one of claims 96 to 99, wherein the inhibitory KIR is KIR3DL1 and activating KIR corresponding thereto is KIR3DS1.

102.
The method according to any one of claims 96 to 99, wherein the inhibitory KIR is KIR2DL1 and activating KIR corresponding thereto is KIR2DS1, KIR2DS2, or KIR2DS4.

# Fig. 1-1

KIR 2DL2-NK92 (cell line)

# Fig. 1-2

KIR 2DL2-NK92 (cell line)

Concentration (μg/mL)

| - | 2.0 hIgG4 | 2.0 Liriliumab |

HLA-Cw3-K562-

# Fig. 2

# Fig. 3

# Fig. 4

KIR-binding capability

# Fig. 5

Agonist activity

Cw3 : HLA-Cw3
Li : Lirilumab
Pa : Pan2D
- : K562-parent

Fig. 6

A

PBMC-A-NK
(2DL2/3, 2DS1/2)

B

PBMC-B-NK
(2DL3)

EP 4 610 277 A1

Fig. 7

Fig. 8

# Fig. 9

Fig. 10

```
                   1                                                                                   100
KIR2DL1*003 HEGVHRKPSLLAHPGRLVKSEETVILQCWSDVMFEHFLLHREGMFNDTLRLIGEHHDGVSKANFSISRMTQDLAGTYRCYGSVTHSPYQVSAPSDPLDIV
KIR2DL2*001 HEGVHRKPSLLAHPGRLVKSEETVILQCWSDVRFEHFLLHREGKFKDTLHLIGEHHDGVSKANFSIGPMMQDLAGTYRCYGSVTHSPYQLSAPSDPLDIV
KIR2DL2*003 HEGVHRKPSLLAHPGRLVKSEETVILQCWSDVRFEHFLLHREGKFKDTLHLIGEHHDGVSKANFSIGPMMQDLAGTYRCYGSVTHSPYQLSAPSDPLDIV
KIR2DL3*001 HEGVHRKPSLLAHPGRLVKSEETVILQCWSDVRFQHFLLHREGKFKDTLHLIGEHHDGVSKANFSIGPMMQDLAGTYRCYGSVTHSPYQLSAPSDPLDIV
KIR2DS1*002 HEGVHRKPSLLAHPGRLVKSEETVILQCWSDVMFEHFLLHREGMFNDTLRLIGEHHDGVSKANFSISRMKQDLAGTYRCYGSVTHSPYQLSAPSDPLDIV
KIR2DS2*001 HEGVHRKPSLLAHPGRLVKSEETVILQCWSDVRFEHFLLHREGKYKDTLHLIGEHHDGVSKANFSIGPMMQDLAGTYRCYGSVTHSPYQLSAPSDPLDIV
KIR2DS4*001 QEGVHRKPSFLALPGHLVKSEETVILQCWSDVMFEHFLLHREGKFNNTLHLIGEHHDGVSKANFSIGPMMPVLAGTYRCYGSVPHSPYQLSAPSDPLDMV

                   101                                                                                 200
KIR2DL1*003 IIGLYEKPSLSAQLGPTVLAGENVTLSCSSRSSYDMYHLSREGEAHERRLPAGPKVNGTFQADFPLGPATHGGTYRCFGSFHDSPYEWSKSSDPLLVSVT
KIR2DL2*001 ITGLYEKPSLSAQPGPTVLAGESVTLSCSSRSSYDMYHLSREGEAHECRFSAGPKVNGTFQADFPLGPATHGGTYRCFGSFRDSPYEWSNSSDPLLVSVT
KIR2DL2*003 ITGLYEKPSLSAQPGPTVLAGESVTLSCSSRSSYDMYHLSREGEAHECRFSAGPKVNGTFQADFPLGPATHGGTYRCFGSFRDSPYEWSNSSDPLLVSVT
KIR2DL3*001 ITGLYEKPSLSAQPGPTVLAGESVTLSCSSRSSYDMYHLSREGEAHERRFSAGPKVNGTFQADFPLGPATHGGTYRCFGSFRDSPYEWSNSSDPLLVSVT
KIR2DS1*002 IIGLYEKPSLSAQPGPTVLAGENVTLSCSSRSSYDMYHLSREGEAHERRLPAGTKVNGTFQANFPLGPATHGGTYRCFGSFRDSPYEWSKSSDPLLVSVT
KIR2DS2*001 ITGLYEKPSLSAQPGPTVLAGESVTLSCSSRSSYDMYHLSREGEAHERRFSAGPKVNGTFQADFPLGPATHGGTYRCFGSFRDSPYEWSNSSDPLLVSVT
KIR2DS4*001 IIGLYEKPSLSAQPGPTVQAGENVTLSCSSRSSYDMYHLSREGEAHERRLPAVRSINGTFQADFPLGPATHGGTYRCFGSFRDAPYEWSNSSDPLLVSVT

                   201                                                                                 300
KIR2DL1*003 GNPSNSWPSPTEPSSKTGNPRHLHILIGTSVVIILFIL-LFFLLHRWCSNKKNAAVMDQESAGNRTANSEDSDEQDPQEVTYTQLNHCVFTQRKITRPSQ
KIR2DL2*001 GNPSNSWPSPTEPSSKTGNPRHLHILIGTSVVIILFIL-LFFLLHRWCSNKKNAAVMDQESAGNRTANSEDSDEQDPQEVTYTQLNHCVFTQRKITRPSQ
KIR2DL2*003 GNPSNSWPSPTEPSSKTGNPRHLHILIGTSVVIILFIL-LFFLLHRWCSNKKNAAVMDQESAGNRTANSEDSDEQDPQEVTYTQLNHCVFTQRKITRPSQ
KIR2DL3*001 GNPSNSWPSPTEPSSETGNPRHLHVLIGTSVVIILFILLLFFLLHRWCNKKNAVVMDQEPAGNRTVNREDSDEQDPQEVTYAQLNHCVFTQRKITRPSQ
KIR2DS1*002 GNPSNSWPSPTEPSSETGNPRHLHVLIGTSVVKIPFTILLFFLLHRWCSDKKNAAVMDQEPAGNRTVNSEDSDEQDHQEVSYA----------------
KIR2DS2*001 GNPSNSWPSPTEPSSKTGNPRHLHVLIGTSVVKIPFTILLFFLLHRWCSNKKNAAVMDQEPAGNRTVNSEDSDEQDHQEVSYA----------------
KIR2DS4*001 GNPSNSWPSPTEPSSKTGNPRHLHVLIGTSVVKIPFTILLFFLLHRWCSDKKNAAVMDQEPAGNRTVNSEDSDEQDHQEVSYA----------------

                   301                        328
KIR2DL1*003 RPKTPPTDIIVYTELPNAESRSKVVSCP (SEQ ID NO: 4)
KIR2DL2*001 RPKTPPTDIIVYAELPNAESRSKVVSCP (SEQ ID NO: 6)
KIR2DL2*003 RPKTPPTDIIVYTELPNAESRSKVVSCP (SEQ ID NO: 57)
KIR2DL3*001 RPKTPPTDIIVYTELPNAEP--------  (SEQ ID NO: 8)
KIR2DS1*002 ---------------------------- (SEQ ID NO: 10)
KIR2DS2*001 ---------------------------- (SEQ ID NO: 12)
KIR2DS4*001 ---------------------------- (SEQ ID NO: 14)
```

# Fig. 11

```
                 1                                                                                                  100
KIR2DL2*001  HEGVHRKPSLLAHPGRLVKSEETVILQCWSDVRFEHFLLHREGKFKDTLHLIGEHHDGVSKANFSIGPMMQDLAGTYRCYGSVTHSPYQLSAPSDPLDIV
KIR2DL2*003  HEGVHRKPSLLAHPGRLVKSEETVILQCWSDVRFEHFLLHREGKFKDTLHLIGEHHDGVSKANFSIGPMMQDLAGTYRCYGSVTHSPYQLSAPSDPLDIV
KIR2DL1*003  HEGVHRKPSLLAHPGRLVKSEETVILQCWSDVMFEHFLLHREGMFNDTLRLIGEHHDGVSKANFSISRMTQDLAGTYRCYGSVTHSPYQVSAPSDPLDIV
KIR2DL3*001  HEGVHRKPSLLAHPGPLVKSEETVILQCWSDVRFQHFLLHREGKFKDTLHLIGEHHDGVSKANFSIGPMMQDLAGTYRCYGSVTHSPYQLSAPSDPLDIV
KIR2DS1*002  HEGVHRKPSLLAHPGRLVKSEETVILQCWSDVMFEHFLLHREGMFNDTLRLIGEHHDGVSKANFSISRMKQDLAGTYRCYGSVTHSPYQLSAPSDPLDIV
KIR2DS2*001  HEGVHRKPSLLAHPGPLVKSEETVILQCWSDVRFEHFLLHREGKYKDTLHLIGEHHDGVSKANFSIGPMMQDLAGTYRCYGSVTHSPYQLSAPSDPLDIV
KIR2DS4*001  QEGVHRKPSFLALPGHLVKSEETVILQCWSDVMFEHFLLHREGKFNNTLHLIGEHHDGVSKANFSIGPMMPVLAGTYRCYGSVPHSPYQLSAPSDPLDMV


                 101                                                                                                200
KIR2DL2*001  ITGLYEKPSLSAQPGPTVLAGESVTLSCSSRSSYDMYHLSREGEAHECRFSAGPKVNGTFQADFPLGPATHGGTYRCFGSFRDSPYEWSNSSDPLLVSVI
KIR2DL2*003  ITGLYEKPSLSAQPGPTVLAGESVTLSCSSRSSYDMYHLSREGEAHECRFSAGPKVNGTFQADFPLGPATHGGTYRCFGSFRDSPYEWSNSSDPLLVSVT
KIR2DL1*003  ITGLYEKPSLSAQLGPTVLAGENVTLSCSSRSSYDMYHLSREGEAHERRLPAGPKVNGTFQADFPLGPATHGGTYRCFGSFHDSPYEWSKSSDPLLVSVT
KIR2DL3*001  ITGLYEKPSLSAQPGPTVLAGESVTLSCSSRSSYDMYHLSREGEAHERRFSAGPKVNGTFQADFPLGPATHGGTYRCFGSFRDSPYEWSNSSDPLLVSVT
KIR2DS1*002  ITGLYEKPSLSAQPGPTVLAGENVTLSCSSRSSYDMYHLSREGEAHERRLPAGTKVNGTFQANFPLGPATHGGTYRCFGSFRDSPYEWSKSSDPLLVSVT
KIR2DS2*001  ITGLYEKPSLSAQPGPTVLAGESVTLSCSSRSSYDMYHLSREGEAHERRFSAGPKVNGTFQADFPLGPATHGGTYRCFGSFRDSPYEWSNSSDPLLVSVT
KIR2DS4*001  ITGLYEKPSLSAQPGPTVQAGENVTLSCSSRSSYDMYHLSREGEAHERRLPAVRSINGTFQADFPLGPATHGGTYRCFGSFRDAPYEWSNSSDPLLVSVT


                 201                                                                                                300
KIR2DL2*001  GNPSNSWPSPTEPSSKTGNPRHLHILIGTSVVIILFIL-LFFLLHRWCSNKKNAAVMDQESAGNRTANSEDSDEQDPQEVTYTQLNHCVFTQRKITRPSQ
KIR2DL2*003  GNPSNSWPSPTEPSSKTGNPRHLHILIGTSVVIILFIL-LFFLLHRWCSNKKNAAVMDQESAGNRTANSEDSDEQDPQEVTYTQLNHCVFTQRKITRPSQ
KIR2DL1*003  GNPSNSWPSPTEPSSKTGNPRHLHILIGTSVVIILFIL-LFFLLHRWCSNKKNAAVMDQESAGNRTANSEDSDEQDPQEVTYTQLNHCVFTQRKITRPSQ
KIR2DL3*001  GNPSNSWPSPTEPSSETGNPRHLHVLIGTSVVIILFILLLFFLLHRWCCNKKNAVVMDQEPAGNRTVNREDSDEQDPQEVTYAQLNHCVFTQRKITRPSQ
KIR2DS1*002  GNPSNSWPSPTEPSSETGNPRHLHVLIGTSVVKIPFTILLFFLLHRWCSDKKNAAVMDQEPAGNRTVNSEDSDEQDHQEVSYA----------------
KIR2DS2*001  GNPSNSWPSPTEPSSKTGNPRHLHVLIGTSVVKIPFTILLFFLLHRWCSNKKNAAVMDQEPAGNRTVNSEDSDEQDHQEVSYA----------------
KIR2DS4*001  GNPSNSWPSPTEPSSKTGNPRHLHVLIGTSVVKIPFTILLFFLLHRWCSDKKNAAVMDQEPAGNRTVNSEDSDEQDHQEVSYA----------------


                 301                      328
KIR2DL2*001  RPKTPPTDIIVYAELPNAESRSKVVSCP (SEQ ID NO: 6)
KIR2DL2*003  RPKTPPTDIIVYTELPNAESRSKVVSCP (SEQ ID NO: 57)
KIR2DL1*003  RPKTPPTDIIVYTELPNAESRSKVVSCP (SEQ ID NO: 4)
KIR2DL3*001  RPKTPPTDIIVYTELPNAEP-------- (SEQ ID NO: 8)
KIR2DS1*002  --------------------------- (SEQ ID NO: 10)
KIR2DS2*001  --------------------------- (SEQ ID NO: 12)
KIR2DS4*001  --------------------------- (SEQ ID NO: 14)
```

Fig. 12

```
            1                                                                                                   100
KIR3DL1*015 HVGGQDKPFLSAWPSAVVPRGGHVTLRCHYRHRFNNFMLYKEDRIHVPIFHGRLFQESFNMSPVTTAHAGNYTCRGSHPHSPTGWSAPSNPVVIMVTGNH
KIR3DL2*002 LMGGQDKPFLSARPSTVVPRGGHVALQCHYRRGFNNFMLYKEDRSHVPIFHGRIFQESFIMGPVTPAHAGTYRCRGSRPHSLTGWSAPSNPLVIMVTGNH
KIR3DS1*013 HMGGQDKPFLSAWPSAVVPRGGHVTLRCHYRHRFNNFMLYKEDRIHVPIFHGRIFQEGFNMSPVTTAHAGNYTCRGSHPHSPTGWSAPSNPMVIMVTGNH

            .........................................................................................................

            101                                                                                                 200
KIR3DL1*015 RKPSLLAHPGPLVKSGERVILQCWSDIMFEHFFLHKEGISKDPSRLVGQIHDGVSKANFSIGPMMLALAGTYRCYGSVTHTPYQLSAPSDPLDIVVTGPY
KIR3DL2*002 RKPSLLAHPGPLLKSGETVILQCWSDVMFEHFFLHRDGISEDPSRLVGQIHDGVSKANFSIGPLMPVLAGTYRCYGSVPHSPYQLSAPSDPLDIVITGLY
KIR3DS1*013 RKPSLLAHPGPLVKSGERVILQCWSDIMFEHFFLHKEWISKDPSRLVGQIHDGVSKANFSIGSMMRALAGTYRCYGSVTHTPYQLSAPSDPLDIVVTGLY

            .........................................................................................................

            201                                                                                                 300
KIR3DL1*015 EKPSLSAQPGPKVQAGESVTLSCSSRSSYDMYHLSREGGAHERRLPAVRKVNRTFQADFPLGPATHGGTYRCFGSFRHSPYEWSDPSDPLLVSVTGNPSS
KIR3DL2*002 EKPSLSAQPGPTVQAGENVTLSCSSWSSYDIYHLSREGEAHERRLRAVPKVNRTFQADFPLGPATHGGTYRCFGSFRALPCVWSNSSDPLLVSVTGNPSS
KIR3DS1*013 EKPSLSAQPGPKVQAGESVTLSCSSRSSYDMYHLSREGGAHERRLPAVRKVNRTFQADFPLGPATHGGTYRCFGSFRHSPYEWSDPSDPLLVSVTGNPSS

            .........................................................................................................

            301                                                                                                 400
KIR3DL1*015 SWPSPTEPSSKSGNPRHLHILIGTSVVIILFILLLFFLLHLWCSNKKNAAVMDQEPAGNRTANSEDSDEQDPEEVTYAQLDHCVFTQRKITRPSQRPKTP
KIR3DL2*002 SWPSPTEPSSKSGICRHLHVLIGTSVVIPLFILLLFFLLYRWCSNKKNAAVMDQEPAGDRTVNRQDSDEQDPQEVTYAQLDHCVFIQRKISRPSQRPKTP
KIR3DS1*013 SWPSPTEPSSKSGNLRHLHILIGTSVVKIPFTILLFFLLHRWCSNKKKCCCNGPRACREQK----------------------------------------

            .........................................................................................................

            401                     434
KIR3DL1*015 PTDTILYTELPNAKPRSKVVSCP----------- (SEQ ID NO: 16)
KIR3DL2*002 LTDTSVYTELPNAEPRSKVVSCPRAPQSGLEGVP (SEQ ID NO: 59)
KIR3DS1*013 ---------------------------------- (SEQ ID NO: 60)
```

# Fig. 13

Amino acid sequence of CD8-derived signal peptide
MALPVTALLLPLALLLHAARP
(SEQ ID NO: 23)


Amino acid sequence of CD8-derived hinge(66 aa)
ALSNSIMYFSHFVPVFLPAKPTTTPAPRPPTPAPTIASQPLSLRPEACRPAAGGAVHTR
GLDFACD
(SEQ ID NO: 24)


Amino acid sequence of G4S linker
GGGGSGGGGSGGGGS
(SEQ ID NO: 25)


Amino acid sequence of CD8-derived hinge
TTTPAPRPPTPAPTIASQPLSLRPEACRPAAGGAVHTRGLDFACD
(SEQ ID NO: 26)


Amino acid sequence of CD8-derived cell membrane domain
IYIWAPLAGTCGVLLLSLVIT
(SEQ ID NO: 27)


Amino acid sequence of CD8-derived intracellular domain
LYCNH
(SEQ ID NO: 28)

# Fig. 14

VH amino acid sequence of Lirilumab (1-7F9)
QVQLVQSGAEVKKPGSSVKVSCKAS<u>GGTFSFYAI</u>SWVRQAPGQGLEWMGG<u>FIPIFGAAN</u>
                        ①                      ②

YAQKFQGRVTITADESTSTAYMELSSLRSDDTAVYYC<u>ARIPSGSYYYDYDMDV</u>WGQGTT
                                  ③

VTVSS
(SEQ ID NO: 29)

① CDR1
② CDR2
③ CDR3


VL amino acid sequence of Lirilumab (1-7F9)
EIVLTQSPVTLSLSPGERATLSCRAS<u>QSVSSYLA</u>WYQQKPGQAPRLLIY<u>DAS</u>NRATGIP
                        ①                      ②

ARFSGSGSGTDFTLTISSLEPEDFAVYYC<u>QQRSNWMYT</u>FGQGTKLEIKRT
                              ③

(SEQ ID NO: 30)

① CDR1
② CDR2
③ CDR3

# Fig. 15

VH amino acid sequence of Pan2D
EVQLQQSGTVLARPGASVKMSCKAS<u>GYTFTSYWMH</u>WMKQRPGQGLEWIGT<u>IYPGNSDTN</u>
                               ①                         ②

YNQKFKGKAKLTAVTSTNTAYMELSSLTNEDSAVYYC<u>SRPTTATRSSAMDY</u>WGQGTSVT
                                        ③

VSS
(SEQ ID NO: 31)

① CDR1
② CDR2
③ CDR3


VL amino acid sequence of Pan2D
QIVLTQSPASMSASLGERVTMTCTASS<u>SVSSSY</u>LYWYQQKPGSSPKLWIY<u>STSNLAS</u>GV
                              ①                     ②

PARFSGSGSGTSYSLTISSMEAEDAATYYC<u>HQYHRSPPT</u>FGGGTKLEIKR
                                    ③

(SEQ ID NO: 32)

① CDR1
② CDR2
③ CDR3

# Fig. 16

VH amino acid sequence of a2DL2/3-DS1
EVQLVQSGAEVKKPGSSVKVSCKAS<u>GGTFSSYAI</u>SWVRQAPGQGLEWMGG<u>IIPIFGTAN</u>
                          ①                                    ②
YAQKFQGRVTITADESTSTAYMELSSLRSEDTAVYYC<u>ARGELDTAIRFDY</u>WGRGTLVTV
                                     ③
SS
(SEQ ID NO: 33)

①  CDR1
②  CDR2
③  CDR3

VL amino acid sequence of a2DL2/3-DS1
QSVLTQPPSASGTPGQRVTISCSGS<u>SSNIGSNT</u>VNWYRQLPGTAPKLLIY<u>SNN</u>QRPSGV
                          ①                                ②
PDRFSGSKSGTSASLAISGLQSEDEADYYC<u>AAWDDSLNGVV</u>FGGGTKVTVL
                              ③
(SEQ ID NO: 34)

①  CDR1
②  CDR2
③  CDR3

# Fig. 17

VH amino acid sequence of a2DL2/3-DS2
QVQLVQSGAGVKKPGSSVKVSCKAS<u>GGTFSSYAI</u>SWVRQAPGQGLEWMGG<u>IIPIFGTSN</u>
                                    ①                                ②

YAQNFQGRVTITADESRNTAYMELSSLRSEDTAMYYC<u>ARGELVDPDAFDI</u>WGQGTLVTV
                                          ③

SS
(SEQ ID NO: 35)

① CDR1
② CDR2
③ CDR3

VL amino acid sequence of a2DL2/3-DS2
QSVLTQPPSVSGAPGQRVTISCTGS<u>SSNIGAGYD</u>VHWYQQLPGTAPKLLIY<u>GNS</u>NRPSG
                                    ①                                ②

VPDRFSGSKSGTSASLAITGLQAEDEADYYC<u>QSYDSSLSGYVV</u>FGGGTKVTVL
                                ③

(SEQ ID NO: 36)

① CDR1
② CDR2
③ CDR3

# Fig. 18

VH amino acid sequence of a2DL2/3-DS3
EVQLVQSGAEVKKPGSSVKVSCKAS<u>GGTFSSYA</u>INWVRQAPGQGLEWMGG<u>IIPIFGTAN</u>
                            ①                   ②

YAQKFQGRVTITADESTSTAYMELSSLRSEDTAVYYC<u>ARDHRPRGSLRGYGMDV</u>WGQGT
                                             ③

TVTVSS
(SEQ ID NO: 37)

① CDR1
② CDR2
③ CDR3

VL amino acid sequence of a2DL2/3-DS3
QSVVTQPPSVSGAPGQRVTISCTGS<u>SSNIGAGYD</u>VHWYQQLPGTAPKLLIY<u>ANNNRPSG</u>
                            ①                   ②

VPDRFSGSKSGTSASLAITGLLPEDEADYYC<u>QSYDSSLSAWV</u>FGGGTKVTVL
                                 ③

(SEQ ID NO: 38)

① CDR1
② CDR2
③ CDR3

# Fig. 19

VH amino acid sequence of a2DL2/3-DS4
EVQLVQSGAEVKKPGSSVKVSCKAS<u>GGTFSSYA</u>ISWVRQAPGQGLEWMGG<u>IIPIFGTAN</u>
                                   ①                             ②

YAQKFQDRVTITADKSTSTAYMELSSLRSEDTAVYYC<u>ARGSRNFDY</u>WGQGTLVTVSS
                                            ③

(SEQ ID NO: 39)
① CDR1
② CDR2
③ CDR3


VL amino acid sequence of a2DL2/3-DS4
AIRMTQSPSSLSASVGDRVTIPCRAS<u>QSIGSY</u>LNWYQQKPGKAPNLLIY<u>AAS</u>SLQSGVP
                                  ①                          ②

SRFSGSGSGTDFTLTISSLQPEDFATYYC<u>QQSYSTPLT</u>FGGGTKVDIK
                             ③

(SEQ ID NO: 40)

① CDR1
② CDR2
③ CDR3

# Fig. 20

VH amino acid sequence of a2DL2/3-DS5
EVQLVQSGAEVKKPGSSVKVSCKAS<u>GGTFSSYAI</u>SWVRQAPGQGLEWMGG<u>IIPIFGTAN</u>
                          ①                         ②

YAQKFQGRVTMTRDTSTSTVYMELSSLRSEDTAVYYC<u>ARSKAAGTRGGMDV</u>WGQGTLVT
                                       ③

VSS
(SEQ ID NO: 41)

① CDR1
② CDR2
③ CDR3

VL amino acid sequence of a2DL2/3-DS5
AIRMTQSPSSLSASVGDGVTITCRAS<u>QSISSYLN</u>WYQQKPGKAPKLLIY<u>AAS</u>SLQSGVP
                           ①                           ②

SRFSGSGSGTDFTLTISSLQPEDFASYYC<u>QQSYSTPLT</u>FGGGTKVEIK
                               ③

(SEQ ID NO: 42)

① CDR1
② CDR2
③ CDR3

# Fig. 21

VH amino acid sequence of a2DL2/3-DS6
QVQLVQSGAEVKKPGSSVKVSCKAS<u>GGTFSSYAI</u>SWVRQAPGQGLEWMGG<u>IIPIFGTAN</u>
                            ①                   ②

YAQKFQGRVTITADESTSTAYMELSSLRSEDTAVYYC<u>ARGLDTPNVVVKGAFDI</u>WGQGT
                                         ③

TVTVSS
(SEQ ID NO: 43)

① CDR1
② CDR2
③ CDR3


VL amino acid sequence of a2DL2/3-DS6
EIVLTQSPGTLSLSPGERATLSCRAS<u>QSVSSSY</u>LAWYQQKPGRAPRLLIY<u>GAS</u>SRATGI
                             ①                    ②

PDRFSGSGSGTDFTLTISRLEPEDFAVYYC<u>QQYGSSPWT</u>FGQGTKLEIK
                                 ③

(SEQ ID NO: 44)

① CDR1
② CDR2
③ CDR3

# Fig. 22

VH amino acid sequence of a2DL2/3-DS7
EVQLVQSGAEVKKPGSSVKVSCKAS<u>GGTFSSYAI</u>SWVRQAPGQGLEWMGG<u>IIPIFGTAN</u>
                            ①                 ②

YAQKFQGRVTITADESTSTAYMELSSLRSEDTAVYYC<u>ASDILTGYSVPYYYYMDV</u>WGQG
                                         ③

TLVTVSS
(SEQ ID NO: 45)

① CDR1
② CDR2
③ CDR3

VL amino acid sequence of a2DL2/3-DS7
DIQLTQSPSSLSASVGDRVTITCQAS<u>QDISNYL</u>NWYQQKPGKAPKLLIY<u>DAS</u>NLETGVP
                              ①                      ②

SRFSGGGSGTDFTFTISSLQPEDIATYYC<u>QQYANLPLT</u>FGGGTKLEIK
                                   ③

(SEQ ID NO: 46)

① CDR1
② CDR2
③ CDR3

# Fig. 23

VH amino acid sequence of a2DL2/3-DS8
QVQLVQSGAEVKKPGSSVKVSCKAS<u>GGTFSSYAI</u>SWVRQAPGQGLELMGG<u>IIPIFGTAN</u>
                        ①                       ②

YAQKFQGRVTITADESTSTAYMELSSLRSEDTAVYYC<u>ATSGGYSYFLSPLDY</u>WGQGTLV
                                        ③

TVSS
(SEQ ID NO: 47)

① CDR1
② CDR2
③ CDR3

VL amino acid sequence of a2DL2/3-DS8
EIVLTQSPGTLSLSPGQRATLSCRAS<u>QSVSSNY</u>LAWYQQKPGQAPRLLIY<u>GAS</u>GRATGI
                         ①                     ②

PDRFSGSGSGTDFTLTISRLEPEDFAVYYC<u>QQYGSSPLT</u>FGGGTKVDIK
                                ③

(SEQ ID NO: 48)

① CDR1
② CDR2
③ CDR3

# Fig. 24

VH amino acid sequence of a2DL2/3-DS9
EVQLVQSGAEVKKPGSSVKVSCKAS<u>GGTFSSYAI</u>SWVRQAPGQGLELMGG<u>IIPIFGTAN</u>
                         ①                      ②

YAQKFQGRVTITADESTSTAYMELSSLRSEDTAVYYC<u>ATSGGYSYFLSPLDY</u>WGQGTLV
                                         ③

TVSS
(SEQ ID NO: 49)

① CDR1
② CDR2
③ CDR3


VL amino acid sequence of a2DL2/3-DS9
EIVLTQSPGTLSLSPGQRATLSCRAS<u>QSVSSNY</u>LAWYQQKPGQAPRLLIY<u>GAS</u>GRATGI
                         ①                      ②

PDRFSGSGSGTDFTLTISRLEPEDFAVYYC<u>QQYGSSPLT</u>FGGGTKVDIK
                                 ③

(SEQ ID NO: 50)

① CDR1
② CDR2
③ CDR3

# Fig. 25

VH amino acid sequence of a2DL2/3-DS10
QVQLVQSGAEVKKPGSSVKVSCKAS<u>GGTFSSFA</u>ISWVRQAPGQGLEWMGG<u>IIPIFGTAN</u>
                           ①                       ②

YAQKFQGRVTITADESTSTAYMELSSLRSEDTAVYYC<u>ARVSGTTGGYYYGMDV</u>WGQGTL
                                            ③

VTVSS
(SEQ ID NO: 51)

① CDR1
② CDR2
③ CDR3


VL amino acid sequence of a2DL2/3-DS10
AIRMTQSPSSVSASVGDRVTITCRAS<u>QGIGNW</u>LAWYQQKPGRAPKLLIS<u>TAS</u>SLQSGVP
                             ①                       ②

SRFSGGGSGRGFTLTISSLQLEDFATYYC<u>QQSYITPWT</u>FGQGTKVEIK
                                  ③

(SEQ ID NO: 52)

① CDR1
② CDR2
③ CDR3

# Fig. 26

VL amino acid sequence of a3DL1-DS1
SYELTQPPSVSVAPGQAATITCAGDNIESKSVNWYQQKPGQAPVLVVYDDTVRPSGIPE
                         ①                        ②

RFSGSNSGNPATLTISRSEAGDEADYYCQVWDNRRDHVVFGGGTKVTVLG
                            ③

(SEQ ID NO: 55)

① CDR1
② CDR2
③ CDR3


VH amino acid sequence of a3DL1-DS1
EVQLVETGGGLVKPGGSLRLSCAASGFTFSDYYMSWIRQAPGKGLEWVSYISSSSSYTN
                         ①                        ②

YADSVKGRFTISRDNAKNSLYLQMNSLRAEDTAVYYCAREGIAAADAFDIWGQGTMVTV
                                    ③

SS
(SEQ ID NO: 56)

① CDR1
② CDR2
③ CDR3

# Fig. 27-1

# Fig. 27-2

A

Raji cells + CAR-T cells

- ●—control
- △—a2DL2/3-DS10
- ◆—CAR
- □—CAR+a2DL2/3-DS10

B

Raji cells + CAR-T cells + NK cells

- ●—control
- △—a2DL2/3-DS10
- ◆—CAR
- □—CAR+a2DL2/3-DS10

# Fig. 28

A

B

# Fig. 29

# Fig. 30

# Fig. 31

PBMC-A-NK
(2DL2/3, 2DS1/2)

# Fig. 32-1

Fab L Chain

KIR 2DL2

Fab H Chain

# Fig. 32-2

```
                  1                                                                                                  100
KIR2DL2*001  HEGVHRKPSLLAHPGRLVKSEETVILQCWSDVRFEHFLLHREGKFKDTLHLIGEHHDGVSKANFSIGPMMQDLAGTYRCYGSVTHSPYQLSAPSDPLDIV
KIR2DL3*001  HEGVHRKPSLLAHPGPLVKSEETVILQCWSDVRFQHFLLHREGKFKDTLHLIGEHHDGVSKANFSIGPMMQDLAGTYRCYGSVTHSPYQLSAPSDPLDIV
KIR2DS1*002  HEGVHRKPSLLAHPGRLVKSEETVILQCWSDVMFEHFLLHREGMFNDTLRLIGEHHDGVSKANFSISRMKQDLAGTYRCYGSVTHSPYQLSAPSDPLDIV
KIR2DS2*001  HEGVHRKPSLLAHPGPLVKSEETVILQCWSDVRFEHFLLHREGKYKDTLHLIGEHHDGVSKANFSIGPMMQDLAGTYRCYGSVTHSPYQLSAPSDPLDIV
KIR2DS4*001  QEGVHRKPSFLALPGHLVKSEETVILQCWSDVMFEHFLLHREGKFNNTLHLIGEHHDGVSKANFSIGPMMPVLAGTYRCYGSVPHSPYQLSAPSDPLDMV

             . . . . . . . . . .

                  101                                                                                                200
KIR2DL2*001  ITGLYEKPSLSAQPGPTVLAGESVTLSCSSRSSYDMYHLSREGEAHECRFSAGPKVNGTFQADFPLGPATHGGTYRCFGSFRDSPYEWSNSSDPLLVSVI
KIR2DL3*001  ITGLYEKPSLSAQPGPTVLAGESVTLSCSSRSSYDMYHLSREGEAHERRFSAGPKVNGTFQADFPLGPATHGGTYRCFGSFRDSPYEWSNSSDPLLVSVT
KIR2DS1*002  IIGLYEKPSLSAQPGPTVLAGENVTLSCSSRSSYDMYHLSREGEAHERRLPAGTKVNGTFQANFPLGPATHGGTYRCFGSFRDSPYEWSKSSDPLLVSVT
KIR2DS2*001  ITGLYEKPSLSAQPGPTVLAGESVTLSCSSRSSYDMYHLSREGEAHERRFSAGPKVNGTFQADFPLGPATHGGTYRCFGSFRDSPYEWSNSSDPLLVSVT
KIR2DS4*001  IIGLYEKPSLSAQPGPTVQAGENVTLSCSSRSSYDMYHLSREGEAHERRLPAVRSINGTFQADFPLGPATHGGTYRCFGSFRDAPYEWSNSSDPLLVSVT

             . . . . . . . . . .

                  201                                                                                                300
KIR2DL2*001  GNPSNSWPSPTEPSSKTGNPRHLHILIGTSVVIILFIL-LFFLLHRWCSNKKNAAVMDQESAGNRTANSEDSDEQDPQEVTYTQLNHCVFTQRKITRPSQ
KIR2DL3*001  GNPSNSWPSPTEPSSETGNPRHLHVLIGTSVVIILFILDLFFLLHRWCQNKKNAVVMDQEPAGNRTVNREDSDEQDPQEVTYAQLNHCVFTQRKITRPSQ
KIR2DS1*002  GNPSNSWPSPTEPSSETGNPRHLHVLIGTSVVKIPFTILLFFLLHRWCSDKKNAAVMDQEPAGNRTVNSEDSDEQDHQEVSYA----------------
KIR2DS2*001  GNPSNSWPSPTEPSSKTGNPRHLHVLIGTSVVKIPFTILLFFLLHRWCSNKKNAAVMDQEPAGNRTVNSEDSDEQDHQEVSYA----------------
KIR2DS4*001  GNPSNSWPSPTEPSSKTGNPRHLHVLIGTSVVKIPFTILLFFLLHRWCSDKKNAAVMDQEPAGNRTVNSEDSDEQDHQEVSYA----------------

             . . . . . . . . . .

                  301                        328
KIR2DL2*001  RPKTPPTDIIVYAELPNAESRSKVVSCP (SEQ ID NO: 6)
KIR2DL3*001  RPKTPPTDIIVYTELPNAEP-------- (SEQ ID NO: 8)
KIR2DS1*002  --------------------------- (SEQ ID NO: 10)
KIR2DS2*001  --------------------------- (SEQ ID NO: 12)
KIR2DS4*001  --------------------------- (SEQ ID NO: 14)
```

EP 4 610 277 A1

# Fig. 33

KIR 2DL2-binding capability

## Fig. 34

# Fig. 35

# Fig. 36

A

B

# Fig. 37-1

VH amino acid sequence of a2DL2/3-DS11
QVQLVQSGAEVKKPGSSVKVSCKAS<u>GGTFSSYA</u>ISWVRQAPGQGLEWMGG<u>IIPIFGTAN</u>
                          ①                        ②

YAQKFQGRVTITADESTSTAYMELSSLRSEDTAVYYC<u>ARLVSSGWYDPSDY</u>WGQGTTVT
                                ③

VSS
(SEQ ID NO: 61)

① CDR1
② CDR2
③ CDR3

VL amino acid sequence of a2DL2/3-DS11
QSVLTQPPSASGTPGQRVTISCSGS<u>SSNIGSNT</u>VNWYQQLPGTAPKLLIY<u>SNN</u>QRPSGV
                          ①                      ②

PDRFSGSKSGTSASLAISGLQSEDEADYYC<u>LTWDDSLTAFV</u>FGTGTKLTVL
                              ③

(SEQ ID NO: 62)

① CDR1
② CDR2
③ CDR3

# Fig. 37-2

VH amino acid sequence of a2DL2/3-DS12
QVQLVQSGAEVKKPGSSVKVSCKAS<u>GGTFSSYA</u>ISWVRQAPGQGLEWMGG<u>IIPIFGTAN</u>
                                    ①                         ②

YAQKFQGRVTITADESTSTAYMELSSLRSEDTAVYYC<u>ARVGSGWYGGDFDY</u>WGQGTLVT
                                          ③

VSS
(SEQ ID NO: 63)

① CDR1
② CDR2
③ CDR3

VL amino acid sequence of a2DL2/3-DS12
QSVLTQPPSVSGTPGQRVTISCSGS<u>SSNIGSNT</u>VNWYQQLPGTAPKLLIY<u>TNN</u>QRPSGV
                                    ①                        ②

PDRFSGSKSGTSASLAISGLQSEDEADYYC<u>AAWDDSLNGWV</u>FGGGTKVTVL
                                      ③

(SEQ ID NO: 64)

① CDR1
② CDR2
③ CDR3

Fig. 38

Amino acid sequence of 3DL2 signal peptide
MSLTVVSMACVGFFLLQGAWP
(SEQ ID NO: 65)


Amino acid sequence of 3DS1 signal peptide
MLLMVVSMACVGLFLVQRAGP
(SEQ ID NO: 66)

# Fig. 39

Amino acid sequence of R99A
AVSGTTGGYYYGMDV
(SEQ ID NO: 67)

Amino acid sequence of Y106A
RVSGTTGGAYYGMDV
(SEQ ID NO: 75)

Amino acid sequence of V99A
RASGTTGGYYYGMDV
(SEQ ID NO: 68)

Amino acid sequence of Y107A
RVSGTTGGYAYGMDV
(SEQ ID NO: 76)

Amino acid sequence of S100A
RVAGTTGGYYYGMDV
(SEQ ID NO: 69)

Amino acid sequence of T108A
RVSGTTGGYYAGMDV
(SEQ ID NO: 77)

Amino acid sequence of G101A
RVSATTGGYYYGMDV
(SEQ ID NO: 70)

Amino acid sequence of G109A
RVSGTTGGYYYAMDV
(SEQ ID NO: 78)

Amino acid sequence of T102A
RVSGATGGYYYGMDV
(SEQ ID NO: 71)

Amino acid sequence of M110A
RVSGTTGGYYYGADV
(SEQ ID NO: 79)

Amino acid sequence of T103A
RVSGTAGGYYYGMDV
(SEQ ID NO: 72)

Amino acid sequence of D111A
RVSGTTGGYYYGMAV
(SEQ ID NO: 80)

Amino acid sequence of G104A
RVSGTTAGYYYGMDV
(SEQ ID NO: 73)

Amino acid sequence of V112A
RVSGTTGGYYYGMDA
(SEQ ID NO: 81)

Amino acid sequence of G105A
RVSGTTGAYYYGMDV
(SEQ ID NO: 74)

# Fig. 40-1

VH amino acid sequence of a2DL2/3-DS1-2
EVQLVQSGAEVKKPGSSVKVSCKAS<u>GGTFSSYAI</u>SWVRQAPGQGLEWMGG<u>IIPIFGTAN</u>
                            ①                                  ②
YAQKFQDRVTITADKSTSTAYMELSSLRSEDTAVYYC<u>ARGELDTAIRFDY</u>WGQGTLVTV
                                      ③
SS
(SEQ ID NO: 82)

① CDR1
② CDR2
③ CDR3

VL amino acid sequence of a2DL2/3-DS1-2
QSVLTQPPSASGTPGQRVTISCSGS<u>SSNIGSNT</u>VNWYQQLPGTAPKLLIY<u>SNN</u>QRPSGV
                          ①                                  ②
PDRFSGSKSGTSASLAISGLRSEDEADYYC<u>AAWDDSLNGVV</u>FGGGTKLTVL
                                ③
(SEQ ID NO: 83)

① CDR1
② CDR2
③ CDR3

# Fig. 40-2

VH amino acid sequence of a2DL2/3-DS2-2
EVQLVQSGAEVKKPGSSVKVSCKAS<u>GGTFSSYAI</u>SWVRQAPGQGLEWMGG<u>IIPIFGTSN</u>
                        ①                       ②

YAQKFQDRVTITADKSTSTAYMELSSLRSEDTAVYYC<u>ARGELVDPDAFDI</u>WGQGTLVTV
                                      ③

SS
(SEQ ID NO: 84)

① CDR1
② CDR2
③ CDR3

VL amino acid sequence of a2DL2/3-DS2-2
QSVLTQPPSASGTPGQRVTISCTGS<u>SSNIGAGYD</u>VHWYQQLPGTAPKLLIY<u>GNS</u>NRPSG
                        ①                      ②

VPDRFSGSKSGTSASLAISGLRSEDEADYYC<u>QSYDSSLSGYVV</u>FGGGTKLTVL
                              ③

(SEQ ID NO: 85)

① CDR1
② CDR2
③ CDR3

# Fig. 40-3

VH amino acid sequence of a2DL2/3-DS3-2
EVQLVQSGAEVKKPGSSVKVSCKAS<u>GGTFSSYA</u>INWVRQAPGQGLEWMGG<u>IIPIFGTAN</u>
                                      ①                         ②

YAQKFQDRVTITADKSTSTAYMELSSLRSEDTAVYYC<u>ARDHRPRGSLRGYGMDV</u>WGQGT
                                            ③

LVTVSS
(SEQ ID NO: 86)

① CDR1
② CDR2
③ CDR3

VL amino acid sequence of a2DL2/3-DS3-2
QSVLTQPPSASGTPGQRVTISCTGS<u>SSNIGAGYD</u>VHWYQQLPGTAPKLLIY<u>ANN</u>QRPSG
                                      ①                         ②

VPDRFSGSKSGTSASLAISGLRSEDEADYYC<u>QSYDSSLSAWV</u>FGGGTKLTVL
                                     ③

(SEQ ID NO: 87)

① CDR1
② CDR2
③ CDR3

# Fig. 41

Amino acid sequence of HLA-G signal peptide-derived peptide
VMAPRTLFL
(SEQ ID NO: 88)


Amino acid sequence of G4S 4 linker
GGGGSGGGGSGGGGSGGGGS
(SEQ ID NO: 89)


Amino acid sequence of HLA-E
GSHSLKYFHTSVSRPGRGEPRFISVGYVDDTQFVRFDNDAASPRMVPRAPWMEQEGSEY
WDRETRSARDTAQIFRVNLRTLRGYYNQSEAGSHTLQWMHGCELGPDRRFLRGYEQFAY
DGKDYLTLNEDLRSWTAVDTAAQISEQKSNDASEAEHQRAYLEDTCVEWLHKYLEKGKE
TLLHLEPPKTHVTHHPISDHEATLRCWALGFYPAEITLTWQQDGEGHTQDTELVETRPA
GDGTFQKWAAVVVPSGEEQRYTCHVQHEGLPEPVTLRWKPASQPTIPIVGIIAGLVLLG
SVVSGAVVAAVIWRKKSSGGKGGSYSKAEWSDSAQGSESHSL
(SEQ ID NO: 90)


Amino acid sequence of IL-2 signal peptide
MYRMQLLSCIALSLALVTNS
(SEQ ID NO: 91)


Amino acid sequence of His tag
HHHHHH
(SEQ ID NO: 92)


VH amino acid sequence of signal peptide
MKHLWFFLLLVAAPRWVLS
(SEQ ID NO: 93)

# Fig. 42

Amino acid sequence of Fab fragment H chain constant region
ASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKKVEPKSCDKTH
(SEQ ID NO: 94)


VL amino acid sequence of signal peptide
MVLQTQVFISLLLWISGAYG
(SEQ ID NO: 95)


Amino acid sequence of Fab fragment L chain constant
region
VAAPSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC
(SEQ ID NO: 96)

# Fig. 43

Amino acid sequence of GGGS linker
GGGS
(SEQ ID NO: 97)


Amino acid sequence of IgG Fc domain
DKTHTCPPCPAPELLGGPSVFLFPPKPKDTLMISRTPEVTCV
VVDVSHEDPEVKFNWYVDGVEVHNAKTKPREEQYNSTYRV
VSVLTVLHQDWLNGKEYKCKVSNKALPAPIEKTISKAKGQ
PREPQVYTLPPSREEMTKNQVSLTCLVKGFYPSDIAVEWE
SNGQPENNYKTTPPVLDSDGSFFLYSKLTVDKSRWQQGNVFSCSVMHEALHNHYTQKSL
SLSPGK
(SEQ ID NO: 98)


Amino acid sequence of Avi tag
GLNDIFEAQKIEWHE
(SEQ ID NO: 99)

# Fig. 44

VL amino acid sequence of CA19 CAR
DIQMTQTTSSLSASLGDRVTISCRASQDISKYLNWYQQKPDGTVKLLIYHTSRLHSGVP
SRFSGSGSGTDYSLTISNLEQEDIATYFCQQGNTLPYTFGGGTKLEIT
(SEQ ID NO: 100)


VH amino acid sequence of CA19 CAR
EVKLQESGPGLVAPSQSLSVTCTVSGVSLPDYGVSWIRQPPRKGLEWLGVIWGSETTYY
NSALKSRLTIIKDNSKSQVFLKMNSLQTDDTAIYYCAKHYYYGGSYAMDYWGQGTSVTV
SS
(SEQ ID NO: 101)

# Fig. 45

Amino acid sequence of CD8-derived cell membrane domain and
intracellular domain
IYIWAPLAGTCGVLLLSLVITLYC
(SEQ ID NO: 102)


Amino acid sequence of intracellular costimulatory factor
KRGRKKLLYIFKQPFMRPVQTTQEEDGCSCRFPEEEEGGCELRVKFSRSADAPAYQQGQ
NQLYNELNLGRREEYDVLDKRRGRDPEMGGKPRRKNPQEGLYNELQKDKMAEAYSEIGM
KGERRRGKGHDGLYQGLSTATKDTYDALHMQALPPR
(SEQ ID NO: 103)


Amino acid sequence of blue fluorescent protein
MSELIKENMHMKLYMEGTVDNHHFKCTSEGEGKPYEGTQTMRIKVVEGGPLPFAFDILA
TSFLYGSKTFINHTQGIPDFFKQSFPEGFTWERVTTYEDGGVLTATQDTSLQDGCLIYN
VKIRGVNFTSNGPVMQKKTLGWEAFTETLYPADGGLEGRNDMALKLVGGSHLIANIKTT
YRSKKPAKNLKMPGVYYVDYRLERIKEANNETYVEQHEVAVARYCDLPSKLGHKLN
(SEQ ID NO: 104)

Fig. 46

Amino acid sequence of IL-21 fragment
HKSSSQGQDRHMIRMRQLIDIVDQLKNYVNDLVPEFLPAPEDVETNCEWSAFSCFQKAQ
LKSANTGNNERIINVSIKKLKRKPPSTNAGRRQKHRLTCPSCDSYEKKPPKEFLERFKS
LLQKMIHQHLSSRTHGSEDS
(SEQ ID NO: 105)


Amino acid sequence of CD8 hinge(62aa)
SIMYFSHFVPVFLPAKPTTTPAPRPPTPAPTIASQPLSLRPEACRPAAGGAVHTRGLDF
ACD
(SEQ ID NO: 106)

# Fig. 47-1

VH amino acid sequence of a2DL1-DS1
QVQLQQSGPELVRASQTLSLTCAIS<u>GDSVSSNSAT</u>WNWIRQSPSRGLEWLGR<u>TYYRSKW</u>
                          ①                                 ②

<u>YN</u>DYAVSVKSRITINPDTSKNQFSLQLNSVTPEDTAVYYC<u>ARDSPSYGRGVFDI</u>WGQGT
                                                    ③

LVTVSS
(SEQ ID NO: 107)

① CDR1
② CDR2
③ CDR3

VL amino acid sequence of a2DL1-DS1
DIVMTQSPDSLAVSLGERATINCKSS<u>QSVLYSSNNKNYLA</u>WYQQKPGQPPKLLIY<u>WAST</u>
                               ①                                ②

RESGVPDRFSGSGSGTDFTLTISSLQAEDVAVYYC<u>QQYYSPPYT</u>FGQGTKLEIK
                                        ③

(SEQ ID NO: 108)

① CDR1
② CDR2
③ CDR3

# Fig. 47-2

```
VH amino acid sequence of a2DL1-DS2
QVQLVQSGAEVKKPGSSVKVSCKASGGTFSSYAISWVRQAPGQGLEWMGGIIPIFGTAN
                          ①                              ②
YAQKFQGRVTITADESTSTAYMELSSLRSEDTAVYYCAREAGFWDGYNFRWFDPWGQGT
                                     ③
LVTVSS
(SEQ ID NO: 109)
```

①  CDR1
②  CDR2
③  CDR3


```
VL amino acid sequence ofa2DL1-DS2
EIVMTQSPATLSVSPGERATLSCRASQSVSSNLAWYQQKPGQAPRLLIYGASTRATGIP
                          ①                          ②
ARFSGSGSGTEFTLTISSLQSEDLAIYYCQQYNNWPRTFGQGTKLEIK
                             ③
(SEQ ID NO: 110)
```

①  CDR1
②  CDR2
③  CDR3

# Fig. 47-3

VH amino acid sequence of a2DL1-DS3
QVQLQQSGPGLVKPSQTLSLTCAIS<u>GDSVSSNSAA</u>WNWIRQSPSRGLEWLGR<u>TYYRSKW</u>
                              ①                        ②

<u>YN</u>DYAVSVKSRITINPDTSKNQFSLQLNSVTPEDTAVYYC<u>ARDQTNWGLLDY</u>WGQGTLV
                                       ③

TVSS
(SEQ ID NO: 111)

①  CDR1
②  CDR2
③  CDR3


VL amino acid sequence of a2DL1-DS3
DIVMTQSPDSLAVSLGERATINCKSS<u>QSVLYSSNNKNYLA</u>WYQQKPGQPPKLLIY<u>WAST</u>
                              ①                        ②

RESGVPDRFSGSGSGTDFTLTISSLQAEDVAVYYC<u>QQYYSSPWT</u>FGQGTKVEIK
                                     ③

(SEQ ID NO: 112)

①  CDR1
②  CDR2
③  CDR3

# Fig. 47-4

VH amino acid sequence of a2DL1-DS4
QVQLVQSGAEVKKPGSSVKVSCKAS<u>GGTFSSYA</u>ISWVRQAPGQGLEWMGG<u>IIPIFGTAN</u>
                         ①                           ②

YAQKFQGRVTITADESTSTAYMELSSLRSEDTAVYYC<u>ARSRSYFDY</u>WGQGTLVTVSS
                                     ③

(SEQ ID NO: 113)


① CDR1
② CDR2
③ CDR3


VL amino acid sequence of a2DL1-DS4
EIVLTQSPGTLSLSPGEGATLSCRAS<u>QSVTSSHL</u>AWYQQKPGQAPRLLIY<u>GAS</u>RRATGI
                          ①                         ②

PDRFSGSGSGTDFTLTISRLEPEDFAVYYC<u>QQYGSSPLT</u>FGGGTKVEIK
                              ③

(SEQ ID NO: 114)


① CDR1
② CDR2
③ CDR3

# Fig. 47-5

VH amino acid sequence of a2DL1-DS5
QVQLVQSGAEVKKPGASVKVSCKAS<u>GYTFTSYGI</u>SWVRQAPGQGLEWMGW<u>ISAYNGNTN</u>
                              ①                          ②

YAQKLQGRVTMTTDTSTSTAYMELRSLRSDDTAVYYC<u>ALSQYYDSSGYKPDAFDI</u>WGQG
                                           ③

TLVTVSS
(SEQ ID NO: 115)

① CDR1
② CDR2
③ CDR3

VL amino acid sequence of a2DL1-DS5
NIQMTQSPSSLSASVGDRVTITCRAS<u>QSISSYL</u>NWYQVKPGKAPKLLIY<u>DASNLQS</u>GVP
                              ①                          ②

SRFSGSGSGTDFTLTISSLQPEDFATYYC<u>QQSYSTPYT</u>FGQGTKLEIK
                                 ③

(SEQ ID NO: 116)

① CDR1
② CDR2
③ CDR3

# Fig. 47-6

VH amino acid sequence of a2DL1-DS6
QVQLVQSGAEVKKPGSSVKVSCKAS<u>GGTFSSYAI</u>SWVRQAPGQGLEWMGG<u>IIPIFGTAN</u>
                                        ①                ②

YAQKFQGRVTITADESTSTAYMELSSLRSEDTAVYYC<u>ARGRSFWFDP</u>WGQGTLVTVSS
                                                ③

TLVTVSS
(SEQ ID NO: 117)

① CDR1
② CDR2
③ CDR3


VL amino acid sequence of a2DL1-DS6
EIVMTQSPATLSVSPGERATLSCRAS<u>QSVSSN</u>LAWYQQKPGQAPRLLIY<u>GAS</u>TRATGIP
                                        ①                ②

ARFSGSGSGTEFTLTISSLQSEDFAVYYC<u>QQYNNWPPIT</u>FGQGTRLEIK
                                    ③

(SEQ ID NO: 118)

① CDR1
② CDR2
③ CDR3

# Fig. 47-7

VH amino acid sequence of a2DL1-DS7
EVQLLESGGGLVQPGGSLRLSCAAS<u>GFTFSSYA</u>MHWVRQAPGKGLEWVST<u>ISGSGGSTY</u>
                               ①                        ②
YADSVKGRFAISRDNSKSTLYLRLTGLRAEDTAVYYC<u>AKGRSRAGAGEDYFDF</u>WGQGTL
                                        ③
VTVSS
(SEQ ID NO: 119)

① CDR1
② CDR2
③ CDR3


VL amino acid sequence of a2DL1-DS7
QSALTQPASVSGSPGQSITISCTGT<u>SSDVGGYNY</u>VSWYQQHPGKAPKLMIY<u>DVSNRPPG</u>
                               ①                        ②
VSNRFSGSKSDNTASLTISGLQDEDEADYYC<u>SSYTSSNTII</u>FGGGTKLTVL
                                 ③

(SEQ ID NO: 120)

① CDR1
② CDR2
③ CDR3

# Fig. 47-8

VH amino acid sequence of a2DL1-DS8

QVQLVQSGAEVKKPGSSVKVSCKAS<u>GGTFSSYA</u>ISWVRQAPGQGLEWMGG<u>IIPIFGTAN</u>
                                ①                ②

YAQKFQGRVTITADESTSTAYMELSSLRSEDTAVYYC<u>ARVYGSGSYDY</u>WGQGTLVTVSS
                                          ③

(SEQ ID NO: 121)

① CDR1
② CDR2
③ CDR3

VL amino acid sequence of a2DL1-DS8

QSVVTQPPSVSAAPGQRVTISCSGS<u>ASNIGSHT</u>VNWYQQLPGTPPKLLIY<u>RNN</u>QRPSGV
                                ①                      ②

PDRFSGSKSGTSASLAISGLQSEDEADYYC<u>AAWDDSLNGVV</u>FGGGTKLTVL
                                ③

(SEQ ID NO: 122)

① CDR1
② CDR2
③ CDR3

# Fig. 47-9

VH amino acid sequence of a2DL1-DS9

QVQLVQSGAEVKKPGSSVKVSCKAS<u>GGTFSSYA</u>ISWVRQAPGQGLEWMGG<u>IIPIFGTAN</u>
                                    ①                           ②

YAQKFQGRVTITADESTSTAYMELSSLRSEDTAVYYC<u>ARGVRPPFPT</u>WGQGTLVTVSS
                                          ③

(SEQ ID NO: 123)

① CDR1
② CDR2
③ CDR3

VL amino acid sequence of a2DL1-DS9

QSVLTQPPSASGTPGQRVTISCSGS<u>SSNIGSNT</u>VNWYQQFPGTAPKLLIY<u>SNN</u>QRPSGV
                                   ①                          ②

PDRFSGSKSGTSASLAISGLQSEDEADYYC<u>AAWDDSLNGWV</u>FGGGTKLTVL
                                  ③

(SEQ ID NO: 124)

① CDR1
② CDR2
③ CDR3

# Fig. 47-10

VH amino acid sequence of a2DL1-DS10
QVQLLESGGGLVQPGGSLRLSCAAS<u>GFTFSTYAT</u>SWVRQAPGKGLEWVST<u>ISGSGGSTY</u>
                               ①                          ②

YADSVKGRFAISRDNSKSTLYLRLTGLRAEDTAVYYC<u>AKGRSRAGAGEDYFDF</u>WGQGTL
                                         ③

VTVSS
(SEQ ID NO: 125)

① CDR1
② CDR2
③ CDR3


VL amino acid sequence of a2DL1-DS10
QSVLTQPPSVSAAPGQKVTISCSGG<u>SSNIGNNY</u>VSWYQQLPGTAPKLLIY<u>DSN</u>KRPSWI
                               ①                          ②

PDRFSGSKSGTSATLGITGLQTGDEADYYC<u>GTWDSSLSAGV</u>FGGGTKLTVL
                                   ③

(SEQ ID NO: 126)

① CDR1
② CDR2
③ CDR3

# Fig. 47-11

VH amino acid sequence of a2DL1-DS11
QMQLVQSGAEVKKPGASVKVSCKAS<u>GYTFTDYY</u>MHWVRQAPGQGLEWMGW<u>INPNSGNTG</u>
                                  ①                            ②
YAQKFQGRVTITADKSTSTAYMELSSLRSEDTAVYYC<u>ARGDSSGGMDV</u>WGRGTLVTVSS
                                            ③

(SEQ ID NO: 127)

① CDR1
② CDR2
③ CDR3

VL amino acid sequence of a2DL1-DS11
QSVVTQPPSVSGAPGQRVTISCTGS<u>SSNIGAGYV</u>VHWYQQLPGTAPKLLIY<u>GNN</u>NRPSG
                                  ①                        ②
VPDRFSGSKSGTSASLAITGLQAEDEADYYC<u>QSYDSSLSGSV</u>FGGGTKLTVL
                                ③

(SEQ ID NO: 128)

① CDR1
② CDR2
③ CDR3

# Fig. 47-12

VH amino acid sequence of a2DL1-DS12
QVQLVQSGAEVKKPGSSVKVSCKAS<u>GGAFSSYAI</u>SWVRQAPGQGLEWMGG<u>IIPIFGTAN</u>
                          ①                         ②

YAQKFQGRVTITADESTSTAYMELSSLRSEDTAVYYC<u>ARDPSSGWGYYGMDV</u>WGQGTMV
                                      ③

TVSS
(SEQ ID NO: 129)

① CDR1
② CDR2
③ CDR3


VL amino acid sequence of a2DL1-DS12
QSVVTQPPSVSGAPGQRVTISCTGD<u>NSNLGAGYN</u>VHWYQHLPGTAPKLLIY<u>GND</u>NRPSG
                          ①                         ②

VPDRFSGSKSGTSASLAITGLQAEDEGDYYC<u>QSYDSSLVV</u>FGGGTKVTVL
                                ③

(SEQ ID NO: 130)

① CDR1
② CDR2
③ CDR3

# Fig. 47-13

VH amino acid sequence of a2DL1-DS13

EVQLVQSGAEVKKPGSSVKVSCKAS<u>GGTFSSYA</u>ISWVRQAPGQGLEWMGG<u>IIPIFGTAN</u>
                          ①                            ②

YAQKFQGRVTITADESTSTAYMELSSLRSEDTAVYYC<u>ARGFNPDY</u>WGQGTLVTVSS
                                        ③

(SEQ ID NO: 131)

① CDR1
② CDR2
③ CDR3


VL amino acid sequence of a2DL1-DS13

QSVLTQPPSASGTPGQRVTISCSGS<u>SSNIGSNT</u>VNWYQQLPQTAPKLLIH<u>HNN</u>QRHSGV
                          ①                            ②

SDRFSASKSGTSASLAISGLQSEDEADYYC<u>AAWDDTLTGLI</u>FGGGTKLTVL
                                ③

(SEQ ID NO: 132)

① CDR1
② CDR2
③ CDR3

# Fig. 47-14

VH amino acid sequence of a2DL1-DS14

QVQLQQSGPGLVKPSQTLSLTCAISGDSVSSNNAAWNWIRQSPSRGLEWLGRTYYRSKW
                         ①                                 ②

YNDYAVSVKSRITINPDTSKNQFSLQLNSVTPEDTAVYYCARVPGDYYYYYGMDVWGQG
                                        ③

TLVTVSS
(SEQ ID NO: 133)

① CDR1
② CDR2
③ CDR3


VL amino acid sequence of a2DL1-DS14

QSVLTQPPSVSGAPGQRVTISCTGSSSNIGAGYDVHWYQHLPGTAPKLLIYGNSNRPSG
                         ①                                 ②

VPARFSGSKSGTSASLAITGLQAEDEADYYCQSYDIGLSGVVFGGGTKVTVL
                               ③

(SEQ ID NO: 134)

① CDR1
② CDR2
③ CDR3

# Fig. 47-15

VH amino acid sequence of a2DL1-DS15
EVQLVQSGAEVKKPGSSVKVSCKAS<u>GGTFSSYAI</u>SWVRQAPGQGLEWMGG<u>IIPIFGTAN</u>
                             ①                                ②

YAQKFQGRVTITADESTSTAYMELSSLRSEDTAVYYC<u>AREELGYSSSSDYYYGMDV</u>WGQ
                                       ③

GTLVTVSS
(SEQ ID NO: 135)

① CDR1
② CDR2
③ CDR3


VL amino acid sequence of a2DL1-DS15
QSVVTQPPSVSGAPGQRVTISCTGS<u>SSNIGAGYD</u>VHWYQQLPGTAPKLLIY<u>GNS</u>NRPSG
                             ①                                  ②

VPDRFSGSKSGTSASLAVTGLQAEDEADYYC<u>QSYDFRLGGFVI</u>FGGGTKVTVL
                                 ③

(SEQ ID NO: 136)

① CDR1
② CDR2
③ CDR3

# Fig. 48-1

VH amino acid sequence of a3DL1-DS2
QVQLQQSGPGLVKPSQTLSLTCAISGDSVSSNSAAWNWIRQSPSRGLEWLGRTYYRSKW
                     ①                  ②

YNDYAVSVKSRITINPDTSKNQFSLQLNSMTPEDTAVYYCAREEGVSDAFDVWGQGTLV
                                       ③

TVSS
(SEQ ID NO: 137)

① CDR1
② CDR2
③ CDR3


VL amino acid sequence of a3DL1-DS2
NIQMTQSPSSLSASVGDRVTITCRASQSISSYLNWYQQKPGKAPKLLIYAASSLQSGVP
                      ①                ②

SRFSGSGSGTDFTLTISSLQPEDFATYYCQQSYSTPPTFGQGTKVEIK
                             ③

(SEQ ID NO: 138)

① CDR1
② CDR2
③ CDR3

156

# Fig. 48-2

VH amino acid sequence of a3DL1-DS3

QVQLQQSGPGLVKPSQTLSLTCAISGDSVSSDSAAWNWIRQSPSRGLEWLGRTYYRSKW

                       ①                            ②

YNDYAVSVKSRITINPDTSKNQFSLQLNSVTPEDTAVYYCAREEGVSDAFDVWGQGTLV

                                        ③

TVSS

(SEQ ID NO: 139)

① CDR1
② CDR2
③ CDR3

VL amino acid sequence of a3DL1-DS3

NIQMTQSPSSLSASVGDRVTITCRASQSISSYLNWYQQKPGKAPKLLIYAASSLQSGVP

                           ①                        ②

SRFSGSGSGTDFTLTISSLQPEDFATYYCQQSYSTPSLTFGGGTKVEIK

                                   ③

(SEQ ID NO: 140)

① CDR1
② CDR2
③ CDR3

# Fig. 48-3

VH amino acid sequence of a3DL1-DS4
EVQLVESGGGVVQPGRSLRLSCAAS<u>GFTFDDYA</u>MHWVRQVPGKGLEWVAV<u>ISYDGSNKY</u>
                              ①                              ②

YADSVKGRFTISRDNSKNTLYLQMNSLRAEDTAVYYC<u>AREGTYGDSIHYYYYGMDV</u>WGQ
                                         ③

GTMVTVSS
(SEQ ID NO: 141)

① CDR1
② CDR2
③ CDR3


VL amino acid sequence of a3DL1-DS4
EIVMTQSPATLSLSPGERATLSCRAS<u>QSVSSSYL</u>SWYQQKPGQAPRLLMY<u>GAS</u>TRATGI
                               ①                              ②

PARFSGSGSGTDFTLTISSLQPEDFAVYYC<u>QQDYNLPLT</u>FGGGTKLEIK
                                 ③

(SEQ ID NO: 142)

① CDR1
② CDR2
③ CDR3

# Fig. 48-4

VH amino acid sequence of a3DL1-DS5
QVQLQQSGPGLVKPSQTLSLTCAISGDSVSSNSAAWNWIRQSPSRGLEWLGRTYYRSKW
                        ①                                   ②
YNDYAVSVKSRITINPDTSKNQFSLQLNSVTPEDTAVYYCAREMGWYDWFDPWGQGTLV
                                        ③

TVSS
(SEQ ID NO: 143)

① CDR1
② CDR2
③ CDR3


VL amino acid sequence of a3DL1-DS5
DIQMTQSPSSLSASVGDRVTITCRASQSISTYLNWYQQKPGKAPKLLIYAASSLQSGVP
                         ①                              ②
SRFSGSGSGTDFTLTISSLQPEDFATYYCQQSYSTPITFGQGTRLEIK
                             ③

(SEQ ID NO: 144)

① CDR1
② CDR2
③ CDR3

# Fig. 48-5

VH amino acid sequence of a3DL1-DS6
QMQLVQSGAEVKKPGESLKISCKGS<u>GYSFTSYW</u>IGWVRQMPGKGPEWMGI<u>IYPGDSYTN</u>
　　　　　　　　　　　　　　　　　　　①　　　　　　　　　　　　　　　　　　　　　　　　②

YSPSFQGHVTISADKSISTAYLQWSSLKASDTAMYYC<u>ARLAPTVEDAFDI</u>WGQGTLVTV
　　　　　　　　　　　　　　　　　　　　　　　　　　　　　　③

SS
(SEQ ID NO: 145)

① CDR1
② CDR2
③ CDR3


VL amino acid sequence of a3DL1-DS6
QSVLTQPPSVSEAPGQRVTISCTGS<u>SSNIGAGYD</u>VHWYQQLPGTAPKLLIY<u>GNS</u>NRPSG
　　　　　　　　　　　　　　　　　①　　　　　　　　　　　　　　　　　　　　　②

VPDRFSGSKSGTSASLAITGLQAEDEADYYC<u>QSYDSSLSEEV</u>FGTGTKVTVL
　　　　　　　　　　　　　　　　　　　　③

(SEQ ID NO: 146)

① CDR1
② CDR2
③ CDR3

## INTERNATIONAL SEARCH REPORT

| International application No. |
|---|
| **PCT/JP2023/038440** |

**A. CLASSIFICATION OF SUBJECT MATTER**

*C07K 16/28*(2006.01)i; *A61K 35/17*(2015.01)i; *A61K 35/22*(2015.01)i; *A61K 35/28*(2015.01)i; *A61K 35/30*(2015.01)i; *A61K 35/32*(2015.01)i; *A61K 35/34*(2015.01)i; *A61K 35/39*(2015.01)i; *A61K 35/407*(2015.01)i; *A61K 35/545*(2015.01)i; *A61K 39/395*(2006.01)i; *A61P 37/06*(2006.01)i; *C12N 5/10*(2006.01)i; *C12N 5/071*(2010.01)i; *C12N 5/0783*(2010.01)i; *C12N 15/13*(2006.01)i; *C12Q 1/02*(2006.01)i

FI: C07K16/28; C12N5/10; C12N5/0783; C12N5/071; C12Q1/02; A61P37/06; A61K39/395 N; A61K35/17; A61K35/545; A61K35/28; A61K35/34; A61K35/32; A61K35/39; A61K35/407; A61K35/22; A61K35/30; C12N15/13 ZNA

According to International Patent Classification (IPC) or to both national classification and IPC

**B. FIELDS SEARCHED**

Minimum documentation searched (classification system followed by classification symbols)

C07K16/28; A61K35/17; A61K35/22; A61K35/28; A61K35/30; A61K35/32; A61K35/34; A61K35/39; A61K35/407; A61K35/545; A61K39/395; A61P37/06; C12N5/10; C12N5/071; C12N5/0783; C12N15/13; C12Q1/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | VITALE, Massimo et al. Isolation of a novel KIR2DL3-specific mAb: comparative analysis of the surface distribution and function of KIR2DL2, KIR2DL3 and KIR2DS2. International Immunology. 2004, vol. 16, no. 10, pp. 1459-1466, doi:10.1093/intimm/dxh147<br>abstract, p. 1461, section "ECM41 mAb specifically recognizes KIR2DL3", p. 1463, "Analysis of NK clones co-expressing the inhibitory and the activating forms of GL183 mAb reactive molecules", p. 1463 "Discussion", fig. 2, table 1 | 1-90 |
| Y | | 96-102 |
| X | US 2017/0334993 A1 (UNIVERSITY CHILDREN'S HOSPITAL TUBINGEN) 23 November 2017 (2017-11-23)<br>claims, paragraphs [0053]-[0057], [0062]-[0067] | 1-4, 7-9, 16, 24-26, 43-44, 49, 57-73, 76, 80-81 |

[✓] Further documents are listed in the continuation of Box C.　　[✓] See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **04 January 2024** | **16 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT

### C. DOCUMENTS CONSIDERED TO BE RELEVANT

| Category* | Citation of document, with indication, where appropriate, of the relevant passages | Relevant to claim No. |
|---|---|---|
| Y | JP 2008-526221 A (NOVO NORDISK A/S) 24 July 2008 (2008-07-24)<br>claims, examples 3, 5 | 96-102 |
| A | EP 3992204 A1 (CRAGE MEDICAL CO., LIMITED) 04 May 2022 (2022-05-04)<br>whole document | 91-95 |
| A | JP 2008-506368 A (NOVO NORDISK A/S) 06 March 2008 (2008-03-06)<br>entire text | 1-102 |
| A | JP 2008-500947 A (INNATE PHARMA) 17 January 2008 (2008-01-17)<br>entire text | 1-102 |

Form PCT/ISA/210 (second sheet) (July 2022)

## INTERNATIONAL SEARCH REPORT

| International application No. |
|---|
| **PCT/JP2023/038440** |

**Box No. I**      **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

     a.   ☑ forming part of the international application as filed.

     b.   ☐ furnished subsequent to the international filing date for the purposes of international search (Rule 13*ter*.1(a)),

         ☐ accompanied by a statement to the effect that the sequence listing does not go beyond the disclosure in the international application as filed.

2.   ☐ With regard to any nucleotide and/or amino acid sequence disclosed in the international application, this report has been established to the extent that a meaningful search could be carried out without a WIPO Standard ST.26 compliant sequence listing.

3.   Additional comments:

EP 4 610 277 A1

## INTERNATIONAL SEARCH REPORT
### Information on patent family members

International application No.

**PCT/JP2023/038440**

| Patent document cited in search report | | | Publication date (day/month/year) | Patent family member(s) | | | Publication date (day/month/year) |
|---|---|---|---|---|---|---|---|
| US | 2017/0334993 | A1 | 23 November 2017 | WO | 2016/069589 | A1 | |
| | | | | claims, p. 15, line 21 to p. 16, line 33, p. 18, line 19 to p. 20, line 15 | | | |
| | | | | EP | 3212227 | A1 | |
| JP | 2008-526221 | A | 24 July 2008 | US | 2008/0305117 | A1 | |
| | | | | claims, examples 3, 5 | | | |
| | | | | WO | 2006/072626 | A1 | |
| | | | | EP | 1836225 | A1 | |
| EP | 3992204 | A1 | 04 May 2022 | US | 2022/0356447 | A1 | |
| | | | | whole document | | | |
| | | | | WO | 2020/259707 | A1 | |
| | | | | KR | 10-2022-0028090 | A | |
| | | | | CA | 3144549 | A | |
| | | | | CN | 114729028 | A | |
| JP | 2008-506368 | A | 06 March 2008 | US | 2009/0081240 | A1 | |
| | | | | whole document | | | |
| | | | | WO | 2006/003179 | A2 | |
| | | | | EP | 1791868 | A2 | |
| JP | 2008-500947 | A | 17 January 2008 | US | 2006/0280740 | A1 | |
| | | | | whole document | | | |
| | | | | WO | 2005/003172 | A2 | |
| | | | | EP | 1673397 | A2 | |

Form PCT/ISA/210 (patent family annex) (July 2022)

## REFERENCES CITED IN THE DESCRIPTION

**Patent documents cited in the description**

- WO 2018148223 A1
- WO 2006003179 A
- WO 2012145384 A
- WO 2021113853 A
- JP 2022170981 A
- WO 9201047 A
- WO 9220791 A
- WO 9306213 A
- WO 9311236 A
- WO 9319172 A
- WO 9501438 A
- WO 9515388 A
- WO 2018018534 A
- WO 2006003179 A2

**Non-patent literature cited in the description**

- **P. PARHAM et al.** NK cell receptors: of missing sugar and missing self. *Curr. Biol.*, 09 March 2000, vol. 10 (5), 195-7
- **YAWATA et al.** MHC class I-specific inhibitory receptors and their ligands structure diverse human NK-cell repertoires toward a balance of missing self-response. *Blood*, 15 September 2008, vol. 112 (6), 2369-80
- **F. BORREGO et al.** The CD94/NKG2 family of receptors: from molecules and cells to clinical relevance. *Immunol. Res.*, 2006, vol. 35 (3), 263-78
- **E. O. LONG et al.** Negative signaling by inhibitory receptors: the NK cell paradigm. *Immunol. Rev.*, August 2008, vol. 224, 70-84
- **F. ROMAGNE et al.** Preclinical characterization of 1-7F9, a novel human anti-KIR receptor therapeutic antibody that augments natural killer-mediated killing of tumor cells. *Blood*, 24 September 2009, vol. 114 (13), 2667-77
- *Annu. Rev. Immunol.*, 1994, vol. 12, 433-455
- **DAVID R. MAASS et al.** *J. Immunol. Methods*, 31 July 2007, vol. 324 (1-2), 13-25
- **LUKAS ROTH et al.** *Methods Mol. Biol.*, 2020, vol. 2070, 173-189
- **ILKAY KOCER et al.** *Biotechnol. Prog.*, March 2021, vol. 37 (2), 33102
- **PLUCKTHUN A.** The Pharmacology of Monoclonal Antibodies. 1994, vol. 113, 269-315
- *Nature Biotechnology*, 2005, vol. 23, 1126-1136
- *Nature Biotechnology*, 2005, vol. 23 (9), 1105-1116
- **LUKAS ROTH et al.** *Methods Mol. Biol.*, 2020, vol. 207, 173-189
- **L. J. COOPER et al.** *Blood*, 15 February 2003, vol. 101 (4), 1637-44